# EUROPEAN PATENT APPLICATION

(11) **EP 4 692 292 A1**
(43) Date of publication of application: **11.02.2026**
(21) Application number: 24193212.8
(22) Date of filing: 06.08.2024
(51) Int. Cl.: C11D 3/38, C12N 1/20, C12N 3/00, C12R 1/07, C12R 1/10, C12R 1/11, C12R 1/125

(54) **IMPROVED METHOD FOR GERMINATING BACTERIAL SPORES**

(71) Applicant: Evonik Operations GmbH, 45128 Essen (DE)
(72) Inventor: PELZER, Stefan, 33335 Gütersloh (DE); FLÜGEL, Monika, 33803 Steinhagen (DE); BRAUNER, Aileen Silvana, 33790 Halle/Westfalen (DE); SCHÖPPNER, Matthias, 63628 Bad Soden Salmünster (DE); FOH, Kerstin, 32139 Spenge (DE)
(74) Representative: Evonik Patent Association

(57) **Abstract**

This invention relates to a method for germinating bacterial spores **SP.** In this method, a surface **5** is contacted with bacterial spores **SP** that were pretreated with at least one biosurfactant **S_{Bio}** and/or are applied in mixture with at least one biosurfactant **S_{Bio}**. **S_{Bio}** is a glycolipid, which is preferably selected from glucolipids, rhamnolipids, sophorolipids. The present invention also relates to the use of at least one biosurfactant **S_{Bio}**, wherein biosurfactant **S_{Bio}** is a glycolipid, for dispersing bacterial spores **SP.** The bacterial spores **SP** are preferably selected from the group consisting of *Bacillus* spores, *Priestia* spores.

The method according to the invention may advantageously be used in a method of cleaning an object **O** or the human or animal body **B.**

## Description

This invention relates to a method for germinating bacterial spores **SP**. In this method, a surface **S** is contacted with bacterial spores **SP** that were pretreated with at least one biosurfactant **S_{Bio}** and/or are applied in mixture with at least one biosurfactant **S_{Bio}**. **S_{Bio}** is a glycolipid, which is preferably selected from glucolipids, rhamnolipids, sophorolipids. The present invention also relates to the use of at least one biosurfactant **S_{Bio}**, wherein biosurfactant **S_{Bio}** is a glycolipid, for dispersing bacterial spores **SP**. The bacterial spores **SP** are preferably selected from the group consisting of *Bacillus* spores, *Priestia* spores. The method according to the invention may advantageously be used in a method of cleaning an object O or the human or animal body **B.**

### Background of the Invention

The quest for sustainability has become a paramount concern in various industries, and the cleaning as well as the personal care sector are no exceptions. As we delve deeper into the microscopic world, the potential of utilizing microorganisms for eco-friendly solutions has become known. Among these microscopic allies, *Bacillus* species and *Priestia* species, stand out for their versatility and efficiency.

These naturally occurring bacteria produce a range of enzymes that can break down organic matter, making them ideal candidates for the formulation of biodegradable and non-toxic cleaning solutions. By tapping into the inherent properties of these microorganisms, we can create products that not only clean effectively but also contribute to the well-being of our planet and its human and animal inhabitants.

Therefore, these robust bacteria have proven to be a treasure trove for the development of cleaning agents and the production of enzymes that pave the way for a long-lasting and sustainable approach to cleanliness.

Products comprising microorganisms for cleaning and disinfection are described in the art.

E. Caselli et al., PLoS One 2018, 13:e0199616 describe probiotic-based cleaning systems for application in hospitals. Similarly, W. Stone et al., Microorganisms 2020, 8:1726 compare the cleaning effect of a cleaning product comprising *Bacillus* spores on microbiome of surfaces found in hospitals.

A. Sp6k, G. Arvanitakis, G. McClung, Food Chem Toxicol. 2018, 116, 10-19 disclose microorganisms as active ingredients in household, professional and industrial cleaning applications.

US 2013/184196 A1 discloses an aqueous cleaning composition comprising spores of certain species of *Bacillus* such as *Bacillus subtilis, Bacillus licheniformis,* and *Bacillus megaterium* (= *Priestia megaterium*)*.*

US 2023/220307 A1 discloses an aqueous microbial cleaning composition comprising *Bacillus* spores.

WO 2008/146958 A1 discloses the use of *Bacillus megaterium* and other *Bacillus* species for malodor removal.

While the prior art thus provides for cleaning products comprising bacteria, there is still a need for further improved cleaning agent based on bacteria such as *Bacillus* and *Priestia* with improved spectrum of activity.

In this context, understanding the application and spreading of cleaning microorganisms on surfaces is a crucial aspect of microbiology. *Bacillus, Priestia,* and other strains that are often commonly referred to as "bio-cleaning agents", play a pivotal role in the degradation and removal of organic compounds, and the maintenance of environmental hygiene. By studying how these microorganisms move and interact with different surfaces, potential strains can be selectively used for different purposes.

As described by O. Erkmen, in Laboratory Practices in Microbiology, 2021, Chapter 11.1, page 107, certain bacteria genus (such as *Bacillus, Clostridium, Desulfotomaculum*) form different kind of cells depending on environmental conditions. Vegetative cells are the structure that grows actively and is formed under favorable environmental conditions. Under unfavorable, adverse environmental conditions, the vegetative cells form spores that are hardy and resilient structures. The spore is formed as a response to adverse conditions. Upon sporulation, each cell canonly produce one spore and, upon germination, each spore, in turn, can germinate into one vegetative cell. Bacterial spores are not reproductive structures, but a structure formed to resist unfavorable environmental conditions. The spore is thus a part of the life cycle of spore forming bacteria under unfavorable conditions. The spores are metabolically inactive and may be dormant for years, but are able to form vegetative cells (one cell per spore) in the suitable environmental conditions.

For another overview, see also S. Sella, L. Vandenberghe, C. Soccol, Microbiological Research 2014, 169, 931-939.

Due to their hardiness and resilience, bacterial spores may conveniently be stored under unfavorable conditions for several time and then be applied, for example in cleaning. *Bacillus, Priestia* and other bacteria that are used in cleaning methods and similar methods provide, on the one hand, the advantage that they form not only vegetative cells, but also spores under nutrient deprivation or conditions that are unfavorable to growth. These spores may then be used as resilient ingredients in cleaning formulations that germinate into vegetative, active cells as soon as the respective formulation is applied, for example to a surface.

Once the spores are applied (typically to a surface) and conditions are adjusted so that the spores germinate and multiply, the resulting vegetative cells may then produce enzymatic activities (such as protease, cellulase, amylase, lipase activity) and/or metabolize certain substrates, thus degrading unwanted molecules such as proteins, starch, cellulose, oil, grease and/or malodorous compounds such as valeric acid.

In such applications (e.g. cleaning), it is crucial that the bacterial spores are spread over a wide surface so that a possibly huge surface comes into contact with the vegetative cells after germination of the spores.

Here, the fact that spores essentially do not exhibit motility, becomes an important impediment in these applications. Motility is the ability to move independently using metabolic energy and must be distinguished from the spreading of vegetative cells which is accomplished by cell division and proliferation, which are functions of which spores are not capable. The spreading of spores over a surface therefore mainly depends on diffusion. It is, however, desirable to have means at hand which would allow the spreading of spores over surface, especially where spores are applied to a surfaces, in particular surfaces with cracks, gaps and crevices. There is hence a need in the art to improve the spreading of spores over surfaces, in particular in methods for germinating bacterial spores on surfaces, such as carried out in the context of cleaning.

The problem to be solved by the present invention was hence to provide a method in which bacterial spores are applied to a surface, and to improve the spreading of these spores.

### Detailed description of the Invention

Surprisingly, it was found that glycolipids, in particular glucolipids, sophorolipids and rhamnolipids, preferably rhamnolipids, can be used to disperse bacterial spores, in particular bacterial endospores. This finding is especially useful where these spores are to be spread on a surface, for example in cleaning.

While not wishing to be bound by a specific theory, the inventors of the present invention suppose that the improved spreading over the surface of spores in the presence of biosurfactants is due to at least the following effects of the biosurfactants on the spores:
- An "anti-clumping" effect that is partly due to the neutralization of adhesive forces between the spores' coats by the glycolipids and partly results from the mutual repulsion of aligned glycolipid residues on the spores' coats;
- A "surfboard" effect of the spore's coat that is coated with glycolipids, which gives them a mobilization on already germinated vegetative cells;
- A "revitalizing" effect of the spores exerted by the glycolipid that leads to earlier/ quicker germination of the spores into a vegetative cell;
- Once spores have germinated into vegetative cells, glycolipids also facilitate the uptake of nutrients to the vegetative cells ("emulsifying effect").

In the context of the invention, it is the anti-clumping effect that is most important for the ability to disperse the bacterial spores **SP**.

The present invention hence relates, in a first aspect, to a method for germinating bacterial spores **SP** on a surface **S**. The present invention, in a second aspect, relates to the use of at least one biosurfactant

**S_{Bio},** wherein biosurfactant **S_{Bio}** is a glycolipid, which is preferably selected from the group of glucolipids, sophorolipids and rhamnolipids, for dispersing bacterial spores **SP**, in particular over a surface **S**.

The present invention hence relates to a method for germinating bacterial spores **SP** on a surface **S** according to **variant 1**, **variant 2**, or **variant 3**,
wherein **variant 1** comprises the following steps:
   a1) providing **SP** and, separately from **SP**, at least one biosurfactant **S_{Bio}**, wherein the biosurfactant **S_{Bio}** is a glycolipid,
   b1) contacting **S** with **SP** and **S_{Bio},** so that a mixture **M₁** comprising **SP** and at least one biosurfactant **S_{Bio}** is obtained, wherein mixture **M₁** is in contact with **S**,
   c1) incubating **M₁** to germinate at least a part of the spores **SP** comprised by mixture **M₁**;
wherein **variant 2** comprises the following steps:
   a2) providing a mixture **M*** comprising **SP** and at least one biosurfactant **S_{Bio}**, wherein the biosurfactant **S_{Bio}** is a glycolipid,
   b2) contacting **S** with the mixture **M***, wherein **M*** is optionally incubated before contacting **S**, so that a mixture **M₂** comprising **SP** and at least one biosurfactant **S_{Bio}** is obtained, wherein mixture **M₂** is in contact with **S**,
   c2) incubating **M₂** to germinate at least a part of the spores **SP** comprised by mixture **M₂**;
wherein **variant 3** comprises the following steps:
   a3) providing a mixture **M*** comprising **SP** and at least one biosurfactant **S_{Bio}**, wherein the biosurfactant **S_{Bio}** is a glycolipid,
   b3)
      o incubating mixture **M***,
      o then separating at least a part **π_{SP}** of the spores **SP** comprised by **M*** from at least a part of the biosurfactant **S_{Bio}** comprised by **M***,
      o contacting **S** with **π_{SP}**,
      so that a mixture **M₃** comprising **SP** and optionally at least one biosurfactant **S_{Bio}** is obtained, wherein mixture **M₃** is in contact with **S**,
   c3) incubating **M₃** to germinate at least a part of the spores **SP** comprised by mixture **M₃**. In a second aspect, the present invention relates to the use of at least one biosurfactant **S_{Bio}**, wherein biosurfactant **S_{Bio}** is a glycolipid, for dispersing bacterial spores **SP** on a surface **S**.

In the course of the studies of the present invention, the following deposited strains were identified by screening of naturally occurring isolates and were deposited at the DSMZ ("Deutsche Sammlung von Mikroorganismen und Zellkulturen"; Leibniz-lnstitute DSMZ-German Collection of Microorganisms and Cell Cultures, Inhoffenstraße 7B, 38124 Braunschweig, Germany) under the provisions of the Budapest Treaty on the International Recognition of the Deposit of Microorganisms for the Purpose of Patent Procedure.
I. *Bacillus velezensis* DSM 34978 has been identified by targeted screening of naturally occurring isolates. It was isolated from the soil and has been deposited with the DSMZ on March 20, 2024 under the provisions of the Budapest Treaty on the International Recognition of the Deposit of Microorganisms for the Purpose of Patent Procedure under the Accession Number as mentioned before in the name of Evonik Operations GmbH.
II. *Bacillus velezensis* DSM 34674 has been identified by targeted screening of naturally occurring isolates. It was isolated from the soil and has been deposited with the DSMZ on July 5, 2023 under the provisions of the Budapest Treaty on the International Recognition of the Deposit of Microorganisms for the Purpose of Patent Procedure under the Accession Number as mentioned before in the name of Evonik Operations GmbH.
III. *Bacillus licheniformis* DSM 34977 has been identified by targeted screening of naturally occurring isolates. It was isolated from a bunny faeces sample and has been deposited with the DSMZ on March 20, 2024 under the provisions of the Budapest Treaty on the International Recognition of the Deposit of Microorganisms for the Purpose of Patent Procedure under the Accession Number as mentioned before in the name of Evonik Operations GmbH.
IV. *Priestia megaterium* DSM 34980 has been identified by targeted screening of naturally occurring isolates. It was isolated from soil rifled by wild boar and has been deposited with the DSMZ on March 20, 2024 under the provisions of the Budapest Treaty on the International Recognition of the Deposit of Microorganisms for the Purpose of Patent Procedure under the Accession Number as mentioned before in the name of Evonik Operations GmbH.
V. *Bacillus subtilis* DSM 34981 has been identified by targeted screening of naturally occurring isolates. It was isolated from an environmental soil sample and has been deposited with the DSMZ on March 20, 2024 under the provisions of the Budapest Treaty on the International Recognition of the Deposit of Microorganisms for the Purpose of Patent Procedure under the Accession Number as mentioned before in the name of Evonik Operations GmbH.

### I. Method for germinating bacterial spores SP

The present invention relates to a method for germinating bacterial spores **SP** on a surface **S**. The method according to the invention is carried out according to one of the three variants: **variant 1, variant 2, variant 3.**

In the following "**variant 1**" will be used as abbreviation for "variant 1 of the method according to the invention".

In the following "**variant 2**" will be used as abbreviation for "variant 2 of the method according to the invention".

In the following "**variant 3**" will be used as abbreviation for "variant 3 of the method according to the invention".

**Variants 1** and **variant 2** are preferred variants of the three variants. **Variant 2** is the most preferred variants of the three variants.

**Variant 1** comprises the following steps:
a1) providing **SP** and, separately from **SP**, at least one biosurfactant **S_{Bio}**, wherein the biosurfactant **S_{Bio}** is a glycolipid,
b1) contacting **S** with **SP** and **S_{Bio},** so that a mixture **M₁** comprising **SP** and at least one biosurfactant **S_{Bio}** is obtained, wherein mixture **M₁** is in contact with **S**,
c1) incubating **M₁** to germinate at least a part of the spores **SP** comprised by mixture **M₁**.

**Variant 2** comprises the following steps:
a2) providing a mixture **M*** comprising **SP** and at least one biosurfactant **S_{Bio}**, wherein the biosurfactant **S_{Bio}** is a glycolipid,
b2) contacting **S** with the mixture **M***, wherein **M*** is optionally incubated before contacting **S**, so that a mixture **M₂** comprising **SP** and at least one biosurfactant **S_{Bio}** is obtained, wherein mixture **M₂** is in contact with **S**,
c2) incubating **M₂** to germinate at least a part of the spores **SP** comprised by mixture **M₂**.

**Variant 3** comprises the following steps:
a3) providing a mixture **M*** comprising **SP** and at least one biosurfactant **S_{Bio}**, wherein the biosurfactant **S_{Bio}** is a glycolipid,
b3) incubating mixture **M*** and then separating at least a part of the spores **SP** comprised by **M*** from at least a part of the biosurfactant **S_{Bio}** comprised by **M*** and contacting **S** with the separated spores, so that a mixture **M₃** comprising **SP** and optionally at least one biosurfactant **S_{Bio}** is obtained, wherein mixture **M₃** is in contact with **S**,
c3) incubating **M₃** to germinate at least a part of the spores **SP** comprised by mixture **M₃**.

Referring to "step **a)**" in the following is to be understood as commonly referring to "step a1) of **variant 1",** "step a2) of **variant 2",** "step a3) of **variant 3**".

Referring to "step **b)**" in the following is to be understood as commonly referring to "step b1) of **variant 1**", "step b2) of **variant 2"**, "step b3) of **variant 3**".

Referring to "step **c)**" in the following is to be understood as commonly referring to "step c1) of **variant 1**", "step c2) of **variant 2**", "step c3) of **variant 3**".

### 1.1 Bacterial Spores SP

The method according to the present invention is a method for germinating bacterial spores **SP** on a surface **S**.

In the method according to the present invention, the bacterial spores **SP** are not particularly limited, and preferably are endospores. They may be spores, in particular endspores, from any bacterium that creates spores in its life cycle, such as *Clostridium, Bacillus, Priestia, Desulfotomaculum, Heyndrickxia.* Preferred *Heyndrickxia* species is *Heyndrickxia coagulans.*

Preferably, the bacterial spores **SP** are selected from *Bacillus* spores, *Priestia* spores.

Preferred *Bacillus* spores are selected from spores of at least one *Bacillus* species selected from the group consisting of *Bacillus velezensis, Bacillus licheniformis,* Bacillus paralicheniformis, *Bacillus subtilis, Bacillus amyloliquefaciens, Bacillus atrophaeus, Bacillus mojavensis, Bacillus laevolacticus, Bacillus pasteurii, Bacillus tequilensis, Bacillus vallismortis, Bacillus pumilus, Bacillus simplex,* preferably selected from the group consisting of *Bacillus velezensis, Bacillus licheniformis, Bacillus subtilis.*

Preferred *Priestia* spores are spores of *Priestia megaterium* (formerly known as "*Bacillus megaterium*")

In the context of this invention, the inventors have identified several *Bacillus* and *Priestia* species with surprisingly advantageous properties that make them predestined for application in cleaning.

A preferred strain of *Bacillus velezensis* is thus selected from *Bacillus velezensis* DSM 34978 and mutants thereof and *Bacillus velezensis* DSM 34674 and mutants thereof.

A preferred strain of *Bacillus licheniformis* is thus selected from *Bacillus licheniformis* DSM 34977 and mutants thereof.

A preferred strain of *Bacillus subtilis* is thus selected from *Bacillus subtilis* DSM 34981 and mutants thereof.

A preferred strain of *Priestia megaterium* is thus selected from *Priestia megaterium* DSM 34980 and mutants thereof.

### 1.2 Surface S

In the method according to the first aspect of the invention, the bacterial spores **SP** are germinated on a surface **S**.

The surface **S** in the method according to the first aspect of the invention is preferably selected from a surface **S_{O}** of an object **O** and a surface **S_{B}** of the human or animal body **B**.

In those embodiments where the surface **S** is a surface **S_{B}** of the human or animal body **B**, it is preferred that the surface **S_{B}** is the surface of a part of the human or animal body selected from skin, hair, most preferred hair.

An "object **O**" is a lifeless, solid, object and preferably comprises at least one material selected from natural fibre, ceramic, metal, plastic, glass, stone.

"Natural fibre" in the context of the invention preferably is one selected from the group consisting of vegetable fibres, animal fibres. Typical vegetable fibres are based on cellulose and in particular selected from wood, cotton, hemp, jute, flax, abaca, piña (pineapple fibre), ramie, sisal, bagasse, banana. Preferably, vegetable fibres are selected from the group consisting of wood, cotton. Vegetable fibres may for example be found in paper, cardboard.

Typical animal fibres are in particular selected from the group consisting of wool, silk.

The object **O** in a preferred embodiment is selected from the group consisting of a woven, a non-woven, a vehicle, crockery, cutlery, food storage containers, furniture, flooring, panelling, paving, domestic machine, garden tool, construction equipment, building for livestock or domestic animal, music instrument, toy, window. The object **O**, in particular, is selected from the group consisting of a woven, a non-woven, furniture, flooring, panelling, even more preferably selected from the group consisting of a woven, a non-woven.
blanket, bedspread, duvet cover.

The non-woven is in particular selected from an article of clothing, curtains, napkins, bed linen, tablecloth, blanket, bedspread, duvet cover.

The woven is in particular selected from an article of clothing, curtains, napkins, bed linen, tablecloth, The article of clothing is in particular selected from the group consisting of sweaters, shirts, pants, shoes, socks, hosiery, coats, haberdashery, suits, hats, caps, bonnets, scarfs, gloves.

The vehicle is in particular selected from the group consisting of car, train (which may be selected from the group consisting of railroad cars, tram cars, underground railway cars), plane, bicycle, motor-bike, rocket, water vehicles (which may be selected from ships, boats, hovercraft).

Crockery is in particular selected from the group consisting of dishes, glassware (such as drinking glasses), cups, platters, mugs, pitchers, pots, cannikins.

Cutlery is in particular selected from the group consisting of knives, forks, spoons, chopsticks.

Food storage containers are in particular selected from the group consisting of barrels, lunchboxes, bottles, jars.

Furniture is in particular selected from the group consisting of shelves, boards, wardrobes, cupboards, desks, tables, chairs, chest of drawers.

Flooring is in particular selected from the group consisting of laminate floor, parquet floor, tiled floor, carpeted floor, ceramic floor.

Panelling is in particular selected from the group consisting of wallpaper, wall tiles, tapestry.

Paving may be any kind of road surface, and is in particular selected from cobblestone, asphalt, gravel.

A domestic machine may be any electric machine used in the household, and is in particular selected from washing machine, dishwasher, vacuum cleaner, sound system, lamp, TV, computer, printer, heater, electrical kitchen appliances such as fridge, oven, stove, boiler, egg boiler, toaster, sandwich maker, waffle iron, mixer, bread machine, yogurt maker, coffee maker, electric kettle.

A garden tool is preferably selected from the group consisting of rake, grate, lawnmower, scythe, spade, shovel.

Construction equipment is in particular any equipment, electrical or non-electrical, which may be used in handcraft or in construction of buildings, roads. Construction equipment is preferably selected from drilling machines, tools such as hammer, rasp, screwdriver.

Building for livestock or domestic animal is in particular any container or building in which pets or farm animals may be kept. In particular, the building for livestock or domestic animal is selected from the group consisting of terrarium, aquarium, cage, pen, kenner, cowshed, hennery, pigsty, coop.

Music instrument may be any keyboard instrument, string instrument, wind instrument, percussion instrument and is preferably selected from piano, guitar, drums.

Toy is preferably selected from video game console, doll.

Window may be any transparent light opening, and is particular selected from outside window of a building, inside window of a building, bull's eye of washing machines, bull's eye of ships, preferably selected from outside window of a building, inside window of a building.

In those embodiments where the method according to the invention is applied to the human or animal body **B**, this means that the method is applied to a living human being or animal. The method according to the invention is non-therapeutic when applied on a surface **S_{B}** of the human or animal body **B**. It goes without saying that in embodiments where the method according to the first aspect of the invention is applied on a surface **S_{O}** of an object **O**, the method is also non-therapeutic, since the object **O** is lifeless.

### I.3 Glycolipid

The at least one biosurfactant **S_{Bio}** provided in step **a)** of the method according to the invention is a glycolipid.

In the context of the method according to the invention, "biosurfactant **S_{Bio}**" is a glycolipid which is preferably selected from the group consisting of glucolipids, rhamnolipids, sophorolipids, even more preferably from the group of rhamnolipids.

Hence, preferred biosurfactants **S_{Bio}** are glycolipids such as glucolipids, rhamnolipids, and sophorolipids. Such glycolipids are described in the art together with their syntheses, for example in EP 0 499 434 A1 (glucolipids are referred to as "glucose lipids" in this document), DE 196 48 439 A1, DE 196 00 743 A1. WO 03/006146 A1, US 2008/0213194 A1, JP H01-304034 A1, CN 1337439 A describe further methods for the synthesis of rhamnolipids. WO 03/002700 A1, US 4,305,961 A, US 7,556,654 B1 describe further methods for the synthesis of sophorolipids.

### I.3.1 Glucolipid

In the context of the method according to the invention, the term "glucolipid" is in particular to be understood as referring to a structure according to formula **(I)**, even more preferred a structure according to formula **(II)**: 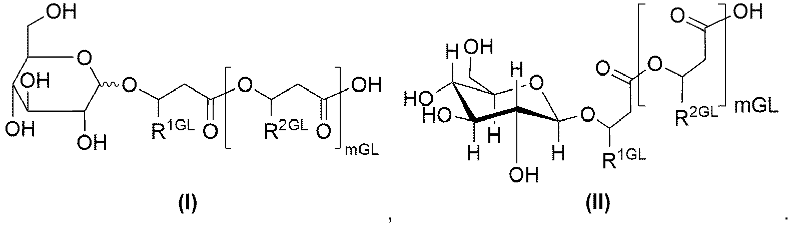 In formulae **(I)** and **(II)**, mGL = 3, 2, 1 or 0, preferably 1 or 0.

Residues R^{1GL} and R^{2GL} are, independently of one another, an organic radical having 2 to 24 carbon atoms, preferably 5 to 20, more preferably 7 to 15, even more preferably 7, carbon atoms,
wherein preferably R^{1GL} and R^{2GL} are independently of one another selected from the group consisting of optionally substituted alkyl radicals with 2 to 24, preferably 5 to 20, more preferably 7 to 15, even more preferably 7, carbon atoms, wherein hydroxy substituted alkyl radicals are preferred substituted alkyl radicals,
optionally substituted alkenyl radicals with 2 to 24 carbon atoms, preferably 5 to 20, more preferably 7 to 15, even more preferably 7, carbon atoms, wherein hydroxy substituted alkenyl radicals are preferred substituted alkenyl radicals,
wherein more preferably R^{1GL} and R^{2GL} are independently of one another selected from the group consisting of pentenyl, heptenyl, nonenyl, undecenyl, tridecenyl and -(CH₂)ₒCH₃ where o = 1 to 23, preferably 4 to 12. Most preferably, R^{1GL} and R^{2GL} are each *n*-heptyl.

Distinct glucolipids for mGL = 0 are abbreviated according to the following nomenclature:
"GL-CX" is understood as meaning glucolipids of the general formulae **(I)** or **(II)** in which mGL = 0 and in which the radical R^{1GL} = -(CH₂)ₒ-CH₃ where o = X-4.

Distinct glucolipids for mGL = 1 are abbreviated according to the following nomenclature:
"GL-CXCY" is understood as meaning glucolipids of the general formulae **(I)** or **(II)** in which mGL = 1 and in which one of the radicals R^{1GL} and R^{2GL} = -(CH₂)ₒ-CH₃ where o = X-4 and the remaining radical R^{1GL} or R^{2GL} = -(CH₂)ₒ-CH₃ where o = Y-4.

The nomenclature used thus does not differentiate between "CXCY" and "CYCX".

If one of the aforementioned indices X and/or Y is provided with ":Z", then this means that the respective radical R^{1GL} and/or R^{2GL} = an unbranched, unsubstituted hydrocarbon radical with X-3 or Y-3 carbon atoms having Z double bonds.

The curvy bond in structure **(I)** [and also structures **(III)**, **(V)**, **(VII)**, **(IX)**, **(XI)** described hereinafter] implies that the respective substituent may be axial or equatorial, preferably is equatorial.

Alkyl radicals may be branched or linear.

Alkenyl radicals may be branched or linear and contain at least one, preferably one to three, double bonds.

In those cases where structures according to formulae **(I)** and **(II)** comprise more than one residue R^{2GL}, these residues R^{2GL} may be identical or different from one another.

In those embodiments where the biosurfactant **S_{Bio}** provided in step **a)** of the method according to the invention comprises glucolipids, it is preferred that 1 % by weight to 30 % by weight, preferably 5 % by weight to 25 % by weight, particularly preferably 10 % by weight to 20 % by weight, of all glucolipids comprised by the biosurfactant **S_{Bio}** provided in step **a)** are GL-C8C10, where the percentages by weight refer to the sum of all of the glucolipids comprised by the biosurfactant **S_{Bio}** provided in step **a)**.

In those embodiments where the biosurfactant **S_{Bio}** provided in step **a)** of the method according to the invention comprises glucolipids, it is alternatively preferred that 0.5 % by weight to 20 % by weight, preferably 3 % by weight to 17 % by weight, particularly preferably 5 % by weight to 15 % by weight, of all glucolipids comprised by the biosurfactant **S_{Bio}** provided in step **a)** are GL-C10C12:1, where the percentages by weight refer to the sum of all of the glucolipids comprised by the biosurfactant **S_{Bio}** provided in step **a)**.

In those embodiments where the biosurfactant **S_{Bio}** provided in step **a)** of the method according to the invention comprises glucolipids, it is alternatively preferred that 0.5 % by weight to 20 % by weight, preferably 2 % by weight to 15 % by weight, particularly preferably 3 % by weight to 12 % by weight, of all glucolipids comprised by the biosurfactant **S_{Bio}** provided in step **a)** are GL-C10C12, where the percentages by weight refer to the sum of all of the glucolipids comprised by the biosurfactant **S_{Bio}** provided in step **a)**.

In those embodiments where the biosurfactant **S_{Bio}** provided in step **a)** of the method according to the invention comprises glucolipids, it is alternatively preferred that
1 % by weight to 30 % by weight, preferably 5 % by weight to 25 % by weight, particularly preferably 10 % by weight to 20 % by weight, of all glucolipids comprised by the biosurfactant **S_{Bio}** provided in step **a)** are GL-C8C10,
0.5 % by weight to 20 % by weight, preferably 3 % by weight to 17 % by weight, particularly preferably 5 % by weight to 15 % by weight, of all glucolipids comprised by the biosurfactant **S_{Bio}** provided in step **a)** are GL-C10C12:1,
0.5 % by weight to 20 % by weight, preferably 2 % by weight to 15 % by weight, particularly preferably 3 % by weight to 12 % by weight, of all glucolipids comprised by the biosurfactant **S_{Bio}** provided in step **a)** are GL-C10C12,
where the percentages by weight refer to the sum of all of the glucolipids comprised by the biosurfactant **S_{Bio}** provided in step **a)**.

In those embodiments where the biosurfactant **S_{Bio}** provided in step **a)** of the method according to theinvention comprises glucolipids, it is alternatively preferred that
10 % by weight to 20 % by weight of all glucolipids comprised by the biosurfactant **S_{Bio}** provided in step **a)** are GL-C8C10,
5 % by weight to 15 % by weight of all glucolipids comprised by the biosurfactant **S_{Bio}** provided in step **a)** are GL-C10C12:1,
3 % by weight to 12 % by weight of all glucolipids comprised by the biosurfactant **S_{Bio}** provided in step **a)** are GL-C10C12,
where the percentages by weight refer to the sum of all of the glucolipids comprised by the biosurfactant **S_{Bio}** provided in step **a)**.

Over and above this, where the biosurfactant **S_{Bio}** provided in step **a)** of the method according to the invention comprises glucolipids, it is alternatively preferred that 0 % by weight to 5 % by weight, preferably 0.01 % by weight to 4 % by weight, particularly preferably 0.1 % by weight to 3 % by weight, of all glucolipids comprised by the biosurfactant **S_{Bio}** provided in step **a)** are GL-C10,
where the percentages by weight refer to the sum of all of the glucolipids comprised by the biosurfactant **S_{Bio}** provided in step **a)**.

### I.3.2 Rhamnolipids

In the context of the method according to the invention, the term "rhamnolipid" is in particular to be understood as referring to compounds of the general formula **(III)** and salts thereof, preferably compounds according to the general structure **(IV)** and salts thereof,

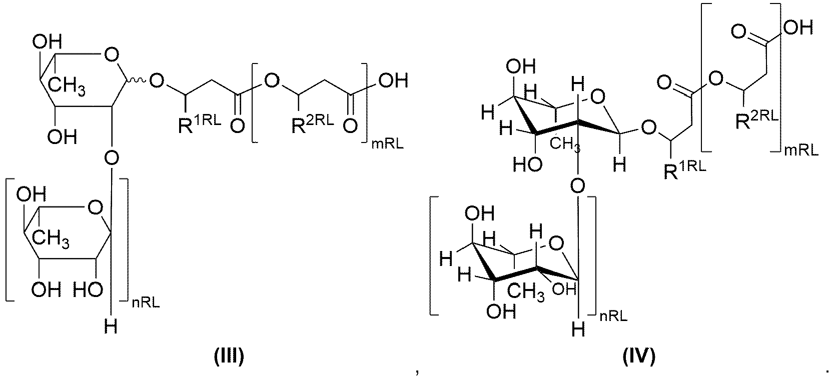

In formulae **(III)** and **(IV),** mRL = 2, 1 or 0, preferably 1 or 0.

nRL = 1 or 0.

Residue R^{1RL} and R^{2RL} are independently of one another, an organic radical having 2 to 24 carbon atoms, preferably 5 to 13, more preferably 7 to 10, even more preferably 7, carbon atoms,
wherein preferably R^{1RL} and R^{2RL} are independently of one another selected from the group consisting of optionally substituted alkyl radicals with 2 to 24, preferably 5 to 13, more preferably 7 to 10, even more preferably 7, carbon atoms, wherein hydroxy substituted alkyl radicals are preferred substituted alkyl radicals,
optionally substituted alkenyl radicals with 2 to 24, preferably 5 to 13, more preferably 7 to 10, even more preferably 7, carbon atoms, wherein hydroxy substituted alkenyl radicals are preferred substituted alkenyl radicals,
wherein more preferably R^{1RL} and R^{2RL} are independently of one another selected from the group consisting of pentenyl, heptenyl, nonenyl, undecenyl, tridecenyl and -(CH₂)ₒ-CH₃ where o = 1 to 23, preferably 4 to 12. Most preferably, R^{1RL} and R^{2RL} are each n-heptyl.

If nRL = 1, the glycosidic bond between the two rhamnose units is preferably in the α-configuration. The optically active carbon atoms of the fatty acids are preferably present as R-enantiomers (e.g. (R)-3-{(R)-3-[2-O-(α-L-rhamnopyranosyl)-α-L-rhamnopyranosyl]oxydecanoyl}oxydecanoate).

The term "di-rhamnolipid" in the context of the present invention is understood to mean compounds of the general formulae **(III)** and **(IV)** or salts thereof, where nRL = 1.

The term "mono-rhamnolipid" in the context of the present invention is understood to mean compounds of the general formulae **(III)** and **(IV)** or salts thereof, where nRL = 0.

Distinct rhamnolipids are abbreviated according to the following nomenclature:
"diRL-CXCY" are understood to mean di-rhamnolipids of the general formulae **(III)** and **(IV)**, in which one of the residues R^{1RL} and R^{2RL} = -(CH₂)ₒ-CH₃ where o = X-4 and the remaining residue R¹ or R² = -(CH₂)ₒ-CH₃ where o = Y-4.
"monoRL-CXCY" are understood to mean mono-rhamnolipids of the general formulae **(III)** and **(IV)**, in which one of the residues R^{1RL} and R^{2RL} = -(CH₂)ₒ-CH₃ where o = X-4 and the remaining residue R^{1RL} or R^{2RL} = -(CH₂)ₒ-CH₃ where o = Y-4.

The nomenclature used therefore does not distinguish between "CXCY" and "CYCX".

For rhamnolipids where mRL=0, the terms "monoRL-CX" or "diRL-CX" are used accordingly.

If one of the abovementioned indices X and/or Y is provided with ":Z", this signifies that the respective residue R^{1RL} and/or R^{2RL} is equal to an unbranched, unsubstituted hydrocarbon residue having X-3 or Y-3 carbon atoms having Z double bonds.

Methods for preparing the relevant rhamnolipids are disclosed, for example, in EP 2 786 743 A1 and EP 2 787 065 A1. Rhamnolipids applicable in the context of the instant invention can also be produced by fermentation of *Pseudomonas,* especially *Pseudomonas aeruginosa,* which are preferably non genetically modified cells, a technology already disclosed in the eighties, as documented e.g. in EP 0 282 942 A1 and DE 41 27 908 A1. Rhamnolipids produced in *Pseudomonas aeruginosa* cells which have been improved for higher rhamnolipid titres by genetical modification can also be used in the context of the instant invention; such cells have for example been disclosed by L. Lei, F. Zhao, S. Han, Y. Zhang, Biotechnol Lett. 2020, 42, 997-1002.

Rhamnolipids produced by *Pseudomonas aeruginosa* are commercially available from Jeneil Biotech Inc., e.g. under the tradename Zonix, from Logos Technologies (technology acquired by Stepan), e.g. under the tradename NatSurFact, from Biotensidion GmbH, e.g. under the tradename Rhapynal, from AGAE technologies, e.g. under the name R90, R95, R95Md, R95Dd, from Locus Bio-Energy Solutions and from Shanghai Yusheng Industry Co. Ltd., e.g. under the tradename Bio-201 Glycolipids.

In those embodiments where the biosurfactant **S_{Bio}** provided in step **a)** of the method according to the invention comprises rhamnolipids, it is preferred that these rhamnolipids provided in step **a)** are mixtures of mono-rhamnolipids and di-rhamnolipids, wherein even more preferably the ratio of the weight of all mono-rhamnolipids provided in step **a)** to the weight of all di-rhamnolipids provided in step **a)** is in the range of from 9 : 1 to 1 : 9, preferably of from 8 : 2 to 2 : 8, more preferably of from 7 : 3 to 3 : 7, more preferred of from 6 : 4 to 4 : 6, most preferred is 1 : 1.

Mono-rhamnolipids are compounds according to structures **(III)**, preferably **(IV)**, wherein nRL = 0. Di-rhamnolipids are compounds according to structures **(III)**, preferably **(IV)**, wherein nRL = 1.

In those embodiments where the biosurfactant **S_{Bio}** provided in step **a)** of the method according to the invention comprises rhamnolipids, it is preferred that 56 % by weight to 95 % by weight, preferably 60 % by weight to 80 % by weight, particularly preferably 66 % by weight to 70 % by weight, of all rhamnolipids comprised by the biosurfactant **S_{Bio}** provided in step **a)** are diRL-C10C10, where the percentages by weight refer to the sum of all of the rhamnolipids comprised by the biosurfactant **S_{Bio}** provided in step **a)**.

In those embodiments where the biosurfactant **S_{Bio}** provided in step **a)** of the method according to the invention comprises rhamnolipids, it is alternatively preferred that 0.5 % by weight to 15 % by weight, preferably 3 % by weight to 12 % by weight, particularly preferably 5 % by weight to 10 % by weight, of all rhamnolipids comprised by the biosurfactant **S_{Bio}** provided in step **a)** are diRL-C10C12:1, where the percentages by weight refer to the sum of all of the rhamnolipids comprised by the biosurfactant **S_{Bio}** provided in step **a)**.

In those embodiments where the biosurfactant **S_{Bio}** provided in step **a)** of the method according to the invention comprises rhamnolipids, it is alternatively preferred that 0.5 to 25 % by weight, preferably 3 % by weight to 15 % by weight, particularly preferably 5 % by weight to 12 % by weight, of all rhamnolipids comprised by the biosurfactant **S_{Bio}** provided in step **a)** are diRL-C10C12, where the percentages by weight refer to the sum of all of the rhamnolipids comprised by the biosurfactant **S_{Bio}** provided in step **a)**.

In those embodiments where the biosurfactant **S_{Bio}** provided in step **a)** of the method according to the invention comprises rhamnolipids, it is alternatively preferred that 0.1 % by weight to 25 % by weight, preferably 2 % by weight to 10 % by weight, particularly preferably 4 % by weight to 8 % by weight, of all rhamnolipids comprised by the biosurfactant **S_{Bio}** provided in step **a)** are diRL-C8C10, where the percentages by weight refer to the sum of all of the rhamnolipids by the biosurfactant **S_{Bio}** provided in step **a)**.

In those embodiments where the biosurfactant **S_{Bio}** provided in step **a)** of the method according to the invention comprises rhamnolipids, it is even further preferred that
0.1 % by weight to 5 % by weight, preferably 0.5 % by weight to 3 % by weight, particularly preferably 0.5 % by weight to 2 % by weight, of all rhamnolipids comprised by the biosurfactant **S_{Bio}** provided in step **a)** are monoRL-C8C10 and/or, preferably and,
0.1 % by weight to 5 % by weight, preferably 0.5 % by weight to 3 % by weight, particularly preferably 0.5 % by weight to 2 % by weight, of all rhamnolipids comprised by the biosurfactant **S_{Bio}** provided in step **a)** are monoRL-C10C10, where the percentages by weight refer to the sum of all of the rhamnolipids comprised by the biosurfactant **S_{Bio}** provided in step **a)**.

In those embodiments where the biosurfactant **S_{Bio}** provided in step **a)** of the method according to the invention comprises rhamnolipids, it is alternatively preferred that 10 % by weight to 30 % by weight, preferably 20 % by weight to 30 % by weight, particularly preferably 25 % by weight to 30 % by weight, of all rhamnolipids comprised by the biosurfactant **S_{Bio}** provided in step **a)** are monoRL-C10C10, where the percentages by weight refer to the sum of all of the rhamnolipids comprised by the biosurfactant **S_{Bio}** provided in step **a)**.

In those embodiments where the biosurfactant **S_{Bio}** provided in step **a)** of the method according to the invention comprises rhamnolipids, it is alternatively preferred that 10 % by weight to 30 % by weight, preferably 12 % by weight to 25 % by weight, particularly preferably 15 % by weight to 20 % by weight, of all rhamnolipids comprised by the biosurfactant **S_{Bio}** provided in step **a)** are diRL-C10C10, where the percentages by weight refer to the sum of all of the rhamnolipids comprised by the biosurfactant **S_{Bio}** provided in step **a)**.

In those embodiments where the biosurfactant **S_{Bio}** provided in step **a)** of the method according to the invention comprises rhamnolipids, it is alternatively preferred that 10 % by weight to 30 % by weight, preferably 12 % by weight to 25 % by weight, particularly preferably 15 % by weight to 20 % by weight, of all rhamnolipids comprised by the biosurfactant **S_{Bio}** provided in step **a)** are monoRL-C8C10, where the percentages by weight refer to the sum of all of the rhamnolipids comprised by the biosurfactant **S_{Bio}** provided in step **a)**.

In those embodiments where the biosurfactant **S_{Bio}** provided in step **a)** of the method according to the invention comprises rhamnolipids, it is alternatively preferred that 3 % by weight to 25 % by weight, preferably 5 % by weight to 20 % by weight, particularly preferably 10 % by weight to 15 % by weight, of all rhamnolipids comprised by the biosurfactant **S_{Bio}** provided in step **a)** are monoRL-C10C12:1, where the percentages by weight refer to the sum of all of the rhamnolipids comprised by the biosurfactant **S_{Bio}** provided in step **a)**.

In those embodiments where the biosurfactant **S_{Bio}** provided in step **a)** of the method according to the invention comprises rhamnolipids, it is alternatively preferred that 1 % by weight to 15 % by weight, preferably 2 % by weight to 10 % by weight, particularly preferably 3 % by weight to 8 % by weight, of all rhamnolipids comprised by the biosurfactant **S_{Bio}** provided in step **a)** are diRL-C10C12, where the percentages by weight refer to the sum of all of the rhamnolipids comprised by the biosurfactant **S_{Bio}** provided in step **a)**.

### I.3.3 Sophorolipids

In the context of the method according to the invention, the term "sophorolipids" is in particular to be understood as referring to compounds of the general formulae **(V)**, **(VII)** and salts thereof, preferably compounds of the general formulae **(VI), (VIII)** and salts thereof:

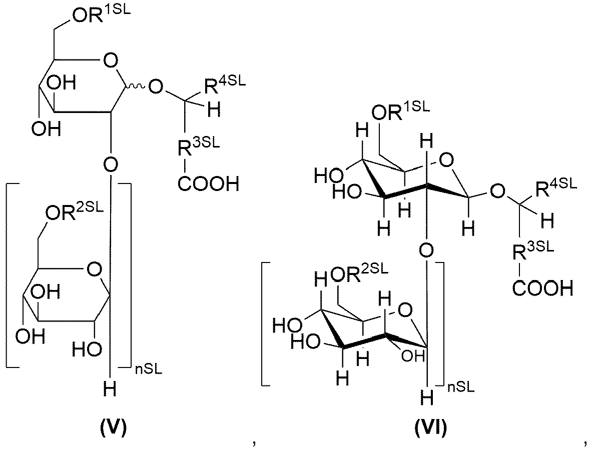

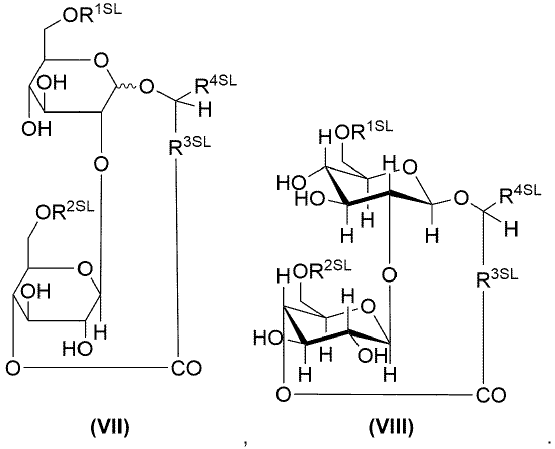

Formulae **(V)**, **(VI)** represent the acid form, Formulae **(VII)**, **(VIII)** represent the lactone form.

In formulae **(V)**, **(VI)**, nSL = 4, 3, 2, 1 or 0, preferably nSL = 1 or 0.
In formulae **(V)**, **(VI)**, **(VII)**, and **(VIII)**,
R^{1SL} = H or -CO-CHs,
R^{2SL} = H or -CO-CHs,
R^{3SL} = a divalent organic moiety which comprises 6 to 32 carbon atoms, preferably 12 to 20, more preferably 14 to 16, most preferably 15.

In those cases where a compound according to one of formulae **(V)**, **(VI)** comprises more than one residue R^{2SL}, these residues R^{2SL} may be the same or different.

R^{3SL} preferably is an optionally substituted, divalent hydrocarbon moiety comprising 6 to 32 carbon atoms, wherein hydroxy substituted hydrocarbon moieties are preferred as substituted hydrocarbon moieties.

R^{3SL} more preferably is selected from the group consisting of
- optionally substituted alkylene radicals with 6 to 32, preferably 12 to 20, more preferably 14 to 16, most preferably 15 carbon atoms, wherein hydroxy-substituted alkylene radicals are preferred substituted alkylene radicals, and wherein it is preferred that the optionally substituted alkylene radicals are unbranched,
- optionally substituted alkenylene radicals with 6 to 32, preferably 12 to 20, more preferably 14 to 16, most preferably 15 carbon atoms, wherein hydroxy-substituted alkenylene radicals are preferred substituted alkenylene radicals, and wherein it is preferred that the optionally substituted alkenylene radicals are unbranched, and wherein it is preferred that the optionally substituted alkenylene radicals comprise one to three double or triple bonds.

R^{3SL} even more preferably is selected from the group consisting of
- unbranched or branched, preferably unbranched, alkylene radicals with 6 to 32, preferably 12 to 20, more preferably 14 to 16, most preferably 15, carbon atoms,
- unbranched or branched, preferably unbranched, alkylene radicals with 6 to 32 preferably 12 to 20, more preferably 14 to 16, most preferably 15, carbon atoms carrying at least one hydroxy group, preferably one hydroxy group,
- unbranched or branched, preferably unbranched, alkenylene radicals with 6 to 32, preferably 12 to 20, more preferably 14 to 16, most preferably 15 carbon atoms, wherein the alkenylene radicals preferably comprise one to three double or triple bonds, wherein the alkenylene radicals more preferably comprise one to three double bonds, even more preferably one double bond,
- unbranched or branched, preferably unbranched, alkenylene radicals with 6 to 32, preferably 12 to 20, more preferably 14 to 16, most preferably 15, carbon atoms carrying at least one hydroxy group, preferably one hydroxy group, wherein the alkenylene radicals preferably comprise one to three double or triple bonds, wherein the alkenylene radicals more preferably comprise one to three double bonds, even more preferably one double bond.

R^{4SL} = H, CH₃ or a monovalent organic radical which comprises 2 to 10 carbon atoms.

R^{4SL} is preferably selected from the group consisting of
- H,
- CH₃,
- optionally substituted alkyl radicals with 2 to 10 carbon atoms, wherein hydroxy-substituted alkyl radicals are preferred substituted alkyl radicals, and wherein it is preferred that the optionally substituted alkyl radicals are unbranched,
- optionally substituted alkenyl radicals with 2 to 10 carbon atoms, wherein hydroxy-substituted alkenyl radicals are preferred substituted alkenyl radicals, and wherein it is preferred that the optionally substituted alkenyl radicals are unbranched, and wherein it is preferred that the optionally substituted alkenyl radicals comprise one to three double or triple bonds.

R^{4SL} is more preferably selected from the group consisting of
- H,
- CH₃,
- unbranched or branched, preferably unbranched, alkyl radicals with 2 to 10 carbon atoms,
- unbranched or branched, preferably unbranched, alkyl radicals with 2 to 10 carbon atoms carrying at least one hydroxy group, preferably one hydroxy group,
- unbranched or branched, preferably unbranched, alkenyl radicals with 2 to 10 carbon atoms, wherein the alkenyl radicals preferably comprise one to three double or triple bonds, wherein the alkenyl radicals more preferably comprise one to three double bonds, even more preferably one double bond,
- unbranched or branched, preferably unbranched, alkenyl radicals with 2 to 10 carbon atoms carrying at least one hydroxy group, preferably one hydroxy group, wherein the alkenyl radicals preferably comprise one to three double or triple bonds, wherein the alkenyl radicals more preferably comprise one to three double bonds, even more preferably one double bond.

R^{4SL} is most preferably selected from the group consisting of H, Methyl, Ethyl.

In an even more preferred embodiment, the term "sophorolipids" is to be understood as referring to compounds of the general formulae **(IX)**, **(XI)** and salts thereof, preferably compounds of the general formulae **(X)**, **(XII)** and salts thereof:

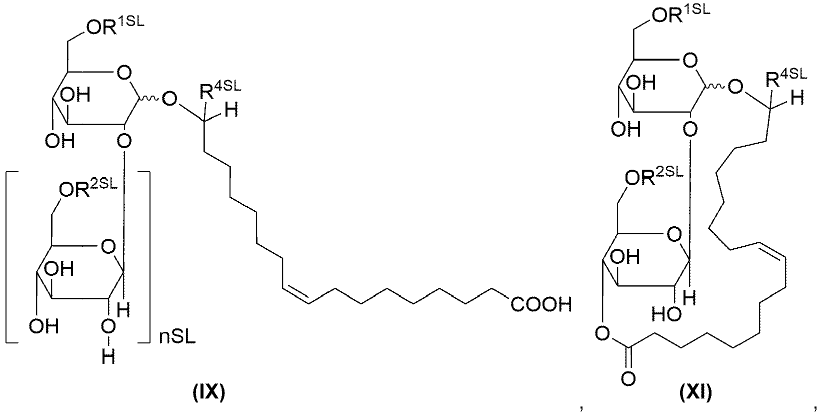

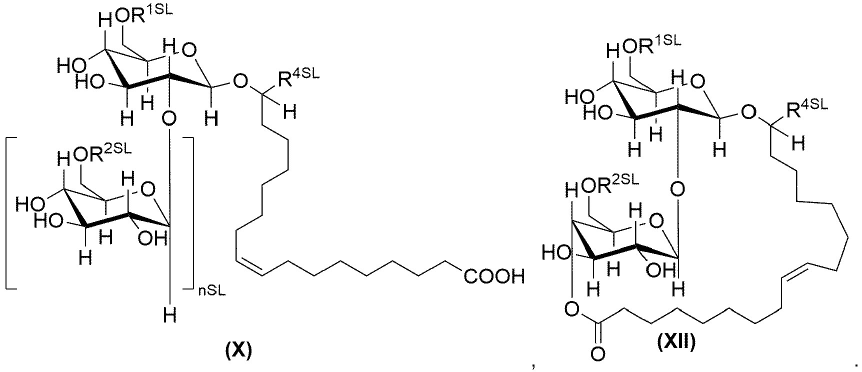

Formulae **(IX)**, **(X)** represent the acid form, Formulae **(XI)**, **(XII)** represent the lactone form.

nSL, R^{1SL}, R^{2SL}, R^{4SL} have the same meaning as described for formulae **(V), (VII), (VI), (VIII).**

Sophorolipids may be used in accordance with the invention in their acid form or their lactone form.

In those embodiments where the biosurfactant **S_{Bio}** provided in step **a)** of the method according to the first aspect of the invention comprises sophorolipids, it is preferred that these sophorolipids comprised by the biosurfactant **S_{Bio}** provided in step **a)** are mixtures of the acid and the lactone form, wherein even more preferably the ratio of the weight of all sophorolipids in the lactone form comprised by the biosurfactant **S_{Bio}** provided in step **a)** to the weight of all sophorolipids in the acid form comprised by the biosurfactant **S_{Bio}** provided in step **a)** is in the range of from 20 : 80 to 80 : 20, especially preferably in the range of from 30 : 70 to 40 : 60.

To determine the content of sophorolipids in the acid or lactone form in a formulation, refer to EP 1 411 111 B1, page 8, paragraph [0053].

Sophorolipids may be obtained as described in EP 1 411 111 A1, paragraphs [0021], [0022].

The biosurfactant **S_{Bio}** provided in step **a)** of the method according to the invention may also comprise derivatives of sophorolipids such as esters of glycerol which are esterified with sophorolipids (mono-, di-, and triesters of sophorolipids with glycerol) and optionally also fatty acids.

The biosurfactant **S_{Bio}** provided in step **a)** of the method according to the invention may therefore comprise derivatives of sophorolipids such as those described in EP 3 034 613 A1 or EP 4 317 448 A1.

### I.4 Variant 1

### I.4.1 Step a1)

In step a1) of **variant 1**, **SP** is provided and, separately from **SP**, at least one biosurfactant **S_{Bio}** is provided. **S_{Bio}** is a glycolipid, in particular selected from rhamnolipids, glucolipids, sophorolipids, preferably selected from rhamnolipids, sophorolipids, even more preferably selected from rhamnolipids.

**SP** may be provided in any form familiar to the skilled person, as long as such form comprises spores **SP** that are still able to germinate. For example, it may be provided in the form of a preparation **P_{SP}** of the respective bacteria, comprising spores **SP**.

In those embodiments where **variant 1** is applied in the context of a cleaning method, the spores **SP** and/or the at least one biosurfactant **S_{Bio}** may also each be provided as a cleaning formulation **F**.

Hence, in particular, the provision of **SP** and **S_{Bio}** in step a1) comprises (but is not limited to) an embodiment in which a, preferably aqueous, mixture **M_{SP1}** comprising **SP** (but not **S_{Bio}**) and a, preferably aqueous, mixture **M_{Bio1}** comprising **S_{Bio}** (but not **SP**) are provided.

**M_{SP1}** and **M_{Bio1}** may each comprise further components, especially depending on the application of the method according to **variant 1**.

In those embodiments where **variant 1** is applied in the context of a method of cleaning, at least one of the mixtures **M_{SP1}** and **M_{Bio1}** may be provided as a cleaning formulation **F**, wherein the preferred components of cleaning formulations **F** are further explained in the following.

In those embodiments where **M_{SP1}** is an aqueous mixture, it is preferred that the content of water in **M_{SP1}** is from 1 wt.-% to 99 wt.-%, more preferably from 10 wt.-% to 99 wt.-%, more preferably from 30 wt.-% to 98 wt.-%, more preferably from 50 wt.-% to 97 wt.-%, more preferably from 60 wt.-% to 96 wt.-%, more preferably from 70 wt.-% to 95 wt.-%, more preferably from 80 wt.-% to 90 wt.-%, wherein the weight-% give the percentage of water relative to the total weight of the mixture **M_{SP1}.**

In those embodiments where **M_{Bio1}** is an aqueous mixture, it is preferred that the content of water in **M_{Bio1}** is from 1 wt.-% to 99 wt.-%, more preferably from 10 wt.-% to 99 wt.-%, more preferably from 30 wt.-% to 98 wt.-%, more preferably from 50 wt.-% to 97 wt.-%, more preferably from 60 wt.-% to 96 wt.-%, more preferably from 70 wt.-% to 95 wt.-%, more preferably from 80 wt.-% to 90 wt.-%, wherein the weight-% give the percentage of water relative to the total weight of the mixture **M_{Bio1}**.

### I.4.2 Step b1)

In step b1) of **variant 1**, surface **S** is contacted with **SP** and **S_{Bio},** so that a mixture **M₁** comprising **SP** and at least one biosurfactant **S_{Bio}** is obtained, wherein mixture **M₁** is in contact with surface **S**.

"Contacting surface **S** with **SP** and **S_{Bio}**" is a further essential feature of **variant 1**. It results in a mixture **M₁** comprising **SP** and at least one biosurfactant **S_{Bio}** that is in contact with **S.** This is to be understood as meaning that the mixture **M₁** and at least a part of the surface **S** are in direct contact. In the context of the present invention "in direct contact" is to be understood as meaning "wetting" of the surface **S** with **M₁**. It will be appreciated that the surface **S** and the mixture **M₁** are thus in direct contact at a contact surface **Sc** of surface **S**.

The contacting step b1) is not further limited. Where two separate mixtures **M_{SP1}** and **M_{Bio1}**, namely a mixture **M_{SP1}** comprising **SP** (but not **S_{Bio}**) and a mixture **M_{Bio1}** comprising **S_{Bio}** (but not **SP**) are provided in step a1) of the method according to **variant 1**, the respective mixtures **M_{SP1}** and **M_{Bio1}** may simply be contacted with **S** and mixed to form mixture **M₁** before or while, preferably while, the two mixtures **M_{SP1}** and **M_{Bio1}** are already in contact with **S**.

In those embodiments where **variant 1** is applied as a method of cleaning, it is preferred that the mixture **M₁** which is in contact with **S** at the end of step b1) of **variant 1**, is also in contact with at least one impurity **I**.

This means in particular that during step b1) and/or during step c1), preferably during step c1), of **variant 1**, at least one impurity **I** which is absorbed or adsorbed on the surface **S**, in particular contact surface **S**_{C}, is at least partially desorbed (and thus removed) from the surface **S** and dispersed or dissolved, in particular dispersed, in the mixture **M₁**. Optionally, the at least one impurity I is then degraded by at least one activity of at least one vegetative cell germinating from **SP** during step c1) of **variant 1**.

In other words, in this preferred embodiment, during step b1) and/or during step c1) of **variant 1**, the mixture **M₁** is brought in direct contact with at least a part **S_{C}** of the surface **S**, wherein at least one impurity **I** is absorbed or adsorbed on **S**_{C}, so that the at least one impurity **I** is at least partially desorbed from the surface **S_{C}** and dispersed or dissolved, in particular dispersed, in the mixture **M₁** (and thus removed) from surface **S_{C}.** The dispersed or dissolved, in particular dispersed, impurity **I** is then optionally degraded in the mixture **M₁** by at least one activity of at least one vegetative cell germinating from **SP** during step c1) of **variant 1.**Therefore, in particular, after step c1) of **variant 1**, a mixture **M₁** is obtained wherein at least one impurity **I** in dissolved or dispersed, preferably dispersed.

The impurity **I** may either be in the form of a liquid (e.g. droplet) or a particle, and preferably is a particle.

In a preferred embodiment, the at least one impurity **I** is an impurity which is inorganic or organic, and is preferably organic.

Organic impurities are preferably selected from oils and fats, which may be of vegetable, animal or synthetic origin, food based particles, soil, pollen, sebum, body fluids, for example, blood, sperm, sweat, urine, feces, liquor.

Inorganic impurities are preferably selected from the group consisting of carbon black, fine dust, plastic particles (e.g. microplastic particles), metal oxides (preferably iron oxide), silica (such as sand), clay, dyes (such as pigments), more preferably clay, in particular kaolin.

*Bacillus velezensis* strain DSM 34978 and mutants of *Bacillus velezensis* strain DSM 34978, *Bacillus velezensis* DSM 34674, mutants of *Bacillus velezensis* DSM 34674 have an outstanding activity to produce at least one, preferably all, of protease activity, amylase activity, or cellulase activity. Where **variant 1** is used as a cleaning method, it is hence preferred that, when the spores **SP** of *Bacillus velezensis* strain DSM 34978, *Bacillus velezensis* DSM 34674, mutants of *Bacillus velezensis* DSM 34674 and/or mutants of *Bacillus velezensis* strain DSM 34978 are selected as spores **SP** in **variant 1**, at least a part of the impurities **I** cleaned during **variant 1** are the substrates of these activities. Hence, it is preferred that in this preferred embodiment, the impurity **I** which is cleaned from the surface **S** during the cleaning method according to **variant 1** is selected from the group consisting of valeric acid, proteins (in particular proteins selected from casein and whey protein), polysaccharides (in particular starch), cellulose. More preferred, it comprises proteins (in particular proteins selected from casein and whey protein), cellulose. Most preferred, it comprises proteins, in particular proteins selected from casein and whey protein.

Valeric acid (abbreviated as "**VA**") is pentanoic acid, i.e. CH₃(CH₂)₃COOH (CAS-No.: 109-52-4).

*Bacillus licheniformis* strain DSM 34977 and mutants of *Bacillus licheniformis* strain DSM 34977 have an outstanding activity to metabolize valeric acid. Where the method according to **variant 1** is used as a cleaning method, it is hence preferred that, when the spores **SP** of *Bacillus licheniformis* strain DSM 34977 and/or mutants of *Bacillus licheniformis* strain DSM 34977 are selected as spores **SP** in **variant 1,** that at least a part of the impurities **I** cleaned during **variant 1** are the substrates of this activity. Hence, it is preferred that in this preferred embodiment, the impurity **I** which is cleaned from the surface **S** during the cleaning method according to **variant 1** is valeric acid.

*Priestia megaterium* strain DSM 34980 and mutants of *Priestia megaterium* strain DSM 34980 have an outstanding activity to produce protease activity. Where the method according to **variant 1** is used as a cleaning method, it is hence preferred that, when the spores **SP** of *Priestia megaterium* strain DSM 34980 and/or mutants of *Priestia megaterium* strain DSM 34980 are selected as spores **SP** in the method according to **variant 1**, that at least a part of the impurities **I** cleaned during **variant 1** are the substrates of these activities. Hence, it is preferred that in this preferred embodiment, the impurity **I** which is cleaned from the surface **S** during the cleaning method according to **variant 1** is selected from the group consisting of valeric acid, proteins (in particular proteins selected from casein and whey protein), in particular proteins, which are even more preferably selected from casein and whey protein.

*Bacillus subtilis* strain DSM 34981 and mutants of *Bacillus subtilis* strain DSM 34981 have an outstanding activity to produce lipase activity. Where the method according to **variant 1** is used as a cleaning method, it is hence preferred that, when the spores **SP** of *Bacillus subtilis* strain DSM 34981 and/or mutants of *Bacillus subtilis* strain DSM 34981 are selected as spores **SP** in the method according to **variant 1,** that at least a part of the impurities **I** cleaned during **variant 1** are the substrates of these activities. Hence, it is preferred that in this preferred embodiment, the impurity **I** which is cleaned from the surface **S** during the cleaning method according to **variant 1** is selected from the group consisting of valeric acid, grease, oil, more preferably grease, oil.

In a further embodiment, where the spores **SP** in the method according to **variant 1** are selected from *Bacillus velezensis* strain DSM 34978 and mutants thereof, *Bacillus velezensis* DSM 34674 and mutants thereof, *Bacillus licheniformis* DSM strain DSM 34977 and mutants thereof, *Priestia megaterium* strain DSM 34980 and mutants thereof and *Bacillus subtilis* strain DSM 34981 and mutants thereof, it is preferred that the impurity **I** is selected from the group consisting of valeric acid, grease, oil, proteins (in particular proteins selected from casein and whey protein), polysaccharides (in particular starch), cellulose.

### I.4.3 Step c1)

In step c1) of **variant 1, M₁** is incubated to germinate at least a part of the spores **SP** comprised by mixture **M₁**.

The skilled person is aware of the conditions to be applied in step c1) that cause at least a part of the spores **SP** to germinate.

It is preferred that mixture **M₁** in steps b1) and c1) of **variant 1** is an aqueous mixture comprising the spores **SP** and the at least one biosurfactant **S_{Bio}**.

This is preferably achieved by simply adding water to the mixture **M₁** during step c1).

Alternatively or additionally, and in those embodiments where in step a1) a mixture **M_{SP1}** comprising **SP** (but not **S_{Bio}**) and a mixture **M_{Bio1}** comprising **S_{Bio}** (but not **SP**) is provided, at least one of these mixture **M_{SP1}**, **M_{Bio1}** may simply be provided as an aqueous mixture.

In addition, in step b1), the respective microorganism may also be contacted with at least one from the group consisting of nutrients, salts.

Preferred nutrients are selected from carbohydrates, amino acids, peptides, peptone, glucose, starch.

Peptides are preferably selected from peptone.

As amino acid, any of the natural amino acids may be selected by the skilled person. Preferably, the amino acid is selected from the group consisting of glutamine, alanine, valine, aspartate, glutamate, methionine, asparagine, preferably alanine, valine, asparagine.

Carbohydrates are preferably sugars, polysaccharides.

Preferred sugars are glucose, saccharose, fructose, galactose.

Preferred polysaccharides are starch, inulin.

Preferred salts are salts selected from sodium chloride, potassium phosphate, di-potassium hydrogen phosphate

Further additives known to the skilled person may also be applied to the mixture **M₁** in step c1), such as tris(hydroxymethyl)aminomethane **(**"**Tris**"**)**.

In addition, the germination of bacteria such as *Bacillus* or *Priestia* is also triggered by contacting the spores with substrates of one or more enzymatic or metabolic activity of these bacteria.

Hence, where spores **SP** of at least one of *Bacillus velezensis* strain DSM 34978, mutants of *Bacillus velezensis* strain DSM 34978, *Bacillus velezensis* DSM 34674, mutants of *Bacillus velezensis* DSM 34674, *Bacillus licheniformis* strain DSM 34977, mutants of *Bacillus licheniformis* strain DSM 34977, *Priestia megaterium* strain DSM 34980, mutants of *Priestia megaterium* strain DSM 34980, *Bacillus subtilis* strain DSM 34981 and mutants of *Bacillus subtilis* strain DSM 34981 are selected as spores **SP** in **variant 1**, it is further preferable that the mixture **M₁** in step c1) comprises the impurities **I** as set forth in the following:
*Bacillus velezensis* strain DSM 34978 and mutants of *Bacillus velezensis* strain DSM 34978, *Bacillus velezensis* DSM 34674, mutants of *Bacillus velezensis* DSM 34674: mixture **M₁** in step c1) preferably comprises at least one impurity **I** selected from the group consisting of valeric acid, proteins (in particular proteins selected from casein and whey protein), polysaccharides (in particular starch), cellulose; more preferred at least one impurity **I** selected from the group consisting of proteins (in particular proteins selected from casein and whey protein), cellulose; most preferred at least one impurity **I** selected from the group consisting of proteins, in particular proteins selected from casein and whey protein.

*Bacillus licheniformis* strain DSM 34977 and mutants of *Bacillus licheniformis* strain DSM 34977: mixture **M₁** in step c1) preferably comprises impurity **I** selected from valeric acid.

*Priestia megaterium* strain DSM 34980 and mutants of *Priestia megaterium* strain DSM 34980: mixture **M₁** in step c1) preferably comprises at least one impurity **I** selected from the group consisting of valeric acid, proteins (in particular proteins selected from casein and whey protein), preferably at least one impurity **I** selected from the group consisting of proteins which even more preferably are selected from casein and whey protein.

*Bacillus subtilis* strain DSM 34981 and mutants of *Bacillus subtilis* strain DSM 34981: mixture **M₁** in step c1) preferably comprises at least one impurity **I** selected from the group consisting of valeric acid, grease, oil, more preferably at least one impurity **I** selected from the group consisting of grease, oil.

The temperature of incubation in step c1) is in particular in the range of from 10 °C to 70 °C, in particular in the range of from 20 °C to 65 °C, preferably in the range of from 25 °C to 55 °C, more preferably in the range of from 30 °C to 45 °C.

Where the surface **S** is the surface **S_{O}** of an object **O**, in particular where **variant 1** is applied as a method for cleaning object **O**, it is preferred that the temperature in step c1) is in a range of from 10 °C to 70 °C, in particular in a range of from 20 °C to 65 °C, preferably in a range of from 25 °C to 55 °C, more preferably in a range of from 30 °C to 45 °C.

Where the surface **S** is the surface **S_{B}** of the human or animal body **B**, in particular where **variant 1** is applied as a method for cleaning the human or animal body **B**, it is preferred that the temperature in step c1) is in a range of from 10 °C to 50 °C, in particular in a range of from 20 °C to 45 °C, preferably in a range of from 25 °C to 40 °C, more preferably in a range of from 30 °C to 38 °C, most preferably 37 °C.

In a further preferred embodiment, the incubation in step c1) is carried out for at least 1 second ("second" = "s"), preferably for at least 10 s, preferably for at least 1 minute ("minute" = "min"), more preferably for at least 5 min, preferably for at least 15 min, preferably for at least 60 min, more preferably from 15 min to 3 d, more preferably from 30 min to 2 d, more preferably from 45 min to 1 d.

Preferably, in those cases where **M₁** comprises water, it is preferred that the pH of the water in **M₁** in step c1) at 25 °C is from 3.5 to 9, preferably from 5 to 9, preferably from 6 to 9, and particularly preferably from 7 to 8.

It is further preferred that the incubation in step c1) is carried out until at least 10 % of the spores **SP** comprised by mixture **M₁** that is contacted with **S** in step b1) have germinated, relative to the number of spores **SP** comprised by mixture **M₁** before mixture **M₁** is subjected to the incubation according to step c1) [i.e. relative to the number of spores **SP** comprised by mixture **M₁** that is obtained directly upon contacting **S** with **SP** and **S_{Bio}** in step b1)]. This "germination rate" in step c1) is preferably determined according to **Assay M-I**.

It is even further preferred that the incubation in step c1) is carried out until at least 20 % of the spores **SP** comprised by mixture **M₁** that is in contact with **S** in step b1) have germinated, more preferably until at least 30 %, even more preferably until at least 40 %, even more preferably until at least 50 %, even more preferably until at least 60 %, even more preferably until at least 70 %, even more preferably until at least 80 %, even more preferably until at least 90 %, even more preferably until at least 95 %, even more preferably until at least 99 % of the spores **SP** comprised by mixture **M₁** that is in contact with **S** in step b1) have germinated, relative to the number of spores **SP** comprised by mixture **M₁** before mixture **M₁** is subjected to the incubation according to step c1).

### I.4.4 Step d1) (optional)

It is preferred that, in a step d1), which is carried out after step c1), at least a part of **M₁** is separated from **S**. The separation is, in a preferred embodiment, carried out so that **M₁** is essentially complete separated from **S**.

This embodiment is particularly preferred where the method according to **variant 1** is applied as a cleaning method, in particular on a surface **S_{O}** of an object **O** or on a surface **S_{B}** of the human or animal body **B**.

The skilled person is aware of how to carry out this separation in each specific context. Generally, this separation is carried out by withdrawing the surface **S** from at least a part of **M₁**.

An object **O** may, in step d1), optionally be subjected to centrifugation (for example in a washing machine).

### I.4.5 Step e1) (optional)

In those optional embodiments of **variant 1** where step d1) is carried out, it is even more preferred to carry out another optional step e1) after step d1).

In an optional step e1) of **variant 1,** the surface **S** is rinsed with further water.

### I.5 Variant 2

### I.5.1 Step a2)

In step a2) of **variant 2**, a mixture **M*** comprising **SP** and at least one biosurfactant **S_{Bio}** is provided. **S_{Bio}** is a glycolipid, in particular selected from rhamnolipids, glucolipids, sophorolipids, preferably selected from rhamnolipids, sophorolipids, even more preferably selected from rhamnolipids.

Mixture **M*** may be provided in **variant 2** in any form familiar to the skilled person, as long as such form comprises at least one biosurfactant **S_{Bio}** and spores **SP** that are still able to germinate. For example, mixture **M*** may be provided in the form of a preparation **P_{SP}** of the respective bacteria, comprising spores **SP**, wherein the preparation **P_{SP}** also comprises at least one biosurfactant **S_{Bio}**.

In those embodiments where **variant 2** is applied in the context of a cleaning method, mixture **M*** may also be provided as a cleaning formulation **F**.

Mixture **M*** may comprise further components, especially depending on the application of the method according to **variant 2.**

In those embodiments where **variant 2** is applied in the context of a method of cleaning, mixture **M*** may be provided as a cleaning formulation **F**, wherein the preferred components of cleaning formulations **F** are further explained in the following.

Mixture **M*** is preferably aqueous.

In those embodiments where mixture **M*** is an aqueous mixture, it is preferred that the content of water in **M*** is from 1 wt.-% to 99 wt.-%, more preferably from 10 wt.-% to 99 wt.-%, more preferably from 30 wt.-% to 98 wt.-%, more preferably from 50 wt.-% to 97 wt.-%, more preferably from 60 wt.-% to 96 wt.-%, more preferably from 70 wt.-% to 95 wt.-%, more preferably from 80 wt.-% to 90 wt.-%, wherein the weight-% give the percentage of water relative to the total weight of the mixture **M***.

### I.5.2 Step b2)

In step b2) of **variant 2,** surface **S** is contacted with the mixture **M***, so that a mixture **M₂** comprising **SP** and at least one biosurfactant **S_{Bio}** is obtained, wherein mixture **M₂** is in contact with surface **S**.

### I.5.2.1 Optional Pre-incubation in Step b2)

In an optional embodiment, mixture **M*** is incubated before mixture **M*** is contacting **S** ("pre-incubation step").

This optional pre-incubation step is preferably performed when mixture **M*** is aqueous. It may be performed to the skilled person's knowledge, for example by incubating the mixture **M*** at a temperature in the range of from 1°C to 95 °C, preferably at a temperature in a range of from 10 °C to 70 °C, in particular in a range of from 20 °C to 65 °C, preferably in a range of from 25 °C to 55 °C, more preferably in a range of from 30 °C to 45 °C.

This optional pre-incubation step is preferably carried out for at least 1 second ("second" = "s"), preferably for at least 10 s, preferably for at least 1 minute ("minute" = "min"), more preferably for at least 5 min, preferably for at least 15 min, preferably for at least 60 min, more preferably from 15 min to 3 d, more preferably from 30 min to 2 d, more preferably from 45 min to 1 d.

During the optional pre-incubation step, mixture **M*** is preferably stirred.

The optional pre-incubation step is advantageous because it contacts the spores **SP** in the mixture **M*** with the at least one biosurfactant **S_{Bio},** before mixture **M*** is applied to surface **S**. The early contacting of the spores **SP** with the at least one biosurfactant is believed to further facilitate the spores' **SP** germination on surface **S** during step c2).

### 1.5.2.2 Contacting in Step b2)

"Contacting surface **S** with the mixture **M***" is a further essential feature of **variant 2**. It results in a mixture **M₂** comprising **SP** and at least one biosurfactant **S_{Bio}** that is in contact with **S**. This is to be understood as meaning that the mixture **M₂** and at least a part of the surface **S** are in direct contact. In the context of the present invention "in direct contact" is to be understood as meaning "wetting" of the surface **S** with **M₂**. It will be appreciated that the surface **S** and the mixture **M₂** are thus in direct contact at a contact surface **S_{C}** of surface **S**.

The contacting step b2) is not further limited. In **variant 2,** the mixtures **M*** may simply be contacted with **S** to form mixture **M₂** in contact with **S**.

In those embodiments where **variant 2** is applied as a method of cleaning, it is preferred that the mixture **M₂** which is in contact with **S** at the end of step b2) of **variant 2,** is also in contact with at least one impurity **I**.

This means in particular that during step b2) and/or during step c2), preferably during step c2), of **variant 2**, at least one impurity **I** which is absorbed or adsorbed on the surface **S**, in particular contact surface **S_{C}**, is at least partially desorbed (and thus removed) from the surface **S** and dispersed or dissolved, in particular dispersed, in the mixture **M₂**. Optionally, the at least one impurity **I** is then degraded by at least one activity of at least one vegetative cell germinating from **SP** during step c2) of **variant 2**.

In other words, in this preferred embodiment, during step b2) and/or during step c2) of **variant 2**, the mixture **M₂** is brought in direct contact with at least a part **S_{C}** of the surface **S**, wherein at least one impurity **I** is absorbed or adsorbed on **S_{C}**, so that the at least one impurity **I** is at least partially desorbed from the surface **S_{C}** and dispersed or dissolved, in particular dispersed, in the mixture **M₂** (and thus removed) from surface **S_{C}**. The dispersed or dissolved, in particular dispersed, impurity **I** is then optionally degraded in the mixture **M₂** by at least one activity of at least one vegetative cell germinating from **SP** during step c2) of **variant 2**.Therefore, in particular, after step c2) of **variant 2**, a mixture **M₂** is obtained wherein at least one impurity **I** in dissolved or dispersed, preferably dispersed.

The impurity **I** may either be in the form of a liquid (e.g. droplet) or a particle, and preferably is a particle.

In a preferred embodiment, the at least one impurity **I** is an impurity which is inorganic or organic, and is preferably organic.

Organic impurities are preferably selected from oils and fats, which may be of vegetable, animal or synthetic origin, food based particles, soil, pollen, sebum, body fluids, for example, blood, sperm, sweat, urine, feces, liquor.

Inorganic impurities are preferably selected from the group consisting of carbon black, fine dust, plastic particles (e.g. microplastic particles), metal oxides (preferably iron oxide), silica (such as sand), clay, dyes (such as pigments), more preferably clay, in particular kaolin.

*Bacillus velezensis* strain DSM 34978 and mutants of *Bacillus velezensis* strain DSM 34978, *Bacillus velezensis* DSM 34674, mutants of *Bacillus velezensis* DSM 34674 have an outstanding activity to produce at least one, preferably all, of protease activity, amylase activity, or cellulase activity. Where the method according to **variant 2** is used as a cleaning method, it is hence preferred that, when the spores **SP** of *Bacillus velezensis* strain DSM 34978, *Bacillus velezensis* DSM 34674, mutants of *Bacillus velezensis* DSM 34674 and/or mutants of *Bacillus velezensis* strain DSM 34978 are selected as spores **SP** in **variant 2,** that at least a part of the impurities **I** cleaned during **variant 2** are the substrates of these activities. Hence, it is preferred that in this preferred embodiment, the impurity **I** which is cleaned from the surface **S** during the cleaning method according to **variant 2** is selected from the group consisting of valeric acid, proteins (in particular proteins selected from casein and whey protein), polysaccharides (in particular starch), cellulose. More preferred, it comprises proteins (in particular proteins selected from casein and whey protein), cellulose. Most preferred, it comprises proteins, in particular proteins selected from casein and whey protein.

*Bacillus licheniformis* strain DSM 34977 and mutants of *Bacillus licheniformis* strain DSM 34977 have an outstanding activity to metabolize valeric acid. Where the method according to **variant 2** is used as a cleaning method, it is hence preferred that, when the spores **SP** of *Bacillus licheniformis* strain DSM 34977 and/or mutants of *Bacillus licheniformis* strain DSM 34977 are selected as spores **SP** in **variant 2,** that at least a part of the impurities **I** cleaned during **variant 2** are the substrates of this activity. Hence, it is preferred that in this preferred embodiment, the impurity **I** which is cleaned from the surface **S** during the cleaning method according to **variant 2** is valeric acid.

*Priestia megaterium* strain DSM 34980 and mutants of *Priestia megaterium* strain DSM 34980 have an outstanding activity to produce protease activity. Where the method according to **variant 2** is used as a cleaning method, it is hence preferred that, when the spores **SP** of *Priestia megaterium* strain DSM 34980 and/or mutants of *Priestia megaterium* strain DSM 34980 are selected as spores **SP** in the method according to **variant 2,** that at least a part of the impurities **I** cleaned during **variant 2** are the substrates of these activities. Hence, it is preferred that in this preferred embodiment, the impurity **I** which is cleaned from the surface **S** during the cleaning method according to **variant 2** is selected from the group consisting of valeric acid, proteins (in particular proteins selected from casein and whey protein), in particular proteins, which are even more preferably selected from casein and whey protein.

*Bacillus subtilis* strain DSM 34981 and mutants of *Bacillus subtilis* strain DSM 34981 have an outstanding activity to produce lipase activity. Where the method according to **variant 2** is used as a cleaning method, it is hence preferred that, when the spores **SP** of *Bacillus subtilis* strain DSM 34981 and/or mutants of *Bacillus subtilis* strain DSM 34981 are selected as spores **SP** in the method according to **variant 2,** that at least a part of the impurities **I** cleaned during **variant 2** are the substrates of these activities. Hence, it is preferred that in this preferred embodiment, the impurity **I** which is cleaned from the surface **S** during the cleaning method according to **variant 2** is selected from the group consisting of valeric acid, grease, oil, more preferably grease, oil.

In a further embodiment, where the spores **SP** in the method according to **variant 2** are selected from *Bacillus velezensis* strain DSM 34978 and mutants thereof, *Bacillus velezensis* DSM 34674 and mutants thereof, *Bacillus licheniformis* DSM strain DSM 34977 and mutants thereof, *Priestia megaterium* strain DSM 34980 and mutants thereof and *Bacillus subtilis* strain DSM 34981 and mutants thereof, it is preferred that the impurity **I** is selected from the group consisting of valeric acid, grease, oil, proteins (in particular proteins selected from casein and whey protein), polysaccharides (in particular starch), cellulose.

### I.5.3 Step c2)

In step c2) of **variant 2, M₂** is incubated to germinate at least a part of the spores **SP** comprised by mixture **M₂**.

The skilled person is aware of the conditions to be applied in step c2) that cause at least a part of the spores **SP** to germinate.

It is preferred that mixture **M₂** in steps b2) and c2) of **variant 2** is an aqueous mixture comprising the spores **SP** and the at least one biosurfactant **S_{Bio}**.

This is preferably achieved by simply adding water to the mixture **M₂** during step c2).

Alternatively or additionally, in step a2) the mixture **M*** may simply be provided as an aqueous mixture.

In addition, in step b2), the respective microorganism may also be contacted with nutrients and/or salts.

Preferred nutrients are selected from carbohydrates, amino acids, peptides, peptone, glucose, starch.

Peptides are preferably selected from peptone.

As amino acid, any of the natural amino acids may be selected by the skilled person. Preferably, the skilled person selects the amino acid from the group consisting of glutamine, alanine, valine, aspartate, glutamate, methionine, asparagine, preferably alanine, valine, asparagine.

Carbohydrates are preferably sugars, polysaccharides.

Preferred sugars are glucose, saccharose, fructose, galactose.

Preferred polysaccharides are starch, inulin.

Preferred salts are salts selected from sodium chloride, potassium phosphate, di-potassium hydrogen phosphate

Further additives known to the skilled person may also be applied to the mixture **M₂** in step c2), such as tris(hydroxymethyl)aminomethane ("**Tris**").

In addition, the germination of bacteria such as *Bacillus* or *Priestia* is also triggered by contacting the spores with substrates of one or more enzymatic or metabolic activity of these bacteria.

Hence, where spores **SP** of at least one of *Bacillus velezensis* strain DSM 34978, mutants of *Bacillus velezensis* strain DSM 34978, *Bacillus velezensis* DSM 34674, mutants of *Bacillus velezensis* DSM 34674, *Bacillus licheniformis* strain DSM 34977, mutants of *Bacillus licheniformis* strain DSM 34977, *Priestia megaterium* strain DSM 34980, mutants of *Priestia megaterium* strain DSM 34980, *Bacillus subtilis* strain DSM 34981 and mutants of *Bacillus subtilis* strain DSM 34981 are selected as spores **SP** in **variant 2**, it is further preferable that the mixture **M₂** in step c2) comprises the impurities **I** as set forth in the following:
*Bacillus velezensis* strain DSM 34978 and mutants of *Bacillus velezensis* strain DSM 34978, *Bacillus velezensis* DSM 34674, mutants of *Bacillus velezensis* DSM 34674: mixture **M₂** in step c2) preferably comprises at least one impurity **I** selected from the group consisting of valeric acid, proteins (in particular proteins selected from casein and whey protein), polysaccharides (in particular starch), cellulose; more preferred at least one impurity **I** selected from the group consisting of proteins (in particular proteins selected from casein and whey protein), cellulose; most preferred at least one impurity **I** selected from the group consisting of proteins, in particular proteins selected from casein and whey protein.

*Bacillus licheniformis* strain DSM 34977 and mutants of *Bacillus licheniformis* strain DSM 34977: mixture **M₂** in step c2) preferably comprises impurity **I** selected from valeric acid.

*Priestia megaterium* strain DSM 34980 and mutants of *Priestia megaterium* strain DSM 34980: mixture **M₂** in step c2) preferably comprises at least one impurity **I** selected from the group consisting of valeric acid, proteins (in particular proteins selected from casein and whey protein), preferably at least one impurity **I** selected from the group consisting of proteins which even more preferably are selected from casein and whey protein.

*Bacillus subtilis* strain DSM 34981 and mutants of *Bacillus subtilis* strain DSM 34981: mixture **M₂** in step c2) preferably comprises at least one impurity **I** selected from the group consisting of valeric acid, grease, oil, more preferably at least one impurity **I** selected from the group consisting of grease, oil.

The temperature of incubation in step c2) is in particular in the range of from 10 °C to 70 °C, in particular in the range of from 20 °C to 65 °C, preferably in the range of from 25 °C to 55 °C, more preferably in the range of from 30 °C to 45 °C.

Where the surface **S** is the surface **S_{O}** of an object **O**, in particular where **variant 2** is applied as a method for cleaning object **O**, it is preferred that the temperature in step c2) is in a range of from 10 °C to 70 °C, in particular in a range of from 20 °C to 65 °C, preferably in a range of from 25 °C to 55 °C, more preferably in a range of from 30 °C to 45 °C.

Where the surface **S** is the surface **S_{B}** of the human or animal body **B,** in particular where **variant 2** is applied as a method for cleaning the human or animal body **B**, it is preferred that the temperature in step c2) is in a range of from 10 °C to 50 °C, in particular in a range of from 20 °C to 45 °C, preferably in a range of from 25 °C to 40 °C, more preferably in a range of from 30 °C to 38 °C, most preferably 37 °C.

In a further preferred embodiment, the incubation in step c2) is carried out for at least 1 second ("second" = "s"), preferably for at least 10 s, preferably for at least 1 minute ("minute" = "min"), more preferably for at least 5 min, preferably for at least 15 min, preferably for at least 60 min, more preferably from 15 min to 3 d, more preferably from 30 min to 2 d, more preferably from 45 min to 1 d.

Preferably, in those cases where **M₂** comprises water, it is preferred that the pH of the water in **M₂** in step c2) at 25 °C is from 3.5 to 9, preferably from 5 to 9, preferably from 6 to 9, and particularly preferably from 7 to 8.

It is further preferred that the incubation in step c2) is carried out until at least 10 % of the spores **SP** comprised by mixture **M₂** that is contacted with **S** in step b2) have germinated, relative to the number of spores **SP** comprised by mixture **M₂** before mixture **M₂** is subjected to the incubation according to step c2) [i.e. relative to the number of spores **SP** comprised by mixture **M₂** that is obtained directly upon contacting **S** with mixture **M*** in step b2)]. This "germination rate" in step c2) is preferably determined according to **Assay M-II.**

It is even further preferred that the incubation in step c2) is carried out until at least 20 % of the spores **SP** comprised by mixture **M₂** that is in contact with **S** in step b2) have germinated, more preferably until at least 30 %, even more preferably until at least 40 %, even more preferably until at least 50 %, even more preferably until at least 60 %, even more preferably until at least 70 %, even more preferably until at least 80 %, even more preferably until at least 90 %, even more preferably until at least 95 %, even more preferably until at least 99 % of the spores **SP** comprised by mixture **M₂** that is in contact with **S** in step b2*) have germinated, relative to the number of spores **SP** comprised by mixture **M₂** before mixture **M₂** is subjected to the incubation according to step c2).

### I.5.4 Step d2) (optional)

It is preferred that, in a step d2), which is carried out after step c2), at least a part of **M₂** is separated from **S**. The separation is, in a preferred embodiment, carried out so that **M₂** is essentially complete separated from **S**.

This embodiment is particularly preferred where the method according to **variant 2** is applied as a cleaning method, in particular on a surface **S_{O}** of an object **O** or on a surface **S_{B}** of the human or animal body **B**.

The skilled person is aware of how to carry out this separation in each specific context. Generally, this separation is carried out by withdrawing the surface **S** from at least a part of **M₂**.

An object **O** may, in step d2), optionally be subjected to centrifugation (for example in a washing machine).

### I.5.5 Step e2) (optional)

In those optional embodiments of **variant 2** where step d2) is carried out, it is even more preferred to carry out another optional step e2) after step d2).

In an optional step e2) of **variant 2**, the surface **S** is rinsed with further water.

### I.6 Variant 3

**Variant 3** reflects a further advantage of the present invention. Namely, in **variant 1** and **variant 2**, the germination of the spores **SP** according to steps c1) and c2), respectively, is carried out in the presence of the at least one biosurfactant **S_{Bio}**. It was now found out that the effect of the at least one biosurfactant **S_{Bio}** on the germination is at least in part already achieved when the spores **SP** are pre-incubated with the at least one biosurfactant **S_{Bio}**. The at least one biosurfactant **S_{Bio}** exerts the positive effect already when used for preincubating the spores.

### I.6.1 Step a3)

In step a3) of **variant 3,** a mixture **M*** comprising **SP** and at least one biosurfactant **S_{Bio}** is provided. **S_{Bio}** is a glycolipid, in particular selected from rhamnolipids, glucolipids, sophorolipids, preferably selected from rhamnolipids, sophorolipids, even more preferably selected from rhamnolipids.

Mixture **M*** may be provided in **variant 3** in any form familiar to the skilled person, as long as such form comprises and at least one biosurfactant **S_{Bio}** and spores **SP** that are still able to germinate. For example, mixture **M*** may be provided in the form of a preparation **P_{SP}** of the respective bacteria, comprising spores **SP**, wherein the preparation **P_{SP}** also comprises at least one biosurfactant **S_{Bio}**.

In those embodiments where **variant 3** is applied in the context of a cleaning method, mixture **M*** may also be provided as a cleaning formulation **F**.

Mixture **M*** may comprise further components, especially depending on the application of the method according to **variant 3.**

In those embodiments where **variant 3** is applied in the context of a method of cleaning, mixture **M*** may be provided as a cleaning formulation **F**, wherein the preferred components of cleaning formulations **F** are further explained in the following.

Mixture **M*** is preferably aqueous.

In those embodiments where mixture **M*** is an aqueous mixture, it is preferred that the content of water in **M*** is from 1 wt.-% to 99 wt.-%, more preferably from 10 wt.-% to 99 wt.-%, more preferably from 30 wt.-% to 98 wt.-%, more preferably from 50 wt.-% to 97 wt.-%, more preferably from 60 wt.-% to 96 wt.-%, more preferably from 70 wt.-% to 95 wt.-%, more preferably from 80 wt.-% to 90 wt.-%, wherein the weight-% give the percentage of water relative to the total weight of the mixture **M***.

### I.6.2 Step b3)

Step b3) of **variant 3** comprises three consecutive sub-steps.

First, mixture **M*** is incubated ("pre-incubation").

Second, at least a part **π_{SP}** of the spores **SP** comprised by the incubated mixture **M*** is separated from at least a part of the biosurfactant **S_{Bio}** comprised by the incubated mixture **M***

Third, surface **S** is contacted with **π_{SP}**.

As a result of step b3), a mixture **M₃** comprising **SP** and optionally at least one biosurfactant **S_{Bio}** is obtained, wherein mixture **M₃** is in contact with surface **S**.

### I.6.3.1 Pre-incubation in Step b3)

Preferably, mixture **M*** that is subjected to the pre-incubation step in the context of step b3) of **variant 3** is aqueous.

This pre-incubation step may be performed according to the skilled person's knowledge, for example by incubating the mixture **M*** at a temperature in the range of from 1 °C to 95 °C, preferably at a temperature in a range of from 10 °C to 70 °C, in particular in a range of from 20 °C to 65 °C, preferably in a range of from 25 °C to 55 °C, more preferably in a range of from 30 °C to 45 °C.

This pre-incubation step is preferably carried out for at least 1 second ("second" = "s"), preferably for at least 10 s, preferably for at least 1 minute ("minute" = "min"), more preferably for at least 5 min, preferably for at least 15 min, preferably for at least 60 min, more preferably from 15 min to 3 d, more preferably from 30 min to 2 d, more preferably from 45 min to 1 d.

During the pre-incubation step, mixture **M*** is preferably stirred.

The pre-incubation step is advantageous because it contacts the spores **SP** in the mixture **M*** with the at least one biosurfactant **S_{Bio},** before mixture **M*** is applied to surface **S**. The early contacting of the spores **SP** with the at least one biosurfactant is believed to further faciliate the spores' **SP** germination on surface **S** during step c3).

### I.6.2.2 Separation Step in Step b3)

Second, at least a part **π_{SP}** of the spores **SP** comprised by the incubated mixture **M*** is separated from at least a part of the biosurfactant **S_{Bio}** comprised by the incubated mixture **M***

This separation step may be performed according to the skilled person's knowledge, for example by at least one step selected from filtration, centrifugation.

In case of centrifugation, at least a part **π_{SP}** of the spores **SP** will form the precipitate, while at least a part of the biosurfactant **S_{Bio}** will remain in the supernatant. The supernatant may then be removed and the precipitate washed, for example with water.

### I.6.2.3 Contacting in Step b3)

"Contacting surface **S** with the at least a part **π_{SP}** of the spores **SP**" is a further essential feature of **variant 3.** It results in a mixture **M₃** comprising **SP**. In those embodiments of step b3) where the separation of **SP** from the biosurfactant **S_{Bio}** was not complete, mixture **M₃** also comprises biosurfactant **S_{Bio}**. Hence, **M₃** comprises at least a part **π_{SP}** of the spores **SP** and optionally at least one biosurfactant **S_{Bio}.** In other words, **M₃** comprises spores **SP** and optionally at least one biosurfactant **S_{Bio}**.

**M₃** is in contact with **S**.

This is to be understood as meaning that the mixture **M₃** and at least a part of the surface **S** are in direct contact. In the context of the present invention "in direct contact" is to be understood as meaning "wetting" of the surface **S** with **M₃**. It will be appreciated that the surface **S** and the mixture **M₃** are thus in direct contact at a contact surface **S_{C}** of surface **S**.

The contacting step b3) is not further limited. In **variant 3,** the at least a part **π_{SP}** of the spores **SP** may simply be contacted with **S** to form mixture **M₃** in contact with **S**.

In those embodiments where **variant 3** is applied as a method of cleaning, it is preferred that the mixture **M₃** which is in contact with **S** at the end of step b3) of **variant 3,** is also in contact with at least one impurity **I**.

This means in particular that during step b3) [in particular during the contacting step of step b3)] and/or during step c3) of **variant 3,** at least one impurity **I** which is absorbed or adsorbed on the surface **S**, in particular contact surface **S_{C}**, is at least partially desorbed (and thus removed) from the surface **S** and dispersed or dissolved, in particular dispersed, in the mixture **M₃**. Optionally, the at least one impurity **I** is then degraded by at least one activity of at least one vegetative cell germinating from **SP** during step c3) of **variant 3.**

In other words, in this preferred embodiment, during step b3) [i.e. the contacting step of step b3)] and/or during step c3) of **variant 3,** the mixture **M₃** is brought in direct contact with at least a part **S_{C}** of the surface S, wherein at least one impurity **I** is absorbed or adsorbed on **S_{C},** so that the at least one impurity **I** is at least partially desorbed from the surface **S_{C}** and dispersed or dissolved, in particular dispersed, in the mixture **M₃** (and thus removed) from surface **S_{C}**. The dispersed or dissolved, in particular dispersed, impurity **I** is then optionally degraded in the mixture **M₃** by at least one activity of at least one vegetative cell germinating from **SP** during step c3) of **variant** 3.Therefore, in particular, after step c3) of **variant 3**, a mixture **M₃** is obtained wherein at least one impurity **I** in dissolved or dispersed, preferably dispersed.

The impurity **I** may either be in the form of a liquid (e.g. droplet) or a particle, and preferably is a particle.

In a preferred embodiment, the at least one impurity **I** is an impurity which is inorganic or organic, and is preferably organic.

Organic impurities are preferably selected from oils and fats, which may be of vegetable, animal or synthetic origin, food based particles, soil, pollen, sebum, body fluids, for example, blood, sperm, sweat, urine, feces, liquor.

Inorganic impurities are preferably selected from the group consisting of carbon black, fine dust, plastic particles (e.g. microplastic particles), metal oxides (preferably iron oxide), silica (such as sand), clay, dyes (such as pigments), more preferably clay, in particular kaolin.

*Bacillus velezensis* strain DSM 34978 and mutants of *Bacillus velezensis* strain DSM 34978, *Bacillus velezensis* DSM 34674, mutants of *Bacillus velezensis* DSM 34674 have an outstanding activity to produce at least one, preferably all, of protease activity, amylase activity, or cellulase activity. Where the method according to **variant 3** is used as a cleaning method, it is hence preferred that, when the spores **SP** of *Bacillus velezensis* strain DSM 34978, *Bacillus velezensis* DSM 34674, mutants of *Bacillus velezensis* DSM 34674 and/or mutants of *Bacillus velezensis* strain DSM 34978 are selected as spores **SP** in **variant 3,** that at least a part of the impurities **I** cleaned during **variant 3** are the substrates of these activities. Hence, it is preferred that in this preferred embodiment, the impurity **I** which is cleaned from the surface **S** during the cleaning method according to **variant 3** is selected from the group consisting of valeric acid, proteins (in particular proteins selected from casein and whey protein), polysaccharides (in particular starch), cellulose. More preferred, it comprises proteins (in particular proteins selected from casein and whey protein), cellulose. Most preferred, it comprises proteins, in particular proteins selected from casein and whey protein.

*Bacillus licheniformis* strain DSM 34977 and mutants of *Bacillus licheniformis* strain DSM 34977 have an outstanding activity to metabolize valeric acid. Where the method according to **variant 3** is used as a cleaning method, it is hence preferred that, when the spores **SP** of *Bacillus licheniformis* strain DSM 34977 and/or mutants of *Bacillus licheniformis* strain DSM 34977 are selected as spores **SP** in **variant 3,** that at least a part of the impurities **I** cleaned during **variant 3** are the substrates of this activity. Hence, it is preferred that in this preferred embodiment, the impurity **I** which is cleaned from the surface **S** during the cleaning method according to **variant 3** is valeric acid.

*Priestia megaterium* strain DSM 34980 and mutants of *Priestia megaterium* strain DSM 34980 have an outstanding activity to produce protease activity. Where the method according to **variant 3** is used as a cleaning method, it is hence preferred that, when the spores **SP** of *Priestia megaterium* strain DSM 34980 and/or mutants of *Priestia megaterium* strain DSM 34980 are selected as spores **SP** in the method according to **variant 3,** that at least a part of the impurities **I** cleaned during **variant 3** are the substrates of these activities. Hence, it is preferred that in this preferred embodiment, the impurity **I** which is cleaned from the surface **S** during the cleaning method according to **variant 3** is selected from the group consisting of valeric acid, proteins (in particular proteins selected from casein and whey protein), in particular proteins, which are even more preferably selected from casein and whey protein.

*Bacillus subtilis* strain DSM 34981 and mutants of *Bacillus subtilis* strain DSM 34981 have an outstanding activity to produce lipase activity. Where the method according to **variant 3** is used as a cleaning method, it is hence preferred that, when the spores **SP** of *Bacillus subtilis* strain DSM 34981 and/or mutants of *Bacillus subtilis* strain DSM 34981 are selected as spores **SP** in the method according to **variant 3**, that at least a part of the impurities **I** cleaned during **variant 3** are the substrates of these activities. Hence, it is preferred that in this preferred embodiment, the impurity **I** which is cleaned from the surface **S** during the cleaning method according to **variant 3** is selected from the group consisting of valeric acid, grease, oil, more preferably grease, oil.

In a further embodiment, where the spores **SP** in the method according to **variant 3** are selected from *Bacillus velezensis* strain DSM 34978 and mutants thereof, *Bacillus velezensis* DSM 34674 and mutants thereof, *Bacillus licheniformis* DSM strain DSM 34977 and mutants thereof, *Priestia megaterium* strain DSM 34980 and mutants thereof and *Bacillus subtilis* strain DSM 34981 and mutants thereof, it is preferred that the impurity **I** is selected from the group consisting of valeric acid, grease, oil, proteins (in particular proteins selected from casein and whey protein), polysaccharides (in particular starch), cellulose.

### I.6.3 Step c3)

In step c3) of **variant 3**, **M₃** is incubated to germinate at least a part of the spores **SP** comprised by mixture **M₃**.

The skilled person is aware of the conditions to be applied in step c3) that cause at least a part of the spores **SP** to germinate.

It is preferred that mixture **M₃** in steps b3) and c3) of **variant 3** is an aqueous mixture comprising the spores **SP** and the at least one biosurfactant **S_{Bio}**.

This is preferably achieved by simply adding water to the mixture **M₃** during step c3).

Alternatively or additionally, in step a3) the mixture **M*** may simply be provided as an aqueous mixture.

In addition, in step b3), the respective microorganism may also be contacted with nutrients and/or salts.

Preferred nutrients are selected from carbohydrates, amino acids, peptides, peptone, glucose, starch.

Peptides are preferably selected from peptone.

As amino acid, any of the natural amino acids may be selected by the skilled person. Preferably, the skilled person selects the amino acid from the group consisting of glutamine, alanine, valine, aspartate, glutamate, methionine, asparagine, preferably alanine, valine, asparagine.

Carbohydrates are preferably sugars, polysaccharides.

Preferred sugars are glucose, saccharose, fructose, galactose.

Preferred polysaccharides are starch, inulin.

Preferred salts are salts selected from sodium chloride, potassium phosphate, di-potassium hydrogen phosphate
Further additives known to the skilled person may also be applied to the mixture **M₁** in step c1), such as tris(hydroxymethyl)aminomethane **(**"**Tris**"**)**.

In addition, the germination of bacteria such as *Bacillus* or *Priestia* is also triggered by contacting the spores with substrates of one or more enzymatic or metabolic activity of these bacteria.

Hence, where spores **SP** of at least one of *Bacillus velezensis* strain DSM 34978, mutants of *Bacillus velezensis* strain DSM 34978, *Bacillus velezensis* DSM 34674, mutants of *Bacillus velezensis* DSM 34674, *Bacillus licheniformis* strain DSM 34977, mutants of *Bacillus licheniformis* strain DSM 34977, *Priestia megaterium* strain DSM 34980, mutants of *Priestia megaterium* strain DSM 34980, *Bacillus subtilis* strain DSM 34981 and mutants of *Bacillus subtilis* strain DSM 34981 are selected as spores **SP** in **variant 3**, it is further preferable that the mixture **M₃** in step c3) comprises the impurities **I** as set forth in the following:
*Bacillus velezensis* strain DSM 34978 and mutants of *Bacillus velezensis* strain DSM 34978, *Bacillus velezensis* DSM 34674, mutants of *Bacillus velezensis* DSM 34674: mixture **M₃** in step c3) preferably comprises at least one impurity **I** selected from the group consisting of valeric acid, proteins (in particular proteins selected from casein and whey protein), polysaccharides (in particular starch), cellulose; more preferred at least one impurity **I** selected from the group consisting of proteins (in particular proteins selected from casein and whey protein), cellulose; most preferred at least one impurity **I** selected from the group consisting of proteins, in particular proteins selected from casein and whey protein.

*Bacillus licheniformis* strain DSM 34977 and mutants of *Bacillus licheniformis* strain DSM 34977: mixture **M₃** in step c3) preferably comprises impurity **I** selected from valeric acid.

*Priestia megaterium* strain DSM 34980 and mutants of *Priestia megaterium* strain DSM 34980: mixture **M₃** in step c3) preferably comprises at least one impurity **I** selected from the group consisting of valeric acid, proteins (in particular proteins selected from casein and whey protein), preferably at least one impurity **I** selected from the group consisting of proteins which even more preferably are selected from casein and whey protein.

*Bacillus subtilis* strain DSM 34981 and mutants of *Bacillus subtilis* strain DSM 34981: mixture **M₃** in step c3) preferably comprises at least one impurity **I** selected from the group consisting of valeric acid, grease, oil, more preferably at least one impurity **I** selected from the group consisting of grease, oil.

The temperature of incubation in step c3) is in particular in the range of from 10 °C to 70 °C, in particular in the range of from 20 °C to 65 °C, preferably in the range of from 25 °C to 55 °C, more preferably in the range of from 30 °C to 45 °C.

Where the surface **S** is the surface **S_{O}** of an object **O**, in particular where **variant 3** is applied as a method for cleaning object **O**, it is preferred that the temperature in step c3) is in a range of from 10 °C to 70 °C, in particular in a range of from 20 °C to 65 °C, preferably in a range of from 25 °C to 55 °C, more preferably in a range of from 30 °C to 45 °C.

Where the surface **S** is the surface **S_{B}** of the human or animal body **B,** in particular where **variant 3** is applied as a method for cleaning the human or animal body **B**, it is preferred that the temperature in step c3) is in a range of from 10 °C to 50 °C, in particular in a range of from 20 °C to 45 °C, preferably in a range of from 25 °C to 40 °C, more preferably in a range of from 30 °C to 38 °C, most preferably 37 °C.

In a further preferred embodiment, the incubation in step c3) is carried out for at least 1 second ("second" = "s"), preferably for at least 10 s, preferably for at least 1 minute ("minute" = "min"), more preferably for at least 5 min, preferably for at least 15 min, preferably for at least 60 min, more preferably from 15 min to 3 d, more preferably from 30 min to 2 d, more preferably from 45 min to 1 d.

Preferably, in those cases where **M₃** comprises water, it is preferred that the pH of the water in **M₃** in step c3) at 25 °C is from 3.5 to 9, preferably from 5 to 9, preferably from 6 to 9, and particularly preferably from 7 to 8.

It is further preferred that the incubation in step c3) is carried out until at least 10 % of the spores **SP** comprised by mixture **M₃** that is contacted with **S** in step b3) have germinated, relative to the number of spores **SP** comprised by mixture **M₃** that is subjected to the incubation according to step c3) [i.e. relative to the number of spores **SP** comprised by mixture **M₃** that is obtained directly upon contacting **S** with **π_{SP}** in step b3]) have germinated.

This "germination rate" in step c3) is preferably determined according to **Assay M-III.**

It is even further preferred that the incubation in step c3) is carried out until at least 20 % of the spores **SP** comprised by mixture **M₃** that is in contact with **S** in step b3) have germinated, more preferably until at least 30 %, even more preferably until at least 40 %, even more preferably until at least 50 %, even more preferably until at least 60 %, even more preferably until at least 70 %, even more preferably until at least 80 %, even more preferably until at least 90 %, even more preferably until at least 95 %, even more preferably until at least 99 % of the spores **SP** comprised by mixture **M₃** that is in contact with **S** in step b3) have germinated, relative to the number of spores **SP** comprised by mixture **M₃** mixture **M₃** is subjected to the incubation according to step c3).

### I.6.4 Step d3) (optional)

It is preferred that, in a step d3), which is carried out after step c3), at least a part of **M₃** is separated from **S**. The separation is, in a preferred embodiment, carried out so that **M₃** is essentially complete separated from **S**.

This embodiment is particularly preferred where the method according to **variant 3** is applied as a cleaning method, in particular on a surface **S_{O}** of an object **O** or on a surface **S_{B}** of the human or animal body **B**.

The skilled person is aware of how to carry out this separation in each specific context. Generally, this separation is carried out by withdrawing the surface **S** from at least a part of **M₃**.

An object **O** may, in step d3), optionally be subjected to centrifugation (for example in a washing machine).

### I.6.5 Step e3) (optional)

In those optional embodiments of **variant 3** where step d3) is carried out, it is even more preferred to carry out another optional step e3) after step d3).

In an optional step e3) of **variant 3**, the surface **S** is rinsed with further water.

### I.7 Preparation P_{SP}

As stated above, in the context of the method according to the first aspect of the invention, spores **SP** may be provided in the form of a preparation **P_{SP}** of the respective bacteria, wherein **P_{SP}** comprises spores **SP**. This may be the case in step a1) of **variant 1.** Likewise, the spores **SP** comprised by mixture **M*** according to step a2) of **variant 2** and step a3) of **variant 3** may also be provided in the form of a preparation **P_{SP}**.

Likewise, as stated below, in the context of the use according to the second aspect of the invention, spores **SP** may be provided in the form of a preparation **P_{SP}** of the respective bacteria, wherein **P_{SP}** comprises spores **SP**. This may be the case in step a1*) of **variant 1*.** Likewise, the spores **SP** comprised by mixture **M*** according to step a2*) of **variant 2*** and step a3*) of **variant 3*** may also be provided in the form of a preparation **P_{SP}**.

The preparation **P_{SP}** of the respective bacteria, may, in addition to the spores **SP** (which are dormant), further comprise at least one of vegetative cells (which are growing), transition state cells (which are transitioning from growth to sporulation phase), in particular all of these types of cells. In a preferred embodiment, the preparation **P_{SP}** of the respective bacteria comprises the respective bacteria mainly or only as spores. "*Mainly as spores*" in particular means that at least 50 %, preferably at least 50 %, preferably at least 55 %, preferably at least 60 %, preferably at least 65 %, preferably at least 70 %, preferably at least 75 %, preferably at least 80 %, preferably at least 85 %, preferably at least 90 %, preferably at least 91 %, preferably at least 92 %, preferably at least 93 %, preferably at least 94 %, preferably at least 95 %, preferably at least 96 %, preferably at least 97 %, preferably at least 98 %, preferably at least 99 %, preferably at least 99.9 %, preferably at least 99.99 % of all cells of the respective bacteria comprised by the respective preparation **P_{SP}** are present as spores.

"Respective bacteria" in particular refers to at least one of *Bacillus, Priestia,* preferably to at least one of *Bacillus velezensis, Bacillus subtilis, Bacillus licheniformis, Priestia megaterium,* more preferably the strains of *Bacillus velezensis* DSM 34978 and mutants thereof, *Bacillus velezensis* DSM 34674 and mutants thereof, *Bacillus licheniformis* DSM 34977 and mutants thereof, *Priestia megaterium* DSM 34980 and mutants thereof, *Bacillus subtilis* DSM 34981 and mutants thereof.

The preparation **P_{SP}** of the respective bacteria, may, in addition to the spores **SP** contain intact cells, inactivated cells, cell debris or mixtures thereof.

It is preferred that the preparation of the respective bacterial comprises the genomic DNA of the respective bacteria.

It is preferred that the preparation of *Bacillus velezensis* comprises the genomic DNA of *Bacillus velezensis.*

It is preferred that the preparation of *Bacillus velezensis* DSM 34978 comprises the genomic DNA of *Bacillus velezensis* DSM 34978.

It is preferred that the preparation of a mutant of *Bacillus velezensis* DSM 34978 comprises the genomic DNA of the mutant of *Bacillus velezensis* DSM 34978.

It is preferred that the preparation of *Bacillus velezensis* DSM 34674 comprises the genomic DNA of *Bacillus velezensis* DSM 34674.

It is preferred that the preparation of a mutant of *Bacillus velezensis* DSM 34674 comprises the genomic DNA of the mutant of *Bacillus velezensis* DSM 34674.

It is preferred that the preparation of *Bacillus licheniformis* comprises the genomic DNA of *Bacillus licheniformis.*

It is preferred that the preparation of *Bacillus licheniformis* DSM 34977 comprises the genomic DNA of *Bacillus licheniformis* DSM 34977.

It is preferred that the preparation of a mutant of *Bacillus licheniformis* DSM 34977 comprises the genomic DNA of the mutant of *Bacillus licheniformis* DSM 34977.

It is preferred that the preparation of *Priestia megaterium* comprises the genomic DNA of *Priestia megaterium.*

It is preferred that the preparation of *Priestia megaterium* DSM 34980 comprises the genomic DNA of *Priestia megaterium* DSM 34980.

It is preferred that the preparation of a mutant of *Priestia megaterium* DSM 34980 comprises the genomic DNA of the mutant of *Priestia megaterium* DSM 34980.

It is preferred that the preparation of *Bacillus subtilis* comprises the genomic DNA of *Bacillus subtilis.*

It is preferred that the preparation of *Bacillus subtilis* DSM 34981 comprises the genomic DNA of *Bacillus subtilis* DSM 34981.

It is preferred that the preparation of a mutant of *Bacillus subtilis* DSM 34981 comprises the genomic DNA of the mutant of *Bacillus subtilis* DSM 34981.

Particularly preferred examples for preparations **P_{SP}** according to the invention are the fermentation broth, as obtained after finishing the fermentation of the cells, the supernatant of the fermentation broth, which is obtained by separating all or the major part of the cells from the fermentation broth, as well as cell lysates and cell extracts, i.e. cytosol preparations, which can be obtained by breaking the microbial cells. Such preparations **P_{SP}** may also be used in concentrated or dried form, wherein the dried form has preferably a total dry matter content of at least 90 wt.-%, more preferably of at least 95 wt.-%.

The term "fermentation broth" according to the invention refers to the product of a cultivation of the respective bacteria in a suitable fermentation medium. Methods for producing such fermentation broths are well known to those skilled in the art. The fermentation broths according to the present invention can for example be obtained by culturing the strains by using the media, conditions and methods as described in US 6,060,051 A, EP 0 287 699 A2, US 2014/0010792 A1. Conventional large-scale microbial culture processes include submerged fermentation, solid state fermentation, or liquid surface culture. Typically, fermentation is carried out, until a certain cell density is reached like an optical density (OD at λ = 600 nm) of about 1 to 5, more preferably 3 to 5, even more preferably 4 to 5. The fermentation broths preferably contain cells in an amount of 10³ to 10¹¹ **CFU**, more preferably in an amount of 10⁵ to 10¹⁰ **CFU**, above all in an amount of 10⁷ to 10⁹ **CFU** per ml of fermentation broth.

The term "fermentation broth" according to the invention refers to the direct product of the cultivation, i.e. a suspension preferably containing cells in the amount as mentioned in the previous paragraph, as well as to concentrated and dried forms of such a fermentation broth, wherein the dried form ("dried fermentation broth") preferably has a total dry matter content of at least 95 wt.-%.

Drying of the preparation **P_{SP}** can be carried out by applying methods as generally known to those skilled in the art, in particular by evaporation of water, freeze-drying, lyophilization, spray drying, spray granulation, fluidized bed drying, vacuum drying and combinations thereof. After drying, the dried preparations as obtained can be further worked up, in particular by grinding and/or granulation.

Preparations **P_{SP}** of the respective bacteria can be obtained by centrifugation, filtration and/or decantation of the fermentation broth and/or by centrifugation, filtration and/or decantation of the suspension as obtained after breaking the microbial cells. Depending on the technique used, these preparations **P_{SP}** may not be completely devoid of cells, but may still comprise a smaller amount of cells, in particular up to 20 wt.-% or up to 10 wt.-% of cells. As the cells produce and secret compounds like metabolites, enzymes and/or peptides into the surrounding medium, the supernatants, extracts and lysates of the cells comprises a mixture of such compounds, in particular metabolites, enzymes and/or peptides, as secreted and/or produced by the cells.

Cell lysates can be prepared either directly by breaking the cells of an optionally dried fermentation broth or by breaking the cells after first separating the cells from the fermentation broth mechanically, in particular by filtration, centrifugation and/or decantation.

Breaking of the cells can be carried out by applying techniques as known to those of skill in the art, for example by mechanical means or by applying high pressure. Depending on the degree of force applied, a composition comprising only ruptured cells or a composition comprising a mixture of cell debris and intact cells is obtained. Homogenization of the cells may be realized for example by utilizing means selected from French cell press, sonicator, homogenizer, microfluidizer, ball mill, rod mill, pebble mill, bead mill, high pressure grinding roll, vertical shaft impactor, industrial blender, high shear mixer, paddle mixer, and/or polytron homogenizer. Suitable alternatives are enzymatic and/or chemical treatment of the cells. After breaking of the cells, the cell debris and remaining cells, if any, can optionally be separated from the cytosol preparation thus obtained to obtain a cell extract, i.e. a cytosol preparation, which is free of cells and cell debris.

The microbial preparations of the invention contain metabolites, enzymes and/or peptides as produced and/or secreted by the microorganisms of the invention, namely bacteria, preferably selected from *Bacillus, Priestia,* more preferably selected from *Bacillus velezensis, Bacillus subtilis, Bacillus licheniformis, Priestia megaterium,* even more preferably selected from the strains of *Bacillus velezensis* DSM 34978 and mutants thereof, *Bacillus velezensis* DSM 34674 and mutants thereof, *Bacillus licheniformis* DSM 34977 and mutants thereof, *Priestia megaterium* DSM 34980 and mutants thereof, *Bacillus subtilis* DSM 34981 and mutants thereof.

The preparations of the invention preferably comprise an effective mixture of at least three, more preferably of at least 5, 8, 12 or 15, in particular of all metabolites of at least one microorganism of the invention. The metabolites possess preferably a molecular weight of between 400 and 100000 Dalton, more preferably of between 500 and 50000 Dalton.

A preparation containing an effective mixture of metabolites as contained in the microorganisms of the invention and/or as contained in the microbial preparations as mentioned before, can be obtained for example according to the methods set forth in US 6,060,051 A. The preparation can in particular be obtained by precipitating the metabolites as contained in the preparations mentioned before by using organic solvents like ethyl acetate and subsequent redissolving of the precipitated metabolites in an appropriate solvent. The metabolites may subsequently be purified by size exclusion filtration that groups metabolites into different fractions based on molecular weight cut-off.

The preparations of the invention can also be provided in combination with a suitable carrier, wherein the carrier is preferably an inert formulation ingredient added to improve recovery, efficacy, or physical properties and/or to aid in packaging or administration of the microorganisms or microbial preparations. Such carriers may be used individually or in combination and can be added either as part of the fermentation medium, in the course of the fermentation or after the fermentation of the microorganisms has been ended.

The carrier is preferably selected from anti-caking agents, antioxidants, bulking agents, binders, structurants, coatings and/or protectants. Examples of useful carriers include polysaccharides (in particular starches, maltodextrins, celluloses, methylcelluloses, gums like guar gum, xanthan gum and gum arabic, wheat middlings, corn cob meal, chitosan and/or inulins), protein sources (in particular skim milk powder, sweet-whey powder, gelatine and/or soy flour), protein hydrolysates (in particular gelatine, yeast extract and/or peptones like soy peptone), peptides, sugars (in particular lactose, trehalose, sucrose, dextrose and/or maltose), lipids (in particular lecithin, vegetable oils and/or mineral oils), salts (in particular sodium chloride, sodium carbonate, calcium carbonate, chalk, limestone, magnesium carbonate, sodium phosphate, calcium phosphate, magnesium phosphate and/or sodium citrate), silicates (in particular clays, zeolites, Fuller's earth, clintpolite, montmorillonite, perlite, vermiculite, diatomaceous earth, talc, bentonites, kaolin clay, silica in particular precipitated silica, hydrophobic silica and/or hydrophilic silica, and/or silicate salts like aluminium, magnesium and/or calcium silicate), silica gel, silica dioxide, activated carbon, lignite, magnesium and calcium oxide.

### I.8 Cleaning Formulation F

As described above, in step **a)** of the method according to the first aspect of the invention, at least one of **SP** and **S_{Bio}** may be provided, either separately (in **variant 1)** from each other or mixed (in **variant 2** and **variant 3)** with each other (as mixture **M***), as a cleaning formulation **F**. This is preferred where the method according to the first aspect of the invention is applied as a method of cleaning.

Likewise, as described below, in step **a***) of preferred embodiments of the use according to the second aspect of the invention, at least one **of SP** and **S_{Bio}** may be provided, either separately (in **variant 1*)** from each other or mixed (in **variant 2*** and **variant 3*)** with each other (as mixture **M***), as a cleaning formulation **F**. This is preferred where the use according to the second aspect of the invention is applied in a method of cleaning.

The cleaning formulation **F** is not further limited and may be in particular selected from a laundry detergent composition, a personal care product (such as an deodorant).

### I.8.1 Optional Ingredients of the Cleaning Formulation F

The cleaning formulation **F** according to the present invention preferably comprises at least one of the following further ingredients. The skilled person may choose from the optional ingredients as described in the following according to his knowledge and according to the intended use of the cleaning formulation **F**.

It goes without saying that the further, optional ingredients are different from the spores **SP** and the at least one biosurfactant **S_{Bio}**.

Hence, in a preferred embodiment, cleaning formulation **F** further comprises at least one of surfactants, enzymes, builders, solvents, preservatives, benefit agents, polymers, bleaching systems, antiredeposition aids, fibre protection agents, soil release agents, dye transfer inhibitors, fabric hueing agents, blueing dyes, enzyme stabilizing agents like boric acid, pH-regulators, emollients, emulsifiers, thickeners/ viscosity regulators /stabilizers, UV photoprotective filters, antioxidants, hydrotropes (or polyols), solids and fillers, film formers, pearlescent additives, deodorant and antiperspirant active ingredients, insect repellents, self-tanning agents, preservatives, conditioners, perfumes, dyes, odour absorbers, cosmetic active ingredients, care additives, superfatting agents, solvents, malodor removers.

### I.8.1.1 Surfactants

In a preferred embodiment, composition **F** comprises at least one surfactant which is not a glycolipid.

This at least one further surfactant may be selected from anionic surfactants, nonionic surfactants, cationic surfactants, amphoteric surfactants, and in particular is selected from anionic surfactants, nonionic surfactants.

### I.8.1.1.1 Nonionic surfactants

In case the cleaning formulation **F** comprises at least one nonionic surfactant, the nonionic surfactants preferably are alkoxylated, advantageously ethoxylated, in particular primary alcohols having preferably 8 to 18 carbon atoms and on average 1 to 12 mol of ethylene oxide **(**"**EO**"**)** per mol of alcohol, in which the alcohol radical can be linear or branched, preferably 2-position methyl-branched, or can contain linear and methyl-branched radicals in a mixture, as are customarily present in oxo alcohol radicals. In particular, however, alcohol ethoxylates with linear radicals from alcohols of native origin having 12 to 18 carbon atoms, for example from coconut, palm, tallow fat or oleyl alcohol, and on average 2 to 8 **EO** per mol of alcohol are preferred. The preferred ethoxylated alcohols include, for example, C₁₂-C₁₄-alcohols with 3 **EO**, 4 **EO** or 7 **EO**, C₉-C₁₁-alcohol with 7 **EO**, C₁₃-C₁₅-alcohols with 3 **EO**, 5 **EO**, 7 **EO** or 8 **EO**, C₁₂-C₁₈-alcohols with 3 **EO**, 5 **EO** or 7 **EO** and mixtures of these, such as mixtures of C₁₂-C₁₄-alcohol with 3 **EO** and C₁₂-C₁₈-alcohol with 7 **EO.** The stated degrees of ethoxylation are statistical average values which can be an integer or a fraction for a specific product. Preferred alcohol ethoxylates have a narrow homologue distribution.

In addition to these examples of nonionic surfactants, it is also possible to use fatty alcohols with more than 12 **EO**. Examples thereof are tallow fatty alcohol with 14 **EO**, 25 **EO**, 30 **EO** or 40 **EO**. Nonionic surfactants which contain **EO** and propylene oxide ("**PO**") groups together in the molecule can also be used. In this connection, it is possible to use block copolymers with **EO-PO** block units or **PO-EO** block units, but also **EO-PO-EO** copolymers or **PO-EO-PO** copolymers.

It is of course also possible to use mixed alkoxylated nonionic surfactants in which **EO** and **PO** units are not distributed blockwise, but randomly. Such products are obtainable as a result of the simultaneous action of ethylene oxide and propylene oxide on fatty alcohols.

Furthermore, alkyl glycosides can also be used as further nonionic surfactants.

A further class of preferably used nonionic surfactants, which are used either as the sole nonionic surfactant or in combination with other nonionic surfactants, are alkoxylated, preferably ethoxylated or ethoxylated and propoxylated fatty acid alkyl esters, preferably having 1 to 4 carbon atoms in the alkyl chain, in particular fatty acid methyl esters, as are described for example in JP S58-217598 A or which are preferably prepared by the process described in WO 90/13533 A1.

Nonionic surfactants of the amine oxide type, for example *N*-cocoalkyl-*N*,*N*-dimethylamine oxide and *N*-tallowalkyl-*N*,*N*-dihydroxyethylamine oxide, and of the fatty acid alkanolamide type may also be suitable. The amount of these nonionic surfactants is preferably not more than that of the ethoxylated fatty alcohols, in particular not more than half thereof.

Further suitable nonionic surfactants are polyhydroxy fatty acid amides; the polyhydroxy fatty acid amides are substances which can usually be obtained by reductive amination of a reducing sugar with ammonia, an alkylamine or an alkanolamine and subsequent acylation with a fatty acid, a fatty acid alkyl ester or a fatty acid chloride.

Further non-limiting examples of non-ionic surfactants include alcohol ethoxylates ("**AE**" or "**AEO**"), alcohol propoxylates, propoxylated fatty alcohols **(**"**PFA**"**)**, alkoxylated fatty acid alkyl esters, such as ethoxylated and/or propoxylated fatty acid alkyl esters, alkylphenol ethoxylates **(**"**APE**"**)**, nonylphenol ethoxylates **(**"**NPE**"**)**, alkylpolyglycosides **(**"**APG**"**)**, alkoxylated amines, fatty acid monoethanolamides **(**"**FAM**"**)**, fatty acid diethanolamides **(**"**FADA**"**)**, ethoxylated fatty acid monoethanolamides **(**"**EFAM**"**)**, polyglycerol esters, glaycerol esters, propoxylated fatty acid monoethanolamides **(**"**PFAM**"**)**, polyhydroxy alkyl fatty acid amides, or *N*-acyl *N*-alkyl derivatives of glucosamine (glucamides, "**GA**", or fatty acid glucamide, "**FAGA**"**),** as well as products available under the trade names "**SPAN**" and "**TWEEN**", and combinations thereof.

### I.8.1.1.2 Anionic surfactants

In case the cleaning formulation **F** comprises at least one anionic surfactant, it is preferred that these anionic surfactants are of the sulphonate and sulphate type.

Suitable surfactants of the sulphonate type here are preferably C₉-C₁₃-alkylbenzenesulphonates, olefinsulphonates, i.e. mixtures of alkene- and hydroxyalkanesulphonates, and also disulphonates, as are obtained, for example, from C₁₂-C₁₈-monoolefins with a terminal or internal double bond by sulphonation with gaseous sulphur trioxide and subsequent alkaline or acidic hydrolysis of the sulphonation products. Also of suitability are alkanesulphonates which are obtained from C₁₂-C₁₈-alkanes, for example by sulphochlorination or sulphoxidation with subsequent hydrolysis or neutralization. Similarly, the esters of α-sulpho fatty acids (ester sulphonates), for example the α-sulphonated methyl esters of hydrogenated coconut, palm kernel or tallow fatty acids, are also suitable.

Further suitable anionic surfactants are sulphated fatty acid glycerol esters. Fatty acid glycerol esters are to be understood as meaning the mono-, di- and triesters, and also mixtures thereof, as are obtained in the preparation by esterification of a monoglycerol with 1 to 3 mol of fatty acid or in the transesterification of triglycerides with 0.3 to 2 mol of glycerol. Preferred sulphated fatty acid glycerol esters here are the sulphation products of saturated fatty acids having 6 to 22 carbon atoms, for example of caproic acid, caprylic acid, capric acid, myristic acid, lauric acid, palmitic acid, stearic acid or behenic acid.

Preferred alkyl sulphates and alkenyl sulphates are the alkali metal and in particular the sodium salts of the sulphuric acid half-esters of the C₁₂-C₁₈-fatty alcohols, for example from coconut fatty alcohol, tallow fatty alcohol, lauryl, myristyl, cetyl or stearyl alcohol or the C₁₀-C₂₀-oxo alcohols and those half-esters of secondary alcohols of these chain lengths. Furthermore, preference is given to alkyl sulphates and alkenyl sulphates of the specified chain length which contain a synthetic straight-chain alkyl radical prepared on a petrochemical basis, and which have an analogous degradation behaviour to the suitable compounds based on fatty chemical raw materials. From the point of view of washing, the C₁₂-C₁₆-alkyl sulphates and C₁₂-C₁₈-alkyl sulphates and also C₁₄-C₁₈-alkyl sulphates are preferred. 2,3-Alkyl sulphates, which are prepared for example in accordance with the US 3,234,258 A or US 5,075,041 A and can be obtained as commercial products of the Shell Oil Company under the name DAN^{®}, are also suitable anionic surfactants.

The sulphuric acid monoesters of the straight-chain or branched C₇-C₂₀-alcohols ethoxylated with 1 to 6 mol of ethylene oxide ("**EO**"), such as 2-methyl-branched C₉-C₁₁-alcohols having on average 3.5 mol of ("**EO**") or C₁₂-C₁₈-fatty alcohols with 1 to 4 **EO**, are also suitable. On account of their high foaming behaviour, they are used in cleaning compositions only in relatively small amounts, for example in amounts of from 1 to 5% by weight.

Further suitable anionic surfactants are also the salts of alkylsulphosuccinic acid, which are also referred to as sulphosuccinates or as sulphosuccinic acid esters and constitute the monoesters and/or diesters of sulphosuccinic acid with alcohols, preferably fatty alcohols and in particular ethoxylated fatty alcohols. Preferred sulphosuccinates contain C₈-C₁₈-fatty alcohol radicals or mixtures of these. Particularly preferred sulphosuccinates contain a fatty alcohol radical which is derived from ethoxylated fatty alcohols. In this connection, sulphosuccinates whose fatty alcohol radicals are derived from ethoxylated fatty alcohols with a narrow homolog distribution are particularly preferred in turn. It is likewise also possible to use alkylsuccinic acid and alkenylsuccinic having preferably 8 to 18 carbon atoms in the alkyl chain/ alkenyl chain or salts thereof.

Particularly preferred anionic surfactants are soaps. Also of suitability are saturated and unsaturated fatty acid soaps, such as the salts of lauric acid, myristic acid, palmitic acid, stearic acid, (hydrogenated) erucic acid and behenic acid, and also soap mixtures derived in particular from natural fatty acids, for example coconut, palm kernel, olive oil or tallow fatty acid.

The anionic surfactants including the soaps can be in the form of their sodium, potassium or ammonium salts, as well as soluble salts of organic bases, such as mono-, di- or triethanolamine. Preferably, the anionic surfactants are in the form of their sodium or potassium salts, in particular in the form of the sodium salts.

Non-limiting examples of anionic surfactants include sulphates and sulphonates, in particular, linear alkylbenzenesulphonates ("**LAS**"), isomers of **LAS**, branched alkylbenzenesulphonates ("**BABS**"), phenylalkanesulphonates, α-olefinsulphonates ("**AOS**"), olefin sulphonates, alkene sulphonates, alkane-2,3-diylbis(sulphates), hydroxyalkanesulphonates and disulphonates, alkyl sulphates ("**AS**") such as sodium dodecyl sulphate ("**SDS**"), fatty alcohol sulphates ("**FAS**"), primary alcohol sulphates ("**PAS**"), alcohol ethersulphates ("**AES**" or "**AEOS**" or "**FES**", also known as alcohol ethoxysulphates or fatty alcohol ether sulphates) such as sodium dodecylpoly(oxyethylene) sulphate ("**SLES**"), secondary alkanesulphonates ("**SAS**"), paraffin sulphonates ("**PS**"), ester sulphonates, sulphonated fatty acid glycerol esters, α-sulpho fatty acid methyl esters ("**α-SFMe**" or "**SES**") including methyl ester sulphonate ("**MES**"), alkyl- or alkenylsuccinic acid, dodecenyl/tetradecenyl succinic acid ("**DTSA**"), fatty acid derivatives of amino acids, diesters and monoesters of sulfo-succinic acid or soap, and combinations thereof.

### I.8.1.1.3 Amphotheric surfactants

In case the cleaning formulation **F** comprises at least one amphotheric surfactant, it is preferred that these amphotheric surfactants are those surface-active compounds which carry at least one quaternary ammonium group and at least one -COO - or -SO_{3⁻} group in the molecule. Particularly preferred amphoteric surfactants in this connection are betaine surfactants such as alkyl- or alkylamidopropylbetaines. In particular, betaines such as the *N*-alkyl-*N*,*N*-dimethylammonium glycinates, e.g. the cocoalkyldimethylammonium glycinate, *N*-acylaminopropyl-*N*,*N*-dimethylammonium glycinates, e.g. the cocoacylaminopropyldimethylammonium glycinate, the C₁₂-C₁₈-alkyldimethylacetobetaine, the cocoamidopropyldimethylacetobetaine, 2-alkyl-3-carboxymethyl-3-hydroxyethylimidazolines and sulphobetaines having in each case 8 to 18 carbon atoms in the alkyl or acyl group, and also the cocoacylaminoethylhydroxyethylcarboxymethyl glycinate are preferred here. A particularly preferred zwitterionic surfactant is the *N*,*N*-dimethyl-*N*-(lauroylamidopropyl)ammoniumacetobetaine known under the INCI name Cocamidopropyl Betaine.

Further suitable amphoteric surfactants are formed by the group of amphoacetates and amphodiacetates, in particular, for example, coco- or laurylamphoacetates or -diacetates, the group of amphopropionates and amphodipropionates, and the group of amino acid-based surfactants such as acyl glutamates, in particular disodium cocoyl glutamate and sodium cocoyl glutamate, acyl glycinates, in particular cocoyl glycinates, and acyl sarcosinates, in particular ammonium lauroyl sarcosinate and sodium cocoyl sarcosinate. Non-limiting examples of amphoteric surfactants include betaine, alkyldimethylbetaine, sulfobetaine.

### I.8.1.1.4 Cationic surfactants

In case the cleaning formulation **F** comprises at least one cationic surfactant, Non-limiting examples of cationic surfactants include alklydimethylethanolamine quat ("**ADMEAQ**"), cetyltrimethylammonium bromide ("**CTAB**"), dimethyldistearylammonium chloride ("**DSDMAC**"), and alkylbenzyldimethylammonium, alkyl quaternary ammonium compounds, alkoxylated quaternary ammonium ("**AQA**") compounds, and combinations thereof.

### 1.8.1.2 Enzymes

In a preferred embodiment, the cleaning formulation **F** comprises at least one enzyme **EY**.

In those embodiments where the cleaning formulation **F** comprises at least one enzyme **EY**, it is further preferred that the enzyme **EY** is selected from the group consisting of protease, amylase, cellulase, mannanase, lipase, cutinase, pectate lyase, peroxidase, oxidase, laccase. It is even more preferred that the at least one enzyme **EY** is selected from the group consisting of protease, amylase, lipase, mannanase. Most preferred, the at least one enzyme **EY** is a lipase.

In case the cleaning formulation **F** comprises at least one enzyme **EY**, it is preferred that the amount of all enzymes **EY** comprised by the cleaning formulation **F** is in the range of 0.1 to 4 wt.-% per the total weight of cleaning formulation **F**.

The at least one enzyme **EY** may be included in the cleaning formulation **F** by adding separate additives ("detergent additives") containing one or more enzymes, or by adding a combined additive comprising all enzymes **EY** comprised by the cleaning formulation **F**. Such a detergent additive as contemplated herein, i.e., a separate additive or a combined additive, can be formulated, for example, as a granulate, liquid, slurry, etc. Preferred detergent additive formulations are granulates, in particular non-dusting granulates, liquids, in particular stabilized liquids, or slurries.

Non-dusting granulates may be produced, e.g., as disclosed in US 4,106,991 A and US 4,661,452 A and may optionally be coated by methods known in the art. Examples of waxy coating materials are poly(ethylene oxide) products (polyethyleneglycol, "**PEG**") with mean molar weights of from about 1000 to about 20000; ethoxylated nonylphenols having from 16 to 50 ethylene oxide units; ethoxylated fatty alcohols in which the alcohol contains from 12 to 20 carbon atoms and in which there are 15 to 80 ethylene oxide units; fatty alcohols; fatty acids; and mono- and di- and triglycerides of fatty acids. Examples of film-forming coating materials suitable for application by fluid bed techniques are given in GB 1,483,591 A. Liquid enzyme preparations may, for instance, be stabilized by adding a polyol such as propylene glycol, a sugar or sugar alcohol, lactic acid or boric acid according to established methods. Protected enzymes may be prepared according to the method disclosed in EP 0 238 216 A1.

### I.8.1.2.1 Cellulases

In those embodiments where the cleaning formulation **F** comprises at least one cellulase, suitable cellulases include those of bacterial or fungal origin. Chemically modified or protein engineered mutants are included. Suitable cellulases include cellulases from the genera *Bacillus, Pseudomonas, Humicola, Fusarium, Thielavia, Acremonium,* e.g., the fungal cellulases produced from *Humicola insolens, Myceliophthora thermophila* and *Fusarium oxysporum* disclosed in US 4,435,307 A, US 5,648,263 A, US 5,691,178 A, US 5,776,757 A and WO 89/09259 A1.

Especially suitable cellulases are the alkaline or neutral cellulases having color care benefits. Examples of such cellulases are cellulases described in EP 0 495 257 A1, WO 91/17243 A1, WO 96/11262 A1, WO 96/29397 A1, WO 98/08940 A1. Other examples are cellulase variants such as those described in WO 94/07998 A1, WO 91/17244 A1, US 5,457,046 A, US 5,686,593 A, US 5,763,254 A,

WO 95/24471 A1, WO 98/12307 A1 and WO 99/01544 A1.

Example of cellulases exhibiting endo-beta-1,4-glucanase activity (EC 3.2.1.4) are those described in WO 02/099091 A2.

Commercially available cellulases include Celluzyme^{™}, and Carezyme^{™} (Novozymes A/S), Clazinase^{™}, and Puradax HA^{™} (Genencor International Inc.), and KAC-500(B)^{™} (Kao Corporation).

### I.8.1.2.2 Proteases

In those embodiments where the cleaning formulation **F** comprises at least one protease, suitable proteases include those of bacterial, fungal, plant, viral or animal origin e.g. vegetable or microbial origin. Microbial origin is preferred. Chemically modified or protein engineered mutants are included. It may be an alkaline protease, such as a serine protease or a metalloprotease. A serine protease may for example be of the S1 family, such as trypsin, or the S8 family such as subtilisin. A metalloproteases protease may for example be a thermolysin from e.g. family M4 or other metalloprotease such as those from M5, M7 or M8 families.

The term "subtilases" refers to a sub-group of serine protease according to R.J. Siezen, W.M. de Vos, J.A.M. Leunissen, B.W. Dijkstra, Protein Engineering, Design and Selection 1991, 4, 719-737 and R.J. Siezen & J.A. Leunissen, Protein Science 1997, 6, 501-523. Serine proteases are a subgroup of proteases characterized by having a serine in the active site, which forms a covalent adduct with the substrate. The subtilases may be divided into 6 sub-divisions, i.e. the Subtilisin family, the Thermitase family, the Proteinase K family, the Lantibiotic peptidase family, the Kexin family and the Pyrolysin family. Examples of subtilases are those derived from *Bacillus* such as *Bacillus lentus, B. alkalophilus, B. subtilis, B. amyloliquefaciens, Bacillus pumilus* and *Bacillus gibsonii* described in: US 7,262,042 B2 and WO 2009/021867 A2, and subtilisin lentus, subtilisin Novo, subtilisin Carlsberg, *Bacillus licheniformis,* subtilisin BPN', subtilisin 309, subtilisin 147 and subtilisin 168 described in WO 89/06279 A1 and protease PD138 described in WO 93/18140 A1. Other useful proteases may be those described in WO 2019/105675 A1, WO 01/016285 A2, and WO 02/016547 A2. Examples of trypsin-like proteases are trypsin (e.g. of porcine or bovine origin) and the *Fusarium* protease described in WO 89/06270 A1, WO 94/25583 A1 and WO 2005/040372 A1, and the chymotrypsin proteases derived from *Cellumonas* described in WO 2005/052161 A2 and WO 2005/052146 A2.

A further preferred protease is the alkaline protease from *Bacillus lentus* DSM 5483, as described for example in WO 95/23221 A1, and variants thereof which are described in WO 92/21760 A2, WO 95/23221 A1, EP 1 921 147 A2 and EP 1 921 148 A2.

Examples of metalloproteases are the neutral metalloprotease as described in WO 2007/044993 A2 (Genencor Int.) such as those derived from *Bacillus amyloliquefaciens.*

Examples of useful proteases are the variants described in: WO 92/19729 A1, WO 96/34946 A1, WO 98/20115 A1, WO 98/20116 A1, WO 99/11768 A1, WO 01/44452 A1, WO 03/006602 A2, WO 2004/03186 A2, WO 2004/041979 A2, WO 2007/006305 A1, WO 2011/036263 A1, WO 2011/036264A1.

Suitable commercially available protease enzymes include those sold under the trade names Alcalase^{®}, Duralase^{™}, Durazym^{™}, Relase^{®}, Relase^{®} Ultra, Savinase^{®}, Savinase^{®} Ultra, Primase, Polarzyme^{®}, Kannase^{®}, Liquanase^{®}, Liquanase^{®} Ultra, Liquanase^{®} 2.5 L, Ovozyme^{®}, Coronase, Coronase^{®} Ultra, Neutrase^{®}, Everlase^{®} and Esperase^{®} (Novozymes A/S), those sold under the tradename Maxatase^{®}, Maxacal^{®}, Maxapem^{®}, Purafect^{®}, Purafect Prime^{®}, Eraser^{®}, Purafect MA^{®}, Purafect Ox^{®}, Purafect OxP^{®}, Puramax^{®}, Properase^{®}, Ultimase^{®}, FN2^{®}, FN3^{®}, FN4^{®}, Excellase^{®}, Opticlean^{®} and Optimase^{®} (Danisco/DuPont), Axapem^{™} (Gist-Brocases N.V.), BLAP (sequence shown in Figure 29 of US 5,352,604 A) and variants hereof (Henkel AG) and KAP (*Bacillus alkalophilus* subtilisin) from Kao.

A protease preferably comprised in the composition according to the instant invention is Liquanase^{®} 2.5 L.

In a preferred embodiments, the proteases comprised by cleaning formulation **F** are stabilized by biosurfactants **S_{Bio}** such as rhamnolipids, as described in US 2018/023040 A1.

### I.8.1.2.3 Lipases and cutinases

In those embodiments where cleaning formulation **F** comprises at least one of lipase, cutinase, suitable lipases and cutinases include those of bacterial or fungal origin. Chemically modified or protein engineered mutant enzymes are included. Examples include lipase from *Thermomyces,* e.g. from *T. lanuginosus* (previously named *Humicola lanuginosa*) as described in EP 0 258 068 A2 and EP 0 305 216 A1, cutinase from *Humicola,* e.g. *H. insolens* (WO 96/13580 A1), lipase from strains of *Pseudomonas* (some of these now renamed to *Burkholderia),* e.g. *P. alcaligenes* or *P. pseudoalcaligenes* (EP 0 218 272 A1), *P. cepacia* (EP 0 331 376 A2), *P. sp.* strain SD705 (US 5,827,718 A & US 5,942,431 A), *P. wisconsinensis* (WO 96/12012 A1), GDSL-type *Streptomyces* lipases (WO 2010/065455 A2), cutinase from *Magnaporthe grisea* (WO 2010/107560 A2), cutinase from *Pseudomonas mendocina* (US 5,389,536 A), lipase from *Thermobifida fusca* (WO 2011/084412 A1), *Geobacillus stearothermophilus* lipase (WO 2011/084417 A1), lipase from *Bacillus subtilis* (WO 2011/084599 A1), and lipase from *Streptomyces griseus* (WO 2011/150157 A2) and *S*. *pristinaespiralis* (WO 2012/137147 A1).

Other examples are lipase variants such as those described in EP 0 407 225 A1, WO 92/05249 A1, WO 94/01541 A1, WO 94/25578 A1, WO 95/14783 A1, WO 95/30744 A2, WO 95/35381 A1, WO 95/22615 A1, WO 96/00292 A1, WO 97/04079 A1, WO 97/07202 A1, WO 00/34450 A1, WO 00/60063 A1, WO 01/92502 A1, WO 2007/87508 A2, CN 104031899 A and WO 2009/109500 A1. Preferred commercial lipase products include Lipolase^{™}, Lipex^{™}; Lipolex^{™}, Lipex^{™} 100 L Evity and Lipoclean^{™} (Novozymes A/S), Lumafast (originally from Genencor) and Lipomax (originally from Gist-Brocades).

Still other examples are lipases sometimes referred to as acyltransferases or perhydrolases, e.g. acyltransferases with homology to *Candida antarctica* lipase A (WO 2010/111143 A2), acyltransferase from *Mycobacterium smegmatis* (WO 2005/56782 A2), perhydrolases from the CE 7 family (WO 2009/67279 A1), and variants of the *Mycobacterium smegmatis* perhydrolase in particular the S54V variant used in the commercial product Gentle Power Bleach from Huntsman Textile Effects Pte Ltd. (WO 2010/100028 A2).

### I.8.1.2.4 Amylases

In those embodiments where cleaning formulation **F** comprises at least one amylase, suitable amylases which can be used herein may be an α-amylase or a glucoamylase and may be of bacterial or fungal origin. Chemically modified or protein engineered mutants are included. Amylases include, for example, α-amylases obtained from *Bacillus,* e.g., a special strain of *Bacillus licheniformis,* described in more detail in GB 1,296,839 A.

Suitable amylases include amylases having SEQ ID NO: 3 in WO 95/10603 A1 or variants having about 90 % sequence identity to SEQ ID NO: 3 thereof. Preferred variants are described in WO 94/02597 A1, WO 94/18314 A1, WO 97/43424 A1 and SEQ ID NO: 4 of WO 99/19467 A1.

Different suitable amylases include amylases having SEQ ID NO: 6 in WO 02/10355 A2 or variants thereof having about 90 % sequence identity thereto.

Other amylases which are suitable are hybrid α-amylase comprising residues 1-33 of the α-amylase derived from B. *amyloliquefaciens* shown in SEQ ID NO: 6 of WO 2006/066594 A2 and residues 36-483 of the *B*. *licheniformis* α -amylase shown in SEQ ID NO: 4 of WO 2006/066594 A2 or variants having about 90 % sequence identity thereof.

Further amylases which are suitable are amylases having SEQ ID NO: 6 in WO 99/019467 A1 or variants thereof having about 90 % sequence identity to SEQ ID NO: 6.

Additional amylases which can be used are those having SEQ ID NO: 1, SEQ ID NO: 3, SEQ ID NO: 2 or SEQ ID NO: 7 of WO 96/23873 A1 or variants thereof having 90 % sequence identity to SEQ ID NO: 1, SEQ ID NO: 2, SEQ ID NO: 3 or SEQ ID NO: 7.

Other amylases which can be used are amylases having SEQ ID NO: 2 of WO 2008/153815 A1, SEQ ID NO: 10 in WO 01/66712 A2 or variants thereof having about 90 % sequence identity to SEQ ID NO: 2 of WO 2008/153815 A1 or about 90 % sequence identity to SEQ ID NO: 10 in WO 01/66712 A2.

Further suitable amylases are amylases having SEQ ID NO: 2 of WO 2009/061380 A2 or variants having about 90 % sequence identity to SEQ ID NO: 2 thereof.

Other suitable amylases are the α-amylase having SEQ ID NO: 12 in WO 01/66712 A2 or a variant having at least about 90 % sequence identity to SEQ ID NO: 12.

Other examples are amylase variants such as those described in WO 2011/098531 A1,

WO 2013/001078 A1 and WO 2013/001087 A2.

Commercially available amylases are Amplify^{™} Prime 100 L, Duramyl^{™}, Termamyl^{™}, Fungamyl^{™}, Stainzyme^{™}, Stainzyme Plus^{™}, Natalase^{™}, Liquozyme X and BAN^{™} (from Novozymes A/S), and Rapidase^{™}, Purastar^{™}/Effectenz^{™}, Powerase and Preferenz S100 (from Genencor International Inc./DuPont).

### 1.8.1.2.5 Peroxidases/ oxidases

In those embodiments where cleaning formulation **F** comprises at least one peroxidase or oxidase, suitable peroxidases/oxidases include those of plant, bacterial or fungal origin. Chemically modified or protein engineered mutants are included. Examples of useful peroxidases include peroxidases from *Coprinus,* e.g., from *C*. *cinereus,* and variants thereof as those described in WO 93/24618 A1, WO 95/10602 A1, and WO 98/15257 A1. Commercially available peroxidases include Guardzyme^{™} (Novozymes A/S).

### 1.8.1.2.6 Mannanases

In those embodiments where cleaning formulation **F** comprises at least one mannanase, mannanases which are particularly preferred according to the invention are mannanases which are sold, for example, under the trade names Mannaway^{®} by the company Novozymes or Purabrite^{®} by the company Genencor. A mannanase preferably comprised in the cleaning formulation **F** is Mannaway^{®} 4.0 L.

### 1.8.1.3 Builders

Cleaning formulation **F** may also comprise at least one builder **B_{Z}**. This is especially useful in those cases where cleaning formulation **F** is applied as laundry composition.

In those embodiments where cleaning formulation **F** comprises at least one builder **B_{Z}**, the amount of all builders **B_{Z}** in cleaning formulation **F** is preferably between 0.1 wt.-% to 10 wt.-%, preferably 1 wt.-% to 7 wt.-%, relative to the total weight of cleaning formulation **F.**

The builder **B_{Z}** may particularly be a chelating agent that forms water-soluble complexes with calcium and magnesium. Non-limiting examples of builders **B_{Z}** include zeolites, diphosphates (pyrophosphates), triphosphates such as sodium triphosphate ("**STP**" or "**STPP**"), carbonates such as sodium carbonate, soluble silicates such as sodium metasilicate, layered silicates (e.g., SKS-6 from Hoechst), ethanolamines such as 2-aminoethan-1-ol ("**MEA**"), diethanolamine ("**DEA**", also known as iminodiethanol), triethanolamine ("**TEA**", also known as 2,2',2"-nitrilotriethanol), and carboxymethyl inulin ("**CMI**"), and combinations thereof.

Non-limiting examples of builders **B_{Z}** include homopolymers of polyacrylates or copolymers thereof, such as poly(acrylic acid) ("**PAA**") or copoly(acrylic acid/maleic acid) ("**PAA/PMA**"). Further non-limiting examples of builders **B_{Z}** include polyaspartic acids and polyglutamic acids and their salts, citrates, ascorbic acid, chelators such as aminocarboxylates, aminopolycarboxylates, like *N*,*N*-dicarboxymethyl glutamic acid and methylglycine *N*,*N*-diacetic acid, and phosphonates, and alkyl- or alkenylsuccinic acid. Additional specific examples of builders **B_{Z}** include 2,2',2"-nitrilotriacetic acid ("**NTA**"), ethylenediaminetetraacetic acid ("**EDTA**"), diethylenetriaminepentaacetic acid ("**DTPA**"), iminodisuccinic acid ("**IDS**"), ethylenediamine-*N*,*N*'-disuccinic acid ("**EDDS**"), glutamic acid-*N*,*N*-diacetic acid ("**GLDA**"), 1-hydroxyethane-1,1-diphosphonic acid ("**HEDP**"), ethylenediaminetetra-(methylenephosphonic acid) ("**EDTMPA**"), diethylenetriaminepentakis(methylenephosphonic acid) ("**DTPMPA**" or "**DTMPA**"), *N*-(2-hydroxyethyl)iminodiacetic acid ("**EDG**"), aspartic acid-*N*-monoacetic acid ("**ASMA**"), aspartic acid-*N*,*N*-diacetic acid ("**ASDA**"), aspartic acid-*N*-monopropionic acid ("**ASMP**"), iminodisuccinic acid ("**IDA**"), *N*-(2-sulfomethyl)-aspartic acid ("**SMAS**"), *N*-(2-sulfoethyl)-aspartic acid ("**SEAS**"), *N*-(2-sulfomethyl)-glutamic acid ("**SMGL**"), *N*-(2-sulfoethyl)-glutamic acid ("**SEGL**"), *N*-methyliminodiacetic acid ("**MIDA**"), α-alanine-*N,N*-diacetic acid ("**α-ALDA**"), serine-*N*,*N*-diacetic acid ("**SEDA**"), isoserine-*N*,*N*-diacetic acid ("**ISDA**"), phenylalanine-*N*,*N*-diacetic acid ("**PHDA**"), anthranilic acid-*N*,*N*-diacetic acid ("**ANDA**"), sulfanilic acid-*N*,*N*-diacetic acid ("**SLDA**"), taurine-*N*,*N*-diacetic acid ("**TUDA**") and sulfomethyl-*N*,*N*-diacetic acid ("**SMDA**"), *N*-(2-hydroxyethyl)-ethylidenediamine-N,N',N'-triacetate ("**HEDTA**"), diethanolglycine ("**DEG**"), diethylenetriamine penta(methylenephosphonic acid) ("**DTPMP**"), aminotris(methylenephosphonic acid) ("**ATMP**"), and combinations and salts thereof.

Preferred builder **B_{Z}** comprised in the cleaning formulation **F** according to the instant invention are selected from the group of aminopolycarboxylates, like *N*,*N*-dicarboxymethyl glutamic acid and methylglycine *N*,*N*-diacetic acid, citrates, polyaspartic acids and polyglutamic acids and their salts.

### I.8.1.4 Solvents

In a preferred embodiment, cleaning formulation **F** may comprise at least one solvent selected from water or non-aqueous solvent. It is preferred that cleaning formulation **F** comprises water, and that it is an aqueous mixture. Such aqueous mixture optionally contains at least one non-aqueous solvent.

In those embodiments where cleaning formulation **F** is an aqueous mixture, it is preferred that the content of water in cleaning formulation **F** is from 1 wt.-% to 99 wt.-%, more preferably from 10 wt.-% to 99 wt.-%, more preferably from 30 wt.-% to 98 wt.-%, more preferably from 50 wt.-% to 97 wt.-%, more preferably from 60 wt.-% to 96 wt.-%, more preferably from 70 wt.-% to 95 wt.-%, more preferably from 80 wt.-% to 90 wt.-%, wherein the weight-% give the percentage of water relative to the total weight of the mixture.

Where cleaning formulation **F** comprises at least one non-aqueous solvent (preferably in addition to water), suitable non-aqueous solvents include monohydric or polyhydric alcohols, alkanolamines or glycol ethers, provided they are miscible with water in the specified concentration range. The solvents are preferably selected from ethanol, *n*-propanol, *iso*-propanol, butanols, glycol, propanediol, butanediol, glycerine, diglycol, propyldiglycol, butyldiglycol, hexylene glycol, ethylene glycol methyl ether, ethylene glycol ethyl ether, ethylene glycol propyl ether, *n*-butyl glycol ether, ethylene glycol mono-glycol ether, diethylene glycol ethyl ether, propylene glycol methyl ether, propylene glycol ethyl ether, propylene glycol propyl ether, dipropylene glycol monomethyl ether, dipropylene glycol monoethyl ether, diisopropylene glycol monomethyl ether, diisopropylene glycol monoethyl ether, methoxy triglycol, ethoxy triglycol, butoxy triglycol, 1-butoxyethoxy-2-propanol, *n*-butoxyethoxy-2-propanol, di-butoxyethoxy-2-propanol, 3-butyl-3-methoxyether solvents, and mixtures of these solvents, butoxyethoxy-2-propanol, 3-butyl-3-butyl ether, propyl glycol, di-octanol ether, di-butoxy-2-propanol, 3-butyl-3-butylether, propanol, propylenglycol, di-butoxyethoxy-2- propanol, di-butoxyethoxy-2-propanol, 3-butoxy-3-methoxyether solvents, diisopropylene glycol monomethylether, diisopropylene glycol monomethylether, and mixtures of these solvents. Cleaning formulation **F** preferably contains a polyol as the non-aqueous solvent. The polyol can comprise glycerol, 1,2-propanediol, 1,3-propanediol, ethylene glycol, diethylene glycol and / or dipropylene glycol.

In those embodiments where cleaning formulation **F** comprises at least one non-aqueous solvent, the amount of all non-aqueous solvent in cleaning formulation **F** is preferably between 0.5 wt.-% to 15 wt.-%, preferably 1 wt.-% to 10 wt.-%, relative to the total weight of cleaning formulation **F.**

### I.8.1.5 Preservatives

In a preferred embodiment, cleaning formulation **F** may comprise at least one preservative. Exemplary preservatives include ascorbic acid, phenoxyethanol, sodium levulinate, sodium benzoate, *p*-anisic acid, potassium sorbate, benzoic acid, glyceryl caprylate, capryl glycol, pentylene glycol, methyl propane diol, bronopol, isothiazolinone (methylisothiazolinone, chloromethylisothiazolinone).

In those embodiments where cleaning formulation **F** comprises at least one preservative, the amount of all preservatives in cleaning formulation **F** is preferably between 0.001 wt.-% to 2 wt.-%, preferably 0.01 wt.-% to 0.1 wt.-%, relative to the total weight of cleaning formulation **F.**

### I.8.1.6 Benefit agent

In a preferred embodiment, cleaning formulation **F** comprises at least one benefit agent. A benefit agent is preferably provided in an encapsulate. The cleaning formulation **F** may also comprise an unconfined (also called non-encapsulated) benefit agent, for example a volatile benefit agent. Where the volatile benefit agent is a perfume, the perfumes are suitable for use as the encapsulated volatile benefit agent and also as the unconfined perfume component.

Preferred encapsulates in this context comprise shear/pressure-sensitive action encapsulates, whereby the sensorial benefit agent is released in response to mechanical force (e.g. friction, pressure, shear stress) on the encapsulate. The encapsulate shell is preferably comprised of materials including but not limited to polyurethane, polyamide, polyolefin, polysaccharide, protein, silicone, lipid, modified cellulose, gums, polyacrylate, polyphosphate, polystyrene, polyesters or combinations of these materials.

Preferably, the benefit agent is selected from the group consisting of a sensorial benefit agent, a skin benefit agent, an olfactory benefit agent, more preferably a sensorial benefit agent. The most preferred benefit agent is a perfume. The benefit agent may be a volatile benefit agent.

Sensorial benefit agents may also have benefits for hair and/or hard surfaces and/or fabrics. The sensorial benefit may have anti-foam properties, and as such it is advantageous for foaming purposes that it is encapsulated so as not to inter with the foam until release by rubbing. Suitable volatile benefit agents include but are not limited to perfumes, insect repellents, essential oils, sensates such as menthol and aromatherapy actives, preferably perfumes. Mixtures of volatile benefit agents may be used.

In those embodiments where cleaning formulation **F** comprises at least one benefit agent, the total amount of benefit agent is preferably from 0.01 wt.-% to 10 wt.-%, more preferably from 0.05 wt.-% to 5 wt.-%, even more preferably from 0.1 wt.-% to 4.0 wt.-%, most preferably from 0.15 wt.-% to 4.0 wt.-%, based on the total weight of the cleaning formulation **F.**

### I.8.1.7 Polymers

In a preferred embodiment, cleaning formulation **F** comprises at least one polymer for use in detergents that are different from the polymers described before. The polymer may function as a co-builder as mentioned above, or may provide a further anti-redeposition effect or one function selected from fiber protection, soil release, dye transfer inhibition, viscosity modifiers, grease cleaning and/or anti-foaming properties. Exemplary polymers include (carboxymethyl)cellulose ("**CMC**"), poly(vinyl alcohol) ("**PVA**"), poly(vinylpyrrolidone) ("**PVP**"), poly(ethyleneglycol) or poly(ethylene oxide) ("**PEG**"), ethoxylated poly(ethyleneimine), carboxymethyl inulin ("**CMI**"), and polycarboxylates such as **PAA, PAA/PMA,** polyaspartic acid, and lauryl methacrylate/acrylic acid copolymers, hydrophobically modified **CMC** ("**HM-CMC**") and silicones, copolymers of terephthalic acid and oligomeric glycols, copolymers of polyethylene terephthalate) and poly(oxyethene terephthalate) ("**PET-POET**"), **PVP,** poly(vinylimidazole) ("**PVI**"), poly(vinylpyridine-*N*-oxide) ("**PVPO**" or "**PVPNO**") and polyvinylpyrrolidone-vinylimidazole ("**PVPVI**"). Further exemplary polymers include sulfonated polycarboxylates, polyethylene oxide and polypropylene oxide ("**PEO-PPO**") and diquaternium ethoxy sulfate. Other exemplary polymers are disclosed in, e.g., WO 2006/130575 A2. Salts of the above-mentioned polymers are also contemplated.

Preferably a cleaning formulation **F** according to the instant invention is characterized in that it comprises at least one selected from anti-redeposition polymers and soil release polymers, with soil release polymers being preferred. This has the technical effect, that the cleaning capabilities of the cleaning formulation **F** according to the instant invention is even more enhanced. It is preferred in the context of the instant invention, that the anti-redeposition polymer or soil release polymer is selected from the group consisting of modified cellulose, preferably carboxymethylcellulose, cellulose acetate and methylcellulose, modified starch, modified inulin, preferably carboxy methyl inulin, polyitaconic acid, polyvinylpyrrolidone, polyvinyl alcohol, and polyethylene glycol, with carboxymethylcellulose and methylcellulose being most preferred.

Further preferred soil release polymers are water soluble polyesters as for example from the TexCare^{®} range commercially available under the name TexCare SRN 260, TexCare SRN 170, TexCare SRN 260 Life and combinations thereof, as well as the soil release polymers disclosed in WO 2016/075178 A1, WO 2016/075179 A1, EP 3 489 340 A1 and EP 3 489 338 A1. Further preferred soil release polymers are selected from carboxy methyl inulins. A commercial example is Carboxyline^{®}CMI.

EP 1 746 109 A2 discloses hybrid polymers of amylose and acrylates, that can also advantageously be used in the cleaning formulation **F** of the instant invention as soil release polymers. A commercial example for this type of soil release polymers is Alcoguard^{®} H 5941.

Non-limiting examples of biopolymers include: starch, like e.g. corn starch, *Zea mays* starch and tapioca starch, modified starch, like e.g. starch hydroxypropyltrimonium chloride and hydrolyzed corn starch, cellulose, bacterial cellulose, modified cellulose, like e.g. microcrystalline cellulose, hydroxypropyl methylcellulose and cetyl hydroxyethylcellulose, guar gum, pectin, inulin, carrageenan, alginate, galactoarabinan, polycitronellol, carboxymethyl inulin, carboxymethyl cellulose, polyitaconic acid and combinations and salts thereof.

In those embodiments where cleaning formulation **F** comprises at least one polymer, the amount of all polymers in cleaning formulation **F** is preferably from 0.05 wt.-% to 8 wt.-%, preferably from 0.1 wt.-% to 5 wt.-%, relative to the total weight of cleaning formulation **F.**

### I.8.1.8 Other Additives

In a preferred embodiment, cleaning formulation **F** comprises at least one further additives selected from the group consisting of bleaching systems, anti-redeposition aids, fibre protection agents, soil release agents, dye transfer inhibitors, fabric hueing agents, blueing dyes, enzyme stabilizing agents like boric acid, pH-regulators, emollients, emulsifiers, thickeners/viscosity regulators /stabilizers,

UV photoprotective filters, antioxidants, hydrotropes (or polyols), solids and fillers, film formers, pearlescent additives, deodorant and antiperspirant active ingredients, insect repellents, self-tanning agents, preservatives, conditioners, perfumes, dyes, odour absorbers, cosmetic active ingredients, care additives, superfatting agents, solvents, malodor removers.

Substances which can be used as exemplary representatives of the individual groups are known to the person skilled in the art and can be found for example in US 2011/0091399 A1.

### I.8.2 Form of the cleaning formulation F

The form of the cleaning formulation **F** depends on the area of use.

In particular in those cases, where the cleaning formulation **F** is used for cleaning an object **O,** in particular in cases where the object **O** is a woven or non-woven article **A** or where article **A** is crockery (e.g. dishes, cups) or cutlery, the cleaning formulation **F** may be in any convenient form, e.g., a bar, a homogenous tablet, a tablet having two or more layers, a pouch having one or more compartments, a regular or compact powder, a granule, a paste, a gel, or a regular, compact or concentrated liquid. There are a number of detergent formulation forms such as layers (same or different phases), pouches, as well as forms for machine dosing unit.

Pouches can be configured as single or multi-compartments. They can be of any form, shape and material which is suitable for holding the cleaning formulation **F,** e.g. without allowing the release of the composition from the pouch prior to water contact. The pouch is made from water soluble film which encloses an inner volume. Said inner volume can be divided into compartments of the pouch. Preferred films are polymeric materials, preferably polymers which are formed into a film or sheet. Preferred polymers, copolymers or derivates thereof are selected polyacrylates, and water soluble acrylate copolymers, methyl cellulose, carboxy methyl cellulose, sodium dextrin, ethyl cellulose, hydroxyethyl cellulose, hydroxypropyl methyl cellulose, maltodextrin, poly methacrylates, most preferably polyvinyl alcohol copolymers and, hydroxypropyl methyl cellulose ("**HPMC**"). Preferably the level of polymer in the film, for example **PVA,** is at least about 60 wt.-%. The preferred average molecular weight of the polymer will typically be from about 20,000 g/mol to about 150,000 g/mol. Films can also be of blend compositions comprising hydrolytically degradable and water soluble polymer blends such as polyactide and polyvinyl alcohol (known under the Trade reference M8630 as sold by Chris Craft In. Prod. Of Gary, Ind., US) plus plasticizers like glycerol, ethylene glycerol, propylene glycol, sorbitol and mixtures thereof. The pouches can comprise a solid laundry detergent composition or part components and/or a liquid cleaning composition or part components separated by the water-soluble film. The compartment for liquid components can be different in composition than compartments containing solids, see for example US 2009/0011970 A1.

Detergent ingredients can be separated physically from each other by compartments in water dissolvable pouches or in different layers of tablets. Thereby negative storage interaction between components can be avoided. Different dissolution profiles of each of the compartments can also give rise to delayed dissolution of selected components in the wash solution.

In those cases where the cleaning formulation **F** is a deodorant or detergent composition for body, i.e. in which the cleaning formulation **F** is used to deodorize or clean the human or animal body **B,** according to the instant invention, the cleaning formulation **F** can be in form of a laundry soap bar and used for hand washing laundry, fabrics and/or textiles. The term laundry soap bar includes laundry bars, soap bars, combo bars, syndet bars and detergent bars. The types of bar usually differ in the type of surfactant they contain, and the term laundry soap bar includes those containing soaps from fatty acids and/or synthetic soaps. The laundry soap bar has a physical form which is solid and not a liquid, gel or a powder at room temperature. The term solid is defined as a physical form which does not significantly change over time, i.e. if a solid object (e.g. laundry soap bar) is placed inside a container, the solid object does not change to fill the container it is placed in. The bar is a solid typically in bar form but can be in other solid shapes such as round or oval.

Cleaning formulation **F,** in particular in those embodiments where it is a detergent composition for household care, can be formulated as a granular detergent as described in WO 2009/092699 A1,

EP 1 705 241 A1, EP 1 382 668 A1, WO 2007/001262 A1, US 6,472,364 B1, WO 2004/074419 A2 or WO 2009/102854 A1.

Nevertheless, in those cases where the cleaning formulation **F** is a detergent composition for body, i.e. in which the cleaning formulation **F** is used for cleaning the human or animal body **B,** according to the instant invention, the cleaning formulation **F** preferably is in the form of a liquid or gel detergent. This may be aqueous, typically containing at least 20 wt.-% water, with the percentages referring to the total cleaning formulation **F.** Other types of liquids, including without limitation, alkanols, amines, diols, ethers and polyols may be included in an aqueous liquid or gel. Where cleaning formulation **F** is an aqueous liquid or gel detergent, it preferably contains from 0 wt-% to 30 wt.-% organic solvent, with the percentages referring to the total weight of cleaning formulation **F.** While a liquid or gel detergent may be non-aqueous, it is preferred that such liquid or gel detergent comprises water.

Where the cleaning formulation **F** is a detergent composition for body, it is characterized in that the pH of such composition at 25 °C is from 3.0 to 9.0, preferably from 4.0 to 7.0 and particularly preferably from 5.0 to 6.6.

Where the cleaning formulation **F** is a deodorant, it may also comprise typical ingredients of deodorants as described in EP 1 051 966 A2 or WO 2013/092186 A2.

Optionally, the cleaning formulation **F** applied in step b. of the method according to the invention is diluted. In those embodiments where the cleaning formulation **F** is diluted, it is preferred that such dilution is carried out with water. The optional dilution may take place before or while **F** is in contact with **S.** Preferably, the optional dilution takes place while **F** is in contact with **S.**

The optional dilution of the cleaning formulation **F** with water **W** may be carried out according the skilled person's knowledge and depends on the context in which the method according to the invention is carried out.

### II. Bacillus velezensis strain DSM 34674 and mutants

In one preferred aspect of the method according to the first aspect of the invention and the use according to the second aspect of the invention, the bacterial spores **SP** are spores of *Bacillus velezensis,* preferably spores of a *Bacillus velezensis* strain selected from the group consisting of *Bacillus velezensis* DSM 34674 and mutants of *Bacillus velezensis* DSM 34674.

*"Bacillus velezensis* DSM 34674" relates to the *Bacillus velezensis* strain as deposited under DSM 34674 at the DSMZ (abbreviated as "*B*. *velezensis* DSM 34674" or *"Bacillus velezensis* DSM 34674" or simply "DSM 34674").

It was surprisingly found that *Bacillus velezensis* DSM 34674 and mutants thereof exhibit a high expression of enzymatic activity for protease and cellulase, and also have good enzymatic activity for amylase (confer point **I.** of the Examples) and therefore are particularly well suited to be used in cleaning formulations.

In addition, it was surprisingly found that *Bacillus velezensis* DSM 34674 and mutants thereof exhibit a high rate of metabolization of valeric acid (1000 ppm), as set forth under point **IV.** of the Examples.

The mutants *of Bacillus velezensis* DSM 34674 are also *Bacillus velezensis* strains and may be obtained by any kind of method, i.e., by genetic modification methods ("**GMO**") or **non-GMO** methods, but preferably the mutants are not genetically modified, i.e., non-**GMO**. It goes without saying that a mutant of *Bacillus velezensis* DSM 34674 is not identical with *Bacillus velezensis* DSM 34674. This means that the mutants of the deposited strain of *Bacillus velezensis* DSM 34674 are preferably either also naturally occurring microorganisms or spontaneous mutants of such naturally occurring microorganisms, i.e. of the deposited strains, or microorganisms which are obtained by another method which is classified as non-**GMO.**

The term "*spontaneous mutant*" refers to mutants that arise from naturally occurring microorganisms and/or parent strains without genetically modifying the microorganisms by applying classical gene technological and/or biotechnological methods like site-directed mutagenesis. Such spontaneous mutants may be obtained by classical methods of natural selection, such as growing the microorganisms in the presence of UV light and/or by applying high temperature or protoplast formation and/or in the presence of a certain antibiotic to which the parent strain is susceptible.

Spontaneous mutants might further, but less preferably, be obtained by using mutagens, i.e., chemical substances which induce the formation of mutants. As formation of spontaneous mutants by using mutagens is less preferred, in a preferred embodiment of the invention the spontaneous mutants and/or non-**GMO** mutants are obtained without the use of such mutagens. If the mutants are obtained by applying gene technological and/or biotechnological methods like site-directed mutagenesis, then preferably methods are applied which are classified as non-**GMO**.

A non-**GMO** method according to the invention is preferably characterized in that the method does not involve introduction of heterologous genetic information into the microorganism. In a particularly preferred embodiment of the invention the microorganisms of the invention are naturally non-occurring mutants, in particular non-**GMO** and/or spontaneous mutants as defined before. The microorganisms of the invention have preferably the same characteristics like the parent strain from which they are derived.

Preferred mutants of *Bacillus velezensis* DSM 34674 display property (β), even more preferably display properties (α) and (β) as described under point II.2.

It is even more preferred that, alternatively or in addition, preferably in addition, the mutants of *Bacillus velezensis* DSM 34674 have at least one of:
- a DNA sequence identity (in particular of the genomic DNA of the mutant) to the genomic DNA sequence of *B*. *velezensis* DSM 34674 as set forth under point II.1.1);
- at least one, preferably at least two, more preferably at least three, even more preferably at least four, even more preferably at least five, most preferably all six characteristics i., ii., iii., iv., v. and vi. as set forth under point II.1.2).

### 11.1 Mutants of Bacillus velezensis DSM 34674 defined by DNA sequence identity

### II.1.1) Sequence identity to genomic DNA

In a preferred embodiment of the present invention, the term "mutant of DSM 34674" refers to *Bacillus velezensis* strains with a DNA sequence identity (in particular of the genomic DNA of the mutant) of at least 95 %, more preferred of at least 97 %, more preferred of at least 98 %, more preferred of at least 99 %, more preferred of at least 99.5 %, more preferred of at least 99.8 %, more preferred of at least 99.9 %, more preferred of at least 99.95 %, more preferred of at least 99.98 %, more preferred of at least 99.99 %, more preferred of at least 99.999 %, more preferred of at least 99.9999 % to the genomic DNA sequence of *Bacillus velezensis* DSM 34674.

The person skilled in the art is aware that various computer programs are available for the calculation of similarity or identity between two nucleotide sequences or amino acid sequences.

Preferred methods for determining the sequence identity initially generate the greatest alignment between the sequences to be compared. Computer programs for determining the identity include, but are not limited to, the GCG program package including
- GAP [J. Devereux, P. Haeberli, O. Smithies, Nucleic Acid Res. 1984, 12, 387-395, Genetics Computer Group University of Wisconsin, Medicine (Wl)], and
- BLASTP, BLASTN and FASTA (S.F. Altschul, W. Gish, W. Miller, E.W. Myers, D.J. Lipman, J Mol Biol. 1990, 215, 403-410; hereinafter "Altschul *et al*."). The BLAST program can be obtained from the National Center for Biotechnology Information (NCBI) and from other sources (BLAST Handbook, Altschul *et al.,* NCBI NLM NIH Bethesda ND 22894).

For instance, the percentage identity between two amino acid sequences can be determined by the algorithm developed by S. B. Needleman & C. D. Wunsch, J Mol Biol. 1970, 48, 443-453 (hereinafter "Needleman & Wunsch"), which has been integrated into the GAP program in the GCG software package, using either a BLOSUM62 matrix or a PAM250 matrix, a gap weight of 16, 14, 12, 10, 8, 6 or 4 and a length weight of 1, 2, 3, 4, 5 or 6. The person skilled in the art will recognize that the use of different parameters will lead to slightly different results, but that the percentage identity between two amino acid sequences overall will not be significantly different. The BLOSUM62 matrix is typically used applying the default settings (gap weight: 12, length weight: 1).

In the context of the present invention, a "*sequence identity of 95 %*" according to the above algorithm means "*95 % homology*"*.* The same applies to higher identities.

Most preferably, the degree of identity between two nucleotide sequences is determined in the context of the present invention by the program "Needle" with the gap opening penalty of 10, and the gap extension penalty of 0.5. The Needle program implements the global alignment algorithm described by Needleman & Wunsch. The preferred version used in the context of this invention is the one presented by F. Madeira, M. Pearce, A.R.N Tivey, P. Basutkar, J. Lee, O. Edbali, N. Madhusoodanan, A. Kolesnikov, R. Lopez, Nucleic Acids Res. 2022, 50, W276-W279, Web Server issue (preferred version accessible online on Jun 1, 2024 via https://www.ebi.ac.uk/jdispatcher/psa/emboss_needle).

The "genomic sequence of *Bacillus velezensis* DSM 34674" is available/ can be determined from the deposited strain *Bacillus velezensis* DSM 34674 by means according to the state of the art (for example, as summarized in the reviews by T. Hu, N. Chitnis, D. Monos, A. Dinh, Human Immunology 2021, 82, 801-811; S. Levy & E. Boone, Cold Spring Harbor Perspectives in Medicine 2019, 9, a025791).

### II.1.2) Sequence identity to conserved DNA sequences

The following sequences are typical, conserved sequences of *Bacillus velezensis* DSM 34674:
1. SEQ ID NO: 1: a DNA sequence coding for the 16S rRNA sequence;
2. SEQ ID NO: 2: a *rpoB* sequence;
3. SEQ ID NO: 3: a *gyrB* sequence;
4. SEQ ID NO: 4: a *groEL* sequence;
5. SEQ ID NO: 5: a *yqfD* sequence;
6. SEQ ID NO: 6: a *uvrC* sequence.

In a further preferred embodiment, the mutant of DSM 34674 exhibits at least one, preferably at least two, more preferably at least three, even more preferably at least four, even more preferably at least five, most preferably all six of the following characteristics:
i. a DNA sequence, which preferably is the DNA sequence coding for the 16S rRNA, with a sequence identity of at least 99 %, preferably at least 99.5 %, more preferably at least 99.9 %, even more preferably 100 %, to the polynucleotide sequence according to SEQ ID NO: 1; and/or
ii. a DNA sequence, which preferably is a *rpoB* DNA sequence, with a sequence identity of at least 99 %, preferably at least 99.5 %, more preferably at least 99.9 %, even more preferably 100 %, to the polynucleotide sequence according to SEQ ID NO: 2; and/or
iii. a DNA sequence, which preferably is a *gyrB* DNA sequence, with a sequence identity of at least 99 %, preferably at least 99.5 %, more preferably at least 99.9 %, even more preferably 100 %, to the polynucleotide sequence according to SEQ ID NO: 3; and/or
iv. a DNA sequence, which preferably is a *groEL* DNA sequence, with a sequence identity of at least 99 %, preferably at least 99.5 %, more preferably at least 99.9 %, even more preferably 100 %, to the polynucleotide sequence according to SEQ ID NO: 4; and/or
v. a DNA sequence, which preferably is a *yqfD* DNA sequence, with a sequence identity of at least 99 %, preferably at least 99.5 %, more preferably at least 99.9 %, even more preferably 100 %, to the polynucleotide sequence according to SEQ ID NO: 5; and/or
vi. a DNA sequence, which preferably is a *uvrC* DNA sequence, with a sequence identity of at least 99 %, preferably at least 99.5 %, more preferably at least 99.9 %, even more preferably 100 %, to the polynucleotide sequence according to SEQ ID NO: 6.

In a most preferred embodiment, the mutant of DSM 34674 exhibits all six characteristics i., ii., iii., iv., v. and vi.

### II.2 Mutants of Bacillus velezensis DSM 34674 defined by function

*Bacillus velezensis* DSM 34674 has high protease activity and cellulase activity, as summarized under point 1.4 of the Example section. Preferably, protease activity in case of *Bacillus velezensis* DSM 34674 and mutants thereof means protease activity in the supernatant, i.e. secreted protease activity, as determined by the procedure of **Assay A-I.** Preferably, cellulase activity in case of *Bacillus velezensis* DSM 34674 and mutants thereof means cellulase activity as determined by the procedure of **Assay C.** *Bacillus velezensis* DSM 34674 according to the invention has excellent amylase activities. Preferably, amylase activity in case of *B*. *velezensis* DSM 34674 and mutants thereof means amylase activities as determined by the procedure of **Assay E.**

In addition, *Bacillus velezensis* DSM 34674 according to the invention has improved activities in terms of metabolization of valeric acid (1000 ppm).

In the context of the method according to the first aspect of the invention and the use according to the second aspect of the invention, those mutants of *Bacillus velezensis* DSM 34674 are preferred that display property (β), even more preferably display property (α) and (β) as follows:
(α) the mutant of *Bacillus velezensis* DSM 34674 is able to metabolize valeric acid (1000 ppm), even more preferably is able to metabolize valeric acid (5000 ppm), wherein, even more preferably, the ability to metabolize valeric acid **VA** (1000 ppm) as well as the ability to metabolize valeric acid **VA** (5000 ppm) is determined by the procedure of **Assay J;**
(β) the mutant of *Bacillus velezensis* DSM 34674 has at least one, preferably at least two, more preferably all three of protease activity (wherein, even more preferably, the protease activity is determined by the procedure of **Assay A-I**,), amylase activity (wherein, even more preferably, the amylase activity is determined by the procedure of **Assay E**), cellulase activity (wherein, even more preferably, the cellulase activity is determined by the procedure of **Assay C**), where it is more preferred that the activities of the mutants of *Bacillus velezensis* DSM 34674 is at least protease activity, where it is most preferred that the activities of the mutants of *Bacillus velezensis* DSM 34674 is at least protease and cellulase activity.

### II.2.1) Mutant of Bacillus velezensis DSM 34674 with ability to metabolize VA

Where a mutant of *Bacillus velezensis* DSM 34674 (= *"Mutant* 34674") is able to metabolize valeric acid at a given concentration **x**_{VA} of **VA**, wherein in particular **x_{VA}** = 1000 ppm and preferably **x_{VA}** = 5000 ppm, it is preferred that, for this concentration **x_{VA}**, the metabolization rate **k**_{XJ%} (**x_{VA}**) of valeric acid exhibited by the *Mutant* 34674 relative to the metabolization rate of valeric acid of *Bacillus velezensis* DSM 34674 is at least 50 %, preferably at least 60 %, preferably at least 65 %, preferably at least 70 %, preferably at least 75 %, preferably at least 85 %, preferably at least 86 %, preferably at least 87 %, preferably at least 88 %, preferably at least 89 %, preferably at least 90 %, more preferably at least 91 %, more preferably at least 92 %, more preferably at least 93 %, more preferably at least 94 %, more preferably at least 95 %, more preferably at least 96 %, more preferably at least 97 %, more preferably at least 98 %, more preferably at least 99 %, more preferably at least 99.6 %, more preferably at least 99.7 %, more preferably at least 99.8 %, more preferably at least 99.9 %, more preferably at least 99.99 %, more preferably at least 99.999 %, more preferably at least 100 %, more preferably at least 110 %, more preferably at least 120 %, wherein **k**_{XJ%} (**x_{VA}**) is, in particular, determined by **Assay L-II** (point VII.5.3.2).

### II.2.2) Mutant of Bacillus velezensis DSM 34674 with protease activity

Where a mutant of *Bacillus velezensis* DSM 34674 has protease activity, it is further preferred that the mutant of *Bacillus velezensis* DSM 34674 has a protease activity which is at least 85 %, preferably at least 86 %, preferably at least 87 %, preferably at least 88 %, preferably at least 89 %, preferably at least 90 %, more preferably at least 91 %, more preferably at least 92 %, more preferably at least 93 %, more preferably at least 94 %, more preferably at least 95 %, more preferably at least 96 %, more preferably at least 97 %, more preferably at least 98 %, more preferably at least 99 %, more preferably at least 99.9 %, more preferably at least 99.99 %, more preferably at least 99.999 %, more preferably at least 100 %, more preferably at least 110 %, more preferably at least 120 % the protease activity of *Bacillus velezensis* DSM 34674, wherein the protease activity of the mutant of *Bacillus velezensis* DSM 34674 with respect to the protease activity of *Bacillus velezensis* DSM 34674 is, in particular, determined by **Assay B-II** (point VII.1.2).

### II.2.3) Mutant of Bacillus velezensis DSM 34674 with cellulase activity

Where a mutant of *Bacillus velezensis* DSM 34674 has cellulase activity, it is further preferred that the mutant of *Bacillus velezensis* DSM 34674 has a cellulase activity which is at least 60 %, preferably at least 65 %, preferably at least 70 %, preferably at least 75 %, preferably at least 85 %, preferably at least 86 %, preferably at least 87 %, preferably at least 88 %, preferably at least 89 %, preferably at least 90 %, more preferably at least 91 %, more preferably at least 92 %, more preferably at least 93 %, more preferably at least 94 %, more preferably at least 95 %, more preferably at least 96 %, more preferably at least 97 %, more preferably at least 98 %, more preferably at least 99 %, more preferably at least 99.6 %, more preferably at least 99.7 %, more preferably at least 99.8 %, more preferably at least 99.9 %, more preferably at least 99.99 %, more preferably at least 99.999 %, more preferably at least 100 %, more preferably at least 110 %, more preferably at least 120 % the cellulase activity of *Bacillus velezensis* DSM 34674, wherein the cellulase activity of the mutant of *Bacillus velezensis* DSM 34674 with respect to the cellulase activity of *Bacillus velezensis* DSM 34674 is, in particular, determined by **Assay D-II** (point VII.2.2).

### II.2.4) Mutant of Bacillus velezensis DSM 34674 with amylase activity

Where a mutant of *Bacillus velezensis* DSM 34674 has amylase activity, it is further preferred that the mutant of *Bacillus velezensis* DSM 34674 has an amylase activity which is at least 99 %, more preferably at least 99.5 %, more preferably at least 99.6 %, more preferably at least 99.7 %, more preferably at least 99.8 %, more preferably at least 99.9 %, more preferably at least 99.99 %, more preferably at least 99.999 %, more preferably at least 100 %, more preferably at least 110 %, more preferably at least 120 % the cellulase activity of *Bacillus velezensis* DSM 34674, wherein the amylase activity of the mutant of *Bacillus velezensis* DSM 34674 with respect to the amylase activity of *Bacillus velezensis* DSM 34674 is, in particular, determined by **Assay F-II** (point VII.3.2).

### III. Bacillus velezensis strain DSM 34978 and mutants

In one preferred aspect of the method according to the first aspect of the invention and the use according to the second aspect of the invention, the bacterial spores **SP** are spores of *Bacillus velezensis,* preferably spores of a *Bacillus velezensis* strain selected from the group consisting of *Bacillus velezensis* DSM 34978 strain and mutants of *Bacillus velezensis* DSM 34978 strain.

*"Bacillus velezensis* DSM 34978" relates to the *Bacillus velezensis* strain as deposited under DSM 34978 at the DSMZ (abbreviated as "*B*. *velezensis* DSM 34978" or *"Bacillus velezensis* DSM 34978" or simply "DSM 34978").

It was surprisingly found that *B*. *velezensis* DSM 34978 and mutants thereof exhibit a high expression of enzymatic activity for protease, amylase, and cellulase (confer point **I.** of the Examples), and therefore are particularly well suited to be used in cleaning formulations.

In addition, it was surprisingly found that *B*. *velezensis* DSM 34978 and mutants thereof exhibit a high rate of metabolization of valeric acid (1000 ppm), as set forth under point **IV.** of the Examples.

The mutants *of Bacillus velezensis* DSM 34978 are also *Bacillus velezensis* strains and may be obtained by any kind of method, i.e., by genetic modification methods ("**GMO**") or non-**GMO** methods, but preferably the mutants are not genetically modified, i.e., non-**GMO**. It goes without saying that a mutant of *Bacillus velezensis* DSM 34978 is not identical with *B*. *velezensis* DSM 34978. This means that the mutants of the deposited strain of *B*. *velezensis* DSM 34978 are preferably either also naturally occurring microorganisms or spontaneous mutants of such naturally occurring microorganisms, i.e. of the deposited strains, or microorganisms which are obtained by another method which is classified as non-**GMO**.

Preferred mutants of *B*. *velezensis* DSM 34978 display property (β), even more preferably display properties (α) and (β) as described under point III.2.

It is even more preferred that, alternatively or in addition, preferably in addition, the mutants of *Bacillus velezensis* DSM 34978 have at least one of:
- a DNA sequence identity (in particular of the genomic DNA of the mutant) to the genomic DNA sequence of *B*. *velezensis* DSM 34978 as set forth under point III.1.1);
- at least one, preferably at least two, more preferably at least three, even more preferably at least four, even more preferably at least five, most preferably all six characteristics i., ii., iii., iv., v. and vi. as set forth under point III.1.2).

### III.1 Mutants of B. velezensis DSM 34978 defined by DNA sequence identity

### III.1.1) Sequence identity to genomic DNA

In a preferred embodiment of the present invention, the term "mutant of DSM 34978" refers to *Bacillus velezensis* strains with a DNA sequence identity (in particular of the genomic DNA of the mutant) of at least 95 %, more preferred of at least 97 %, more preferred of at least 98 %, more preferred of at least 99 %, more preferred of at least 99.5 %, more preferred of at least 99.8 %, more preferred of at least 99.9 %, more preferred of at least 99.95 %, more preferred of at least 99.98 %, more preferred of at least 99.99 %, more preferred of at least 99.999 %, more preferred of at least 99.9999 % to the genomic DNA sequence of *B*. *velezensis* DSM 34978.

The "genomic sequence of *B*. *velezensis* DSM 34978" is available/ can be determined from the deposited strain *B*. *velezensis* DSM 34978 by means according to the state of the art (for example, as summarized in the reviews by T. Hu, N. Chitnis, D. Monos, A. Dinh, Human Immunology 2021, 82, 801-811; S. Levy & E. Boone, Cold Spring Harbor Perspectives in Medicine 2019, 9, a025791).

### 111.1.2) Sequence identity to conserved DNA sequences

The following sequences are typical, conserved sequences of *Bacillus velezensis* DSM 34978;
- SEQ ID NO: 7: a DNA sequence coding for the 16S rRNA sequence;
- SEQ ID NO: 8: a *rpoB* DNA sequence;
- SEQ ID NO: 9: a *gyrB* DNA sequence;
- SEQ ID NO: 10: a *groEL* DNA sequence;
- SEQ ID NO: 11: a DNA sequence coding for an ATP synthase C chain;
- SEQ ID NO: 12: a DNA sequence coding for a chemotaxis protein sequence.

In a further preferred embodiment, the mutant of *Bacillus velezensis* DSM 34978 exhibits at least one, preferably at least two, more preferably at least three, even more preferably at least four, even more preferably at least five, most preferably all six of the following characteristics i. to vi.:
i. a DNA sequence, which preferably is the DNA sequence coding for a 16S rRNA, with a sequence identity of at least 99 %, preferably at least 99.5 %, more preferably at least 99.9 %, even more preferably 100 %, to the polynucleotide sequence according to SEQ ID NO: 7;
ii. a DNA sequence, which preferably is a *rpoB* DNA sequence, with a sequence identity of at least 99 %, preferably at least 99.5 %, more preferably at least 99.9 %, even more preferably 100 %, to the polynucleotide sequence according to SEQ ID NO: 8;
iii. a DNA sequence, which preferably is a *gyrB* DNA sequence, with a sequence identity of at least 99 %, preferably at least 99.5 %, more preferably at least 99.9 %, even more preferably 100 %, to the polynucleotide sequence according to SEQ ID NO: 9;
iv. a DNA sequence, which preferably is a *groEL* DNA sequence, with a sequence identity of at least 99 %, preferably at least 99.5 %, more preferably at least 99.9 %, even more preferably 100 %, to the polynucleotide sequence according to SEQ ID NO: 10;
v. a DNA sequence, which preferably is a DNA sequence coding for an ATP synthase C chain, with a sequence identity of at least 99 %, preferably at least 99.5 %, more preferably at least 99.9 %, even more preferably 100 %, to the polynucleotide sequence according to SEQ ID NO: 11;
vi. a DNA sequence, which preferably is a DNA sequence coding for a chemotaxis protein, with a sequence identity of at least 99 %, preferably at least 99.5 %, more preferably at least 99.9 %, even more preferably 100 %, to the polynucleotide sequence according to SEQ ID NO: 12.

In a most preferred embodiment, the mutant of *B*. *velezensis* DSM 34978 exhibits all six characteristics i., ii., iii., iv., v. and vi.

### III.2 Mutants of B. velezensis DSM 34978 defined by function

*Bacillus velezensis* DSM 34978 has high activities in terms of protease activity, amylase activity, cellulase activity, as summarized under point **I.4** of the Example section. Preferably, protease activity in case of *Bacillus velezensis* DSM 34978 and mutants thereof means protease activity in the supernatant, i.e. secreted protease activity, as determined by the procedure of **Assay A-I.** Preferably, cellulase activity in case of *Bacillus velezensis* DSM 34978 and mutants thereof means cellulase activity as determined by the procedure of **Assay C.** Preferably, amylase activity in case of *B*. *velezensis* DSM 34978 and mutants thereof means amylase activity as determined by the procedure of **Assay E.**

In addition, *B*. *velezensis* DSM 34978 according to the invention has improved activities in terms of metabolization of valeric acid (1000 ppm).

In the context of the method according to the first aspect of the invention and the use according to the second aspect of the invention, those mutants of *Bacillus velezensis* DSM 34978 are preferred that display property (β), even more preferably display property (α) and (β) as follows:
(α) the mutant of *Bacillus velezensis* DSM 34978 is able to metabolize valeric acid (1000 ppm), even more preferably is able to metabolize valeric acid (5000 ppm), wherein, even more preferably, the ability to metabolize valeric acid **VA** (1000 ppm) as well as the ability to metabolize valeric acid **VA** (5000 ppm) is determined by the procedure of **Assay J;**
(β) the mutant of *Bacillus velezensis* DSM 34978 has at least one, preferably at least two, more preferably all three of protease activity (wherein, even more preferably, the protease activity is determined by the procedure of **Assay A-I**,), amylase activity (wherein, even more preferably, the amylase activity is determined by the procedure of **Assay E**), cellulase activity (wherein, even more preferably, the cellulase activity is determined by the procedure of **Assay C**), where it is more preferred that the activities of the mutants of *Bacillus velezensis* DSM 34978 is at least protease activity, where it is most preferred that the activities of the mutants of *Bacillus velezensis* DSM 34978 is at least protease and cellulase activity.

### III.2.1) Mutant of Bacillus velezensis DSM 34978 with ability to metabolize VA

Where a mutant of *Bacillus velezensis* DSM 34978 (= *"Mutant* 34978") is able to metabolize valeric acid at a given concentration **x_{VA}** of valeric acid (= "**VA**"), wherein in particular **x_{VA}** = 1000 ppm and preferably **x_{VA}** = 5000 ppm, it is preferred that, for this concentration **x_{VA}**, the metabolization rate **k**_{XJ%} (**x_{VA}**) of valeric acid exhibited by the *Mutant* 34978 relative to the metabolization of valeric acid of *Bacillus velezensis* DSM 34978 is at least 50 %, at least 60 %, preferably at least 65 %, preferably at least 70 %, preferably at least 75 %, preferably at least 85 %, preferably at least 86 %, preferably at least 87 %, preferably at least 88 %, preferably at least 89 %, preferably at least 90 %, more preferably at least 91 %, more preferably at least 92 %, more preferably at least 93 %, more preferably at least 94 %, more preferably at least 95 %, more preferably at least 96 %, more preferably at least 97 %, more preferably at least 98 %, more preferably at least 99 %, more preferably at least 99.6 %, more preferably at least 99.7 %, more preferably at least 99.8 %, more preferably at least 99.9 %, more preferably at least 99.99 %, more preferably at least 99.999 %, more preferably at least 100 %, more preferably at least 110 %, more preferably at least 120 %, wherein **k**_{XJ%} (**x_{VA}**) is, in particular, determined by **Assay L-I** (point VII.5.3.1).

### III.2.2) Mutant of Bacillus velezensis DSM 34978 with protease activity

Where a mutant of *Bacillus velezensis* DSM 34978 has protease activity, it is further preferred that such mutant of *Bacillus velezensis* DSM 34978 has a protease activity which is at least 85 %, preferably at least 86 %, preferably at least 87 %, preferably at least 88 %, preferably at least 89 %, preferably at least 90 %, more preferably at least 91 %, more preferably at least 92 %, more preferably at least 93 %, more preferably at least 94 %, more preferably at least 95 %, more preferably at least 96 %, more preferably at least 97 %, more preferably at least 98 %, more preferably at least 99 %, more preferably at least 99.9 %, more preferably at least 99.99 %, more preferably at least 99.999 %, more preferably at least 100 %, more preferably at least 110 %, more preferably at least 120 % the protease activity of *Bacillus velezensis* DSM 34978, wherein the protease activity of the mutant of *Bacillus velezensis* DSM 34978 with respect to the protease activity of *Bacillus velezensis* DSM 34978 is, in particular, determined by **Assay B-I** (point VII.1.2)

### III.2.3) Mutant of Bacillus velezensis DSM 34978 with cellulase activity

Where a mutant of *Bacillus velezensis* DSM 34978 has cellulase activity, it is further preferred that such mutant of *Bacillus velezensis* DSM 34978 has a cellulase activity which is at least 60 %, preferably at least 65 %, preferably at least 70 %, preferably at least 75 %, preferably at least 85 %, preferably at least 86 %, preferably at least 87 %, preferably at least 88 %, preferably at least 89 %, preferably at least 90 %, more preferably at least 91 %, more preferably at least 92 %, more preferably at least 93 %, more preferably at least 94 %, more preferably at least 95 %, more preferably at least 96 %, more preferably at least 97 %, more preferably at least 98 %, more preferably at least 99 %, more preferably at least 99.6 %, more preferably at least 99.7 %, more preferably at least 99.8 %, more preferably at least 99.9 %, more preferably at least 99.99 %, more preferably at least 99.999 %, more preferably at least 100 %, more preferably at least 110 %, more preferably at least 120 % the cellulase activity of *Bacillus velezensis* DSM 34978, wherein the cellulase activity of the mutant of *Bacillus velezensis* DSM 34978 with respect to the cellulase activity of *Bacillus velezensis* DSM 34978 is, in particular, determined by **Assay D-I** (point VII.2.2).

### III.2.4) Mutant of Bacillus velezensis DSM 34978 with amylase activity

Where a mutant of *B*. *velezensis* DSM 34978 has amylase activity, it is further preferred that such mutant of *Bacillus velezensis* DSM 34978 has an amylase activity which is at least 99 %, more preferably at least 99.5 %, more preferably at least 99.6 %, more preferably at least 99.7 %, more preferably at least 99.8 %, more preferably at least 99.9 %, more preferably at least 99.99 %, more preferably at least 99.999 %, more preferably at least 100 %, more preferably at least 110 %, more preferably at least 120 % the cellulase activity of *Bacillus velezensis* DSM 34978, wherein the amylase activity of the mutant of *Bacillus velezensis* DSM 34978 with respect to the amylase activity of *Bacillus velezensis* DSM 34978 is, in particular, determined by **Assay F-I** (point VII.3.2).

### IV. Bacillus licheniformis strain DSM 34977 and mutants thereof

In one preferred aspect of the method according to the first aspect of the invention and the use according to the second aspect of the invention, the bacterial spores **SP** are spores of *Bacillus licheniformis,* preferably spores of a *Bacillus licheniformis* strain selected from the group consisting of *Bacillus licheniformis* DSM 34977 and mutants of *Bacillus licheniformis* DSM 34977.

"*Bacillus licheniformis* DSM 34977" relates to the *Bacillus licheniformis* DSM 34977 strain as deposited under DSM 34977 at the DSMZ (abbreviated as "*B. licheniformis* DSM 34977" or "*Bacillus licheniformis* DSM 34977" or simply "DSM 34977").

It was surprisingly found that *B*. *licheniformis* DSM 34977 and mutants thereof exhibit a high ability to grow on valeric acid (1000 ppm) and exhibit a high rate of metabolization of valeric acid (1000 ppm), as set forth under point **IV.** of the Examples, and therefore are particularly well suited to be used in cleaning formulations.

The mutants of *B*. *licheniformis* DSM 34977 are also *Bacillus licheniformis* strains and may be obtained by any kind of method, i.e., by genetic modification methods ("**GMO**") or non-**GMO** methods, but preferably the mutants are not genetically modified, i.e., non-**GMO**. It goes without saying that a mutant of *B. licheniformis* DSM 34977 is not identical with *B. licheniformis* DSM 34977. This means that the mutants of the deposited strain of *B*. *licheniformis* DSM 34977 are preferably either also naturally occurring microorganisms or spontaneous mutants of such naturally occurring microorganisms, i.e. of the deposited strains, or microorganisms which are obtained by another method which is classified as non-**GMO.**

Preferred mutants of *Bacillus licheniformis* DSM 34977 display property (γ), even more preferably display properties (γ) and (δ) as described under point IV.2.

It is even more preferred that, alternatively or in addition, preferably in addition, the mutants of *Bacillus licheniformis* DSM 34977 have at least one of:
- a DNA sequence identity (in particular of the genomic DNA of the mutant) to the genomic DNA sequence of *Bacillus licheniformis* DSM 34977 as set forth under point IV.1.1);
- at least one, preferably at least two, more preferably at least three, even more preferably at least four, even more preferably at least five, most preferably all six characteristics i., ii., iii., iv., v. and vi. as set forth under point IV.1.2).

### IV.1 Mutants of B. licheniformis DSM 34977 defined by DNA sequence identity

### IV.1.1) Sequence identity to genomic DNA

In a preferred embodiment of the present invention, the term "mutant of DSM 34977" refers to *Bacillus licheniformis* strains with a DNA sequence identity (in particular of the genomic DNA of the mutant) of at least 95 %, more preferred of at least 97 %, more preferred of at least 98 %, more preferred of at least 99 %, more preferred of at least 99.5 %, more preferred of at least 99.8 %, more preferred of at least 99.9 %, more preferred of at least 99.95 %, more preferred of at least 99.98 %, more preferred of at least 99.99 %, more preferred of at least 99.999 %, more preferred of at least 99.9999 % to the genomic DNA sequence of *B*. *licheniformis* DSM 34977.

The "genomic sequence of *B*. *licheniformis* DSM 34977" is available/ can be determined from the deposited strain *B*. *licheniformis* DSM 34977 by means according to the state of the art (for example, as summarized in the reviews by T. Hu, N. Chitnis, D. Monos, A. Dinh, Human Immunology 2021, 82, 801-811; S. Levy & E. Boone, Cold Spring Harbor Perspectives in Medicine 2019, 9, a025791).

### IV.1.2) Sequence identity to conserved DNA sequences

The following sequences are typical, conserved sequences of *B*. *licheniformis* DSM 34977:
- SEQ ID NO: 19: a DNA sequence coding for the 16S rRNA sequence;
- SEQ ID NO: 20: a *rpoB* sequence;
- SEQ ID NO: 21: a *gyrB* sequence;
- SEQ ID NO: 22: a groELsequence;
- SEQ ID NO: 23: a *yqfD* sequence;
- SEQ ID NO: 24: an *adaA* sequence.

In a further preferred embodiment, the mutant of DSM 34977 exhibits at least one, preferably at least two, more preferably at least three, even more preferably at least four, even more preferably at least five, most preferably all six of the following characteristics i. to vi.:
i. a DNA sequence, which preferably is the DNA sequence coding for the 16S rRNA, with a sequence identity of at least 99 %, preferably at least 99.5 %, more preferably at least 99.9 %, even more preferably 100 %, to the polynucleotide sequence according to SEQ ID NO: 19; and/or
ii. a DNA sequence, which preferably is a *rpoB* DNA sequence, with a sequence identity of at least 99 %, preferably at least 99.5 %, more preferably at least 99.9 %, even more preferably 100 %, to the polynucleotide sequence according to SEQ ID NO: 20; and/or
iii. a DNA sequence, which preferably is a *gyrB* DNA sequence, with a sequence identity of at least 99 %, preferably at least 99.5 %, more preferably at least 99.9 %, even more preferably 100 %, to the polynucleotide sequence according to SEQ ID NO: 21; and/or
iv. a DNA sequence, which preferably is a *groEL* DNA sequence, with a sequence identity of at least 99 %, preferably at least 99.5 %, more preferably at least 99.9 %, even more preferably 100 %, to the polynucleotide sequence according to SEQ ID NO: 22; and/or
v. a DNA sequence, which preferably is a *yqfD* DNA sequence, with a sequence identity of at least 99 %, preferably at least 99.5 %, more preferably at least 99.9 %, even more preferably 100 %, to the polynucleotide sequence according to SEQ ID NO: 23; and/or
vi. a DNA sequence, which preferably is an *adaA* DNA sequence, with a sequence identity of at least 99 %, preferably at least 99.5 %, more preferably at least 99.9 %, even more preferably 100 %, to the polynucleotide sequence according to SEQ ID NO: 24.

In a most preferred embodiment, the mutant of *B*. *licheniformis* DSM 34977 exhibits all six characteristics i., ii., iii., iv., v. and vi.

### IV.2 Mutants of B. licheniformis DSM 34977 defined by function

*Bacillus licheniformis* DSM 34977 according to the invention has improved activities in terms of growth on 1000 ppm valeric acid and metabolization of valeric acid, as shown under point **IV.** of the Example section. Preferably, the ability to grow on valeric acid (1000 ppm) and the metabolization of valeric acid in case of *Bacillus licheniformis* DSM 34977 and mutants thereof is determined by the procedure of **Assay J** (point Vll.5.1).

In the context of the method according to the first aspect of the invention and the use according to the second aspect of the invention, those mutants of *Bacillus licheniformis* DSM 34977 are preferred that display property (γ), more preferably display properties (γ) and (δ) as follows:
(γ) the mutant of *Bacillus licheniformis* DSM 34977 is able to grow on valeric acid (1000 ppm), even more preferably is able to grow on valeric acid (5000 ppm), wherein, even more preferably, the ability to grow on **VA** (1000 ppm) as well as the ability to grow on **VA** (5000 ppm) is determined by the procedure of **Assay J,** wherein "able to grow on valeric acid (1000 ppm)" means in particular that they fulfill the condition **"Γ_{XJ;1000 ppm}** > 1" for **x**_{VA} = 1000 ppm as determined by **Assay J** (point VII.5.1.4.1) and wherein "able to grow on valeric acid (5000 ppm)" means in particular that they fulfill the condition **"Γ_{XJ;5000 ppm}** > 1 for **x**_{VA} = 5000 ppm as determined by **Assay J** (point VII.5.1.4.2);
(δ) the mutant of *Bacillus licheniformis* DSM 34977 has an effectiveness **E**_{XJ%} (**x_{VA}**) to reduce valeric acid [wherein **x**_{VA} = 1000 ppm, preferably **x**_{VA} = 5000 ppm] which is at least 85 %, preferably at least 86 %, preferably at least 87 %, preferably at least 88 %, preferably at least 89 %, preferably at least 90 %, more preferably at least 91 %, more preferably at least 92 %, more preferably at least 93 %, more preferably at least 94 %, more preferably at least 95 %, more preferably at least 96 %, more preferably at least 97 %, more preferably at least 98 %, more preferably at least 99 %, more preferably at least 99.9 %, more preferably at least 99.99 %, more preferably at least 99.999 %, more preferably at least 100 %, more preferably at least 110 %, more preferably at least 120 % with respect to the effectiveness of *Bacillus licheniformis* DSM 34977 to reduce valeric acid, wherein **E_{XJ%}** (**x_{VA}**) [wherein **x_{VA}** = 1000 ppm, preferably **x_{VA}** = 5000 ppm] is, in particular, determined by **Assay K-I** (point VII.5.2.1).

### V. Priestia meqaterium strain DSM 34980 and mutants

In one preferred aspect of the method according to the first aspect of the invention and the use according to the second aspect of the invention, the bacterial spores **SP** are spores of *Priestia megaterium,* preferably spores of a *Priestia megaterium* strain selected from the group consisting of *Priestia megaterium* DSM 34980 and mutants of *Priestia megaterium* DSM 34980.

"*Priestia megaterium* DSM 34980" strain relates to the *Priestia megaterium* DSM 34980 strain as deposited under DSM 34980 at the DSMZ (abbreviated as *"P. megaterium* DSM 34980" or "*Priestia megaterium* DSM 34980" or simply "DSM 34980").

It was surprisingly found that *Priestia megaterium* DSM 34980 and mutants thereof exhibit a high expression of enzymatic activity for protease (confer point **II.** of the Examples) and therefore are particularly well suited to be used in cleaning formulations.

In addition, it was surprisingly found that *Priestia megaterium* DSM 34980 and mutants thereof exhibit a high ability to grow on valeric acid (1000 ppm) and exhibit a high rate of metabolization of valeric acid (1000 ppm), as set forth under point **IV.** of the Examples.

The mutants *of P. megaterium* DSM 34980 are also *Priestia megaterium* strains and may be obtained by any kind of method, i.e., by genetic modification methods ("**GMO**") or non-**GMO** methods, but preferably the mutants are not genetically modified, i.e., non-**GMO**. It goes without saying that a mutant of *P. megaterium* DSM 34980 is not identical with *P. megaterium* DSM 34980. This means that the mutants of the deposited strain of *P*. *megaterium* DSM 34980 are preferably either also naturally occurring microorganisms or spontaneous mutants of such naturally occurring microorganisms, i.e. of the deposited strains, or microorganisms which are obtained by another method which is classified as non-**GMO**.

Preferred mutants of *Priestia megaterium* DSM 34980 display property (ε), more preferably display properties (ε) and (γ), even more preferably display properties (ε), (γ), and (δ) as described under point V.2.

It is even more preferred that, alternatively or in addition, preferably in addition, the mutants of *Priestia megaterium* DSM 34980 have at least one of:
- a DNA sequence identity (in particular of the genomic DNA of the mutant) to the genomic DNA sequence of *Priestia megaterium* DSM 34980 as set forth under point V.1.1);
- at least one, preferably at least two, more preferably at least three, even more preferably at least four, even more preferably at least five, most preferably all six characteristics i., ii., iii., iv., v. and vi. as set forth under point V.1.2).

### V.1 Mutants of P. megaterium DSM 34980 defined by DNA sequence identity

### IV.1.1) Sequence identity to genomic DNA

In a preferred embodiment of the present invention, the term "mutant of DSM 34980" refers to *Priestia megaterium* strains with a DNA sequence identity (in particular of the genomic DNA of the mutant) of at least 95 %, more preferred of at least 97 %, more preferred of at least 98 %, more preferred of at least 99 %, more preferred of at least 99.5 %, more preferred of at least 99.8 %, more preferred of at least 99.9 %, more preferred of at least 99.95 %, more preferred of at least 99.98 %, more preferred of at least 99.99 %, more preferred of at least 99.999 %, more preferred of at least 99.9999 % to the genomic DNA sequence of *P. megaterium* DSM 34980.

The "genomic sequence of *P*. *megaterium* DSM 34980" is available/ can be determined from the deposited strain *P. megaterium* DSM 34980 by means according to the state of the art (for example, as summarized in the reviews by T. Hu, N. Chitnis, D. Monos, A. Dinh, Human Immunology 2021, 82, 801-811; S. Levy & E. Boone, Cold Spring Harbor Perspectives in Medicine 2019, 9, a025791).

### IV.1.2) Sequence identity to conserved DNA sequences

The following sequences are typical, conserved sequences of *P*. *megaterium* DSM 34980:
1. SEQ ID NO: 25: a DNA sequence coding for the 16S rRNA sequence;
2. SEQ ID NO: 26: a *rpoB* sequence;
3. SEQ ID NO: 27: a *gyrB* sequence;
4. SEQ ID NO: 28: a *groEL*equence;
5. SEQ ID NO: 29: a *yqfD* sequence.

In a further preferred embodiment, the mutant of DSM 34980 exhibits at least one, preferably at least two, more preferably at least three, even more preferably at least four, most preferably all five of the following characteristics i. to v:
i. a DNA sequence, which preferably is the DNA sequence coding for the 16S rRNA, with a sequence identity of at least 99 %, preferably at least 99.5 %, more preferably at least 99.9 %, even more preferably 100 %, to the polynucleotide sequence according to SEQ ID NO: 25; and/or
ii. a DNA sequence, which preferably is a *rpoB* DNA sequence, with a sequence identity of at least 99 %, preferably at least 99.5 %, more preferably at least 99.9 %, even more preferably 100 %, to the polynucleotide sequence according to SEQ ID NO: 26; and/or
iii. a DNA sequence, which preferably is a *gyrB* DNA sequence, with a sequence identity of at least 99 %, preferably at least 99.5 %, more preferably at least 99.9 %, even more preferably 100 %, to the polynucleotide sequence according to SEQ ID NO: 27; and/or
iv. a DNA sequence, which preferably is a *groEL* DNA sequence, with a sequence identity of at least 99 %, preferably at least 99.5 %, more preferably at least 99.9 %, even more preferably 100 %, to the polynucleotide sequence according to SEQ ID NO: 28; and/or
v. a DNA sequence, which preferably is a *yqfD* DNA sequence, with a sequence identity of at least 99 %, preferably at least 99.5 %, more preferably at least 99.9 %, even more preferably 100 %, to the polynucleotide sequence according to SEQ ID NO: 29.

In a most preferred embodiment, the mutant of DSM 34980 exhibits all five characteristics i., ii., iii., iv., and v.

### V.2 Mutants of P. megaterium DSM 34980 defined by function

*Priestia megaterium* DSM 34980 has improved activities in terms of protease activity, as shown under point **II.** of the Example section. Preferably, protease activity in case of *P*. *megaterium* DSM 34980 and mutants thereof means total protease activities, i.e. secreted protease activity as well as cell-bound protease

In addition, *Priestia megaterium* DSM 34980 according to the invention has improved activities in terms of growth on 1000 ppm valeric acid and metabolization of valeric acid, as shown under point **IV.** of the Example section. Preferably, the ability to grow on valeric acid (1000 ppm) and the metabolization of valeric acid in case of *Priestia megaterium* DSM 34980 and mutants thereof is determined by the procedure of **Assay J.**

In the context of the method according to the first aspect of the invention and the use according to the second aspect of the invention, those mutants of *Priestia megaterium* DSM 34980 are preferred that display property (ε), more preferably display properties (ε) and (γ), even more preferably display properties (ε), (γ), and (δ) as follows:
(γ) the mutant of *Priestia megaterium* DSM 34980 is able to grow on valeric acid (1000 ppm), even more preferably is able to grow on valeric acid (5000 ppm), wherein, even more preferably, the ability to grow on **VA** (1000 ppm) as well as the ability to grow on **VA** (5000 ppm) is determined by the procedure of **Assay J,** wherein "able to grow on valeric acid (1000 ppm)" means in particular that they fulfill the condition **"Γ_{XJ;1000 ppm}** > 1" for **x_{VA}** = 1000 ppm as determined by **Assay J** (point VII.5.1.4.1)" and wherein "able to grow on valeric acid (5000 ppm)" means in particular that they fulfill the condition **"Γ_{XJ;5000 ppm}** > 1" for **x_{VA}** = 5000 ppm as determined by **Assay J** (point VII.5.1.4.2);
(δ) the mutant of *Priestia megaterium* DSM 34980 has an effectiveness **E_{XJ%}** (**x_{VA}**) to reduce valeric acid [wherein **x_{VA}** = 1000 ppm, preferably **x_{VA}** = 5000 ppm] which is at least 85 %, preferably at least 86 %, preferably at least 87 %, preferably at least 88 %, preferably at least 89 %, preferably at least 90 %, more preferably at least 91 %, more preferably at least 92 %, more preferably at least 93 %, more preferably at least 94 %, more preferably at least 95 %, more preferably at least 96 %, more preferably at least 97 %, more preferably at least 98 %, more preferably at least 99 %, more preferably at least 99.9 %, more preferably at least 99.99 %, more preferably at least 99.999 %, more preferably at least 100 %, more preferably at least 110 %, more preferably at least 120 % with respect to the effectiveness of *Priestia megaterium* DSM 34980 to reduce valeric acid, wherein **E_{XJ%}** (**x_{VA}**) [wherein **x_{VA}** = 1000 ppm, preferably **x_{VA}** = 5000 ppm] is, in particular, determined by **Assay K-II** (point VII.5.2.2);
(ε) the mutant of *Priestia megaterium* DSM 34980 has protease activity, wherein it is further preferred that the mutant of *Priestia megaterium* DSM 34980 has a protease activity which is at least 45 %, preferably at least 50 %, preferably at least 55 %, preferably at least 60 %, preferably at least 65 %, preferably at least 70 %, preferably at least 75 %, preferably at least 78 %, preferably at least 79 %, more preferably at least 80 %, more preferably at least 81 %, more preferably at least 82 %, more preferably at least 83 %, more preferably at least 84 %, more preferably at least 85 %, more preferably at least 86 %, more preferably at least 87 %, more preferably at least 88 %, more preferably at least 89 %, more preferably at least 90 %, more preferably at least 91 %, more preferably at least 92 %, more preferably at least 93 %, more preferably at least 94 %, more preferably at least 95 %, more preferably at least 96 %, more preferably at least 97 %, more preferably at least 98 %, more preferably at least 99 %, more preferably at least 99.9 %, more preferably at least 99.99 %, more preferably at least 99.999 %, more preferably at least 100 %, more preferably at least 110 %, more preferably at least 120 % the protease activity of *Priestia megaterium* DSM 34980, wherein the protease activity of the mutant of *Priestia megaterium* DSM 34980 with respect to the protease activity of *Priestia megaterium* DSM 34980 is, in particular, determined by **Assay B-III** (point VII.1.4).

### VI. Bacillus subtilis strain DSM 34981 and mutants

In one preferred aspect of the method according to the first aspect of the invention and the use according to the second aspect of the invention, the bacterial spores **SP** are spores of *Bacillus subtilis,* preferably spores of a *Bacillus subtilis* strain selected from the group consisting of *Bacillus subtilis* DSM 34981 strain and mutants of *Bacillus subtilis* DSM 34981 strain.

"*Bacillus subtilis* DSM 34981" strain relates to the *Bacillus subtilis* DSM 34981 strain as deposited under DSM 34981 at the DSMZ (abbreviated as "*B*. *subtilis* DSM 34981" or "*Bacillus subtilis* DSM 34981" or simply "DSM 34981").

It was surprisingly found that *Bacillus subtilis* DSM 34981 and mutants thereof exhibit a high expression of enzymatic activity for lipase (confer point **III.** of the Examples) and therefore are particularly well suited to be used in cleaning formulations.

In addition, it was surprisingly found that *Bacillus subtilis* DSM 34981 and mutants thereof exhibit a high ability to grow on valeric acid (1000 ppm) and exhibit a high rate of metabolization of valeric acid (1000 ppm), as set forth under point **IV.** of the Examples.

The mutants *of B. subtilis* DSM 34981 are also *Bacillus subtilis* strains and may be obtained by any kind of method, i.e., by genetic modification methods ("**GMO**") or non-**GMO** methods, but preferably the mutants are not genetically modified, i.e., non-**GMO**. It goes without saying that a mutant of *B*. *subtilis* DSM 34981 is not identical with *B. subtilis* DSM 34981. This means that the mutants of the deposited strain of *B*. *subtilis* DSM 34981 are preferably either also naturally occurring microorganisms or spontaneous mutants of such naturally occurring microorganisms, i.e. of the deposited strains, or microorganisms which are obtained by another method which is classified as non-**GMO**.

Preferred mutants of *Bacillus subtilis* DSM 34981 display property (ζ), more preferably display properties (γ) and (ζ), even more preferably display properties (γ), (δ), and (ζ) as described under point VI.2. It is even more preferred that, alternatively or in addition, preferably in addition, the mutants of *Bacillus subtilis* DSM 34981 have at least one of:
- a DNA sequence identity (in particular of the genomic DNA of the mutant) to the genomic DNA sequence of *Bacillus subtilis* DSM 34981 as set forth under point VI.1.1);
- at least one, preferably at least two, more preferably at least three, even more preferably at least four, even more preferably at least five, most preferably all six characteristics i., ii., iii., iv., v. and vi. as set forth under point VI.1.2).

### VI.1 Mutants of B. subtilis DSM 34981 defined by DNA sequence identity

### VI.1.1) Sequence identity to genomic DNA

In a preferred embodiment of the present invention, the term "mutant of DSM 34981" refers to *Bacillus subtilis* strains with a DNA sequence identity (in particular of the genomic DNA of the mutant) of at least 95 %, more preferred of at least 97 %, more preferred of at least 98 %, more preferred of at least 99 %, more preferred of at least 99.5 %, more preferred of at least 99.8 %, more preferred of at least 99.9 %, more preferred of at least 99.95 %, more preferred of at least 99.98 %, more preferred of at least 99.99 %, more preferred of at least 99.999 %, more preferred of at least 99.9999 % to the genomic DNA sequence of *B*. *subtilis* DSM 34981.

The "genomic sequence of B. *subtilis* DSM 34981" is available/ can be determined from the deposited strain *B*. *subtilis* DSM 34981 by means according to the state of the art (for example, as summarized in the reviews by T. Hu, N. Chitnis, D. Monos, A. Dinh, Human Immunology 2021, 82, 801-811; S. Levy & E. Boone, Cold Spring Harbor Perspectives in Medicine 2019, 9, a025791).

### V. 1.2) Sequence identity to conserved DNA sequences

The following sequences are typical, conserved sequences of *B*. *subtilis* DSM 34981:
1. SEQ ID NO: 13: a DNA sequence coding for the 16S rRNA sequence;
2. SEQ ID NO: 14: a *rpoB* sequence;
3. SEQ ID NO: 15: a *gyrB* sequence;
4. SEQ ID NO: 16: a *groEL*sequence;
5. SEQ ID NO: 17: a *lipC* sequence;
6. SEQ ID NO: 18: a *yqcA* sequence.

In a further preferred embodiment, the mutant of DSM 34981 exhibits at least one, preferably at least two, more preferably at least three, even more preferably at least four, even more preferably at least five, most preferably all six of the following characteristics i. to vi.:
i. a DNA sequence, which preferably is the DNA sequence coding for the 16S rRNA, with a sequence identity of at least 99 %, preferably at least 99.5 %, more preferably at least 99.9 %, even more preferably 100 %, to the polynucleotide sequence according to SEQ ID NO: 13; and/or
ii. a DNA sequence, which preferably is a *rpoB* DNA sequence, with a sequence identity of at least 99 %, preferably at least 99.5 %, more preferably at least 99.9 %, even more preferably 100 %, to the polynucleotide sequence according to SEQ ID NO: 14; and/or
iii. a DNA sequence, which preferably is a *gyrB* DNA sequence, with a sequence identity of at least 99 %, preferably at least 99.5 %, more preferably at least 99.9 %, even more preferably 100 %, to the polynucleotide sequence according to SEQ ID NO: 15; and/or
iv. a DNA sequence, which preferably is a *groEL* DNA sequence, with a sequence identity of at least 99 %, preferably at least 99.5 %, more preferably at least 99.9 %, even more preferably 100 %, to the polynucleotide sequence according to SEQ ID NO: 16; and/or
v. a DNA sequence, which preferably is a *lipC* DNA sequence, with a sequence identity of at least 99 %, preferably at least 99.5 %, more preferably at least 99.9 %, even more preferably 100 %, to the polynucleotide sequence according to SEQ ID NO: 17; and/or
vi. a DNA sequence, which preferably is a *yqcA* DNA sequence, with a sequence identity of at least 99 %, preferably at least 99.5 %, more preferably at least 99.9 %, even more preferably 100 %, to the polynucleotide sequence according to SEQ ID NO: 18.

In a most preferred embodiment, the mutant of *B*. *subtilis* DSM 34981 exhibits all six characteristics i., ii., iii., iv., v. and vi.

### VI.2 Mutants of B. subtilis DSM 34981 defined by function

*Bacillus subtilis* DSM 34981 has improved activities in terms of lipase activity, as shown under point **III.** of the Example section. Preferably, lipase activity in case of *Bacillus subtilis* DSM 34981 and mutants thereof is determined by the procedure of **Assay G.**

In addition, *Bacillus subtilis* DSM 34981 according to the invention has improved activities in terms of growth on 1000 ppm valeric acid and metabolization of valeric acid, as shown under point **IV.** of the Example section. Preferably, the ability to grow on valeric acid (1000 ppm) and the metabolization of valeric acid in case of *Bacillus subtilis* DSM 34981 and mutants thereof is determined by the procedure of **Assay J.**

In the context of the method according to the first aspect of the invention and the use according to the second aspect of the invention, those mutants of *Bacillus subtilis* DSM 34981 are preferred that display property (ζ), more preferably display properties (ζ) and (γ), even more preferably display properties (ζ), (γ), and (δ) as follows:
(γ) the mutant of *Bacillus subtilis* DSM 34981 is able to grow on valeric acid (1000 ppm), even more preferably is able to grow on valeric acid (5000 ppm), wherein, even more preferably, the ability to grow on **VA** (1000 ppm) as well as the ability to grow on **VA** (5000 ppm) is determined by the procedure of **Assay J,** wherein "able to grow on valeric acid (1000 ppm)" means in particular that they fulfill the condition **"Γ_{XJ;1000 ppm}** > 1" for **x_{VA}** = 1000 ppm as determined by **Assay J** (point VII.5.1.4.1) and wherein "able to grow on valeric acid (5000 ppm)" means in particular that they fulfill the condition **"Γ_{XJ;5000 ppm}** > 1" for **x_{VA}** = 5000 ppm as determined by **Assay J** (point VII.5.1.4.2);
(δ) the mutant of *Bacillus subtilis* DSM 34981 has an effectiveness **E**_{XJ%} (**x_{VA}**) to reduce valeric acid [wherein **x_{VA}** = 1000 ppm, preferably **x_{VA}** = 5000 ppm] which is at least 85 %, preferably at least 86 %, preferably at least 87 %, preferably at least 88 %, preferably at least 89 %, preferably at least 90 %, more preferably at least 91 %, more preferably at least 92 %, more preferably at least 93 %, more preferably at least 94 %, more preferably at least 95 %, more preferably at least 96 %, more preferably at least 97 %, more preferably at least 98 %, more preferably at least 99 %, more preferably at least 99.9 %, more preferably at least 99.99 %, more preferably at least 99.999 %, more preferably at least 100 %, more preferably at least 110 %, more preferably at least 120 % with respect to the effectiveness of *Bacillus subtilis* DSM 34981 to reduce valeric acid, wherein **E_{XJ%}** (**x_{VA}**) [wherein **x_{VA}** = 1000 ppm, preferably **x_{VA}** = 5000 ppm] is, in particular, determined by **Assay K-III** (point VII.5.2.3);
(ζ) the mutant of *Bacillus subtilis* DSM 34981 has lipase activity, it is further preferred that the mutant of *Bacillus subtilis* DSM 34981 has a lipase activity which is at least 91 %, more preferably at least 92 %, more preferably at least 93 %, more preferably at least 94 %, more preferably at least 95 %, more preferably at least 96 %, more preferably at least 97 %, more preferably at least 98 %, more preferably at least 99 %, more preferably at least 99.9 %, more preferably at least 99.99 %, more preferably at least 99.999 %, more preferably at least 100 %, more preferably at least 110 %, more preferably at least 120 % the lipase activity of *B*. *subtilis* DSM 34981, wherein the lipase activity of the mutant of *Bacillus subtilis* DSM 34981 with respect to the lipase activity of *Bacillus subtilis* DSM 34981 is, in particular, determined by **Assay H** (point VII.4.2).

### VII. Assays for qualification and quantification of catalytic activities, valeric acid metabolism, germination, spreading on a surface

### VII.1 Protease activity

"Protease activity" in the sense of the present invention is deemed to be an activity that catalyzes the hydrolytic cleavage of a peptide bond linking two amino acid residues ("proteolytic enzymatic activity"). US 2018/023040 A1 discloses cleaning compositions comprising proteases, which provide a biocatalytic composition comprising biosurfactants and a protease.

The prior art describes different assays to qualitatively and quantitatively determine protease activity, for example in Zhang et al., ACS Omega 2021, 6, 3675-3680.

In the context of the present invention, two different protease activities may be determined, namely the protease activity that is secreted by a certain strain as well as the total protease activity exerted by a certain strain (i.e. secreted and cell-bound).

**Assay A-I** is the preferred qualitative test for determination of secreted protease activity according to the invention.

**Assay A-II** is the preferred qualitative test for determination of total protease activity according to the invention.

Whether a mutant of *Bacillus velezensis* DSM 34978 exhibits "protease activity" is preferably determined by **Assay A-I** further explained in the following.

Whether a mutant of *Bacillus velezensis* DSM 34674 exhibits "protease activity" is preferably determined by **Assay A-I** further explained in the following.

Whether a mutant of *Priestia megaterium* DSM 34980 exhibits "protease activity" is preferably determined by **Assay A-II** further explained in the following.

In order to determine the protease activity (in %) of a given mutant of *Bacillus velezensis* DSM 34978 related to the protease activity of *Bacillus velezensis* DSM 34978, **Assay B-I** is preferably used.

In order to determine the protease activity (in %) of a given mutant of *Bacillus velezensis* DSM 34674 related to the protease activity of *Bacillus velezensis* DSM 34674, **Assay B-II** is preferably used.

In order to determine the protease activity (in %) of a given mutant of *Priestia megaterium* DSM 34980 related to the protease activity of *Priestia megaterium* DSM 34980, **Assay B-III** is preferably used.

### VII. 1.1 Assay A-I

**Assay A-I** may be carried out by the following steps to determine whether a given **Strain X_{AI}**, for example *Bacillus velezensis* DSM 34978 or a given mutant of *Bacillus velezensis* DSM 34978, or *Bacillus velezensis* DSM 34674 or a given mutant of *Bacillus velezensis* DSM 34674 exhibit protease activity.

**Assay A-I** may be used to determine secreted protease activity.
**(A-I-1) Strain X_{AI}** is cultivated in 10 ml tryptic-soy-broth ("**TSB**"; Merck - 105459; peptone from casein 17.0 g/L; peptone from soymeal 3.0 g/L; D(+) glucose monohydrate 2.5 g/L; sodium chloride 5.0 g/L; di-potassium hydrogen phosphate 2.5 g/L) at 37 °C and 200 rpm for 16 h. As inoculum, 20 µl of a cryo-conservation culture was used for each **strain X_{AI}**.
**(A-I-2)** After the overnight cultivation, the optical density is measured at λ = 660 nm using a cuvette photometer. A culture volume **CM** for OD 1 (in 1 ml) is obtained as follows:
   The culture medium **CM** is obtained by centrifugation (5000 x g, 3 min, room temperature). The filter-sterile (filtered by 0.2 µm) supernatant is obtained, adjusted (by dilution with **TSB** medium) to OD 1 and then used (abbreviated as "**SN_{AI}**") for the qualitative protease test on specific selective agar plates [step **(A-I-3)**].

**(A-I-3)** 10% Crossley milk agar ("**CM0213**", Oxoid; 10 g/L skim milk powder, 1 g/L peptone, 0.01 g/L bromocresol purple, 13 g/L agar-agar) is prepared and 5 µl of the supernatant **SN_{AI}** [adjusted to OD 1] is dropped on the agar plates.

**(A-I-4)** The agar plates are incubated at 37 °C for 24 h. Through the indicator dye in the agar medium, the elucidation by protease degradation is directly visible. The protease activity is indicated by the extent (area) of the circular / elliptic clearance zone (also referred to as "halo of degradation"). For the determination of the area of the clearance zone, the diameter (in case of a circular clearance zone) or the average value of the largest and smallest diameter (in case of an elliptic circular clearance zone) may be determined. Values are assessed in millimeters (mm) of two independent biological replicates.

**(A-I-5)** As blank control, steps **(A-I-1)** and **(A-I-2)** is repeated except that no **Strain X_{AI}** is cultivated in **TSB.** In step **(A-I-2)**, the filter-sterile supernatant is diluted in the same ratio as for the tested **Strain X_{AI}** to give blank culture medium **CM_{AI,Blank}**. Then, step **(A-I-3)** is repeated with the difference that 5 µl of **CM_{AI,Blank}** is dropped on 10% Crossley milk agar **CM0213** prepared as described in step **(A-I-3)** and incubated for 24 h at 37 °C.

**(A-I-6)** Optionally, as further control and for quantification of the protease activity, the area of a clearance zone may be compared to the area of a clearance zone of a pure protease from *Bacillus licheniformis* with protease activity of known quantity (typically in the range of from 7-15 U/mg). For this, pure protease from *Bacillus licheniformis* (Subtilisin, P5380, Sigma-Aldrich) is concentrated to 1 mg/ml, and steps **(A-I-3)** and **(A-I-4)** are repeated, except that instead of 5 µl of **SN_{AI},** 5 µl of the 1 mg/ml solution is used (typically having a calculated activity of around 35-75 µU). By this, area size of a clearance zone may be correlated to the quantitative value of protease activity.

Qualitative assessment of protease activity according to **Assay A-I**:
Any **Strain X_{AI}** that shows visible protease degradation in step **(A-I-4)** of **Assay A** is, in particular, deemed to exhibit the respective protease activity according to the invention. Preferably, any **Strain X_{A}** that in step **(A-I-4)** shows a clearance zone the area of which is larger, preferably at least 10 %, more preferably at least 50 % larger, than the area of the clearance zone observed for **CM_{AI,Blank}** in step **(A-I-5)** is deemed to exhibit protease activity.

### VII. 1.2 Assays B-I, B-II

**Assay B-I** is the preferred quantitative test for assessing the protease activity of *Bacillus velezensis* DSM 34978 (hereinafter *"Mutant* 34978") with respect to the protease activity of *Bacillus velezensis* DSM 34978.

In **Assay B-I**, the following steps are carried out:
**(B-I-1)** Steps **(A-I-1)** to **(A-I-4)** of **Assay A-I** are carried out, wherein **Strain X_{AI}** = *Bacillus velezensis* DSM 34978. The area of the clearance zone obtained at the end of step **(A-I-4)** is measured and abbreviated as "**H_{BI1}**". Step **(B-I-1)** is repeated three times and the average value of the three measurements of **H_{BI1}** is calculated and abbreviated as "**Ĥ_{BI1}**".

**(B-I-2)** Steps **(A-I-1)** to **(A-I-4)** of **Assay A-I** are carried out, wherein **Strain X_{AI}** = *Mutant* 34978. The area of the clearance zone obtained at the end of step **(A-I-4)** is measured and abbreviated as **"H_{BI2}**". Step **(B-I-2)** is repeated three times and the average value of the three measurements of **H_{BI2}** is calculated and abbreviated as "**Ĥ_{BI2}**".

The percentage of protease activity **Aₚᵣₒₜ** exhibited by the *Mutant* 34978 with respect to *Bacillus velezensis* DSM 34978 is then calculated by **Aₚᵣₒₜ** = (**Ĥ_{BI2}**/ **Ĥ**_{BI1})*100.

**Assay B-II** is the preferred quantitative test for assessing the protease activity of a mutant of *Bacillus velezensis* DSM 34674 (hereinafter *"Mutant* 34674") with respect to the protease activity of *Bacillus velezensis* DSM 34674.

In **Assay B-II,** the following steps are carried out:
**(B-II-1)** Steps **(A-I-1)** to **(A-I-4)** of **Assay A-I** are carried out, wherein **Strain X_{AI}** = *Bacillus velezensis* DSM 34674. The area of the clearance zone obtained at the end of step **(A-I-4)** is measured and abbreviated as "**H_{BII1}**". Step **(B-II-1)** is repeated three times and the average value of the three measurements of **H_{BII1}** is calculated and abbreviated as "**Ĥ_{BII1}**".

**(B-II-2)** Steps **(A-I-1)** to **(A-I-4)** of **Assay A-I** are carried out, wherein **Strain X_{AI}** = *Mutant* 34674. The area of the clearance zone obtained at the end of step **(A-I-4)** is measured and abbreviated as "**H_{BII2}**". Step **(B-II-2)** is repeated three times and the average value of the three measurements of **H_{BII2}** is calculated and abbreviated as "**Ĥ_{BII2}**".

The percentage of protease activity **A_{Prot}** exhibited by the *Mutant* 34674 with respect to *Bacillus velezensis* DSM 34674 is then calculated by **A_{Prot}** = (**Ĥ_{BII2}**/ **Ĥ_{BII1}**)*100.

### VII.1.3 Assay A-II

**Assay A-II** may be carried out by the following steps to determine whether a given **Strain X_{AII}**, for example *Priestia megaterium* DSM 34980 or a given mutant of *Priestia megaterium* DSM 34980, exhibits protease activity.

**Assay A-II** may be used to determine total protease activity of an organism (i.e. protease activity that is secreted and cell-bound).

**(A-II-1) Strain X_{AII}** is cultivated on **TSA** agar medium ("**TSA**"; peptone from casein 17.0 g/L; peptone from soymeal 3.0 g/L; D(+) glucose monohydrate 2.5 g/L; sodium chloride 5.0 g/L; di-potassium hydrogen phosphate 2.5 g/L) at 37 °C for 16 h. Then the **strain X_{AII}** is collected and washed three times with phosphate-buffered saline ("**PBS**"; Sigma, P4417-100TAB) at 8000 x g centrifugation, 3 min, room temperature, and adjusted by dilution with **PBS** to an optical density (OD at λ = 600) equal to 0.1. The thus obtained suspension is then used as culture medium **CM_{AII}** in step **(A-II-2).**

**(A-II-2)** Skim milk agar medium (skim milk powder 28 g/L, casein enzymatic hydrolysate 5 g/L, yeast extract 2.5 g/L, glucose 1 g/L, agar 15 g/L) is prepared and 10 µl of the respective culture medium **CM_{AII}** is dropped on the agar plates.

**(A-II-3)** The agar plates are incubated at room temperature for 7 d. The protease activity is indicated by the extent (area) of the circular / elliptic clearance zone (also referred to as "halo of degradation"). For the determination of the area of the clearance zone, the diameter (in case of a circular clearance zone) or the average value of the largest and smallest diameter (in case of an elliptic circular clearance zone) may be determined. Values are assessed in millimeters (mm) of two independent biological replicates.

**(A-II-4)** As blank control, step **(A-II-2)** and step **(A-II-3)** is repeated except that **PBS** medium free of any **Strain X_{AII}** is cultivated on skim milk agar medium.

**(A-II-5)** Optionally, as further control and for quantification of the protease activity, the area of a clearance zone may be compared to the area of a clearance zone of a pure protease from *Bacillus licheniformis* with protease activity of known quantity (typically in the range of from 7-15 U/mg). For this, pure protease from *Bacillus licheniformis* (subtilisin, P5380, Sigma-Aldrich) is concentrated to 1 mg/ml, and steps **(A-II-2)** and **(A-II-3)** are repeated, except that instead of 10 µl of **CM_{AII}**, 5 µl of the 1 mg/ml solution is used (typically having a calculated activity of around 35-75 µU). By this, area size of a clearance zone may be correlated to the quantitative value of protease activity.

Qualitative assessment of protease activity according to **Assay A-II**:
Any **Strain X_{AII}** that shows visible protease degradation in step **(A-II-4)** of **Assay A-II** is, in particular, deemed to exhibit the respective protease activity according to the invention. Preferably, any **Strain X_{AII}** that in step **(A-II-3)** shows a clearance zone the area of which is larger, preferably at least 10 %, more preferably at least 50 % larger, than the area of the clearance zone observed for the blank control in step **(A-II-4)** is deemed to exhibit protease activity.

### VII.1.4 Assays B-III

**Assay B-III** is the preferred quantitative test for assessing the protease activity of a mutant of *Priestia megaterium* DSM 34980 (hereinafter *"Mutant* 34980") with respect to the protease activity of *Priestia megaterium* DSM 34980.

In **Assay B-III,** the following steps are carried out:
**(B-III-1)** Steps **(A-II-1)** to **(A-II-3)** of **Assay A-II** are carried out, wherein **Strain X_{AII}** = *Priestia megaterium* DSM 34980. The area of the clearance zone obtained at the end of step **(A-II-3)** is measured and abbreviated as "**H_{BIII1}**". Step **(B-III-1)** is repeated three times and the average value of the three measurements of **H_{BIII1}** is calculated and abbreviated as "**Ĥ_{BIII1}**".

**(B-III-2)** Steps **(A-II-1)** to **(A-II-3)** of **Assay A-II** are carried out, wherein **Strain X_{AII}** = *Mutant* 34980. The area of the clearance zone obtained at the end of step **(A-II-3)** is measured and abbreviated as "**H_{BIII2}**". Step **(B-III-2)** is repeated three times and the average value of the three measurements of **H_{BIII2}** is calculated and abbreviated as "**Ĥ_{BIII2}**".

The percentage of protease activity **A_{Prot}** exhibited by the *Mutant* 34980 with respect to *Priestia megaterium* DSM 34980 is then calculated by **A_{Prot}** = (**Ĥ_{BII2}**/ **Ĥ_{BII1}**)*100.

### VII.2 Cellulase activity: Assay C and Assays D-I,D-II

"Cellulase activity" in the sense of the present invention is deemed to be an activity that catalyzes the hydrolytic cleavage of the β-1,4-glykosidic bond of cellulose.

The prior art describes different assays to qualitatively and quantitatively determine cellulase activity, for example in Wood & Mahalingeshwara Bhat, Methods in Enzymology 1988, 160, 87-112.

**Assay C** is the preferred qualitative test for cellulase activity according to the invention.

Whether a mutant of *Bacillus velezensis* DSM 34978 or a mutant of *Bacillus velezensis* DSM 34674 exhibit "cellulase activity" is preferably determined by the following **Assay C.**

In order to determine the cellulase activity (in %) of a given mutant of *Bacillus velezensis* DSM 34978 related to the cellulase activity of *Bacillus velezensis* DSM 34978, **Assay D-I** is preferably used.

In order to determine the cellulase activity (in %) of a given mutant of *Bacillus velezensis* DSM 34674 related to the cellulase activity of *Bacillus velezensis* DSM 34674, **Assay D-II** is preferably used.

### VII.2.1 Assay C

**Assay C** may be carried out by the following steps to determine whether a given **Strain Xc,** for example *Bacillus velezensis* DSM 34978 or a given mutant of *Bacillus velezensis* DSM 34978, or *Bacillus velezensis* DSM 34674 or a given mutant of *Bacillus velezensis* DSM 34674, exhibit cellulase activity.

**(C-1) Strain X**_{C} is incubated in a pre-culture in 10 ml **VIB** medium (Difco Veal Infusion Broth, Ref. 234420) at 37 °C and 200 rpm for 16 h.

**(C-2)** After the incubation, a further culture of 2.5 ml **CMC** Growth Medium [minimal-salt medium with 10 g/L carboxymethyl cellulose (Sigma 419311)] is inoculated with 125 µl of the pre-culture medium using a small deep-well incubation plate. After inoculation, the further culture is incubated at 37 °C for 24 h at 300 rpm. After 24 h, the OD of the further culture is measured at λ = 660 nm.

**(C-3)** Then, the supernatant **SN_{C}** of the further culture is harvested by centrifugating of 1 ml aliquot of the further culture in a 1.5 ml Eppendorf tube for 5 min at 13000 rpm.

**(C-4)** 500 µl of the supernatant **SN_{C}** are subjected to a cellulase activity test using the EnzChek^{®} Cellulase kit from Molecular Probes/Life technologies according to the manufacturer's instructions (E33953). The principle of this test kit is based on the cellulase-catalyzed conversion of the EnzChek^{®} Cellulase Substrate, blue fluorescent, 339/452, whereby the fluorescence is enhanced. The detection limit is 4 mU/ml to 3 U/ml. To prepare a standard curve for quantification, a 2 U/ml cellulase from *Aspergillus niger* (Sigma-Aldrich, C1184) and dilutions thereof are prepared.

The measurement is performed in black 96-well microtiter plates (Greiner FIA-Plate, black 96K, F-form, 655076-225) and in the Tecan plate reader infinite M1000 Pro. Subsequently, the fluorescence is measured at an excitation of λ = 339 nm and an emission of λ = 452 nm. The cellulase activity is calculated based on the standard curve and the activity is normalized to OD1 (λ = 660 nm) to normalize over cell growth differences after 24 hours incubation at the end of step **(C-2).**

**(C-5)** As blank control, steps **(C-1)** to **(C-4)** are repeated except that the preculture (and logically also the further culture) are cultivated without **Strain Xc** and at the end of step **(C-3),** a blank supernatant **SN_{C,Blank}** is obtained. Step **(C-4)** is repeated with the difference that 500 µl **SN_{C,Blank}** is used instead of 500 µl of **SNc.**

Qualitative assessment of cellulase activity according to **Assay C:**
Any **Strain X_{C}**, the supernatant **SN_{C}** of which shows a higher, preferably at least 10 % higher, more preferably at least 50 % higher, cellulase activity than **SN_{C,Blank}** in step **(C-4)** of **Assay C** is, in particular, deemed to exhibit cellulase activity according to the invention.

### VI.2.2 Assays D-I, D-II

**Assay D-I** is the preferred quantitative test for assessing the cellulase activity of a mutant of *Bacillus velezensis* DSM 34978 (hereinafter *"Mutant* 34978") with respect to the cellulase activity of *Bacillus velezensis* DSM 34978.

In **Assay D-I**, the following steps are carried out:
**(D-I-I)** Steps **(C-1)** to **(C-4)** of **Assay C** are carried out, wherein **Strain X_{C}** = *Bacillus velezensis* DSM 34978. The cellulase activity in step **(C-4)** is measured and abbreviated as "**H_{CI1}**". Step **(D-I-1)** is repeated three times, and the average value of the three measurements of **H_{CI1}** is calculated and abbreviated as "**Ĥ_{CI1}**".

**(D-I-2)** Steps **(C-1)** to **(C-4)** of **Assay C** are carried out, wherein **Strain X_{C}** = *Mutant* 34978. The cellulase activity in step **(C-4)** is measured and abbreviated as "**H_{CI2}**". Step **(D-I-2)** is repeated three times and the average value of the three measurements of **H_{CI2}** is calculated and abbreviated as "**Ĥ_{CI2}**".

The percentage of cellulase activity **A_{Cell}** exhibited by the *Mutant* 34978 with respect to *Bacillus velezensis* DSM 34978 is then calculated by **A_{Cell}** = (**Ĥ_{CI2}**/ **Ĥ_{CI1}**)*100.

**Assay D-II** is the preferred quantitative test for assessing the cellulase activity of a mutant of *Bacillus velezensis* DSM 34674 with respect to the cellulase activity of *Bacillus velezensis* DSM 34674.

In **Assay D-II**, the following steps are carried out:
**(D-II-1)** Steps **(C-1)** to **(C-4)** of **Assay C** are carried out, wherein **Strain X_{C}** = *Bacillus velezensis* DSM 34674. The cellulase activity in step **(C-4)** is measured and abbreviated as "**H_{BII1}**". Step **(D-II-1)** is repeated three times and the average value of the three measurements of **H_{CII1}** is calculated and abbreviated as "**Ĥ_{CII1}**".

**(D-II-2)** Steps **(C-1)** to **(C-4)** of **Assay C** are carried out, wherein **Strain X_{C}** = *Mutant* 34674. The area of the clearance zone obtained at the end of step **(C-4)** is measured and abbreviated as "**H_{CII2}**". Step **(D-II-2)** is repeated three times and the average value of the three measurements of **H_{CII2}** is calculated and abbreviated as "**Ĥ_{CII2}**".

The percentage of cellulase activity **A_{Cell}** exhibited by the *Mutant* 34674 with respect to *Bacillus velezensis* DSM 34674 is then calculated by **A_{Cell}** = (**Ĥ_{CII2}**/ **Ĥ_{CII1}**)*100.

### VII.3 Amylase activity: Assay E and Assays F-I, F-II:

"Amylase activity" in the sense of the present invention is deemed to be an activity that catalyzes the hydrolytic cleavage of a glycosidic bond within a polysaccharide such as starch ("glycosidic enzymatic activity").

The prior art describes different assays to qualitatively and quantitatively determine amylase activity, for example in Oliveira et al., MethodsX 2019, 6, 246-258.

**Assay E** is the preferred qualitative test for amylase activity according to the invention.

Whether a mutant of *Bacillus velezensis* DSM 34978 exhibits "amylase activity" is preferably determined by the following **Assay E.**

Whether a mutant of *Bacillus velezensis* DSM 34674 exhibits "amylase activity" is preferably determined by the following **Assay E.**

In order to determine the amylase activity (in %) of a given mutant of *Bacillus velezensis* DSM 34978 related to the amylase activity of *Bacillus velezensis* DSM 34978, **Assay F-I** is preferably used.

In order to determine the amylase activity (in %) of a given mutant of *Bacillus velezensis* DSM 34674 related to the amylase activity of *Bacillus velezensis* DSM 34674, **Assay F-II** is preferably used.

### VI.3.1 Assay E

**Assay E** may be carried out by the following steps to determine whether a given **Strain Xε,** for example *Bacillus velezensis* DSM 34978 or a given mutant of *Bacillus velezensis* DSM 34978, or *Bacillus velezensis* DSM 34674 or a given mutant of *Bacillus velezensis* DSM 34674, exhibit amylase activity.

**(E-1) Strain X_{E}** is cultivated in 10 ml tryptic-soy-broth ("**TSB**"; Merck - 105459; peptone from casein 17.0 g/L; peptone from soymeal 3.0 g/L; D(+) glucose monohydrate 2.5 g/L; sodium chloride 5.0 g/L; di-potassium hydrogen phosphate 2.5 g/L), wherein the **TSB** medium further contains 2.5 g/L soluble starch (Merck) at 37 °C and 200 rpm for 16 h. As inoculum, 20 µl of a cryo-conservation culture is used for each **strain X_{E}**.

**(E-2)** After the overnight cultivation, the optical density is measured at λ = 660 nm using a cuvette photometer and a culture volume for OD1 (in 1 ml) is obtained by centrifugation (5000 x g, 3 min, room temperature). The filter-sterile supernatant (abbreviated as **SN_{E}**) is obtained, adjusted (by dilution with **TSB** medium) to OD1 and sterilized using a 0.2 µm filter and then used for the qualitative amylase test on specific selective agar plates [step **(E-3)**].

**(E-3)** Starch agar plates are prepared by using 37 g/L Luria-Bertani ("**LB**") agar (Merck-110283) with 10 g/L soluble starch (Merck, CAS: 9005-84-9). 5 µl of the supernatant **SN_{E}** is dropped on the agar plates.

**(E-4)** The plates are incubated at 37 °C for 24 h, and then flooded with 10 % Lugol's solution (5 g iodine, 10 g potassium iodide in 10 ml distilled water), which is removed directly thereafter. The amylase activity is indicated by the extent (area) of the circular / elliptic clearance zone (also referred to as "halo of degradation"). For the determination of the area of the clearance zone, the diameter (in case of a circular clearance zone) or the average value of the largest and smallest diameter (in case of an elliptic circular clearance zone) may be determined. Values are assessed in millimeters (mm) of two independent biological replicates.

**(E-5)** As blank control, step **(E-1)** is repeated except that no **Strain Xε** is cultivated in **TSB.** The filter-sterile supernatant is diluted in the same ratio as in the case of the **Strain Xε** in step **(E-2)** to be tested to give blank supernatant **SN_{E,Blank}**. Then, step **(E-3)** is repeated with the difference that 5 µl of **SN_{E,Blank}** is dropped on the agar plate prepared as described in step **(E-3)** and incubated for 24 h at 37 °C.

**(E-6)** Optionally, as further control and for quantification of the amylase activity, the area of a clearance zone may be compared to the area of a clearance zone of a pure α-amylase from *Bacillus licheniformis* (CAS: 9000-85-5) with an amylase activity of known quantity. For this, steps **(E-3)** and **(E-4)** are repeated, except that instead of 5 µl of **SN_{E}**, 5 µl of a prepared solution of the α-amylase (0.1 mg/ml) is used. By this, the area size of a clearance zone may be correlated to a quantitative value of amylase activity.

Qualitative assessment of amylase activity according to **Assay E:**
Any **Strain X_{E}** that shows visible amylase activity in step **(E-4)** of **Assay E** is, in particular, deemed to exhibit amylase activity according to the invention. Preferably, any **Strain Xε** that in step **(E-4)** shows a halo of degradation the area of which is larger, preferably at least 10 %, more preferably at least 50 % larger, than the area of the clearance zone observed for **SN_{E,Blank}** in step **(E-5)** is deemed to exhibit amylase activity.

### VI.3.2 Assays F-I, F-II

**Assay F-I** is the preferred quantitative test for assessing the amylase activity of a mutant of *Bacillus velezensis* DSM 34978 (hereinafter *"Mutant* 34978") with respect to the amylase activity of *Bacillus velezensis* DSM 34978.

In **Assay F-I**, the following steps are carried out:
**(F-I-1)** Steps **(E-1)** to **(E-4)** of **Assay E** are carried out, wherein **Strain Xε** = *Bacillus velezensis* DSM 34978. The area of the clearance zone obtained at the end of step **(E-4)** is measured and abbreviated as "**H_{EI1}**". Step **(F-I-1)** is repeated three times and the average value of the three measurements of **H_{EI1}** is calculated and abbreviated as **"Ĥ_{EI1}".**

**(F-I-2)** Steps **(E-1)** to **(E-4)** of **Assay E** are carried out, wherein **Strain Xε** = *Mutant* 34978. The area of the clearance zone obtained at the end of step **(E-4)** is measured and abbreviated as "**H_{EI2}**". Step **(F-I-2)** is repeated three times and the average value of the three measurements of **H_{EI2}** is calculated and abbreviated as "**Ĥ_{EI2}**".

The percentage of amylase activity **A_{Amy}** exhibited by the *Mutant* 34978 with respect to *Bacillus velezensis* DSM 34978 is then calculated by **A_{Amy}** = (**Ĥ_{EI2}**/ **Ĥ_{EI1}**)*100.

**Assay F-II** is the preferred quantitative test for assessing the amylase activity of a mutant of *Bacillus velezensis* DSM 34674 with respect to the amylase activity of *Bacillus velezensis* DSM 34674.

In **Assay F-II**, the following steps are carried out:
**(F-II-1)** Steps **(E-1)** to **(E-4)** of **Assay E** are carried out, wherein **Strain Xε** = *Bacillus velezensis* DSM 34674. The area of the clearance zone obtained at the end of step **(E-4)** is measured and abbreviated as "**H_{EII1}**". Step **(F-II-1)** is repeated three times and the average value of the three measurements of **H_{EII1}** is calculated and abbreviated as "**Ĥ_{EII1}**".

**(F-II-2)** Steps **(E-1)** to **(E-4)** of **Assay E** are carried out, wherein **Strain Xε** = *Mutant* 34674. The area of the clearance zone obtained at the end of step **(E-4)** is measured and abbreviated as "**H_{EII2}**". Step **(F-II-2)** is repeated three times and the average value of the three measurements of **H_{EII2}** is calculated and abbreviated as "**Ĥ_{EII2}**".

The percentage of amylase activity **A_{Amy}** exhibited by the *Mutant* 34674 with respect to *Bacillus velezensis* DSM 34674 is then calculated by **A_{Amy}** = (**Ĥ_{EII2}**/ **Ĥ_{EII1}**)*100.

### VII.4 Lipase activity: Assays G, H

"Lipase activity" in the sense of the present invention is deemed to be an activity that catalyzes the hydrolytic cleavage of an ester bond within triglycerides, diglycerides, and monoglycerides. P. Chandra et al., Microb Cell Fact. 2020, 19, 169 review the applications of microbial lipases, for example in waste water treatment and as cleaning agents.

The prior art describes different assays to qualitatively and quantitatively determine lipase activity, for example in Guo et al., Enzyme and Microbial Technology 2015, 71, 8-12; Javed et al. Prog Biophys Mol Biol. 2018 :132, 23-34 (doi: 10.1016/j.pbiomolbio.2017.07.014.).

**Assay G** is the preferred qualitative test for lipase activity according to the invention.

Whether a mutant of *Bacillus subtilis* DSM 34981 exhibits "lipase activity" is preferably determined by the following **Assay G.** A variety of para-nitrophenyl ("**p-NP**") esters having two to 16 carbon atoms (p-NP acetate to p-NP palmitate) in their fatty acid side chain can be hydrolyzed by bacterial lipases (Javed et al., Progress in Biophysics and Molecular Biology 2018, 132, 23-34). In **Assay G** the lipase activity is measured by the amount of free **p-NP** which is the product of the hydrolysis of 4-nitrophenyloctanonate ("**4-NP-C8**"). In **Assay G,** free **p-NP** is directly measured via a photometric assay.

In order to determine the lipase activity (in %) of a given mutant of *Bacillus subtilis* DSM 34981 related to the lipase activity of *Bacillus subtilis* DSM 34981, **Assay H** is preferably used.

### VII.4.1 Assay G

**Assay G** may be carried out by the following steps to determine whether a given **Strain X_{G}**, for example a given mutant of *Bacillus subtilis* DSM 34981, exhibits lipase activity.

**(G-1) Strain X_{G}** is incubated in a pre-culture in 10 ml tryptic-soy-broth ("**TSB**"; Merck - 105459; peptone from casein 17.0 g/L; peptone from soymeal 3.0 g/L; D(+) glucose monohydrate 2.5 g/L; sodium chloride 5.0 g/L; di-potassium hydrogen phosphate 2.5 g/L), at 37 °C and 200 rpm for 16 h.

**(G-2)** After the incubation, the pre-culture is centrifugated twice and washed with main culture medium **MCM₀** (described in the context of **Assay J**). The main culture is incubated at 37 °C and 200 rpm agitation for 4 hours. Afterwards, the culture is harvested by centrifugation (5000 x g, 3 min, room temperature) and adjusted to OD 0.25 (A = 660 nm) in 180 µl 0.1 M Tris-buffer. 180 µl of this suspension **S_{B}** of bacterial cells is then used in step **(G-4).**

**(G-3)** As stock solution **S_{4NPC8}** for the substrate a 4 mmol/L 4-nitrophenyloctanoate ("**4-NP-C8**"; Sigma-Aldrich, CAS:1956-10-1) is prepared. A solution of **4-NP-C8** in *iso*-propanol is prepared, and this stock solution is further diluted in 0.1 M Tris-buffer pH 8.5 to give **S_{4NPC8}** as follows: 28.8 mg **4-NP-C8** are mixed with 6.25 ml *iso*-propanol, and 18.75 ml 0.1 M Tris-buffer (pH 8.5) are added.

**(G-4)** 180 µl bacterial cell suspension **S_{B}** is mixed with 20 µl **S_{4NPC8}** in a 96-well microliter plate (Greiner bio one, Ref. 655101) and incubated at 25 °C for 60 min in a Tecan spark plate reader. The determination is carried out in the Tecan plate reader at λ = 410 nm. Every 2 min, a measurement is done and after 60 min, the final absorbance at A = 410 nm is used for the evaluation.

For the evaluation, a p-nitrophenol ("**p-NP**") standard series (0 to 10 mmol/L) is prepared and used to quantify the amount of released **p-NP** to determine the lipase activity in the *Bacillus* suspension **S_{B}**. The activity of expressed lipase is given in mU/ml as mean value from two independent biological experiments.

**(G-5)** For the determination of the baseline, a blank solution **S_{G,blank}** of 20 µl substrate solution **S_{4NPC8}** in 180 µl 0.1 M Tris-buffer is used.

As a positive control, different concentrations of a pure lipase from *Candida rugosa* (Sigma-Aldrich, L174) are used.

Qualitative assessment of cellulase activity according to **Assay G:**
Any **Strain X_{G}**, the suspension **S_{B}** of which shows a higher, preferably at least 10 % higher, more preferably at least 50 % higher, lipase activity than **SN_{G,Blank}** in step **(G-4)** of **Assay G** is, in particular, deemed to exhibit lipase activity according to the invention.

### VI. 4.2 Assay H

**Assay H** is the preferred quantitative test for assessing the lipase activity of a mutant of *Bacillus subtilis* DSM 34981 (hereinafter *"Mutant* 34981") with respect to the lipase activity of *Bacillus subtilis* DSM 34981.

In **Assay H,** the following steps are carried out:
**(H-1)** Steps **(G-1)** to **(G-4)** of **Assay G** are carried out, wherein **Strain X_{G}** = *Bacillus subtilis* DSM 34981. The lipase activity in step **(G-4)** is measured and abbreviated as "**H_{H1}**". Step **(H-1)** is repeated three times, and the average value of the three measurements of **H_{H1}** is calculated and abbreviated as "**Ĥ_{H1}**".

**(H-2)** Steps **(G-1)** to **(G-4)** of **Assay G** are carried out, wherein **Strain X_{G}** = *Mutant* 34981. The lipase activity in step **(G-4)** is measured and abbreviated as "**H_{H2}**". Step **(H-2)** is repeated three times and the average value of the three measurements of **H_{H2}** is calculated and abbreviated as "**Ĥ_{H2}**".

The percentage of lipase activity **A_{Lipa}** exhibited by the *Mutant* 34981 with respect to *Bacillus subtilis* DSM 34981 is then calculated by **A_{Lipa}** = (**Ĥ_{H2}**/ **Ĥ_{H1}**)*100.

### VII.5 Valeric acid metabolism rate and ability to grow on valeric acid (1000 ppm): Assay J

### VII. 5.1 Assay J

**Assay J** allows to determine two different properties of a given strain, namely
1) A strain's ability to grow in the presence of a certain concentration of **VA** (exemplified in **Assay J** on the basis of a concentration of 1000 ppm **VA**). This ability to grow is expressed as factor **Γ_{XJ}**, which is positive for those strains that are deemed to be able to grow on **VA** with a certain concentration;
2) The ability to reduce **VA** of a certain maximal concentration (exemplified in **Assay J** on the basis of a concentration of 1000 ppm **VA**). This ability to reduce **VA** may then be quantified by a constant **k_{XJ}**, wherein **k_{XJ} ≤** 1.

### VII.5.1.1 Ability to grow on valeric acid, factor Γ_{XJ}

**Assay J** may be carried out by the following steps to determine whether a given **Strain X_{J}**, for example *B*. *velezensis* DSM 34978 or a given mutant of *B*. *velezensis* DSM 34978, *B*. *velezensis* DSM 34674 or a given mutant of *B*. *velezensis* DSM 34674, *Bacillus licheniformis* DSM 34977 or a given mutant of *Bacillus licheniformis* DSM 34977, *Priestia megaterium* DSM 34980 or a given mutant of *Priestia megaterium* DSM 34980, *Bacillus subtilis* DSM 34981 or a given mutant of *Bacillus subtilis* DSM 34981, grows on valeric acid of a certain concentration **x_{VA}**, in particular of a concentration **x_{VA}** of 1000 ppm.

The concentration of valeric acid in the medium **MCM_{+Val}** that is used in **Assay J** is **x_{VA}** = 1000 ppm. Thus, **Assay J** allows to determine whether a given **Strain X** is able to grow in the presence of 1000 ppm **VA.** "Able to grow in the presence of 1000 ppm **VA"** may be abbreviated as "to grow on 1000 ppm valeric acid" or "to grow on valeric acid (1000 ppm)".

In order to determine the ability to grow at lower or higher, in particular higher, concentrations **x_{VA}** of **VA** (for example **x_{VA}** = 5000 ppm), **Assay J** is easily adapted by substituting **MCM_{+Val}** by another medium **MCM*_{+Val}** which is identical to **MCM_{+Val}**, but has a lower or higher, in particular higher, concentration **x_{VA}** of **VA**. For example, for **x_{VA}** = 5000 ppm, a medium **MCM*_{+Val}** with 5000 ppm **VA** may be used which is obtained by the same procedure as stated below for **MCM_{+Val}**, except that in step 5., 5 g valeric acid are deployed.

If such medium **MCM*_{+Val}** is used in **Assay J, Assay J** allows to determine whether a given **Strain X** is able to grow in the presence of 5000 ppm **VA.** "Able to grow in the presence of 5000 ppm **VA**" may be abbreviated as "to grow on 5000 ppm valeric acid" or "to grow on valeric acid (5000 ppm)".

The same applies, logically, to any other variation of **Assay J** where yet another medium **MCM****_{+Val} with a yet different concentration **x_{VA}** is used. The skilled person knows that such medium is simply obtained by the same procedure as described for **MCM_{+Val}**, by simply adjusting the amount of **VA** used in step 5.

### VII.5.1.2 Rate of metabolization of valeric acid, constant k_{XJ}

**Assay J** may furthermore be carried out by the following steps to determine whether a given **Strain X_{J}**, for example *B*. *velezensis* DSM 34978 or a given mutant of *B*. *velezensis* DSM 34978, *B*. *velezensis* DSM 34674 or a given mutant of *B*. *velezensis* DSM 34674, *Bacillus licheniformis* DSM 34977 or a given mutant of *Bacillus licheniformis* DSM 34977, *Priestia megaterium* DSM 34980 or a given mutant of *Priestia megaterium* DSM 34980, *Bacillus subtilis* DSM 34981 or a given mutant of *Bacillus subtilis* DSM 34981 is able to metabolize (and thus reduce) valeric acid of a certain concentration **x_{VA}**, in particular a concentration of **x_{VA}** = 1000 ppm.

The concentration of valeric acid in the medium **MCM_{+Val}** that is used in **Assay J** is **x_{VA}** = 1000 ppm. Thus, **Assay J** allows to determine the ability to metabolize valeric acid of a given **Strain X,** wherein the concentration of **VA** is **x_{VA}** = 1000 ppm. "Ability to metabolize valeric acid [...], wherein the concentration of **VA** is 1000 ppm" may be abbreviated as "able to metabolize 1000 ppm valeric acid" or "able to metabolize valeric acid (1000 ppm)".

In order to determine the ability to metabolize **VA** at lower or higher, in particular higher, concentrations **x_{VA}** of **VA** (for example **x_{VA}** = 5000 ppm), **Assay J** is easily adapted by substituting **MCM_{+Val}** by another medium **MCM*_{+Val}** which is identical to **MCM_{+Val}**, but has a lower or higher, in particular higher, concentration **x_{VA}** of **VA.** For example, for **x_{VA}** = 5000 ppm, a medium **MCM*_{+Val}** with 5000 ppm **VA** may be used which is obtained by the same procedure as stated below for **MCM_{+Val}**, except that in step 5., 5 g valeric acid are deployed.

If such medium **MCM*_{+Val}** is used in **Assay J, Assay J** allows to determine whether a given **Strain X** is able to metabolize 5000 ppm **VA.** "Ability to metabolize valeric acid [...], wherein the concentration of **VA** is 5000 ppm" may be abbreviated as "able to metabolize 5000 ppm valeric acid" or "able to metabolize valeric acid (5000 ppm)".

The same applies, logically, to any other variation of **Assay J** where yet another medium **MCM****_{+Val} with a yet different concentration **x_{VA}** is used. The skilled person knows that such medium is simply obtained by the same procedure as described for **MCM_{+Val}**, by simply adjusting the amount of **VA** used in step 5.

### VII.5.1.3 Steps according to Assay J

The following culture media **MCM_{+Val}** and **MCM₀** are prepared:
**MCM_{+Val}** is a main culture medium containing valeric acid obtained by combining the following solutions/ substances:
1. 200 g aqueous salt solution 1 with 20 g/L KH₂PO₄, 61 g/L K₂HPO₄, 16 g/L (NH₄)₂SO₄;
2. 10 g aqueous salt solution 2 with 0.176 g/L Mn(II)SO₄*H₂O and 12.3 g/L MgSO₄*H₂O;
3. 10 g of an aqueous ammonium iron (III) citrate solution with a concentration of 2.2 g/L of ammonium iron (III) citrate;
4. 20 g of a 50 % (*w*/*v*) aqueous glucose monohydrate solution (50 g glucose monohydrate are dissolved in water to give a final volume of aqueous solution of 100 ml);
5. 1 g valeric acid (Sigma-Aldrich, 240370).

The stocks 1. to 5. are combined and filled up to 1 L; the pH is adjusted to 7.

**MCM₀** is a main culture medium containing no valeric acid obtained by combining the stocks 1. to 4. and filling them up to 1 L; the pH is adjusted to 7.

In **Assay J,** the following steps are carried out:
**(J-1) Strain X_{J}** is incubated in a pre-culture **PC_{J}** in 10 ml tryptic-soy-broth ["**TSB**"; Merck - 105459; peptone from casein 17.0 g/L; peptone from soymeal 3.0 g/L; D(+) glucose monohydrate 2.5 g/L; sodium chloride 5.0 g/L; di-potassium]. **PC_{J}** is then divided one half each, **PC_{J+}** and **PC_{J-}.**

**(J-2) PC_{J+}** and **PC_{J-}** are each centrifugated and the respective pellet is redispersed in aqueous 0.9 % (*w*/*v*) NaCl solution ("aqueous 0.9 % (*w*/*v*) NaCl solution" means that 0.9 g NaCl are dissolved in water to give a final volume of aqueous solution of 100 ml). Then, each dispersion is centrifugated for 5 min at 5000 x g and the NaCl solution is removed from the obtained pellet (1^{st} washing).

**(J-3)** Each pellet is redispersed in fresh aqueous 0.9 % (*w*/*v*) NaCl solution. Then, each dispersion is centrifugated for 5 min at 5000 x g and the NaCl solution is removed (2^{nd} washing). The pellet from **PC_{J+}** is used in step **(J-4).** The pellet from **PC_{J-}** is used in step **(J-5).**

**(J-4)** The washed pellet from **PCj+** from step **(J-3)** is diluted with main culture medium **MCM_{+Val}** until an OD of 0.1 at λ = 660 nm (**v_{Start}** = OD 0.1). **MCM_{+Val}** contains valeric acid (Sigma-Aldrich, 240370) in a concentration of **c₀** = 1 g/L. The incubation is at 30 °C with 225 rpm for 48 h in a small cultivation system (Woulter-Deutz). Then, the OD at λ = 660 nm is measured ("**v_{+Val}**"). Then, the culture is centrifugated at 13000 x g for 3 minutes at room temperature ("**RT**") and subsequently the supernatant **SN_{+Val}** is harvested. The concentration **c_{+Val}** of valeric acid in **SN_{+Val}** is quantified using an analytical method.

**(J-5)** The washed pellet from **PC_{J-}** from step **(J-3)** is diluted with main culture medium **MCM₀** until an OD of 0.1 at λ = 660 nm (**v_{Start}** = OD 0.1). **MCM₀** comprises no valeric acid. The incubation is at 30 °C with 225 rpm for 48 h in a small cultivation system (Woulter-Deutz). Then, the OD at λ = 660 nm is measured ("**v_{-Val}**"). Then, the culture is centrifugated at 13000 x g for 3 minutes at **RT** and subsequently the supernatant **SN_{-Val}** is harvested. The concentration **c_{-Val}** of valeric acid in **SN_{-Val}** is quantified using an analytical method (this may be made as a control and/or standardization, since the concentration should be **c_{-Val}**= 0).

**(J-6)** For a blank control, the same volume of main culture medium **MCM_{+Val}** as in step **(J-4),** but without **Strain X_{J}**, is incubated at 30 °C with 225 rpm for 48 h in a small cultivation system (Woulter-Deutz). Then, the culture medium is centrifugated at 13000 x g for 3 minutes at **RT** and subsequently the supernatant **SN_{Blank}** is harvested. The concentration **c_{Val0}** of valeric acid in **SN_{Blank}** is quantified using an analytical method as a control.

### VII.5.1.4 Evaluation of Assay J

### VII.5.1.4.1 ...at 1000 ppm VA

Where **Assay J** is carried out with **MCM+vai,** namely a concentration of valeric acid of **x_{VA}** = 1000 ppm, the following evaluation may be made:
→ If **v_{+Val}** is higher than **v_{-Val}** , then **X_{J}** is deemed to grow on 1000 ppm valeric acid according to the invention. The improved growth of **X_{J}** on valeric acid medium (1000 ppm) may then be expressed as factor **Γ**_{**XJ**; **1000 ppm**} = **v_{+Val}** / **v_{-Val}**. In particular, according to the invention, in order for a strain **X_{J}** to be able to grow on valeric acid medium (1000 ppm), the condition **Γ**_{**XJ**; **1000ppm**} > 1 is fulfilled.
→ If the concentration of valeric acid **c_{+Val}** in **SN_{+Val}** is lower than the concentration of valeric acid **c_{Val0}** in **SN_{Blank}**, then **X_{J}** is able to metabolize valeric acid (1000 ppm). The rate of metabolization of valeric acid (1000 ppm) by **X_{J}** may then be expressed as constant **k**_{**XJ**; **1000 ppm**} = 1 - **c_{+Val}** / **c_{Val0}**.

The effectiveness **E_{XJ; 1000 ppm}** to reduce valeric acid (1000 ppm) of a strain **X_{J}**, in particular a strain **X_{J}** which fulfills the condition "**Γ_{XJ; 1000 ppm}** > 1", may then be expressed as the ratio **E_{XJ; 1000 ppm}** as follows: **E_{XJ; 1000} ppm = k_{XJ; 1000} ppm * Γ_{XJ; 1000} ppm.**

### VII.5.1.4.2 ...at 5000 ppm VA

Where **Assay J** is carried out with another medium **MCM*_{+Val}** instead of **MCM_{+Val}**, namely at a concentration of valeric acid of **x_{VA}** = 5000 ppm, the following evaluation may be made:
→ If **v_{+Val}** is higher than **v_{-Val}** , then **X_{J}** is deemed to grow on 5000 ppm valeric acid according to the invention. The improved growth **of X_{J}** on valeric acid medium (5000 ppm) may then be expressed as factor **Γ**_{**XJ**; **5000 ppm**} = **v_{+Val}** / **v_{-Val}**. In particular, according to the invention, in order for a strain **X_{J}** to be able to grow on valeric acid medium (5000 ppm), the condition **Γ**_{**XJ**; **5000 ppm**} > 1 is fulfilled.
→ If the concentration of valeric acid **c_{+Val}** in **SN_{+Val}** is lower than the concentration of valeric acid **c_{Val0}** in **SN_{Blank}**, then **X_{J}** is able to metabolize valeric acid (5000 ppm). The rate of metabolization of valeric acid (5000 ppm) by **X_{J}** may then be expressed as constant **k_{XJ; 5000 ppm}** = 1 - **c_{+Val}** / **c_{Val0}**.

The effectiveness **E_{XJ; 5000 ppm}** to reduce valeric acid (5000 ppm) of a strain **X_{J}**, in particular a strain **X_{J}** which fulfills the condition "**Γ_{XJ; 5000 ppm}** > 1", may then be expressed as the ratio **E_{XJ; 5000 ppm}** as follows:
**E_{XJ; 5000} ppm = k_{XJ; 5000} ppm * Γ_{XJ; 5000} ppm.**

### VII.5.1.4.3 ...at a general concentration of VA, i.e. x_{VA}

Where **Assay J** is carried out with another medium **MCM****_{+Val} instead of **MCM_{+Val}**, namely at a concentration of valeric acid of **x_{VA}**, the following evaluation may be made:
→ If **v_{+Val}** is higher than **v_{-Val}** , then **X_{J}** is deemed to grow at **x_{VA}** valeric acid according to the invention. The improved growth of **X_{J}** on valeric acid medium (**x_{VA}**) may then be expressed as factor **Γ_{XJ;xVA}** = **v_{+Val}** / **v_{-Val}**. In particular, according to the invention, in order for a strain **X_{J}** to be able to grow on valeric acid medium (**x_{VA}**), the condition **Γ_{XJ;xVA}** > 1 is fulfilled.
→ If the concentration of valeric acid **c_{+Val}** in **SN_{+Val}** is lower than the concentration of valeric acid **c_{Val0}** in **SN_{Blank}**, then **X_{J}** is able to metabolize valeric acid (**x_{VA}**). The rate of metabolization of valeric acid (**x_{VA}**) by **X_{J}** may then be expressed as constant **kxj; _{xVA}** = 1 - **c_{+Val}** / **c_{Val0}**.

The effectiveness **E_{XJ;XVA}** to reduce valeric acid (**x_{VA}**) of a strain **X_{J}**, in particular a strain **X_{J}** which fulfills the condition "**Γ_{XJ;xVA}** > 1", may then be expressed as the ratio **E_{XJ;XVA}** as follows:
**E_{XJ;XVA} = k_{XJ; 5000 ppm} * Γ_{XJ; 5000} ppm.**

### VII.5.1.5 Analysis of VA concentrations in the context of Assay J

In **Assay J,** the quantification of valeric acid was done by an analytical method as follows:
The respective sample and a series of calibration standards are mixed with an internal standard (2-methylhexanoic acid). An excess of NaCl is added to the sample, and it is then diluted with 1 part 50 wt.-% sulfuric acid to 4 parts sample. The resulting mixture is extracted in a ratio of 1 part diethyl ether to 1.25 parts sample for 10 minutes and then centrifuged for 10 minutes at 3000 g. The organic supernatant is removed and, if necessary, further diluted to a concentration range of 1 g/L. Samples and calibration standards are measured using an Agilent GC 8860 equipped with split/spitless injector and FID detector on a GC column (BP21 30m; ID 0.32 mm; film 0.25 µm) at a flow rate of 1 ml/min He. The separation is carried out by means of a temperature gradient from 130 °C to 180 °C within 8.5 min. The chromatograms are evaluated by means of a multi-point area evaluation linear without zero crossing with 2-methylhexanoic acid as the internal standard. Valeric acid is eluting at 5.4 min within the program.

### VII.5.2 Assays K-I, K-II, K-III

### VII.5.2.1 Assay K-I

**Assay K-I** is the preferred quantitative test for assessing the effectiveness **E_{XJ%}** (**x_{VA}**) to reduce valeric acid of a mutant of *Bacillus licheniformis* DSM 34977 (hereinafter *"Mutant* 34977") relative to the effectiveness to reduce valeric acid of *Bacillus licheniformis* DSM 34977, which may be determined for a given concentration **x_{VA}**, in particular of a concentration **x_{VA}** = 1000 ppm, preferably a concentration **x_{VA}** = 5000 ppm.

In **Assay K-I,** the following steps are carried out:
**(K-I-1)** Steps **(J-1)** to **(J-5)** of **Assay J** are carried out for a given concentration **x_{VA}**, in particular a concentration **x_{VA}** = 1000 ppm, preferably a concentration **x_{VA}** = 5000 ppm, wherein **Strain X_{J}** = *Bacillus licheniformis* DSM 34977. Step **(K-I-1)** is repeated three times, and the average value of the three measurements of **E_{XJ}** = **Γ_{XJ} *k_{XJ}** is calculated and abbreviated as **"Ê_{KI1}".**

**(K-I-2)** Steps **(J-1)** to **(J-5)** of **Assay J** are carried out for a given concentration **x_{VA}**, in particular a concentration **x_{VA}** = 1000 ppm, preferably a concentration **x_{VA}** = 5000 ppm, wherein **Strain X_{J}** = mutant of *Bacillus licheniformis* DSM 34977. Step **(K-I-2)** is repeated three times, and the average value of the three measurements of **E_{XJ}** = **Γ_{XJ}** ***k_{XJ}** is calculated and abbreviated as "**Ê_{KI2}**".

The effectiveness **E_{XJ%}** (**x_{VA}**) exhibited by the *Mutant* 34977 to reduce valeric acid with respect to the effectiveness of *Bacillus licheniforms* DSM 34977 to reduce valeric acid for a given concentration **x_{VA}** is then calculated by **E_{XJ%}** (**x_{VA}**) = (**Ê_{KI2}**/ **Ê_{KI1}**)*100.

### VII.5.2.2 Assay K-II

**Assay K-II** is the preferred quantitative test for assessing the effectiveness **E_{XJ%}** (**x_{VA}**) to reduce valeric acid of a mutant of *Priestia megaterium* DSM 34980 (hereinafter "*Mutant 34980*") relative to the effectiveness to reduce valeric acid of *Priestia megaterium* DSM 34980, which may be determined for a given concentration **x_{VA}**, in particular of a concentration **x_{VA}** = 1000 ppm, preferably a concentration **x_{VA}** = 5000 ppm.

In **Assay K-II,** the following steps are carried out:
**(K-II-1)** Steps **(J-1)** to **(J-5)** of **Assay J** are carried out for a given concentration **x_{VA}**, in particular a concentration **x_{VA}** = 1000 ppm, preferably a concentration **x_{VA}** = 5000 ppm, wherein **Strain X_{J}** = *Priestia megaterium* DSM 34980. Step **(K-II-1)** is repeated three times, and the average value of the three measurements of **E_{XJ}** = **Γ_{XJ} *kxj** is calculated and abbreviated as "**Ê_{KII1}**".

**(K-II-2)** Steps **(J-1)** to **(J-5)** of **Assay J** are carried out for a given concentration **x_{VA}**, in particular a concentration **x_{VA}** = 1000 ppm, preferably a concentration **x_{VA}** = 5000 ppm, wherein **Strain X_{J}** = mutant of *Priestia megaterium* DSM 34980. Step **(K-II-2)** is repeated three times, and the average value of the three measurements of **E_{XJ}** = **Γ_{XJ} *kxj** is calculated and abbreviated as "**Ê_{KII2}**".

The effectiveness **E_{XJ%}** exhibited by the *Mutant* 34980 to reduce valeric acid with respect to the effectiveness of *Priestia megaterium* DSM 34980 to reduce valeric acid for a given concentration **x_{VA}** is then calculated by **E_{XJ%}** = (**Ê_{KI2}**/ **Ê_{KI1}**)*100.

### VII.5.2.3 Assay K-III

**Assay K-III** is the preferred quantitative test for assessing the effectiveness **E_{XJ%}** (**x_{VA}**) to reduce valeric acid of a mutant of *Bacillus subtilis* DSM 34981 (hereinafter *"Mutant* 34981") relative to the effectiveness to reduce valeric acid of *Bacillus subtilis* DSM 34981, which may be determined for a given concentration **x_{VA}**, in particular of a concentration **x_{VA}** = 1000 ppm, preferably a concentration **x_{VA}** = 5000 ppm.

In **Assay K-III,** the following steps are carried out:
**(K-III-1)** Steps **(J-1)** to **(J-5)** of **Assay J** are carried out for a given concentration **x_{VA}**, in particular a concentration **x_{VA}** = 1000 ppm, preferably a concentration **x_{VA}** = 5000 ppm, wherein **Strain X_{J}** = *Bacillus subtilis* DSM 34981. Step **(K-III-1)** is repeated three times, and the average value of the three measurements of **E_{XJ}** = **Γ_{XJ} *kxj** is calculated and abbreviated as **"Ê_{KIII1}".**

**(K-III-2)** Steps **(J-1)** to **(J-5)** of **Assay J** are carried out for a given concentration **x_{VA}**, in particular a concentration **x_{VA}** = 1000 ppm, preferably a concentration **x_{VA}** = 5000 ppm, wherein **Strain X_{J}** = mutant of *Bacillus subtilis* DSM 34981. Step **(K-III-2)** is repeated three times, and the average value of the three measurements of **E_{XJ}** = **Γ_{XJ} *kxj** is calculated and abbreviated as "**Ê_{KIII2}**".

The effectiveness **E_{XJ%}** (**x_{VA}**) exhibited by the *Mutant* 34981 to reduce valeric acid with respect to the effectiveness of *Bacillus subtilis* DSM 34981 to reduce valeric acid for a given concentration **x_{VA}** is then calculated by **E_{XJ%}** (**x_{VA}**) = (**Ê_{KI2}**/ **Ê_{KI1}**)*100.

### VII.5.3 Assays L-I, L-II

### VII.5.3.1 Assay L-I

**Assay L-I** is the preferred quantitative test for assessing **k_{XJ%}** (**x_{VA}**) i.e. the metabolization rate of a valeric acid medium of a mutant of *Bacillus velezensis* DSM 34978 (hereinafter *"Mutant* 34978") relative to the metabolization rate of a valeric acid medium of *Bacillus velezensis* DSM 34978, which may be determined for a given concentration **x_{VA}**, in particular for a concentration **x_{VA}** = 1000 ppm, preferably a concentration **x_{VA}** = 5000 ppm.

In **Assay L-I,** the following steps are carried out:
**(L-I-1)** Steps **(J-1)** to **(J-4)** and **(J-6)** of **Assay J** are carried out for a given concentration **x_{VA}**, in particular a concentration **x_{VA}** = 1000 ppm, preferably a concentration **x_{VA}** = 5000 ppm, wherein **Strain X_{J}** = *Bacillus velezensis* DSM 34978. Step **(L-I-1)** is repeated three times, and the average value of the three measurements of **k_{XJ}** is calculated and abbreviated as **"_{KLI1}".**

**(L-I-2)** Steps **(J-1)** to **(J-4)** and **(J-6)** of **Assay J** are carried out for a given concentration **x_{VA}**, in particular a concentration **x_{VA}** = 1000 ppm, preferably a concentration **x_{VA}** = 5000 ppm, wherein **Strain X_{J}** = mutant of *Bacillus velezensis* DSM 34978. Step **(L-I-2)** is repeated three times, and the average value of the three measurements of **k_{XJ}** is calculated and abbreviated as **"κ_{LI2}".**

The relative metabolization rate **k_{XJ%}** (**x_{VA}**) of valeric acid for a given concentration **x_{VA}** exhibited by the *Mutant* 34978 with respect to the metabolization of valeric acid of *Bacillus velezensis* DSM 34978 is then calculated by **k_{XJ%}** (**x_{VA}**) = (**κ_{LI2}**/ **κ_{LI1}**)*100.

### VII.5.3.2 Assay L-II

**Assay L-II** is the preferred quantitative test for assessing **k_{XJ%}** (**x_{VA}**) i.e. the metabolization rate of a valeric acid medium of a mutant of *Bacillus velezensis* DSM 34674 (hereinafter *"Mutant* 34674") relative to the metabolization rate of a valeric acid medium of *Bacillus velezensis* DSM 34674, which may be determined for a given concentration **x_{VA}**, in particular for a concentration **x_{VA}** = 1000 ppm, preferably a concentration **x_{VA}** = 5000 ppm.

In **Assay L-II,** the following steps are carried out:
**(L-II-1)** Steps **(J-1)** to **(J-4)** and **(J-6)** of **Assay J** are carried out for a given concentration **x_{VA}**, in particular a concentration **x_{VA}** = 1000 ppm, preferably a concentration **x_{VA}** = 5000 ppm, wherein **Strain X_{J}** = *Bacillus velezensis* DSM 34674. Step **(L-II-1)** is repeated three times, and the average value of the three measurements of **k_{XJ}** is calculated and abbreviated as "**κ_{LII1}**".

**(L-II-2)** Steps **(J-1)** to **(J-4)** and **(J-6)** of **Assay J** are carried out for a given concentration **x_{VA}**, in particular a concentration **x_{VA}** = 1000 ppm, preferably a concentration **x_{VA}** = 5000 ppm, wherein **Strain X_{J}** = mutant of *Bacillus velezensis* DSM 34674. Step **(L-II-2)** is repeated three times, and the average value of the three measurements of **k_{XJ}** is calculated and abbreviated as "**κ_{LII2}**".

The relative metabolization rate of valeric acid **k_{XJ%}** (**x_{VA}**) exhibited by the *Mutant* 34674 with respect to the metabolization of valeric acid for a given concentration **x_{VA}** of *Bacillus velezensis* DSM 34674 is then calculated by **k_{XJ%}** (**x_{VA}**) = (**κ_{LII2}**/ **κ_{LII1}**)*100.

### VII.6 Determination of Germination rate: Assay M

In a preferred embodiment of **variant 1/ variant 1*,** the incubation in step c1)/ c1*) is carried out until at least 10 % of the spores **SP** that are comprised by mixture **M₁** have germinated, relative to the number of spores **SP** comprised by mixture **M₁** before mixture **M₁** is subjected to the incubation according to step c1)/ c1*).

In a preferred embodiment of **variant 2/ variant 2*,** the incubation in step c2)/ c2*) is carried out until at least 10 % of the spores **SP** that are comprised by mixture **M₂** have germinated, relative to the number of spores **SP** comprised by mixture **M₂** before mixture **M₂** is subjected to the incubation according to step c2)/ c2*), and

In a preferred embodiment of **variant 3/ variant 3*,** is carried out until at least 10 % of the spores **SP** that are comprised by mixture **M₃** have germinated, relative to the number of spores **SP** comprised by mixture **M₃** mixture **M₃** is subjected to the incubation according to step c3)/ c3*).

The skilled person is aware of methods to determine the amount of spores **SP** in a given mixture **M₁**, **M₂**, or **M₃**, and, depending on the specific application of the method according to the first aspect of the invention, the skilled person can choose the respective methods to be used, such as CFU determination, ammonium iron (II) sulfate assay, counting chamber and cell count by algorithm (cf. R. Biermann, L. Niemeyer, L. Rösner, C. Ude, P. Lindner, I. Bicet, S. Beutel, Eng Life Sci. 2022, 22, 299-307; Hereinafter "Biermann *et al.*")*.*

Methods for counting spores numbers in a given sample are further described by K.C. Alves et al., J. Vis. Exp. (196), e65141, doi:10.3791/65141 (2023).

### VII.6.1 Assay M-I

In particular, the number of the spores **SP** that have germinated after a certain point of time **tₓ** after start of the incubation according to step c1)/ step c1*), relative to the number of spores **SP** comprised by mixture **M₁** that is contacted with **S** in step b1)/ step b1*) [i.e. before mixture **M₁** is subjected to the incubation according to step c1]/ c1*)], may be determined by **Assay M-I** as follows (following Biermann *et al.,* page 301, paragraph 2.4):
**(M-I-1)** A sample S_{M1,t=0} of M₁ is withdrawn from **M₁** at the end of step b1)/ b1*), before incubation according to step c1)/ c1*). Sample **S_{M1,t=0}** may be diluted with 0.9 % (*w*/*v*) aqueous NaCl-solution in those cases were the concentration of spores in the sample turns out to be too high so that no purposeful counting of **CFUs** is possible in steps **(M-I-6)** and **(M-I-7).**
**(M-I-2)** A sample **S**_{**M1,t=**x} of **M₁** is withdrawn from **M₁** at the time of **tₓ** after start of the incubation according to step c1)/ c1*). The volume of S_{M1,t=0} and S_{M1,t=x} are the same. Where sample **S_{M1,t=0}** is diluted with 0.9 % (*w*/*v*) aqueous NaCl-solution, the same dilution ratio is applied to sample **S_{M1,t=x}**.
**(M-I-3)** Sample **S_{M1,t=0}** and sample **S**_{**M1,t=**x} are heated at 80 °C for 30 min at 500 rpm, so only germinated spores occur as colonies on the plates after the incubation according to step **(M-I-5)**.
**(M-I-4)** A given volume (e.g. 100 µl) of sample **S_{M1,t=0}** and the same volume of sample **S**_{**M1,t=**x} are evenly spread on petri dishes using a cell spreader. The petri dishes contain **PNY**-medium prepared in advance and stored at 4 °C. The composition of the **PNY**-medium is as follows:
   in [g*L⁻¹]: peptone (caseine) 5.0, yeast extract 5.0, dextrose 5.0, KH₂PO₄ 0.5, K₂HPO₄ 0.5, MgSO₄ 0.25, MnSO₄ 0.01, FeSO₄ 0.01, NaCl 0.01, CuSO₄ 0.001, ZnSO₄ 0.001, CoSO₄ 0.001; pH 6.0 in purified water] with 1.5 % (*w*/*v*) agar.
**(M-I-5)** Inoculated petri-dishes are incubated at 37 °C for the same amount of time (24 h to 48 h).
**(M-I-6)** For sample **S_{M1,t=0}** the colony forming units ("CFU") are counted and denoted as "**ω_{M1,t=0}**".
**(M-I-7)** For sample **S**_{**M1,t=**x} the colony forming units ("CFU") are counted and denoted as "**ω_{M1,t=x}**".

Steps **(M-I-1)** to **(M-I-7)** and steps **(M-I-2)** to **(M-I-7)** are carried out for three samples of S_{M1,t=0} and S_{M1,t=x}, respectively. The average value of the three measurements of ω_{M1,t=0} and ω_{M1,t=x} are calculated and abbreviated as "**ω_{M1,t=0}**" and "**ω̃_{M1,t=x}**", respectively.

The number **Σ_{M1,t=x}** of spores **SP** (in %) in mixture **M₁** that have germinated at a given point of time **tₓ** after the start of the incubation in step c1)/ c1*), relative to the number of spores **SP** comprised by mixture **M₁** that is contacted with **S** in step b1)/ b1*) is then calculated by **Σ**_{**M1,t**=x} = **[(ω̃_{M1,t=0} - ω_{M1,t=x})/ ω_{M1,t=0}]*100.**

### VII.6.2 Assay M-II

In particular, the number of the spores **SP** that have germinated after a certain point of time **tₓ** after start of the incubation according to step c2)/ c2*), relative to the number of spores **SP** comprised by mixture **M₂** that is contacted with **S** in step b2)/b2*) [i.e. before mixture **M₂** is subjected to the incubation according to step c2]/ c2*)], may be determined by **Assay M-II** as follows (following Biermann *et al.,* page 301, paragraph 2.4):
**(M-II-1)** A sample **S_{M2,t=0}** of **M₂** is withdrawn from **M₂** at the end of step b2)/ b2*), before incubation according to step c2)/ c2*). Sample **S_{M2,t=0}** may be diluted with 0.9 % (*w*/*v*) aqueous NaCl-solution in those cases were the concentration of spores in the sample turns out to be too high so that no purposeful counting of **CFUs** is possible in steps **(M-II-6)** and **(M-II-7).**
**(M-II-2)** A sample **S_{M2,t=x}** of **M₂** is withdrawn from **M₂** at the time of **tₓ** after start of the incubation according to step c2)/ c2*). The volume of **S**_{**M2**,t=0} and **S_{M2,t=x}** are the same. Where sample **S_{M2,t=0}** is diluted with 0.9 % (wlv) aqueous NaCl-solution, the same dilution ratio is applied to sample **S_{M2,t=x}**.
**(M-II-3)** Sample **S**_{**M2**,t=0} and sample **S_{M2,t=x}** are heated at 80 °C for 30 min at 500 rpm, so only germinated spores occur as colonies on the plates after the incubation according to step **(M-II-5).**
**(M-II-4)** A given volume (e.g. 100 µl) of sample **S**_{**M2**,t=0} and the same volume of sample S_{M2,t=x} are evenly spread on petri dishes using a cell spreader. The petri dishes contain **PNY**-medium prepared in advance and stored at 4 °C [the composition of the **PNY**-medium is described above, in the context of step **(M-I-4)**].
**(M-II-5)** Inoculated petri-dishes are incubated at 37 °C for the same amount of time (24 h to 48 h).
**(M-II-6)** For sample **S_{M2,t=0}** the colony forming units **("CFU")** are counted and denoted as "**ω**_{M2,t=0}".
**(M-II-7)** For sample S_{M2,t=x} the colony forming units **("CFU")** are counted and denoted as "**ω_{M2,t=x}**".

Steps **(M-II-1)** to **(M-II-7)** and steps **(M-II-2)** to **(M-II-7)** are carried out for three samples of **S**_{**M2**,t=0} and **S_{M2,t=x}**, respectively. The average value of the three measurements of **ω_{M2,t=0}** and **ω_{M2,t=x}** are calculated and abbreviated as "**ω_{M2,t=0}**" and "**ω̃_{M2,t=x}**", respectively.

The number **Σ_{M2,t=x}** of spores **SP** (in %) in mixture **M₂** that have germinated at a given point of time **tₓ** after the start of the incubation in step c2)/ c2*), relative to the number of spores **SP** comprised by mixture **M₂** that is contacted with **S** in step b2)/ b2*) is then calculated by Σ_{M2,t=x} = [(ω̃_{M2,t=0} - **ω_{M2,t=x}**)/ ω_{M2,t=0}]*100.

### VII. 6.3 Assay M-III

In particular, the number of the spores **SP** that have germinated after a certain point of time **tₓ** after start of the incubation according to step c3)/ c3*), relative to the number of spores **SP** comprised by mixture **M₃** that is contacted with **S** in step b3)/b3*) [i.e. before mixture **M₃** is subjected to the incubation according to step c3]/ c3*)], may be determined by Assay **M-III** as follows (following Biermann *et al.,* page 301, paragraph 2.4):
**(M-III-1)** A sample **S_{M3,t=0}** of **M₃** is withdrawn from **M₃** at the end of step b3), before incubation according to step c3)/c3*). Sample **S**_{**M3,t**=0} may be diluted with 0.9 % (w/v) aqueous NaCl-solution in those cases were the concentration of spores in the sample turns out to be too high so that no purposeful counting of CFUs is possible in steps **(M-III-6)** and **(M-III-7)**.
**(M-III-2)** A sample **S_{M3,t=x}** of **M₃** is withdrawn from **M₃** at the time of **tₓ** after start of the incubation according to step c3)/c3*). The volume of **S**_{**M3,t**=0} and **S_{M3,t=x}** are the same. Where sample **S_{M3,t=0}** is diluted with 0.9 % (wlv) aqueous NaCl-solution, the same dilution ratio is applied to sample **S_{M3,t=x}**.
**(M-III-3)** Sample **S**_{**M3,t**=0} and sample **S_{M3,t=x}** are heated at 80 °C for 30 min at 500 rpm, so only germinated spores occur as colonies on the plates after the incubation according to step **(M-III-5)**.
**(M-III-4)** A given volume (e.g. 100 µl) of sample **S**_{**M3,t**=0} and the same volume of sample **S_{M3,t=x}** are evenly spread on petri dishes using a cell spreader. The petri dishes contain **PNY**-medium prepared in advance and stored at 4 °C [the composition of the **PNY**-medium is described above, in the context of step **(M-I-4)]**.
**(M-III-5)** Inoculated petri-dishes are incubated at 37 °C for the same amount of time (24 h to 48 h).
**(M-III-6)** For sample **S_{M3,t=0}** the colony forming units **("CFU")** are counted and denoted as "**ω_{M3,t=0}**".
**(M-III-7)** For sample **S_{M3,t=x}** the colony forming units **("CFU")** are counted and denoted as "**ω_{M3,t=x}**".

Steps **(M-III-1)** to **(M-III-7)** and steps **(M-III-2)** to **(M-III-7)** are carried out for three samples of **S**_{**M3,t**=0} and **S_{M3,t=x},** respectively. The average value of the three measurements of **ω_{M3,t=0}** and **ω_{M3,t=x}** are calculated and abbreviated as **"ω̃_{M3,t=0}"** and "**ω**_{**M3**,t=x}", respectively.

The number **Σ_{M3,t=x}** of spores **SP** (in %) in mixture **M₃** that have germinated at a given point of time **tₓ** after the start of the incubation in step c3)/c3*), relative to the number of spores **SP** comprised by mixture **M₃** that is contacted with **S** in step b3)/b3*) is then calculated by **Σ_{M3,t=x} = [(ω̃_{M3,t=0} - ω_{M3,t=x})/ ω_{M3,t=0]}*100.**

### VII.7 Determination of spreading on a surface S: Assay N

According to the second aspect of the invention, the at least one biosurfactant **S_{Bio}** is used to disperse bacterial spores **SP,** in particular on a surface **S.**

Preferably, the at least one biosurfactant **S_{Bio}** is used to spread bacterial spores **SP** on a surface **S,** wherein even more preferably the at least one biosurfactant **S_{Bio}** is used to improve the spreading of bacterial spores **SP** on a surface **S.**

Whether a certain additive **Z in** fact may be used for dispersing or spreading the bacterial spores **SP,** in particular may be used for an improvement in spreading of the bacterial spores **SP,** is preferably determined by comparing the spreading of spores **SP** in presence of a certain additive **Z** as compared to the spreading of the same number of the same spores **SP** in absence of the additive **Z.**

This improvement may be determined by investigating the increase of the spreading/ dispersion of the respective spores **SP** on a surface **S** in an aqueous mixture **M_{z}** comprising the additive **Z** to be investigated (such as the at least one biosurfactant **S_{Bio}**) as compared to the spreading of bacterial spores **SP** on a surface **S** in an aqueous mixture **M₀** wherein **M_{z}** and **M₀** are identical except that **M₀** is obtained from **M_{z}** by substituting all the additives **Z** in **M_{z}** by the same weight of water.

The dispersing, in particular spreading, preferably the improvement of spreading of bacterial spores **SP** on a surface **S** in presence of a certain additive **Z** is preferably determined by **Assay N** as follows according to the invention.

**Assay N** is based on the observation that more colony forming units develop from spores that are spread out over a larger area as compared to the same amount of spores which are clumped together.

**(N-1)** Two test mixtures **M₀** and **M_{z}** are prepared: For **M₀**, 10⁹ spores **SP** (spore count determined by Biermann *et al.)* are mixed with water (100 ml). For **M_{z},** 10⁹ spores **SP** (spore count determined by Biermann *et al.)* are mixed with additive **Z** and with water (100 ml).

**(N-2) M₀** and **M_{z}** are homogenized by shaking (1 hour and 200 rpm) and incubated at **RT** without shaking for a further 24 hours.

**(N-3)** Samples of **M₀** and **M_{z}** (undiluted or diluted by the same dilution factor with 0.9 % (wlv) aqueous NaCl) are plated onto motility agar. Motility agar plates contain 37 g/L Luria-Bertani (LB) agar (Merck-110283) with a reduced concentration of agar-agar (0.7 %).

**(N-4)** The plates are incubated for 10 h at 30 °C.

**(N-5)** The number of colonies (i.e. colony forming units, **CFU) A_{M0}** of the bacteria for **M₀** and the number of colonies (i.e. colony forming units, **CFU) A_{MZ}** of the bacteria for **M_{z}** are counted and compared.

Steps **(N-1)** to **(N-5)** are repeated two times and the average value **Â_{MZ}** of the three measurements **Â_{MZ}** as well as the average value **Â**_{M0} of the three measurements **Â_{M0}** are calculated.

If **Â_{M0}** < **Â**_{MZ}, in particular in those cases where **Â**_{MZ} is at least 10 % larger than **Â_{M0}**, preferably at least 50 % larger than **Â_{M0}**, **Z** is deemed to disperse the bacterial spores **SP,** and in particular is deemed to increase the spreading of bacterial spores **SP** on a surface **S.**

### VIII. Use

In a second aspect, the present invention relates to the use of at least one biosurfactant **S_{Bio}**, wherein the biosurfactant **S_{Bio}** is a glycolipid, for dispersing bacterial spores **SP,** in particular on a surface **S.** Preferably, the present invention relates to the use of at least one biosurfactant **S_{Bio}**, wherein biosurfactant **S_{Bio}** is a glycolipid, for dispersing bacterial spores **SP** on a surface **S** during germinating the bacterial spores **SP** on a surface **S.**

### VIII.1 Glycolipid

The at least one biosurfactant **S_{Bio}** used according to the second aspect of the invention is a glycolipid.

In the context of the use according to the second aspect of the invention, "biosurfactant **S_{Bio}**" is a glycolipid which is preferably selected from the group consisting of glucolipids, rhamnolipids, sophorolipids, even more preferably from the group of rhamnolipids.

Hence, preferred biosurfactants **S_{Bio}** are glycolipids such as glucolipids, rhamnolipids, and sophorolipids. Such glycolipids are described in the art together with their syntheses, for example in EP 0 499 434 A1 (glucolipids are referred to as "glucose lipids" in this document), DE 196 48 439 A1, DE 196 00 743 A1. WO 03/006146 A1, US 2008/0213194 A1, JP H01-304034 A1, CN 1337439 A describe further methods for the synthesis of rhamnolipids. WO 03/002700 A1, US 4,305,961 A, US 7,556,654 B1 describe further methods for the synthesis of sophorolipids.

### VIII. 1.1 Glucolipid

In the context of the use according to the second aspect of the invention, the term "glucolipid" is in particular to be understood as referring to a structure according to formula **(I)**, even more preferred a structure according to formula **(II)**:

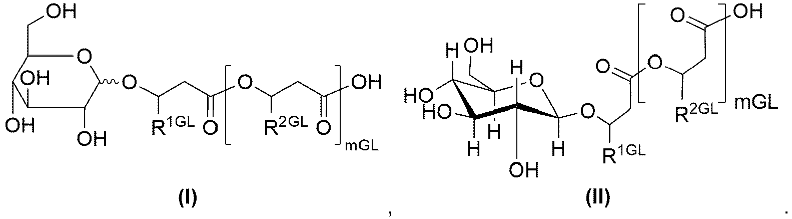

In formulae **(I)** and **(II)**, mGL = 3, 2, 1 or 0, preferably 1 or 0.

Residues R^{1GL} and R^{2GL} are, independently of one another, an organic radical having 2 to 24 carbon atoms, preferably 5 to 20, more preferably 7 to 15, even more preferably 7, carbon atoms,
wherein preferably R^{1GL} and R^{2GL} are independently of one another selected from the group consisting of optionally substituted alkyl radicals with 2 to 24, preferably 5 to 20, more preferably 7 to 15, even more preferably 7, carbon atoms, wherein hydroxy substituted alkyl radicals are preferred substituted alkyl radicals,
optionally substituted alkenyl radicals with 2 to 24 carbon atoms, preferably 5 to 20, more preferably 7 to 15, even more preferably 7, carbon atoms, wherein hydroxy substituted alkenyl radicals are preferred substituted alkenyl radicals,
wherein more preferably R^{1GL} and R^{2GL} are independently of one another selected from the group consisting of pentenyl, heptenyl, nonenyl, undecenyl, tridecenyl and -(CH₂)ₒCH₃ where o = 1 to 23, preferably 4 to 12. Most preferably, R^{1GL} and R^{2GL} are each n-heptyl.

Distinct glucolipids for mGL = 0 are abbreviated according to the following nomenclature:
"GL-CX" is understood as meaning glucolipids of the general formulae **(I)** or **(II)** in which mGL = 0 and in which the radical R^{1GL} = -(CH₂)ₒ-CH₃ where o = X-4.

Distinct glucolipids for mGL = 1 are abbreviated according to the following nomenclature:
"GL-CXCY" is understood as meaning glucolipids of the general formulae **(I)** or **(II)** in which mGL = 1 and in which one of the radicals R^{1GL} and R^{2GL} = -(CH₂)ₒ-CH₃ where o = X-4 and the remaining radical R^{1GL} or R^{2GL} = -(CH₂)ₒ-CH₃ where o = Y-4.

The nomenclature used thus does not differentiate between "CXCY" and "CYCX".

If one of the aforementioned indices X and/or Y is provided with ":Z", then this means that the respective radical R^{1GL} and/or R^{2GL} = an unbranched, unsubstituted hydrocarbon radical with X-3 or Y-3 carbon atoms having Z double bonds.

The curvy bond in structure **(I)** [and also structures **(III)**, **(V), (VII), (IX), (XI)** described hereinafter] implies that the respective substituent may be axial or equatorial, preferably is equatorial.

Alkyl radicals may be branched or linear.

Alkenyl radicals may be branched or linear and contain at least one, preferably one to three, double bonds.

In those cases where structures according to formulae **(I)** and **(II)** comprise more than one residue R^{2GL}, these residues R^{2GL} may be identical or different from one another.

In those embodiments where the biosurfactant **S_{Bio}**, used according to the second aspect of the invention comprises glucolipids, it is preferred that 1 % by weight to 30 % by weight, preferably 5 % by weight to 25 % by weight, particularly preferably 10 % by weight to 20 % by weight, of all glucolipids comprised by the biosurfactant **S_{Bio}** that are used according to the second aspect of the invention are GL-C8C10, where the percentages by weight refer to the sum of all of the glucolipids comprised by the biosurfactant **S_{Bio}** used according to the second aspect of the invention.

In those embodiments where the biosurfactant **S_{Bio}** used according to the second aspect of the invention comprises glucolipids, it is alternatively preferred that 0.5 % by weight to 20 % by weight, preferably 3 % by weight to 17 % by weight, particularly preferably 5 % by weight to 15 % by weight, of all glucolipids comprised by the biosurfactant **S_{Bio}** that are used according to the second aspect of the invention are GL-C10C12:1, where the percentages by weight refer to the sum of all of the glucolipids comprised by the biosurfactant **S_{Bio}** used according to the second aspect of the invention.

In those embodiments where the biosurfactant **S_{Bio}** used according to the second aspect of the invention comprises glucolipids, it is alternatively preferred that 0.5 % by weight to 20 % by weight, preferably 2 % by weight to 15 % by weight, particularly preferably 3 % by weight to 12 % by weight, of all glucolipids comprised by the biosurfactant **S_{Bio}** that are used according to the second aspect of the invention are GL-C10C12, where the percentages by weight refer to the sum of all of the glucolipids comprised by the biosurfactant **S_{Bio}** used according to the second aspect of the invention.

In those embodiments where the biosurfactant **S_{Bio}** used according to the second aspect of the invention comprises glucolipids, it is alternatively preferred that
1 % by weight to 30 % by weight, preferably 5 % by weight to 25 % by weight, particularly preferably 10 % by weight to 20 % by weight, of all glucolipids comprised by the biosurfactant **S_{Bio}** that are used according to the second aspect of the invention are GL-C8C10,
0.5 % by weight to 20 % by weight, preferably 3 % by weight to 17 % by weight, particularly preferably 5 % by weight to 15 % by weight, of all glucolipids comprised by the biosurfactant **S_{Bio}** that are used according to the second aspect of the invention are GL-C10C12:1,
0.5 % by weight to 20 % by weight, preferably 2 % by weight to 15 % by weight, particularly preferably 3 % by weight to 12 % by weight, of all glucolipids comprised by the biosurfactant **S_{Bio}** that are used according to the second aspect of the invention are GL-C10C12,
where the percentages by weight refer to the sum of all of the glucolipids comprised by the biosurfactant **S_{Bio}** used according to the second aspect of the invention.

In those embodiments where the biosurfactant **S_{Bio}** used according to the second aspect of the invention comprises glucolipids, it is alternatively preferred that
10 % by weight to 20 % by weight of all glucolipids comprised by the biosurfactant **S_{Bio}** that are used according to the second aspect of the invention are GL-C8C10,
5 % by weight to 15 % by weight of all glucolipids comprised by the biosurfactant **S_{Bio}** that are used according to the second aspect of the invention are GL-C10C12:1,
3 % by weight to 12 % by weight of all glucolipids comprised by the biosurfactant **S_{Bio}** that are used according to the second aspect of the invention are GL-C10C12,
where the percentages by weight refer to the sum of all of the glucolipids comprised by the biosurfactant **S_{Bio}** used according to the second aspect of the invention.

Over and above this, where the biosurfactant **S_{Bio}** used according to the second aspect of the invention comprises glucolipids, it is alternatively preferred that 0 % by weight to 5 % by weight, preferably 0.01 % by weight to 4 % by weight, particularly preferably 0.1 % by weight to 3 % by weight, of all glucolipids comprised by the biosurfactant **S_{Bio}** that are used according to the second aspect of the invention are GL-C10,
where the percentages by weight refer to the sum of all of the glucolipids comprised by the biosurfactant **S_{Bio}** used according to the second aspect of the invention.

### VIII. 1.2 Rhamnolipids

In the context of the use according to the second aspect of the invention, the term "rhamnolipid" is in particular to be understood as referring to compounds of the general formula **(III)** and salts thereof, preferably compounds according to the general structure (IV) and salts thereof,

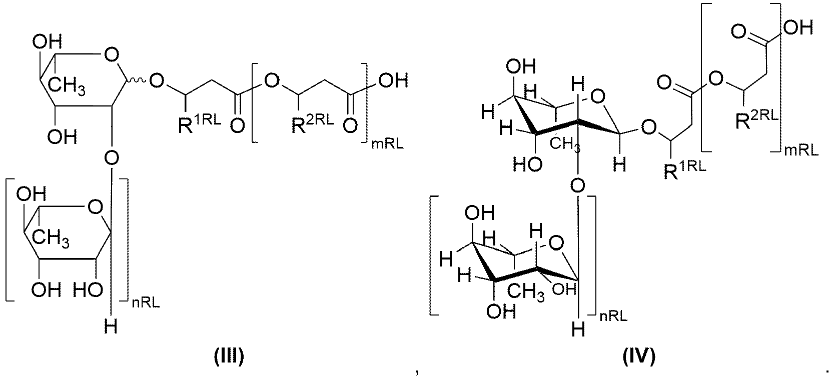

In formulae **(III)** and **(IV),** mRL = 2, 1 or 0, preferably 1 or 0.

nRL = 1 or 0.

Residue R^{1RL} and R^{2RL} are independently of one another, an organic radical having 2 to 24 carbon atoms, preferably 5 to 13, more preferably 7 to 10, even more preferably 7, carbon atoms,
wherein preferably R^{1RL} and R^{2RL} are independently of one another selected from the group consisting of optionally substituted alkyl radicals with 2 to 24, preferably 5 to 13, more preferably 7 to 10, even more preferably 7, carbon atoms, wherein hydroxy substituted alkyl radicals are preferred substituted alkyl radicals,
optionally substituted alkenyl radicals with 2 to 24, preferably 5 to 13, more preferably 7 to 10, even more preferably 7, carbon atoms, wherein hydroxy substituted alkenyl radicals are preferred substituted alkenyl radicals,
wherein more preferably R^{1RL} and R^{2RL} are independently of one another selected from the group consisting of pentenyl, heptenyl, nonenyl, undecenyl, tridecenyl and -(CH₂)ₒ-CH₃ where o = 1 to 23, preferably 4 to 12. Most preferably, R^{1RL} and R^{2RL} are each n-heptyl.

If nRL = 1, the glycosidic bond between the two rhamnose units is preferably in the α-configuration. The optically active carbon atoms of the fatty acids are preferably present as R-enantiomers (e.g. (R)-3-{(R)-3-[2-O-(α-L-rhamnopyranosyl)-α-L-rhamnopyranosyl]oxydecanoyl}oxydecanoate).

The term "di-rhamnolipid" in the context of the present invention is understood to mean compounds of the general formulae **(III)** and **(IV)** or salts thereof, where nRL = 1.

The term "mono-rhamnolipid" in the context of the present invention is understood to mean compounds of the general formulae **(III)** and **(IV)** or salts thereof, where nRL = 0.

Distinct rhamnolipids are abbreviated according to the following nomenclature:
"diRL-CXCY" are understood to mean di-rhamnolipids of the general formulae **(III)** and (IV), in which one of the residues R^{1RL} and R^{2RL} = -(CH₂)ₒ-CH₃ where o = X-4 and the remaining residue R¹ or R² = -(CH₂)ₒ-CH₃ where o = Y-4.

"monoRL-CXCY" are understood to mean mono-rhamnolipids of the general formulae **(III)** and **(IV),** in which one of the residues R^{1RL} and R^{2RL} = -(CH₂)ₒ-CH₃ where o = X-4 and the remaining residue R^{1RL} or R^{2RL} = -(CH₂)ₒ-CH₃ where o = Y-4.

The nomenclature used therefore does not distinguish between "CXCY" and "CYCX".

For rhamnolipids where mRL=0, the terms "monoRL-CX" or "diRL-CX" are used accordingly.

If one of the abovementioned indices X and/or Y is provided with ":Z", this signifies that the respective residue R^{1RL} and/or R^{2RL} is equal to an unbranched, unsubstituted hydrocarbon residue having X-3 or Y-3 carbon atoms having Z double bonds.

Methods for preparing the relevant rhamnolipids are disclosed, for example, in EP 2 786 743 A1 and

EP 2 787 065 A1. Rhamnolipids applicable in the context of the instant invention can also be produced by fermentation of *Pseudomonas,* especially *Pseudomonas aeruginosa,* which are preferably non genetically modified cells, a technology already disclosed in the eighties, as documented e.g. in EP 0 282 942 A1 and DE 41 27 908 A1. Rhamnolipids produced in *Pseudomonas aeruginosa* cells which have been improved for higher rhamnolipid titres by genetical modification can also be used in the context of the instant invention; such cells have for example been disclosed by L. Lei, F. Zhao, S. Han, Y. Zhang, Biotechnol Lett. 2020, 42, 997-1002.

Rhamnolipids produced by *Pseudomonas aeruginosa* are commercially available from Jeneil Biotech Inc., e.g. under the tradename Zonix, from Logos Technologies (technology acquired by Stepan), e.g. under the tradename NatSurFact, from Biotensidion GmbH, e.g. under the tradename Rhapynal, from AGAE technologies, e.g. under the name R90, R95, R95Md, R95Dd, from Locus Bio-Energy Solutions and from Shanghai Yusheng Industry Co. Ltd., e.g. under the tradename Bio-201 Glycolipids.

In those embodiments where the biosurfactant **S_{Bio}**, used according to the second aspect of the invention comprises rhamnolipids, it is preferred that these rhamnolipids that are used according to the second aspect of the invention are mixtures of mono-rhamnolipids and di-rhamnolipids, wherein even more preferably the ratio of the weight of all mono-rhamnolipids that are used according to the second aspect of the invention to the weight of all di-rhamnolipids that are used according to the second aspect of the invention is in the range of from 9 : 1 to 1 : 9, preferably of from 8 : 2 to 2 : 8, more preferably of from 7 : 3 to 3 : 7, more preferred of from 6 : 4 to 4 : 6, most preferred is 1 : 1.

In those embodiments where the biosurfactant **S_{Bio}** used according to the second aspect of the invention comprises rhamnolipids, it is preferred that 56 % by weight to 95 % by weight, preferably 60 % by weight to 80 % by weight, particularly preferably 66 % by weight to 70 % by weight, of all rhamnolipids comprised by the biosurfactant **S_{Bio}** that are used according to the second aspect of the invention are diRL-C10C10, where the percentages by weight refer to the sum of all of the rhamnolipids comprised by the biosurfactant **S_{Bio}** that are used according to the second aspect of the invention.

In those embodiments where the biosurfactant **S_{Bio}** used according to the second aspect of the invention comprises rhamnolipids, it is alternatively preferred that 0.5 % by weight to 15 % by weight, preferably 3 % by weight to 12 % by weight, particularly preferably 5 % by weight to 10 % by weight, of all rhamnolipids comprised by the biosurfactant **S_{Bio}** that are used according to the second aspect of the invention are diRL-C10C12:1, where the percentages by weight refer to the sum of all of the rhamnolipids comprised by the biosurfactant **S_{Bio}** that are used according to the second aspect of the invention.

In those embodiments where the biosurfactant **S_{Bio}** used according to the second aspect of the invention comprises rhamnolipids, it is alternatively preferred that 0.5 to 25 % by weight, preferably 3 % by weight to 15 % by weight, particularly preferably 5 % by weight to 12 % by weight, of all rhamnolipids comprised by the biosurfactant **S_{Bio}** that are used according to the second aspect of the invention are diRL-C10C12, where the percentages by weight refer to the sum of all of the rhamnolipids by the biosurfactant **S_{Bio}** that are used according to the second aspect of the invention.

In those embodiments where the biosurfactant **S_{Bio}** used according to the second aspect of the invention comprises rhamnolipids, it is alternatively preferred that 0.1 % by weight to 25 % by weight, preferably 2 % by weight to 10 % by weight, particularly preferably 4 % by weight to 8 % by weight, of all rhamnolipids comprised by the biosurfactant **S_{Bio}** that are used according to the second aspect of the invention are diRL-C8C10, where the percentages by weight refer to the sum of all of the rhamnolipids by the biosurfactant **S_{Bio}** that are used according to the second aspect of the invention.

In those embodiments where the biosurfactant **S_{Bio}** used according to the second aspect of the invention comprises rhamnolipids, it is even further preferred that
0.1 % by weight to 5 % by weight, preferably 0.5 % by weight to 3 % by weight, particularly preferably 0.5 % by weight to 2 % by weight, of all rhamnolipids comprised by the biosurfactant **S_{Bio}** that are used according to the second aspect of the invention are monoRL-C8C10 and/or, preferably and,
0.1 % by weight to 5 % by weight, preferably 0.5 % by weight to 3 % by weight, particularly preferably 0.5 % by weight to 2 % by weight, of all rhamnolipids comprised by the biosurfactant **S_{Bio}** that are used according to the second aspect of the invention are monoRL-C10C10, where the percentages by weight refer to the sum of all of the rhamnolipids comprised by the biosurfactant **S_{Bio}** that are used according to the second aspect of the invention.

In those embodiments where the biosurfactant **S_{Bio}** used according to the second aspect of the invention comprises rhamnolipids, it is alternatively preferred that 10 % by weight to 30 % by weight, preferably 20 % by weight to 30 % by weight, particularly preferably 25 % by weight to 30 % by weight, of all rhamnolipids comprised by the biosurfactant **S_{Bio}** that are used according to the second aspect of the invention are monoRL-C10C10, where the percentages by weight refer to the sum of all of the rhamnolipids comprised by the biosurfactant **S_{Bio}** that are used according to the second aspect of the invention.

In those embodiments where the biosurfactant **S_{Bio}**, used according to the second aspect of the invention comprises rhamnolipids, it is alternatively preferred that 10 % by weight to 30 % by weight, preferably 12 % by weight to 25 % by weight, particularly preferably 15 % by weight to 20 % by weight, of all rhamnolipids comprised by the biosurfactant **S_{Bio}** that are used according to the second aspect of the invention are diRL-C10C10, where the percentages by weight refer to the sum of all of the rhamnolipids comprised by the biosurfactant **S_{Bio}** that are used according to the second aspect of the invention.

In those embodiments where the biosurfactant **S_{Bio}** used according to the second aspect of the invention comprises rhamnolipids, it is alternatively preferred that 10 % by weight to 30 % by weight, preferably 12 % by weight to 25 % by weight, particularly preferably 15 % by weight to 20 % by weight, of all rhamnolipids comprised by the biosurfactant **S_{Bio}** that are used according to the second aspect of the invention are monoRL-C8C10, where the percentages by weight refer to the sum of all of the rhamnolipids comprised by the biosurfactant **S_{Bio}** that are used according to the second aspect of the invention.

In those embodiments where the biosurfactant **S_{Bio}** used according to the second aspect of the invention comprises rhamnolipids, it is alternatively preferred that 3 % by weight to 25 % by weight, preferably 5 % by weight to 20 % by weight, particularly preferably 10 % by weight to 15 % by weight, of all rhamnolipids comprised by the biosurfactant **S_{Bio}** that are used according to the second aspect of the invention are monoRL-C10C12:1, where the percentages by weight refer to the sum of all of the rhamnolipids comprised by the biosurfactant **S_{Bio}** that are used according to the second aspect of the invention.

In those embodiments where the biosurfactant **S_{Bio}** used according to the second aspect of the invention comprises rhamnolipids, it is alternatively preferred that 1 % by weight to 15 % by weight, preferably 2 % by weight to 10 % by weight, particularly preferably 3 % by weight to 8 % by weight, of all rhamnolipids comprised by the biosurfactant **S_{Bio}** that are used according to the second aspect of the invention are diRL-C10C12, where the percentages by weight refer to the sum of all of the rhamnolipids comprised by the biosurfactant **S_{Bio}** that are used according to the second aspect of the invention.

### VIII. 1.3 Sophorolipid

In the context of the use according to the second aspect of the invention, the term "sophorolipids" is in particular to be understood as referring to compounds of the general formulae **(V), (VII)** and salts thereof, preferably compounds of the general formulae **(VI), (VIII)** and salts thereof:

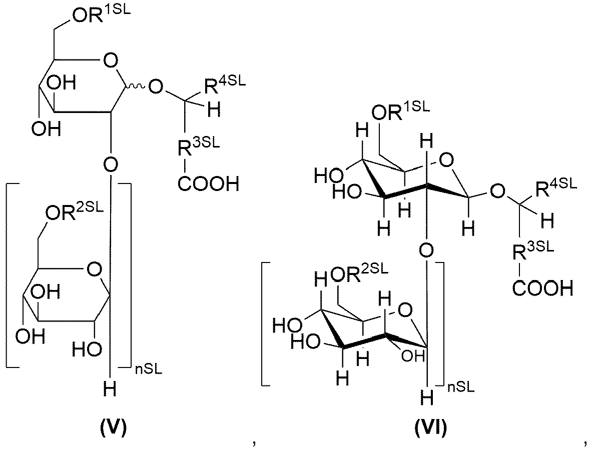

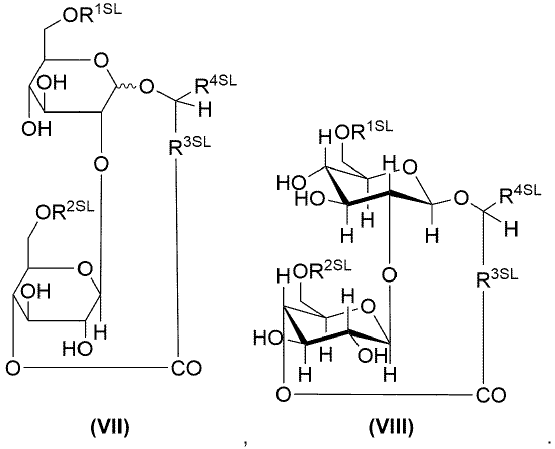

Formulae **(V), (VI)** represent the acid form, Formulae **(VII), (VIII)** represent the lactone form.

In formulae **(V), (VI),** nSL = 4, 3, 2, 1 or 0, preferably nSL = 1 or 0.
In formulae **(V), (VI), (VII),** and **(VIII),**
R^{1SL} = H or -CO-CHs,
R^{2SL} = H or -CO-CHs,
R^{3SL} = a divalent organic moiety which comprises 6 to 32 carbon atoms, preferably 12 to 20, more preferably 14 to 16, most preferably 15.

In those cases where a compound according to one of formulae **(V), (VI),** comprises more than one residue R^{2SL}, these residues R^{2SL} may be the same or different.

R^{3SL} preferably is an optionally substituted, divalent hydrocarbon moiety comprising 6 to 32 carbon atoms, wherein hydroxy substituted hydrocarbon moieties are preferred as substituted hydrocarbon moieties.

R^{3SL} more preferably is selected from the group consisting of
- optionally substituted alkylene radicals with 6 to 32, preferably 12 to 20, more preferably 14 to 16, most preferably 15 carbon atoms, wherein hydroxy-substituted alkylene radicals are preferred substituted alkylene radicals, and wherein it is preferred that the optionally substituted alkylene radicals are unbranched,
- optionally substituted alkenylene radicals with 6 to 32, preferably 12 to 20, more preferably 14 to 16, most preferably 15 carbon atoms, wherein hydroxy-substituted alkenylene radicals are preferred substituted alkenylene radicals, and wherein it is preferred that the optionally substituted alkenylene radicals are unbranched, and wherein it is preferred that the optionally substituted alkenylene radicals comprise one to three double or triple bonds.

R^{3SL} even more preferably is selected from the group consisting of
- unbranched or branched, preferably unbranched, alkylene radicals with 6 to 32, preferably 12 to 20, more preferably 14 to 16, most preferably 15, carbon atoms,
- unbranched or branched, preferably unbranched, alkylene radicals with 6 to 32 preferably 12 to 20, more preferably 14 to 16, most preferably 15, carbon atoms carrying at least one hydroxy group, preferably one hydroxy group,
- unbranched or branched, preferably unbranched, alkenylene radicals with 6 to 32, preferably 12 to 20, more preferably 14 to 16, most preferably 15 carbon atoms, wherein the alkenylene radicals preferably comprise one to three double or triple bonds, wherein the alkenylene radicals more preferably comprise one to three double bonds, even more preferably one double bond,
- unbranched or branched, preferably unbranched, alkenylene radicals with 6 to 32, preferably 12 to 20, more preferably 14 to 16, most preferably 15, carbon atoms carrying at least one hydroxy group, preferably one hydroxy group, wherein the alkenylene radicals preferably comprise one to three double or triple bonds, wherein the alkenylene radicals more preferably comprise one to three double bonds, even more preferably one double bond.

R^{4SL} = H, CH₃ or a monovalent organic radical which comprises 2 to 10 carbon atoms.

R^{4SL} is preferably selected from the group consisting of
- H,
- CH₃,
- optionally substituted alkyl radicals with 2 to 10 carbon atoms, wherein hydroxy-substituted alkyl radicals are preferred substituted alkyl radicals, and wherein it is preferred that the optionally substituted alkyl radicals are unbranched,
- optionally substituted alkenyl radicals with 2 to 10 carbon atoms, wherein hydroxy-substituted alkenyl radicals are preferred substituted alkenyl radicals, and wherein it is preferred that the optionally substituted alkenyl radicals are unbranched, and wherein it is preferred that the optionally substituted alkenyl radicals comprise one to three double or triple bonds.

R^{4SL} is more preferably selected from the group consisting of
- H,
- CH₃,
- unbranched or branched, preferably unbranched, alkyl radicals with 2 to 10 carbon atoms,
- unbranched or branched, preferably unbranched, alkyl radicals with 2 to 10 carbon atoms carrying at least one hydroxy group, preferably one hydroxy group,
- unbranched or branched, preferably unbranched, alkenyl radicals with 2 to 10 carbon atoms, wherein the alkenyl radicals preferably comprise one to three double or triple bonds, wherein the alkenyl radicals more preferably comprise one to three double bonds, even more preferably one double bond,
- unbranched or branched, preferably unbranched, alkenyl radicals with 2 to 10 carbon atoms carrying at least one hydroxy group, preferably one hydroxy group, wherein the alkenyl radicals preferably comprise one to three double or triple bonds, wherein the alkenyl radicals more preferably comprise one to three double bonds, even more preferably one double bond.

R^{4SL} is most preferably selected from the group consisting of H, Methyl, Ethyl.

In an even more preferred embodiment, the term "sophorolipids" is to be understood as referring to compounds of the general formulae **(IX), (XI)** and salts thereof, preferably compounds of the general formulae **(X), (XII)** and salts thereof:

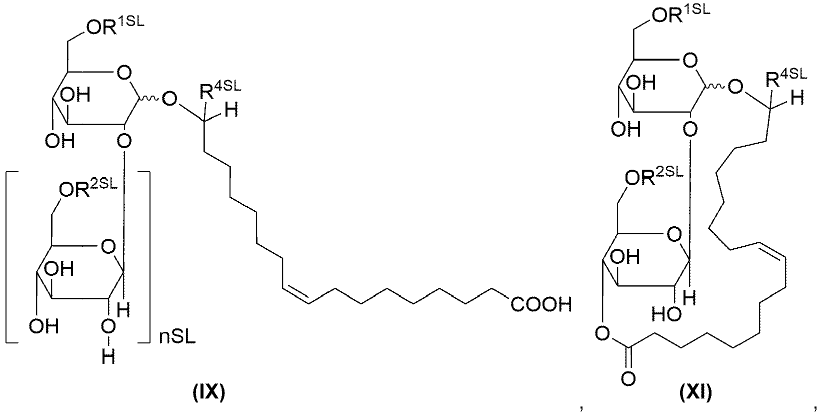

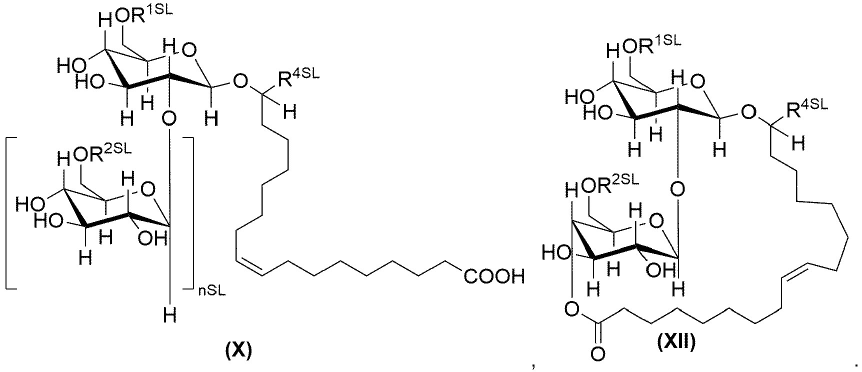

Formulae **(IX), (X)** represent the acid form, Formulae **(XI), (XII)** represent the lactone form.

nSL, R^{1SL}, R^{2SL}, R^{4SL} have the same meaning as described for formulae **(V), (VII), (VI), (VIII).**

Sophorolipids may be used in accordance with the invention in their acid form or their lactone form.

In those embodiments where the biosurfactant **S_{Bio}**, used according to the second aspect of the invention comprises sophorolipids, it is preferred that these sophorolipids comprised by the biosurfactant **S_{Bio}** that are used according to the second aspect of the invention are mixtures of the acid and the lactone form, wherein even more preferably the ratio of the weight of all sophorolipids in the lactone form comprised by the biosurfactant **S_{Bio}** that are used according to the second aspect of the invention to the weight of all sophorolipids in the acid form comprised by the biosurfactant **S_{Bio}** that are used according to the second aspect of the invention is in the range of from 20 : 80 to 80 : 20, especially preferably in the range of from 30 : 70 to 40 : 60.

To determine the content of sophorolipids in the acid or lactone form in a formulation, refer to EP 1 411 111 B1, page 8, paragraph [0053].

Sophorolipids may be obtained as described in EP 1 411 111 A1, paragraphs [0021], [0022].

The biosurfactant **S_{Bio}** used according to the second aspect of the invention may also comprise derivatives of sophorolipids such as esters of glycerol which are esterified with sophorolipids (mono-, di-, and triesters of sophorolipids with glycerol) and optionally also fatty acids.

The biosurfactant **S_{Bio}** used according to the second aspect of the invention may therefore comprise derivatives of sophorolipids such as those described in EP 3 034 613 A1 or EP 4 317 448 A1.

### VIII.2 Bacterial spores SP

The use according to the second aspect of the method of the present invention is for dispersing bacterial spores **SP,** in particular on a surface **S.**

In particular, the second aspect of the present invention relates to the use of at least one biosurfactant **S_{Bio}**, which is a glycolipid, preferably selected from the group of glucolipids, sophorolipids and rhamnolipids, for improving the dispersing of bacterial spores **SP,** in particular on a surface **S.**

Preferably, the second aspect of the present invention relates to the use of at least one biosurfactant **S_{Bio}**, which is a glycolipid, more preferably selected from the group of glucolipids, sophorolipids and rhamnolipids, for spreading of bacterial spores **SP** on a surface **S,** in particular for improving the spreading of bacterial spores on a surface **S.**

The use according to the second aspect of the invention is preferably carried out during germinating the bacterial spores **SP** on a surface **S.**

In the use according to the second aspect of the invention, the bacterial spores **SP** are not particularly limited, and preferably are endospores. They may be spores, in particular endospores, from any bacterium that creates spores in its life cycle, such as *Clostridium, Bacillus, Priestia, Desulfotomaculum, Heyndrickxia.* The preferred *Heyndrickxia* species is *Heyndrickxia coagulans.*

Preferably, the bacterial spores **SP** are selected from *Bacillus* spores, *Priestia* spores.

Preferred *Bacillus* spores are selected from spores of at least one *Bacillus* species selected from the group consisting of *Bacillus velezensis, Bacillus licheniformis, Bacillus paralicheniformis, Bacillus subtilis, Bacillus amyloliquefaciens, Bacillus atrophaeus, Bacillus mojavensis, Bacillus laevolacticus, Bacillus pasteurii, Bacillus tequilensis, Bacillus vallismortis, Bacillus pumilus, Bacillus simplex,* preferably selected from the group consisting of *Bacillus velezensis, Bacillus licheniformis, Bacillus subtilis.*

Preferred *Priestia* spores are spores of *Priestia megaterium* (formerly known as *"Bacillus megaterium").*

In the context of this invention, the inventors have identified several *Bacillus* and *Priestia* species with surprisingly advantageous properties that make them predestined for application in cleaning.

A preferred strain of *Bacillus velezensis* is thus selected from *Bacillus velezensis* DSM 34978 and mutants thereof and *Bacillus velezensis* DSM 34674 and mutants thereof.

A preferred strain of *Bacillus licheniformis* is thus selected from *Bacillus licheniformis* DSM 34977 and mutants thereof.

A preferred strain of *Bacillus subtilis* is thus selected from *Bacillus subtilis* DSM 34981 and mutants thereof.

A preferred strain of *Priestia megaterium* is thus selected from *Priestia megaterium* DSM 34980 and mutants thereof.

### VIII.3 Surface 5

In the use according to the second aspect of the invention, the bacterial spores **SP** are dispersed on a surface **S,** in particular spread on a surface **S.**

The surface **S** in the use according to the second aspect of the invention is preferably selected from a surface **Sₒ** of an object **O** and a surface **S_{B}** of the human or animal body **B.**

In those embodiments where the surface **S** is a surface **S_{B}** of the human or animal body **B,** it is preferred that the surface **S_{B}** is the surface of a part of the human or animal body selected from skin, hair, most preferred hair.

An "object **O"** is a lifeless, solid, object and preferably comprises at least one material selected from natural fibre, ceramic, metal, plastic, glass, stone.

"Natural fibre" in the context of the invention preferably is one selected from the group consisting of vegetable fibres, animal fibres. Typical vegetable fibres are based on cellulose and in particular selected from wood, cotton, hemp, jute, flax, abaca, piña (pineapple fibre), ramie, sisal, bagasse, banana. Preferably, vegetable fibres are selected from the group consisting of wood, cotton. Vegetable fibres may for example be found in paper, cardboard.

Typical animal fibres are in particular selected from the group consisting of wool, silk.

The object **O** in a preferred embodiment is selected from the group consisting of a woven, a non-woven, a vehicle, crockery, cutlery, food storage containers, furniture, flooring, panelling, paving, domestic machine, garden tool, construction equipment, building for livestock or domestic animal, music instrument, toy, window. The object **O,** in particular, is selected from the group consisting of a woven, a non-woven, furniture, flooring, panelling, even more preferably selected from the group consisting of a woven, a non-woven.

The woven is in particular selected from an article of clothing, curtains, napkins, bed linen, tablecloth, blanket, bedspread, duvet cover.

The non-woven is in particular selected from an article of clothing, curtains, napkins, bed linen, tablecloth, blanket, bedspread, duvet cover.

The article of clothing is in particular selected from the group consisting of sweaters, shirts, pants, shoes, socks, hosiery, coats, haberdashery, suits, hats, caps, bonnets, scarfs, gloves.

The vehicle is in particular selected from the group consisting of car, train (which may be selected from the group consisting of railroad cars, tram cars, underground railway cars), plane, bicycle, motor-bike, rocket, water vehicles (which may be selected from ships, boats, hovercraft).

Crockery is in particular selected from the group consisting of dishes, glassware (such as drinking glasses), cups, platters, mugs, pitchers, pots, cannikins.

Cutlery is in particular selected from the group consisting of knives, forks, spoons, chopsticks.

Food storage containers are in particular selected from the group consisting of barrels, lunchboxes, bottles, jars.

Furniture is in particular selected from the group consisting of shelves, boards, wardrobes, cupboards, desks, tables, chairs, chest of drawers.

Flooring is in particular selected from the group consisting of laminate floor, parquet floor, tiled floor, carpeted floor, ceramic floor.

Panelling is in particular selected from the group consisting of wallpaper, wall tiles, tapestry.

Paving may be any kind of road surface, and is in particular selected from cobblestone, asphalt, gravel.

A domestic machine may be any electric machine used in the household, and is in particular selected from washing machine, dishwasher, vacuum cleaner, sound system, lamp, TV, computer, printer, heater, electrical kitchen appliances such as fridge, oven, stove, boiler, egg boiler, toaster, sandwich maker, waffle iron, mixer, bread machine, yogurt maker, coffee maker, electric kettle.

A garden tool is preferably selected from the group consisting of rake, grate, lawnmower, scythe, spade, shovel.

Construction equipment is in particular any equipment, electrical or non-electrical, which may be used in handcraft or in construction of buildings, roads. Construction equipment is preferably selected from drilling machines, tools such as hammer, rasp, screwdriver.

Building for livestock or domestic animal is in particular any container or building in which pets or farm animals may be kept. In particular, the building for livestock or domestic animal is selected from the group consisting of terrarium, aquarium, cage, pen, kenner, cowshed, hennery, pigsty, coop.

Music instrument may be any keyboard instrument, string instrument, wind instrument, percussion instrument and is preferably selected from piano, guitar, drums.

Toy is preferably selected from video game console, doll.

Window may be any transparent light opening, and is particular selected from outside window of a building, inside window of a building, bull's eye of washing machines, bull's eye of ships, preferably selected from outside window of a building, inside window of a building.

In those embodiments where the at least one biosurfactant **S_{Bio}** is used for dispersing bacterial spores **SP,** in particular on a surface **S,** according to the second aspect of the invention, and wherein the surface **S** is a surface **S_{B}** to the human or animal body **B,** this means that the at least one biosurfactant **S_{Bio}** is used with a living human being or animal. In this embodiment of the use according to the second aspect of the invention, the use is non-therapeutic. It goes without saying that in embodiments where the at least one biosurfactant **S_{Bio}** is used for dispersing bacterial spores **SP,** in particular on a surface **S,** according to the second aspect of the invention, and wherein the surface **S** is a surface **So** of an object **O,** the method is also non-therapeutic, since the object **O** is lifeless.

### VIII.4 Dispersing

It was surprisingly observed that bacterial spores **SP** are dispersed better, in particular on a surface **S,** preferably over a wider area of surface **S,** when these spores **SP** are incubated before and/or during the dispersing with at least one biosurfactant **S_{Bio}**.

"Dispersing bacterial spores **SP"** in the sense of the present invention is to be understood in the sense of creating repulsive forces and/or reducing adhesive forces between a multitude of bacterial spores **SP** (i.e. at least two bacterial spores **SP).** In particular, it means that the tendency of bacterial spores **SP** to clump together is reduced.

This dispersing of the bacterial spores **SP** is improved in the context of the use according to the second aspect of the invention. This improvement of the dispersing is at least in part a result of the effect of the at least one biosurfactant **S_{Bio}** on the coating of the spores, which weakens the adhesive forces between the spores **SP** and/or creates repulsive forces between the spores **SP.**

Hence, in particular, the present invention relates to the use of at least one biosurfactant **S_{Bio}**, which is a glycolipid, preferably selected from the group of glucolipids, sophorolipids and rhamnolipids, for improving the dispersing of bacterial spores **SP,** in particular on a surface **S.**

Preferably, the present invention relates to the use of at least one biosurfactant **S_{Bio}**, which is a glycolipid preferably selected from the group of glucolipids, sophorolipids and rhamnolipids, for spreading of bacterial spores **SP** on a surface **S,** in particular for improving the spreading of bacterial spores on a surface **S.**

"Improvement" of the dispersing, in particular of the spreading, preferably on a surface **S,** is to be understood in that the average distance **δ_{SP}** between a certain number **x_{SP}** of bacterial spores **SP** after spreading the **x_{SP}** of bacterial spores **SP** on a surface is greater when the **x_{SP}** spores are brought into contact according to the second aspect of the invention with the at least one biosurfactant **S_{Bio}**, compared to the average distance **δ_{SP}** after spreading of **x_{SP}** of the same bacterial spores **S_{Bio}** that were not brought into contact with the at least one biosurfactant **S_{Bio}**, wherein the method of spreading the bacterial spores **SP** is identical in both cases.

**x_{SP}** is preferably in the range of from 10³ to 10¹¹, more preferably in the range of from 10⁵ to 10¹⁰, above all in the range of from 10⁷ to 10⁹.

Surprisingly, the average distance **δ_{SP}** between a certain number **x_{SP}** of bacterial spores **SP** after spreading the **x_{SP}** of bacterial spores **SP** on a surface when the **x_{SP}** spores are brought into contact according to the second aspect of the invention with the at least one biosurfactant **S_{Bio}** is also greater compared to the average distance **δ_{SP}** after spreading of **x_{SP}** of the same bacterial spores **S_{Bio}** that were brought into contact with other surfactants **(SLES,** ethoxylated alcohols), wherein the method of spreading the bacterial spores **SP** is identical in both cases.

The average distance **δ_{SP}** between the **x_{SP}** spores is determined by measuring the distance between each of the **x_{SP}** spores and the other (**x_{SP}** -1) spores [wherein the distance between two spores **xᵢ** and **xⱼ** is only counted once] and dividing it by the number of spores **x_{SP}**. The distances between the **x_{SP}** spores may be determined by a method known to the skilled person, for example by a microscope.

Whether the average distance **δ_{SP}** is greater may also be observed by germinating the spores spread on a certain surface **S** into colonies and counting the colony forming units (= **"CFU").** Where the average distance **δ_{SP}** is greater, more colony forming units are observed, because where the spores are spread out wider, more **CFUs** form from each spore after the spore's germination, relative to the number of spores.

The dispersing, in particular the spreading, and the improvement of spreading is preferably determined by **Assay N** described under point VII.7.

It was surprisingly observed that, when the protocol of **Assay N** is used and the dispersing, in particular spreading, of bacterial spores **SP** which were incubated with different additives **(SLES,** ethoxylated alcohol, rhamnolipid, water), that the most **CFU** form upon pre-incubation of bacterial spores **SP** with rhamnolipids. As this improvement is also observed with respect to the preincubation in water, it can be concluded that this is at least in part due to an improved dispersing effect of the bacterial spores **SP** over the surface **S.**

### VIII.5 Variants

In the second aspect, this invention relates to the use of at least one biosurfactant **S_{Bio}**, wherein biosurfactant **S_{Bio}** is a glycolipid, for dispersing bacterial spores **SP,** in particular on a surface **S.**

In the context of the use according the second aspect of the invention, the form in which the bacterial spores **SP** are provided is not particularly limited.

In one preferred embodiment, the bacterial spores **SP** are provided as an aqueous mixture **M_{SP}**, wherein it is more preferred that the content of water in **M_{SP}** is from 1 wt.-% to 99 wt.-%, more preferably from 10 wt.-% to 99 wt.-%, more preferably from 30 wt.-% to 98 wt.-%, more preferably from 50 wt.-% to 97 wt.-%, more preferably from 60 wt.-% to 96 wt.-%, more preferably from 70 wt.-% to 95 wt.-%, more preferably from 80 wt.-% to 90 wt.-%, wherein the weight-% give the percentage of water relative to the total weight of the mixture **M_{SP}**.

This aqueous mixture **M_{SP}** may then be dispersed, in particular on a surface **S,** and the bacterial spores **SP** may be treated with the at least one biosurfactant **S_{Bio}** before or while the aqueous mixture **M_{SP}** contacts the surface **S.**

The preferred conditions applied in the use according to the second aspect of the invention are those described for the method according to the first aspect of the invention.

Hence, the use according to the second aspect of the invention is preferably carried out in the context of one of the three variants: **variant 1*, variant 2*, variant 3*.**

In the following **"variant 1*"** will be used as abbreviation for "variant 1* of the use according to the second aspect of the invention".

In the following **"variant 2*"** will be used as abbreviation for "variant 2* of the use according to the second aspect of the invention".

In the following **"variant 3*"** will be used as abbreviation for "variant 3* of the use according to the second aspect of the invention".

**Variants 1*** and **variant 2*** are preferred variants of the three variants. **Variant 2*** is the most preferred variants of the three variants.

**Variant 1*** relates to the use of at least one biosurfactant **S_{Bio}**, wherein biosurfactant **S_{Bio}** is a glycolipid, for dispersing bacterial spores **SP,** in particular on a surface **S,** in particular during germinating the bacterial spores **SP** on a surface **S,** wherein in **variant 1*,** the following steps a1*), b1*), c1*) are carried out:
a1*) providing **SP** and, separately from **SP,** at least one biosurfactant **S_{Bio}**, wherein the biosurfactant **S_{Bio}** is a glycolipid,
b1*) contacting **S** with **SP** and **S_{Bio}**, so that a mixture **M₁** comprising **SP** and at least one biosurfactant **S_{Bio}** is obtained, wherein mixture **M₁** is in contact with **S,**
c1*) incubating **M₁** to germinate at least a part of the spores **SP** comprised by mixture **M₁**.

**Variant 2*** relates to the use of at least one biosurfactant **S_{Bio}**, wherein biosurfactant **S_{Bio}** is a glycolipid, for dispersing bacterial spores **SP** on a surface **S,** in particular during germinating the bacterial spores **SP** on a surface **S,** wherein in **variant 2*,** the following steps a2*), b2*), c2*) are carried out:
a2*) providing a mixture **M*** comprising **SP** and at least one biosurfactant **S_{Bio}**, wherein the biosurfactant **S_{Bio}** is a glycolipid,
b2*) contacting **S** with the mixture **M*,** wherein **M*** is optionally incubated before contacting **S,** so that a mixture **M₂** comprising **SP** and at least one biosurfactant **S_{Bio}** is obtained, wherein mixture **M₂** is in contact with **S,**
c2*) incubating **M₂** to germinate at least a part of the spores **SP** comprised by mixture **M₂**.

**Variant 3*** relates to the use of at least one biosurfactant **S_{Bio}**, wherein biosurfactant **S_{Bio}** is a glycolipid, for dispersing bacterial spores **SP** on a surface **S,** in particular during germinating the bacterial spores **SP** on a surface **S,** wherein in **variant 3*,** the following steps a3*), b3*), c3*) are carried out:
a3*) providing a mixture **M*** comprising **SP** and at least one biosurfactant **S_{Bio}**, wherein the biosurfactant **S_{Bio}** is a glycolipid,
b3*) incubating mixture **M*** and then separating at least a part of the spores **SP** comprised by **M*** from at least a part of the biosurfactant **S_{Bio}** comprised by **M*** and contacting **S** with the separated spores, so that a mixture **M₃** comprising **SP** and optionally at least one biosurfactant **S_{Bio}** is obtained, wherein mixture **M₃** is in contact with **S,**
c3*) incubating **M₃** to germinate at least a part of the spores **SP** comprised by mixture **M₃**.

Referring to "step **a*)"** is to be understood as commonly referring to "step a1*) of **variant 1*",** "step a2*) of **variant 2*",** "step a3*) of **variant 3*".**

Referring to "step **b*)"** is to be understood as commonly referring to "step b1*) of **variant 1*",** "step b2*) of **variant 2*",** "step b3*) of **variant 3*".**

Referring to "step **c*)"** is to be understood as commonly referring to "step c1*) of **variant 1*",** "step c2*) of **variant 2*",** "step c3*) of **variant 3*".**

### VIII.5.1 Variant 1*

### VIII.5.1.1 Step a1*)

In step a1*) of **variant 1*, SP** is provided and, separately from **SP,** at least one biosurfactant **S_{Bio}**, is provided. **S_{Bio}** is a glycolipid, in particular selected from rhamnolipids, glucolipids, sophorolipids, preferably selected from rhamnolipids, sophorolipids, even more preferably selected from rhamnolipids.

**SP** may be provided in any form familiar to the skilled person, as long as such form comprises spores **SP** that are still able to germinate. For example, it may be provided in the form of a preparation **P_{SP}** of the respective bacteria, comprising spores **SP.**

In those embodiments where **variant 1*** is applied in the context of a use according to the second aspect of the invention in a cleaning method, the spores **SP** and/or the at least one biosurfactant **S_{Bio}** may also each be provided as a cleaning formulation **F.**

Hence, in particular, the provision of **SP** and **S_{Bio}** in step a1*) comprises (but is not limited to) an embodiment in which a, preferably aqueous, mixture **M_{SP1}** comprising **SP** (but not **S_{Bio}**) and a, preferably aqueous, mixture **M_{Bio1}** comprising **S_{Bio}** (but not **SP)** are provided.

**M_{SP1}** and **M_{Bio1}** may each comprise further components, especially depending on the application of the use according to **variant 1*.**

In those embodiments where **variant 1*** is applied in the context of a use according to the second aspect of the invention in a cleaning method, at least one of the mixtures **M_{SP1}** and **M_{Bio1}** may be provided as a cleaning formulation **F,** wherein the preferred components of cleaning formulations F are further explained above.

In those embodiments where **M_{SP1}** is an aqueous mixture, it is preferred that the content of water in **M_{SP1}** is from 1 wt.-% to 99 wt.-%, more preferably from 10 wt.-% to 99 wt.-%, more preferably from 30 wt.-% to 98 wt.-%, more preferably from 50 wt.-% to 97 wt.-%, more preferably from 60 wt.-% to 96 wt.-%, more preferably from 70 wt.-% to 95 wt.-%, more preferably from 80 wt.-% to 90 wt.-%, wherein the weight-% give the percentage of water relative to the total weight of the mixture **M_{SP1}.**

In those embodiments where **M_{Bio1}** is an aqueous mixture, it is preferred that the content of water in **M_{Bio1}** is from 1 wt.-% to 99 wt.-%, more preferably from 10 wt.-% to 99 wt.-%, more preferably from 30 wt.-% to 98 wt.-%, more preferably from 50 wt.-% to 97 wt.-%, more preferably from 60 wt.-% to 96 wt.-%, more preferably from 70 wt.-% to 95 wt.-%, more preferably from 80 wt.-% to 90 wt.-%, wherein the weight-% give the percentage of water relative to the total weight of the mixture **M_{Bio1}**.

### VIII.5.1.2 Step b1*)

In step b1*) of **variant 1*,** surface **S** is contacted with **SP** and **S_{Bio}**, so that a mixture **M₁** comprising SP and at least one biosurfactant **S_{Bio}** is obtained, wherein mixture **M₁** is in contact with surface S.

"Contacting surface **S** with **SP** and **S_{Bio}**" is a further essential feature of **variant 1.** It results in a mixture **M₁** comprising **SP** and at least one biosurfactant **S_{Bio}** that is in contact with **S.** This is to be understood as meaning that the mixture **M₁** and at least a part of the surface **S** are in direct contact. In the context of the present invention "in direct contact" is to be understood as meaning "wetting" of the surface **S** with **M₁**. It will be appreciated that the surface **S** and the mixture **M₁** are thus in direct contact at a contact surface **Sc** of surface **S.**

The contacting step b1*) is not further limited. Where two separate mixtures **M_{SP1}** and **M_{Bio1}**, namely a mixture **M_{SP1}** comprising **SP** (but not **S_{Bio}**) and a mixture **M_{Bio1}** comprising **S_{Bio}** (but not **SP)** are provided in step a1*) in the use according to **variant 1*,** the respective mixtures **M_{SP1}** and **M_{Bio1}** may simply be contacted with **S** and mix to form mixture **M₁** before or while, preferably while, the two mixtures **M_{SP1}** and **M_{Bio1}** are already in contact with **S.**

In those embodiments where **variant 1*** is applied in the context of a use according to the second aspect of the invention in a method of cleaning, it is preferred that the mixture **M₁** which is in contact with **S** at the end of step b1*) of **variant 1*,** is also in contact with at least one impurity I.

This means in particular that during step b1*) and/or during step c1*), preferably during step c1*), of **variant 1*,** at least one impurity **I** which is absorbed or adsorbed on the surface **S,** in particular contact surface **S_{C}**, is at least partially desorbed (and thus removed) from the surface **S** and dispersed or dissolved, in particular dispersed, in the mixture **M₁**. Optionally, the at least one impurity **I** is then degraded by at least one activity of at least one vegetative cell germinating from **SP** during step c1*) of **variant 1*.**

In other words, in this preferred embodiment, during step b1*) and/or during step c1*) of **variant 1*,** the mixture **M₁** is brought in direct contact with at least a part **S_{C}** of the surface **S,** wherein at least one impurity **I** is absorbed or adsorbed on **S_{C}**, so that the at least one impurity **I** is at least partially desorbed from the surface **S_{C}** and dispersed or dissolved, in particular dispersed, in the mixture **M₁** (and thus removed from surface **S_{C}**). The dispersed or dissolved, in particular dispersed, impurity I is then optionally degraded in the mixture **M₁** by at least one activity of at least one vegetative cell germinating from **SP** during step c1*) of **variant 1*.Therefore,** in particular, after step c1*) of **variant 1*,** a mixture **M₁** is obtained wherein at least one impurity **I** in dissolved or dispersed, preferably dispersed.

The impurity **I** may either be in the form of a liquid (e.g. droplet) or a particle, and preferably is a particle.

In a preferred embodiment, the at least one impurity **I** is an impurity which is inorganic or organic, and is preferably organic.

Organic impurities are preferably selected from oils and fats, which may be of vegetable, animal or synthetic origin, food based particles, soil, pollen, sebum, body fluids, for example, blood, sperm, sweat, urine, feces, liquor.

Inorganic impurities are preferably selected from the group consisting of carbon black, fine dust, plastic particles (e.g. microplastic particles), metal oxides (preferably iron oxide), silica (such as sand), clay, dyes (such as pigments), more preferably clay, in particular kaolin.

Where **variant 1*** is used according to the second aspect of the invention in a method of cleaning, it is preferred that, when the spores **SP** of *Bacillus velezensis* strain DSM 34978, *Bacillus velezensis* DSM 34674, mutants of *Bacillus velezensis* DSM 34674 and/or mutants of *Bacillus velezensis* strain DSM 34978 are selected as spores **SP** in **variant 1*,** at least a part of the impurities **I** cleaned during **variant 1*** are the substrates of these activities. Hence, it is preferred that in this preferred embodiment, the impurity **I** which is cleaned from the surface S during the use according to the second aspect of the invention in a cleaning method according to **variant 1*** is selected from the group consisting of valeric acid, proteins (in particular proteins selected from casein and whey protein), polysaccharides (in particular starch), cellulose. More preferred, it comprises proteins (in particular proteins selected from casein and whey protein), cellulose. Most preferred, it comprises proteins, in particular proteins selected from casein and whey protein.

Where **variant 1*** is used according to the second aspect of the invention in a method of cleaning, it is hence preferred that, when the spores **SP** of *Bacillus licheniformis* strain DSM 34977 and/or mutants of *Bacillus licheniformis* strain DSM 34977 are selected as spores **SP** in **variant 1*,** that at least a part of the impurities **I** cleaned during **variant 1*** are the substrates of this activity. Hence, it is preferred that in this preferred embodiment, the impurity **I** which is cleaned from the surface S during the use according to the second aspect of the invention in a cleaning method according to **variant 1*** is valeric acid.

Where **variant 1*** is used according to the second aspect of the invention in a method of cleaning, it is hence preferred that, when the spores **SP** of *Priestia megaterium* strain DSM 34980 and/or mutants of *Priestia megaterium* strain DSM 34980 are selected as spores **SP** in **variant 1*,** that at least a part of the impurities **I** cleaned during **variant 1*** are the substrates of these activities. Hence, it is preferred that in this preferred embodiment, the impurity **I** which is cleaned from the surface S during the use according to the second aspect of the invention in a cleaning method according to **variant 1*** is selected from the group consisting of valeric acid, proteins (in particular proteins selected from casein and whey protein), in particular proteins, which are even more preferably selected from casein and whey protein.

Where **variant 1*** is used according to the second aspect of the invention in a method of cleaning, it is hence preferred that, when the spores **SP** of *Bacillus subtilis* strain DSM 34981 and/or mutants of *Bacillus subtilis* strain DSM 34981 are selected as spores **SP** in **variant 1*,** that at least a part of the impurities **I** cleaned during **variant 1*** are the substrates of these activities. Hence, it is preferred that in this preferred embodiment, the impurity **I** which is cleaned from the surface **S** during the use according to the second aspect of the invention in a cleaning method according to **variant 1*** is selected from the group consisting of valeric acid, grease, oil, more preferably grease, oil.

In a further embodiment, where the spores **SP** in the use according to **variant 1*** are selected from *Bacillus velezensis* strain DSM 34978 and mutants thereof, *Bacillus velezensis* DSM 34674 and mutants thereof, *Bacillus licheniformis* DSM strain DSM 34977 and mutants thereof, *Priestia megaterium* strain DSM 34980 and mutants thereof and *Bacillus subtilis* strain DSM 34981 and mutants thereof, it is preferred that the impurity **I** is selected from the group consisting of valeric acid, grease, oil, proteins (in particular proteins selected from casein and whey protein), polysaccharides (in particular starch), cellulose.

### VIII.5.1.3 Step c1*)

In step c1*) of **variant 1*, M₁** is incubated to germinate at least a part of the spores **SP** comprised by mixture **M₁**.

The skilled person is aware of the conditions to be applied in step c1 *) that cause at least a part of the spores **SP** to germinate.

It is preferred that mixture **M₁** in steps b1*) and c1*) of **variant 1*** is an aqueous mixture comprising the spores **SP** and the at least one biosurfactant **S_{Bio}**.

This is preferably achieved by simply adding water to the mixture **M₁** during step c1*).

Alternatively or additionally, and in those embodiments where in step a1*) a mixture **M_{SP1}** comprising **SP** (but not **S_{Bio}**) and a mixture **M_{Bio1}** comprising **S_{Bio}** (but not **SP)** is provided, at least one of these mixture **M_{SP1}**, **M_{Bio1}** may simply be provided as an aqueous mixture.

In addition, in step b1*), the respective microorganism may also be contacted with nutrients and/or salts.

Preferred nutrients are selected from carbohydrates, amino acids, peptides, peptone, glucose, starch.

Peptides are preferably selected from peptone.

As amino acid, any of the natural amino acids may be selected by the skilled person. Preferably, the skilled person selects the amino acid from the group consisting of glutamine, alanine, valine, aspartate, glutamate, methionine, asparagine, preferably alanine, valine, asparagine.

Carbohydrates are preferably sugars, polysaccharides.

Preferred sugars are glucose, saccharose, fructose, galactose.

Preferred polysaccharides are starch, inulin.

Preferred salts are salts selected from sodium chloride, potassium phosphate, di-potassium hydrogen phosphate
Further additives known to the skilled person may also be applied to the mixture **M₁** in step c1*), such as tris(hydroxymethyl)aminomethane **("Tris").**

In addition, the germination of bacteria such as *Bacillus* or *Priestia* is also triggered by contacting the spores with substrates of one or more enzymatic or metabolic activity of these bacteria.

Hence, where spores **SP** of at least one of *Bacillus velezensis* strain DSM 34978, mutants of *Bacillus velezensis* strain DSM 34978, *Bacillus velezensis* DSM 34674, mutants of *Bacillus velezensis* DSM 34674, *Bacillus licheniformis* strain DSM 34977, mutants of *Bacillus licheniformis* strain DSM 34977, *Priestia megaterium* strain DSM 34980, mutants of *Priestia megaterium* strain DSM 34980, *Bacillus subtilis* strain DSM 34981 and mutants of *Bacillus subtilis* strain DSM 34981 are selected as spores **SP** in **variant 1*,** it is further preferable that the mixture **M₁** in step c1*) comprises the impurities **I** as set forth in the following:
*Bacillus velezensis* strain DSM 34978 and mutants of *Bacillus velezensis* strain DSM 34978, *Bacillus velezensis* DSM 34674, mutants of *Bacillus velezensis* DSM 34674: mixture **M₁** in step c1*) preferably comprises at least one impurity **I** selected from the group consisting of valeric acid, proteins (in particular proteins selected from casein and whey protein), polysaccharides (in particular starch), cellulose; more preferred at least one impurity **I** selected from the group consisting of proteins (in particular proteins selected from casein and whey protein), cellulose; most preferred at least one impurity **I** selected from the group consisting of proteins, in particular proteins selected from casein and whey protein.

*Bacillus licheniformis* strain DSM 34977 and mutants of *Bacillus licheniformis* strain DSM 34977: mixture **M₁** in step c1*) preferably comprises impurity **I** selected from valeric acid.

*Priestia megaterium* strain DSM 34980 and mutants of *Priestia megaterium* strain DSM 34980: mixture **M₁** in step c1*) preferably comprises at least one impurity **I** selected from the group consisting of valeric acid, proteins (in particular proteins selected from casein and whey protein), preferably at least one impurity I selected from the group consisting of proteins which even more preferably are selected from casein and whey protein.

*Bacillus subtilis* strain DSM 34981 and mutants of *Bacillus subtilis* strain DSM 34981: mixture **M₁** in step c1 *) preferably comprises at least one impurity **I** selected from the group consisting of valeric acid, grease, oil, more preferably at least one impurity **I** selected from the group consisting of grease, oil.

The temperature of incubation in step c1*) is in particular in the range of from 10 °C to 70 °C, in particular in the range of from 20 °C to 65 °C, preferably in the range of from 25 °C to 55 °C, more preferably in the range of from 30 °C to 45 °C.

Where the surface **S** is the surface **S_{O}** of an object **O,** in particular where **variant 1*** is applied as a use according to the second aspect of the invention in a method for cleaning object **O,** it is preferred that the temperature in step c1*) is in a range of from 10 °C to 70 °C, in particular in a range of from 20 °C to 65 °C, preferably in a range of from 25 °C to 55 °C, more preferably in a range of from 30 °C to 45 °C.

Where the surface **S** is the surface **S_{B}** of the human or animal body **B,** in particular where **variant 1*** is applied as a use according to the second aspect of the invention in a method for cleaning the human or animal body **B,** it is preferred that the temperature in step c1*) is in a range of from 10 °C to 50 °C, in particular in a range of from 20 °C to 45 °C, preferably in a range of from 25 °C to 40 °C, more preferably in a range of from 30 °C to 38 °C, most preferably 37 °C.

In a further preferred embodiment, the incubation in step c1*) is carried out for at least 1 second ("second" = "s"), preferably for at least 10 s, preferably for at least 1 minute ("minute" = "min"), more preferably for at least 5 min, preferably for at least 15 min, preferably for at least 60 min, more preferably from 15 min to 3 d, more preferably from 30 min to 2 d, more preferably from 45 min to 1 d.

Preferably, in those cases where **M₁** comprises water, it is preferred that the pH of the water in **M₁** in step c1*) at 25 °C is from 3.5 to 9, preferably from 5 to 9, preferably from 6 to 9, and particularly preferably from 7 to 8.

It is further preferred that the incubation in step c1*) is carried out until at least 10 % of the spores **SP** comprised by mixture **M₁** that is contacted with **S** in step b1*) have germinated, relative to the number of spores **SP** comprised by mixture **M₁** before mixture **M₁** is subjected to the incubation according to step c1*) [i.e. relative to the number of spores **SP** comprised by mixture **M₁** that is obtained directly upon contacting **S** with **SP** and **S_{Bio}** in step b1*)]. This "germination rate" in step c1*) is preferably determined according to **Assay M-I.**

It is even further preferred that the incubation in step c1 *) is carried out until at least 20 % of the spores **SP** comprised by mixture **M₁** that is in contact with **S** in step b1*) have germinated, more preferably until at least 30 %, even more preferably until at least 40 %, even more preferably until at least 50 %, even more preferably until at least 60 %, even more preferably until at least 70 %, even more preferably until at least 80 %, even more preferably until at least 90 %, even more preferably until at least 95 %, even more preferably until at least 99 % of the spores **SP** comprised by mixture **M₁** that is in contact with S in step b1*) have germinated, relative to the number of spores **SP** comprised by mixture **M₁** before mixture **M₁** is subjected to the incubation according to step c1*).

### VIII.5.1.4 Step dD (optional)

It is preferred that, in a step d1*), which is carried out after step c1*), at least a part of **M₁** is separated from **S.** The separation is, in a preferred embodiment, carried out so that **M₁** is essentially complete separated from **S.**

This embodiment is particularly preferred where the use according to **variant 1*** is applied in the context of a cleaning method, in particular on a surface **S_{O}** of an object **O** or on a surface **S_{B}** of the human or animal body **B.**

The skilled person is aware of how to carry out this separation in each specific context. Generally, this separation is carried out by withdrawing the surface **S** from at least a part of **M₁**.

An object **O** may, in step d1*), optionally be subjected to centrifugation (for example in a washing machine).

### VIII.5.1.5 Step e1*) (optional)

In those optional embodiments of **variant 1*** where step d1*) is carried out, it is even more preferred to carry out another optional step e1*) after step d1*).

In an optional step e1*) of **variant 1*,** the surface **S** is rinsed with further water.

### VIII. 5.2 Variant 2*

### VIII.5.2.1 Step a2*)

In step a2*) of **variant 2*,** a mixture **M*** comprising **SP** and at least one biosurfactant **S_{Bio}**, is provided. **S_{Bio}** is a glycolipid, in particular selected from rhamnolipids, glucolipids, sophorolipids, preferably selected from rhamnolipids, sophorolipids, even more preferably selected from rhamnolipids.

Mixture **M*** may be provided in **variant 2*** in any form familiar to the skilled person, as long as such form comprises at least one biosurfactant **S_{Bio}** and spores **SP** that are still able to germinate. For example, mixture **M*** may be provided in the form of a preparation **P_{SP}** of the respective bacteria, comprising spores **SP,** wherein the preparation **P_{SP}** also comprises at least one biosurfactant **S_{Bio}**.

In those embodiments where **variant 2*** is applied in the context of a use according to the second aspect of the invention in a cleaning method, mixture **M*** may also be provided as a cleaning formulation **F.**

Mixture **M*** may comprise further components, especially depending on the use according to **variant 2*.**

In those embodiments where **variant 2*** is applied in the context of a use according to the second aspect of the invention in a cleaning method, mixture **M*** may be provided as a cleaning formulation **F,** wherein the preferred components of cleaning formulations **F** are further explained above.

Mixture **M*** is preferably aqueous.

In those embodiments where mixture **M*** is an aqueous mixture, it is preferred that the content of water in **M*** is from 1 wt.-% to 99 wt.-%, more preferably from 10 wt.-% to 99 wt.-%, more preferably from 30 wt.-% to 98 wt.-%, more preferably from 50 wt.-% to 97 wt.-%, more preferably from 60 wt.-% to 96 wt.-%, more preferably from 70 wt.-% to 95 wt.-%, more preferably from 80 wt.-% to 90 wt.-%, wherein the weight-% give the percentage of water relative to the total weight of the mixture **M*.**

### VIII.5.2.2 Step b2*)

In step b2*) of **variant 2*,** surface **S** is contacted with the mixture **M*,** so that a mixture **M₂** comprising **SP** and at least one biosurfactant **S_{Bio}** is obtained, wherein mixture **M₂** is in contact with surface **S.**

### VIII.5.2.2.1 Optional Pre-incubation in Step b2*)

In an optional embodiment, mixture **M*** is incubated before mixture **M*** is contacting **S** ("pre-incubation step").

This optional pre-incubation step is preferably performed when mixture **M*** is aqueous. It may be performed to the skilled person's knowledge, for example by incubating the mixture **M*** at a temperature in the range of from 1°C to 95 °C, preferably at a temperature in a range of from 10 °C to 70 °C, in particular in a range of from 20 °C to 65 °C, preferably in a range of from 25 °C to 55 °C, more preferably in a range of from 30 °C to 45 °C.

This optional pre-incubation step is preferably carried out for at least 1 second ("second" = "s"), preferably for at least 10 s, preferably for at least 1 minute ("minute" = "min"), more preferably for at least 5 min, preferably for at least 15 min, preferably for at least 60 min, more preferably from 15 min to 3 d, more preferably from 30 min to 2 d, more preferably from 45 min to 1 d.

During the optional pre-incubation step, mixture **M*** is preferably stirred.

The optional pre-incubation step is advantageous because it contacts the spores **SP** in the mixture **M*** with the at least one biosurfactant **S_{Bio}**, before mixture **M*** is applied to surface **S.** The early contacting of the spores **SP** with the at least one biosurfactant is believed to further facilitate the spores' **SP** germination on surface S during step c2).

### VIII.5.2.2.2 Contacting in Step b2*)

"Contacting surface **S** with the mixture **M*"** is a further essential feature of **variant 2*.** It results in a mixture **M₂** comprising **SP** and at least one biosurfactant **S_{Bio}** that is in contact with **S.** This is to be understood as meaning that the mixture **M₂** and at least a part of the surface **S** are in direct contact. In the context of the present invention "in direct contact" is to be understood as meaning "wetting" of the surface **S** with **M₂**. It will be appreciated that the surface **S** and the mixture **M₂** are thus in direct contact at a contact surface **S_{C}** of surface **S.**

The contacting step b2*) is not further limited. In **variant 2*,** the mixtures **M*** may simply be contacted with **S** to form mixture **M₂** in contact with **S.**

In those embodiments where **variant 2*** is applied in the context of a use according to the second aspect of the invention in a method of cleaning, it is preferred that the mixture **M₂** which is in contact with S at the end of step b2*) of **variant 2*,** is also in contact with at least one impurity **I.**

This means in particular that during step b2*) and/or during step c2*), preferably during step c2*), of **variant 2*,** at least one impurity **I** which is absorbed or adsorbed on the surface **S,** in particular contact surface **S_{C}**, is at least partially desorbed (and thus removed) from the surface **S** and dispersed or dissolved, in particular dispersed, in the mixture **M₂**. Optionally, the at least one impurity **I** is then degraded by at least one activity of at least one vegetative cell germinating from **SP** during step c2*) of **variant 2*.**

In other words, in this preferred embodiment, during step b2*) and/or during step c2*) of **variant 2*,** the mixture **M₂** is brought in direct contact with at least a part **S_{C}** of the surface **S,** wherein at least one impurity **I** is absorbed or adsorbed on **S_{C}**, so that the at least one impurity I is at least partially desorbed from the surface **S_{C}** and dispersed or dissolved, in particular dispersed, in the mixture **M₂** (and thus removed from surface **S_{C}**). The dispersed or dissolved, in particular dispersed, impurity **I** is then optionally degraded in the mixture **M₂** by at least one activity of at least one vegetative cell germinating from **SP** during step c2*) of **variant 2***.Therefore, in particular, after step c2*) of **variant 2*,** a mixture **M₂** is obtained wherein at least one impurity **I** in dissolved or dispersed, preferably dispersed.

The impurity **I** may either be in the form of a liquid (e.g. droplet) or a particle, and preferably is a particle.

In a preferred embodiment, the at least one impurity **I** is an impurity which is inorganic or organic, and is preferably organic.

Organic impurities are preferably selected from oils and fats, which may be of vegetable, animal or synthetic origin, food based particles, soil, pollen, sebum, body fluids, for example, blood, sperm, sweat, urine, feces, liquor.

Inorganic impurities are preferably selected from the group consisting of carbon black, fine dust, plastic particles (e.g. microplastic particles), metal oxides (preferably iron oxide), silica (such as sand), clay, dyes (such as pigments), more preferably clay, in particular kaolin.

Where **variant 2*** is used according to the second aspect of the invention in a method of cleaning, it is hence preferred that, when the spores **SP** of *Bacillus velezensis* strain DSM 34978, *Bacillus velezensis* DSM 34674, mutants of *Bacillus velezensis* DSM 34674 and/or mutants of *Bacillus velezensis* strain DSM 34978 are selected as spores **SP** in **variant 2*,** that at least a part of the impurities **I** cleaned during **variant 2*** are the substrates of these activities. Hence, it is preferred that in this preferred embodiment, the impurity **I** which is cleaned from the surface S during the use according to the second aspect of the invention in a cleaning method according to **variant 2*** is selected from the group consisting of valeric acid, proteins (in particular proteins selected from casein and whey protein), polysaccharides (in particular starch), cellulose. More preferred, it comprises proteins (in particular proteins selected from casein and whey protein), cellulose. Most preferred, it comprises proteins, in particular proteins selected from casein and whey protein.

Where **variant 2*** is used according to the second aspect of the invention in a method of cleaning, it is hence preferred that, when the spores **SP** of *Bacillus licheniformis* strain DSM 34977 and/or mutants of *Bacillus licheniformis* strain DSM 34977 are selected as spores **SP** in **variant 2*,** that at least a part of the impurities **I** cleaned during **variant 2*** are the substrates of this activity. Hence, it is preferred that in this preferred embodiment, the impurity **I** which is cleaned from the surface **S** during the use according to the second aspect of the invention in a cleaning method according to **variant 2*** is valeric acid.

Where **variant 2*** is used according to the second aspect of the invention in a method of cleaning, it is hence preferred that, when the spores **SP** of *Priestia megaterium* strain DSM 34980 and/or mutants of *Priestia megaterium* strain DSM 34980 are selected as spores **SP** in **variant 2*,** that at least a part of the impurities **I** cleaned during **variant 2*** are the substrates of these activities. Hence, it is preferred that in this preferred embodiment, the impurity **I** which is cleaned from the surface **S** during the use according to the second aspect of the invention in a cleaning method according to **variant 2*** is selected from the group consisting of valeric acid, proteins (in particular proteins selected from casein and whey protein), in particular proteins, which are even more preferably selected from casein and whey protein.

Where **variant 2*** is used according to the second aspect of the invention in a method of cleaning, it is hence preferred that, when the spores **SP** of *Bacillus subtilis* strain DSM 34981 and/or mutants of *Bacillus subtilis* strain DSM 34981 are selected as spores **SP** in **variant 2*,** that at least a part of the impurities I cleaned during **variant 2*** are the substrates of these activities. Hence, it is preferred that in this preferred embodiment, the impurity **I** which is cleaned from the surface **S** during the use according to the second aspect of the invention in a cleaning method according to **variant 2***is selected from the group consisting of valeric acid, grease, oil, more preferably grease, oil.

In a further embodiment, where the spores **SP** in the method according to **variant 2*** are selected from *Bacillus velezensis* strain DSM 34978 and mutants thereof, *Bacillus velezensis* DSM 34674 and mutants thereof, *Bacillus licheniformis* DSM strain DSM 34977 and mutants thereof, *Priestia megaterium* strain DSM 34980 and mutants thereof and *Bacillus subtilis* strain DSM 34981 and mutants thereof, it is preferred that the impurity **I** is selected from the group consisting of valeric acid, grease, oil, proteins (in particular proteins selected from casein and whey protein), polysaccharides (in particular starch), cellulose.

### VIII.5.2.3 Step c2*)

In step c2*) of **variant 2*, M₂** is incubated to germinate at least a part of the spores **SP** comprised by mixture **M₂**.

The skilled person is aware of the conditions to be applied in step c2*) that cause at least a part of the spores **SP** to germinate.

It is preferred that mixture **M₂** in steps b2*) and c2*) of **variant 2*** is an aqueous mixture comprising the spores **SP** and the at least one biosurfactant **S_{Bio}**.

This is preferably achieved by simply adding water to the mixture **M₂** during step c2*).

Alternatively or additionally, in step a2*) the mixture **M*** may simply be provided as an aqueous mixture.

In addition, in step b2*), the respective microorganism may also be contacted with nutrients and/or salts.

Preferred nutrients are selected from carbohydrates, amino acids, peptides, peptone, glucose, starch.

Peptides are preferably selected from peptone.

As amino acid, any of the natural amino acids may be selected by the skilled person. Preferably, the skilled person selects the amino acid from the group consisting of glutamine, alanine, valine, aspartate, glutamate, methionine, asparagine, preferably alanine, valine, asparagine.

Carbohydrates are preferably sugars, polysaccharides.

Preferred sugars are glucose, saccharose, fructose, galactose.

Preferred polysaccharides are starch, inulin.

Preferred salts are salts selected from sodium chloride, potassium phosphate, di-potassium hydrogen phosphate
Further additives known to the skilled person may also be applied to the mixture **M₂** in step c2*), such as tris(hydroxymethyl)aminomethane **("Tris").**

In addition, the germination of bacteria such as *Bacillus* or *Priestia* is also triggered by contacting the spores with substrates of one or more enzymatic or metabolic activity of these bacteria.

Hence, where spores **SP** of at least one of *Bacillus velezensis* strain DSM 34978, mutants of *Bacillus velezensis* strain DSM 34978, *Bacillus velezensis* DSM 34674, mutants of *Bacillus velezensis* DSM 34674, *Bacillus licheniformis* strain DSM 34977, mutants of *Bacillus licheniformis* strain DSM 34977, *Priestia megaterium* strain DSM 34980, mutants of *Priestia megaterium* strain DSM 34980, *Bacillus subtilis* strain DSM 34981 and mutants of *Bacillus subtilis* strain DSM 34981 are selected as spores **SP** in **variant 2*,** it is further preferable that the mixture **M₂** in step c2*) comprises the impurities **I** as set forth in the following:
*Bacillus velezensis* strain DSM 34978 and mutants of *Bacillus velezensis* strain DSM 34978, *Bacillus velezensis* DSM 34674, mutants of *Bacillus velezensis* DSM 34674: mixture **M₂** in step c2*) preferably comprises at least one impurity **I** selected from the group consisting of valeric acid, proteins (in particular proteins selected from casein and whey protein), polysaccharides (in particular starch), cellulose; more preferred at least one impurity **I** selected from the group consisting of proteins (in particular proteins selected from casein and whey protein), cellulose; most preferred at least one impurity **I** selected from the group consisting of proteins, in particular proteins selected from casein and whey protein.

*Bacillus licheniformis* strain DSM 34977 and mutants of *Bacillus licheniformis* strain DSM 34977: mixture **M₂** in step c2*) preferably comprises impurity **I** selected from valeric acid.

*Priestia megaterium* strain DSM 34980 and mutants of *Priestia megaterium* strain DSM 34980: mixture **M₂** in step c2*) preferably comprises at least one impurity **I** selected from the group consisting of valeric acid, proteins (in particular proteins selected from casein and whey protein), preferably at least one impurity **I** selected from the group consisting of proteins which even more preferably are selected from casein and whey protein.

*Bacillus subtilis* strain DSM 34981 and mutants of *Bacillus subtilis* strain DSM 34981: mixture **M₂** in step c2*) preferably comprises at least one impurity **I** selected from the group consisting of valeric acid, grease, oil, more preferably at least one impurity **I** selected from the group consisting of grease, oil.

The temperature of incubation in step c2*) is in particular in the range of from 10 °C to 70 °C, in particular in the range of from 20 °C to 65 °C, preferably in the range of from 25 °C to 55 °C, more preferably in the range of from 30 °C to 45 °C.

Where the surface **S** is the surface **S_{O}** of an object **O,** in particular where **variant 2*** is applied as a use according to the second aspect of the invention in a method for cleaning object **O,** it is preferred that the temperature in step c2*) is in a range of from 10 °C to 70 °C, in particular in a range of from 20 °C to 65 °C, preferably in a range of from 25 °C to 55 °C, more preferably in a range of from 30 °C to 45 °C.

Where the surface **S** is the surface **S_{B}** of the human or animal body **B,** in particular where **variant 2*** is applied as a use according to the second aspect of the invention in a method for cleaning the human or animal body **B,** it is preferred that the temperature in step c2*) is in a range of from 10 °C to 50 °C, in particular in a range of from 20 °C to 45 °C, preferably in a range of from 25 °C to 40 °C, more preferably in a range of from 30 °C to 38 °C, most preferably 37 °C.

In a further preferred embodiment, the incubation in step c2*) is carried out for at least 1 second ("second" = "s"), preferably for at least 10 s, preferably for at least 1 minute ("minute" = "min"), more preferably for at least 5 min, preferably for at least 15 min, preferably for at least 60 min, more preferably from 15 min to 3 d, more preferably from 30 min to 2 d, more preferably from 45 min to 1 d.

Preferably, in those cases where **M₂** comprises water, it is preferred that the pH of the water in **M₂** in step c2*) at 25 °C is from 3.5 to 9, preferably from 5 to 9, preferably from 6 to 9, and particularly preferably from 7 to 8.

It is further preferred that the incubation in step c2*) is carried out until at least 10 % of the spores **SP** comprised by mixture **M₂** that is contacted with S in step b2*) have germinated, relative to the number of spores **SP** comprised by mixture **M₂** before mixture **M₂** is subjected to the incubation according to step c2*) [i.e. relative to the number of spores **SP** comprised by mixture **M₂** that is obtained directly upon contacting **S** with mixture **M*** in step b2*)]. This "germination rate" in step c2*) is preferably determined according to **Assay M-II.**

It is even further preferred that the incubation in step c2*) is carried out until at least 20 % of the spores **SP** comprised by mixture **M₂** that is in contact with **S** in step b2*) have germinated, more preferably until at least 30 %, even more preferably until at least 40 %, even more preferably until at least 50 %, even more preferably until at least 60 %, even more preferably until at least 70 %, even more preferably until at least 80 %, even more preferably until at least 90 %, even more preferably until at least 95 %, even more preferably until at least 99 % of the spores **SP** comprised by mixture **M₂** that is in contact with **S** in step b2*) have germinated, relative to the number of spores **SP** comprised by mixture **M₂** before mixture **M₂** is subjected to the incubation according to step c2*).

### VIII.5.2.4 Step d2*) (optional)

It is preferred that, in a step d2*), which is carried out after step c2*), at least a part of **M₂** is separated from **S.** The separation is, in a preferred embodiment, carried out so that **M₂** is essentially complete separated from **S.**

This embodiment is particularly preferred where the method according to **variant 2*** is applied as a cleaning method, in particular on a surface **S_{O}** of an object **O** or on a surface **S_{B}** of the human or animal body **B.**

The skilled person is aware of how to carry out this separation in each specific context. Generally, this separation is carried out by withdrawing the surface **S** from at least a part of **M₂**.

An object **O** may, in step d2*), optionally be subjected to centrifugation (for example in a washing machine).

### VIII.5.2.5 Step e2*) (optional)

In those optional embodiments of **variant 2*** where step d2*) is carried out, it is even more preferred to carry out another optional step e2*) after step d2*).

In an optional step e2*) of **variant 2*,** the surface **S** is rinsed with further water.

### VIII.5.3 Variant 3*

**Variant 3*** reflects a further advantage of the present invention. Namely, in **variant 1*** and **variant 2,** the germination of the spores **SP** according to steps c1*) and c2*), respectively, is carried out in the presence of the at least one biosurfactant **S_{Bio}**. It was now found out that the effect of the at least one biosurfactant **S_{Bio}** on the germination is at least in part already achieved when the spores **SP** are pre-incubated with the at least one biosurfactant **S_{Bio}**. The at least one biosurfactant **S_{Bio}** exerts the positive effect even when not present during the germination step, but only during the pre-incubation.

### VIII.5.3.1 Step a3*)

In step a3*) of **variant 3*,** a mixture **M*** comprising **SP** and at least one biosurfactant **S_{Bio}**, is provided. **S_{Bio}** is a glycolipid, in particular selected from rhamnolipids, glucolipids, sophorolipids, preferably selected from rhamnolipids, sophorolipids, even more preferably selected from rhamnolipids.

Mixture **M*** may be provided in **variant 3*** in any form familiar to the skilled person, as long as such form comprises and at least one biosurfactant **S_{Bio}** and spores **SP** that are still able to germinate. For example, mixture **M*** may be provided in the form of a preparation **P_{SP}** of the respective bacteria, comprising spores **SP,** wherein the preparation **P_{SP}** also comprises at least one biosurfactant **S_{Bio}**.

In those embodiments where **variant 3*** is applied in the context of a use according to the second aspect of the invention in a cleaning method, mixture **M*** may also be provided as a cleaning formulation **F.**

Mixture **M*** may comprise further components, especially depending on the application of the method according to **variant 3*.**

In those embodiments where **variant 3*** is applied in the context of a use according to the second aspect of the invention in a cleaning method, mixture **M*** may be provided as a cleaning formulation **F,** wherein the preferred components of cleaning formulations **F** are further explained in the following.

Mixture **M*** is preferably aqueous.

In those embodiments where mixture **M*** is an aqueous mixture, it is preferred that the content of water in **M*** is from 1 wt.-% to 99 wt.-%, more preferably from 10 wt.-% to 99 wt.-%, more preferably from 30 wt.-% to 98 wt.-%, more preferably from 50 wt.-% to 97 wt.-%, more preferably from 60 wt.-% to 96 wt.-%, more preferably from 70 wt.-% to 95 wt.-%, more preferably from 80 wt.-% to 90 wt.-%, wherein the weight-% give the percentage of water relative to the total weight of the mixture **M*.**

### VIII.5.3.2 Step b3*)

Step b3*) of **variant 3*** comprises three consecutive sub-steps.

First, mixture **M*** is incubated ("pre-incubation").

Second, at least a part **π_{SP}** of the spores **SP** comprised by the incubated mixture **M*** is separated from at least a part of the biosurfactant **S_{Bio}** comprised by the incubated mixture **M***

Third, surface **S** is contacted with **π_{SP}**.

As a result of step b3*), a mixture **M₃** comprising **SP** and optionally at least one biosurfactant **S_{Bio}** is obtained, wherein mixture **M₃** is in contact with surface **S.**

### I.5.3.2.1 Pre-incubation in Step b3*)

Preferably, mixture **M*** that is subjected to the pre-incubation step in the context of step b3*) of **variant 3*** is aqueous.

This pre-incubation step may be performed according to the skilled person's knowledge, for example by incubating the mixture **M*** at a temperature in the range of from 1°C to 95 °C, preferably at a temperature in a range of from 10 °C to 70 °C, in particular in a range of from 20 °C to 65 °C, preferably in a range of from 25 °C to 55 °C, more preferably in a range of from 30 °C to 45 °C.

This pre-incubation step is preferably carried out for at least 1 second ("second" = "s"), preferably for at least 10 s, preferably for at least 1 minute ("minute" = "min"), more preferably for at least 5 min, preferably for at least 15 min, preferably for at least 60 min, more preferably from 15 min to 3 d, more preferably from 30 min to 2 d, more preferably from 45 min to 1 d.

During the pre-incubation step, mixture **M*** is preferably stirred.

The pre-incubation step is advantageous because it contacts the spores **SP** in the mixture **M*** with the at least one biosurfactant **S_{Bio}**, before mixture **M*** is applied to surface **S.** The early contacting of the spores **SP** with the at least one biosurfactant is believed to further facilitate the spores' **SP** germination on surface **S** during step c3*).

### I.5.3.2.2 Separation Step in Step b3*)

Second, at least a part **π_{SP}** of the spores **SP** comprised by the incubated mixture **M*** is separated from at least a part of the biosurfactant **S_{Bio}** comprised by the incubated mixture **M***

This separation step may be performed according to the skilled person's knowledge, for example by at least one step selected from filtration, centrifugation.

In case of centrifugation, at least a part **π_{SP}** of the spores **SP** will form the precipitate, while at least a part of the biosurfactant **S_{Bio}** will remain in the supernatant. The supernatant may then be removed and the precipitate washed, for example with water.

### 1.5.3.2.3 Contacting in Step b3*)

"Contacting surface **S** with the at least a part **π_{SP}** of the spores **SP"** is a further essential feature of **variant 3*.** It results in a mixture **M₃** comprising **SP.** In those embodiments of step b3*) where the separation of SP from the biosurfactant **S_{Bio}** was not complete, mixture **M₃** also comprises biosurfactant **S_{Bio}**. Hence, **M₃** comprises at least a part **π_{SP}** of the spores **SP** and optionally at least one biosurfactant **S_{Bio}**. In other words, **M₃** comprises spores **SP** and optionally at least one biosurfactant **S_{Bio}**.

**M₃** is in contact with **S.**

This is to be understood as meaning that the mixture **M₃** and at least a part of the surface S are in direct contact. In the context of the present invention "in direct contact" is to be understood as meaning "wetting" of the surface **S** with **M₃**. It will be appreciated that the surface **S** and the mixture **M₃** are thus in direct contact at a contact surface **S_{C}** of surface **S.**

The contacting step b3*) is not further limited. In **variant 3*,** the at least a part **π_{SP}** of the spores **SP** may simply be contacted with **S** to form mixture **M₃** in contact with **S.**

In those embodiments where **variant 3*** is applied in the context of a use according to the second aspect of the invention in a method of cleaning, it is preferred that the mixture **M₃** which is in contact with **S** at the end of step b3*) of **variant 3*,** is also in contact with at least one impurity **I.**

This means in particular that during step b3*) [in particular during the contacting step of step b3*)] and/or during step c3*) of **variant 3*,** at least one impurity **I** which is absorbed or adsorbed on the surface **S,** in particular contact surface **S_{C}**, is at least partially desorbed (and thus removed) from the surface **S** and dispersed or dissolved, in particular dispersed, in the mixture **M₃**. Optionally, the at least one impurity I is then degraded by at least one activity of at least one vegetative cell germinating from SP during step c3*) of **variant 3*.**

In other words, in this preferred embodiment, during step b3*) [i.e. the contacting step of step b3*)] and/or during step c3*) of **variant 3*,** the mixture **M₃** is brought in direct contact with at least a part **S_{C}** of the surface **S,** wherein at least one impurity **I** is absorbed or adsorbed on **Sc,** so that the at least one impurity **I** is at least partially desorbed from the surface **S_{C}** and dispersed or dissolved, in particular dispersed, in the mixture **M₃** (and thus removed from surface **S_{C}**). The dispersed or dissolved, in particular dispersed, impurity **I** is then optionally degraded in the mixture **M₃** by at least one activity of at least one vegetative cell germinating from **SP** during step c3*) of **variant 3*.**Therefore, in particular, after step c3*) of **variant 3*,** a mixture **M₃** is obtained wherein at least one impurity **I** in dissolved or dispersed, preferably dispersed.

The impurity **I** may either be in the form of a liquid (e.g. droplet) or a particle, and preferably is a particle.

In a preferred embodiment, the at least one impurity **I** is an impurity which is inorganic or organic, and is preferably organic.

Organic impurities are preferably selected from oils and fats, which may be of vegetable, animal or synthetic origin, food based particles, soil, pollen, sebum, body fluids, for example, blood, sperm, sweat, urine, feces, liquor.

Inorganic impurities are preferably selected from the group consisting of carbon black, fine dust, plastic particles (e.g. microplastic particles), metal oxides (preferably iron oxide), silica (such as sand), clay, dyes (such as pigments), more preferably clay, in particular kaolin.

Where **variant 3*** is used according to the second aspect of the invention in a method of cleaning, it is hence preferred that, when the spores **SP** of *Bacillus velezensis* strain DSM 34978, *Bacillus velezensis* DSM 34674, mutants of *Bacillus velezensis* DSM 34674 and/or mutants of *Bacillus velezensis* strain DSM 34978 are selected as spores **SP** in **variant 3*,** that at least a part of the impurities **I** cleaned during **variant 3*** are the substrates of these activities. Hence, it is preferred that in this preferred embodiment, the impurity **I** which is cleaned from the surface S during the use according to the second aspect of the invention in a cleaning method according to **variant 3*** is selected from the group consisting of valeric acid, proteins (in particular proteins selected from casein and whey protein), polysaccharides (in particular starch), cellulose. More preferred, it comprises proteins (in particular proteins selected from casein and whey protein), cellulose. Most preferred, it comprises proteins, in particular proteins selected from casein and whey protein.

Where **variant 3*** is used according to the second aspect of the invention in a method of cleaning, it is hence preferred that, when the spores **SP** of *Bacillus licheniformis* strain DSM 34977 and/or mutants of *Bacillus licheniformis* strain DSM 34977 are selected as spores **SP** in **variant 3*,** that at least a part of the impurities **I** cleaned during **variant 3*** are the substrates of this activity. Hence, it is preferred that in this preferred embodiment, the impurity **I** which is cleaned from the surface S during the use according to the second aspect of the invention in a cleaning method according to **variant 3*** is valeric acid.

Where **variant 3*** is used according to the second aspect of the invention in a method of cleaning, it is hence preferred that, when the spores **SP** of *Priestia megaterium* strain DSM 34980 and/or mutants of *Priestia megaterium* strain DSM 34980 are selected as spores **SP** in the method according to **variant 3*,** that at least a part of the impurities **I** cleaned during **variant 3*** are the substrates of these activities. Hence, it is preferred that in this preferred embodiment, the impurity **I** which is cleaned from the surface S during the use according to the second aspect of the invention in a cleaning method according to **variant 3*** is selected from the group consisting of valeric acid, proteins (in particular proteins selected from casein and whey protein), in particular proteins, which are even more preferably selected from casein and whey protein.

Where **variant 3*** is used according to the second aspect of the invention in a method of cleaning, it is hence preferred that, when the spores **SP** of *Bacillus subtilis* strain DSM 34981 and/or mutants of *Bacillus subtilis* strain DSM 34981 are selected as spores **SP** in the method according to **variant 3*,** that at least a part of the impurities **I** cleaned during **variant 3*** are the substrates of these activities. Hence, it is preferred that in this preferred embodiment, the impurity **I** which is cleaned from the surface S during the use according to the second aspect of the invention in a cleaning method according to **variant 3*** is selected from the group consisting of valeric acid, grease, oil, more preferably grease, oil.

In a further embodiment, where the spores **SP** in the method according to **variant 3*** are selected from *Bacillus velezensis* strain DSM 34978 and mutants thereof, *Bacillus velezensis* DSM 34674 and mutants thereof, *Bacillus licheniformis* DSM strain DSM 34977 and mutants thereof, *Priestia megaterium* strain DSM 34980 and mutants thereof and *Bacillus subtilis* strain DSM 34981 and mutants thereof, it is preferred that the impurity **I** is selected from the group consisting of valeric acid, grease, oil, proteins (in particular proteins selected from casein and whey protein), polysaccharides (in particular starch), cellulose.

### VIII.5.3.3 Step c3*)

In step c3*) of **variant 3*, M₃** is incubated to germinate at least a part of the spores **SP** comprised by mixture **M₃**.

The skilled person is aware of the conditions to be applied in step c3*) that cause at least a part of the spores **SP** to germinate.

It is preferred that mixture **M₃** in steps b3*) and c3*) of **variant 3** is an aqueous mixture comprising the spores **SP** and the at least one biosurfactant **S_{Bio}**.

This is preferably achieved by simply adding water to the mixture **M₃** during step c3*).

Alternatively or additionally, in step a3*) the mixture **M*** may simply be provided as an aqueous mixture.

In addition, in step b3*), the respective microorganism may also be contacted with nutrients and/or salts.

Preferred nutrients are selected from carbohydrates, amino acids, peptides, peptone, glucose, starch.

Peptides are preferably selected from peptone.

As amino acid, any of the natural amino acids may be selected by the skilled person. Preferably, the skilled person selects the amino acid from the group consisting of glutamine, alanine, valine, aspartate, glutamate, methionine, asparagine, preferably alanine, valine, asparagine.

Carbohydrates are preferably sugars, polysaccharides.

Preferred sugars are glucose, saccharose, fructose, galactose.

Preferred polysaccharides are starch, inulin.

Preferred salts are salts selected from sodium chloride, potassium phosphate, di-potassium hydrogen phosphate

Further additives known to the skilled person may also be applied to the mixture **M₁** in step c1), such as tris(hydroxymethyl)aminomethane **("Tris").**

In addition, the germination of bacteria such as *Bacillus* or *Priestia* is also triggered by contacting the spores with substrates of one or more enzymatic or metabolic activity of these bacteria.

Hence, where spores **SP** of at least one of *Bacillus velezensis* strain DSM 34978, mutants of *Bacillus velezensis* strain DSM 34978, *Bacillus velezensis* DSM 34674, mutants of *Bacillus velezensis* DSM 34674, *Bacillus licheniformis* strain DSM 34977, mutants of *Bacillus licheniformis* strain DSM 34977, *Priestia megaterium* strain DSM 34980, mutants of *Priestia megaterium* strain DSM 34980, *Bacillus subtilis* strain DSM 34981 and mutants of *Bacillus subtilis* strain DSM 34981 are selected as spores **SP** in **variant 3*,** it is further preferable that the mixture **M₃** in step c3*) comprises the impurities **I** as set forth in the following:
*Bacillus velezensis* strain DSM 34978 and mutants of *Bacillus velezensis* strain DSM 34978, *Bacillus velezensis* DSM 34674, mutants of *Bacillus velezensis* DSM 34674: mixture **M₃** in step c3*) preferably comprises at least one impurity **I** selected from the group consisting of valeric acid, proteins (in particular proteins selected from casein and whey protein), polysaccharides (in particular starch), cellulose; more preferred at least one impurity **I** selected from the group consisting of proteins (in particular proteins selected from casein and whey protein), cellulose; most preferred at least one impurity **I** selected from the group consisting of proteins, in particular proteins selected from casein and whey protein.

*Bacillus licheniformis* strain DSM 34977 and mutants of *Bacillus licheniformis* strain DSM 34977: mixture **M₃** in step c3*) preferably comprises impurity **I** selected from valeric acid.

*Priestia megaterium* strain DSM 34980 and mutants of *Priestia megaterium* strain DSM 34980: mixture **M₃** in step c3*) preferably comprises at least one impurity **I** selected from the group consisting of valeric acid, proteins (in particular proteins selected from casein and whey protein), preferably at least one impurity I selected from the group consisting of proteins which even more preferably are selected from casein and whey protein.

*Bacillus subtilis* strain DSM 34981 and mutants of *Bacillus subtilis* strain DSM 34981: mixture **M₃** in step c3*) preferably comprises at least one impurity **I** selected from the group consisting of valeric acid, grease, oil, more preferably at least one impurity **I** selected from the group consisting of grease, oil.

The temperature of incubation in step c3*) is in particular in the range of from 10 °C to 70 °C, in particular in the range of from 20 °C to 65 °C, preferably in the range of from 25 °C to 55 °C, more preferably in the range of from 30 °C to 45 °C.

Where the surface **S** is the surface **S_{O}** of an object **O,** in particular where **variant 3*** is applied as a use according to the second aspect of the invention in a method for cleaning object **O,** it is preferred that the temperature in step c3*) is in a range of from 10 °C to 70 °C, in particular in a range of from 20 °C to 65 °C, preferably in a range of from 25 °C to 55 °C, more preferably in a range of from 30 °C to 45 °C.

Where the surface **S** is the surface **S_{B}** of the human or animal body **B,** in particular where **variant 3*** is applied as a use according to the second aspect of the invention in a method for cleaning the human or animal body **B,** it is preferred that the temperature in step c3*) is in a range of from 10 °C to 50 °C, in particular in a range of from 20 °C to 45 °C, preferably in a range of from 25 °C to 40 °C, more preferably in a range of from 30 °C to 38 °C, most preferably 37 °C.

In a further preferred embodiment, the incubation in step c3*) is carried out for at least 1 second ("second" = "s"), preferably for at least 10 s, preferably for at least 1 minute ("minute" = "min"), more preferably for at least 5 min, preferably for at least 15 min, preferably for at least 60 min, more preferably from 15 min to 3 d, more preferably from 30 min to 2 d, more preferably from 45 min to 1 d.

Preferably, in those cases where **M₃** comprises water, it is preferred that the pH of the water in **M₃** in step c3*) at 25 °C is from 3.5 to 9, preferably from 5 to 9, preferably from 6 to 9, and particularly preferably from 7 to 8.

It is further preferred that the incubation in step c3*) is carried out until at least 10 % of the spores **SP** comprised by mixture **M₃** that is contacted with **S** in step b3*) have germinated, relative to the number of spores **SP** comprised by mixture **M₃** that is subjected to the incubation according to step c3*) [i.e. relative to the number of spores **SP** comprised by mixture **M₃** that is obtained directly upon contacting **S** with **π_{SP}** in step b3*]) have germinated.

This "germination rate" in step c3*) is preferably determined according to **Assay M-III.**

It is even further preferred that the incubation in step c3*) is carried out until at least 20 % of the spores **SP** comprised by mixture **M₃** that is in contact with **S** in step b3*) have germinated, more preferably until at least 30 %, even more preferably until at least 40 %, even more preferably until at least 50 %, even more preferably until at least 60 %, even more preferably until at least 70 %, even more preferably until at least 80 %, even more preferably until at least 90 %, even more preferably until at least 95 %, even more preferably until at least 99 % of the spores **SP** comprised by mixture **M₃** that is in contact with **S** in step b3*) have germinated, relative to the number of spores **SP** comprised by mixture **M₃** mixture **M₃** is subjected to the incubation according to step c3*).

### VIII.5.3.4 Step d3*) (optional)

It is preferred that, in a step d3*), which is carried out after step c3*), at least a part of **M₃** is separated from **S.** The separation is, in a preferred embodiment, carried out so that **M₃** is essentially complete separated from **S.**

This embodiment is particularly preferred where the method according to **variant 3** is applied as a cleaning method, in particular on a surface **S_{O}** of an object **O** or on a surface **S_{B}** of the human or animal body **B**.

The skilled person is aware of how to carry out this separation in each specific context. Generally, this separation is carried out by withdrawing the surface **S** from at least a part of **M₃.**

An object **O** may, in step d3*), optionally be subjected to centrifugation (for example in a washing machine).

### VIII.5.3.5 Step e3*) (optional)

In those optional embodiments of **variant 3** where step d3*) is carried out, it is even more preferred to carry out another optional step e3*) after step d3*).

In an optional step e3*) of **variant 3,** the surface **S** is rinsed with further water.

### Examples

Examples **I.** to **III.** show advantageous enzymatic activities of *Bacillus* and *Priestia* strains which may advantageously be used in cleaning where metabolization of different substrates underlies the cleaning effect. Preferably, they are used in cleaning.

Example **IV**. shows the surprising properties of the different bacterial species and strains in terms of positive or negative growth rate in presence of **VA** and **VA** reduction rate.

Example **V.** shows the surprising properties of the glycolipids, in particular rhamnolipids, to enhance the spreading of spores of different bacterial species. The surprising findings described in Example **V.** underlie the present invention.

### I. Determination of protease, amylase, and cellulase activity of Bacillus velezensis DSM 34978 and DSM 34674

Five different *Bacillus velezensis* strains were tested for protease, amylase and cellulase activity in a testing procedure according to **Assay A-I, Assay E,** and **Assay C,** respectively.

Two *Bacillus velezensis* strains DSM 34978 and DSM 34674 were isolated from the soil, respectively. Three further *Bacillus velezensis* strains (abbreviated as "*Bacillus velezensis* strain 1", "*Bacillus velezensis* strain 2", and "*Bacillus velezensis* strain 3", respectively) were isolated from commercially available cleaning products A, B, and C, respectively, and investigated as comparisons.

### 1.1 Determination of the protease activity

The testing for protease activity was carried out as follows:
Each of the strains of interest was cultivated in 10 ml tryptic-soy-broth [**TSB**; Merck - 105459 (peptone from casein 17.0 g/L; peptone from soymeal 3.0 g/L; D (+) glucose monohydrate 2.5 g/L; sodium chloride 5.0 g/L; di-potassium hydrogen phosphate 2.5 g/L)] at 37 °C and 200 rpm overnight (16 h) separately. As inoculum, 20 µl of a cryo-conservation culture was used for each strain. After the overnight cultivation, the optical density was measured at λ = 660 nm using a cuvette photometer and a culture volume for OD 1 (in 1 ml) was adjusted by centrifugation (5000 x g, 3 min, room temperature). The filter-sterile supernatant (previously adjusted to OD1) was used for the qualitative protease test on specific selective agar plates.

10 % Crossley milk agar (**CM0213**, Oxoid; 10 g/L skim milk powder, 1 g/L peptone, 0.01 g/L bromocresol purple, 13 g/L agar-agar) was prepared and 5 µl of the supernatant of each strain of interest was dropped on the agar plates. After an incubation at 37 °C for 24 h, the protease activity was indicated by a clearance zone ("halo of degradation"), which was measured and compared to a pure protease from *Bacillus licheniformis* (Subtilisin, P5380, Sigma-Aldrich) concentrated to 1 mg/ml. Through the indicator dye in the agar medium, the elucidation by protease degradation is directly visible. The clearance zone is given as an average of the horizontal and vertical diameters. Values are given in millimeters (mm) of two independent biological replicates. The results are summarized in table 1.

### 1.2 Determination of the amylase activity

Like the protease activity test, the amylase activity was also checked in a qualitative plate test.

The strains of interest were cultivated in 10 ml tryptic-soy-broth with soluble starch [**TSB**; Merck- 105459 (peptone from casein 17.0 g/L; peptone from soymeal 3.0 g/L; D(+)glucose monohydrate 2.5 g/L; sodium chloride 5.0 g/L; di-potassium hydrogen phosphate 2.5 g/L) + 2.5 g/L soluble starch (Merck) at 37 °C and 200 rpm overnight (16 h) separately. As inoculum, 20 µl of a cryo-conservation culture was used for each strain. After the overnight cultivation, the optical density was measured at λ = 660 nm using a cuvette photometer and a culture volume for OD1 (in 1ml) was adjusted by centrifugation (5000 x g, 3 min, room temperature). The filter-sterile supernatant (previously adjusted to OD1 and sterilized using a 0.2 µm filter) was used for the qualitative amylase test on specific selective agar plates.

Starch agar plates were prepared by using 37 g/L Luria-Bertani ("**LB**") agar (Merck-110283) with10 g/L soluble starch (Merck, CAS: 9005-84-9). 5 µl of the supernatant of each strain were dropped on the agar plate. Plates were incubated at 37 °C for 24 h. The α-amylase from *Bacillus licheniformis* (CAS: 9000-85-5) was used as control. 5 µl of a prepared 0.1 mg/ml solution was dropped similarly. Specifically, the amylase activity was observed as a clear zone around the bacterial supernatant spots or the α-amylase spot, becoming evident after application of 10 ml of 10 % Lugol's solution (5 g iodine, 10 g potassium iodide in 10 ml distilled water) to the plate. After removing the Lugol's solution, the diameter of the clearance zones was measured horizontally and vertically, and the mean thereof used for assessing the area of the clearance zone. The test is performed in two independent biological replicates. The results are summarized in table 1.

### 1.3 Determination of the cellulase activity

The cell free supernatant of the *Bacillus* strains of interest was tested regarding cellulase activity. Cellulase activity produced by the different *Bacillus* strains of interest and released in the supernatant was quantified with the EnzChek^{®} Cellulase kit from Molecular Probes/life technologies (E33953) following the working protocol. The principle of this test is based on the cellulase-catalyzed conversion of the substrate EnzChek^{®} Cellulase Substrate, blue fluorescent, 339/452, whereby the fluorescence is enhanced.

The supernatant was prepared beforehand. A pre-culture of each strain of interest was done in 10 ml **VIB** medium (Difco Veal Infusion Broth, Ref. 234420) at 37 °C and 200 rpm overnight (16 h). After the incubation, a new main culture with 2.5 ml **CMC** Growth Medium [minimal-salt medium with 10 g/L carboxymethyl cellulose (Sigma 419311)] was inoculated with 125 µl pre-culture using a small deep-well incubation plate and incubated at 37 °C for 24 h at 300 rpm. After 24 hours, the OD at λ = 660 nm of each culture was measured and the supernatant harvested by centrifugation. This was done with a 1 ml aliquot of each culture in a 1.5 ml Eppendorf tube for 5 min at 13000 rpm. After that, approx. 500 µl of culture supernatant was used for the enzyme test.

The detection limit of the cellulase kit was determined as 4mU-3U/ml activity. Therefore, a 2U/ml cellulase from *Aspergillus niger* (Sigma-Aldrich, C1184) and dilutions were prepared for a standard curve for quantification. The assay was done according to the kit manual. The measurement was performed in black 96-well microtiter plates (Greiner FIA-Plate, black 96K, F-form, 655076-225) and in the Tecan plate reader infinite M1000 Pro. Subsequently, the fluorescence was measured at an excitation of λ = 339 nm and an emission of λ = 452 nm. The cellulase activity was calculated based on the standard curve and the activity was normalized to OD 1 (λ = 660 nm) to standardize over cell growth differences after 24 hours incubation.

### 1.4 Results

The results are summarized in the following table 1:

**Table 1: Protease and amylase activities in supernatants ("SN") measured via a qualitative plate assay by determining the halo of degradation size (Ø in mm). Cellulase activity of supernatants is given as quantitative values in mU/OD1. The experiments were performed in two biological replicates and the means and standard deviations ("σ") were given. Comparative strains 1, 2, 3 from product A, B and C, respectively, belong to the Bacillus species velezensis as DSM 34978 and DSM 34674.**

| **Strain** | **Protease activity SN** | **Amylase activity SN** | **Cellulase activity SN** |
|---|---|---|---|
| | **Ø** halo of degradation [mm] | **Ø** halo of degradation [mm] | mU/OD 1 |
| **DSM 34978** | 16.97 (**σ** 0.32) | 7.54 (**σ** 0.58) | 1660.76 (**σ** 440.15) |
| **DSM 34674** | 15.89 (**σ** 0.32) | 7.34 (**σ** 1.26) | 1084.81 (**σ** 153.33) |
| **Strain 1** | 12.9 (**σ** 0.765) | 7.50 (**σ** 1.96) | 944.01 (**σ** 271.23) |
| **Strain 2** | 14.30 (**σ** 0.53) | 5.99 (**σ** 0.75) | 764.29 (**σ** 113.04) |
| **Strain 3** | 13.74 (**σ** 0.18) | 7.50 (**σ** 0.45) | 751.43 (**σ** 77.52) |

### I.4.1 Protease Activities

*Bacillus velezensis* strains DSM 34978 and DSM 34674 showed remarkably high activity for protease compared to the other screened *Bacillus* strains. As described under point **I**.1, the supernatants were harvested from a culture of each strain and adjusted to an optical density of OD1 to standardize over growth effects. The degradation or clearance zones is measured as the diameter ("**Ø**") measured (diameter in mm) have a **Ø** size of 16.97 mm and 15.89 mm for DSM 34978 and DSM 34674, respectively. Comparative *Bacillus velezensis* strains 1, 2 and 3 were tested in the same way and it could be shown that the protease activity of all of these strains was lower (table 1).

It was hence surprisingly found that DSM 34978 and DSM 34674 supernatants contained the highest levels of proteases which can degrade casein and whey proteins very effectively.

These halo sizes were compared to the halo of a pure protease from *Bacillus licheniformis* with an activity of 7-15 U/mg. The 5 µl of the 1 mg/ml solutions have a calculated activity of around 35-75 µU, that is represented by a degradation halo **Ø** size of 22.12 mm.

The qualitative plate assay hence showed that the two *Bacillus velezensis* strains DSM 34978 and DSM 34674 produced protease activity that have the potential to compete with pure industrial enzymes. Simultaneously, the expression level of each activity was higher than of the respective expression level of prior art *Bacillus velezensis* strains which were already used in cleaning products.

### 1.4.2 Amylase Activities

Likewise, as in case of the protease activity, the two *Bacillus velezensis* strains DSM 34978 and DSM 34674 also proved to be very good in producing amylase activity. The tested supernatants, harvested from a 16 h cultivation, were found to contain amylases which degrade starch and produce a halo of degradation of the **Ø** sizes 7.54 mm and 7.34 mm for the strains DSM 34978 and DSM 34674, respectively. The corresponding α-amylase from *Bacillus licheniformis* with an activity of 250 mU was found to produce a halo of degradation of **Ø** 13.13 mm size.

The qualitative plate assay hence showed that the two *Bacillus velezensis* strains DSM 34978 and DSM 34674 produced amylase activity that can compete with pure industrial enzymes. Simultaneously, the expression level of each activity was well in the range of (and in case of DSM 34978 even higher than) the respective expression level of prior art *Bacillus velezensis* strains 1, 2, and 3, which were already used in cleaning products. *Bacillus velezensis* strains DSM 34978 showed the highest expression level of amylase compared to all other tested strains.

### 1.4.3 Cellulase Activities

The cellulase activity was measured with a quantitative assay.

Just as in the case of the protease and amylase activity test, the supernatants of the two *Bacillus velezensis* strains DSM 34978 and DSM 34674 showed very high cellulase activity, which may be due to the fact that both strains were isolated from the soil containing different fiber and cellulose compounds which must be broken down to fuel their energy metabolism and keep them alive.

The normalized supernatant (OD 1) of *Bacillus velezensis* DSM 34978 showed an activity of 1660.76 mU, whereas the *Bacillus velezensis* DSM 34674 produced less cellulase, but overall an activity of 1084.81 mU. These data were compared with the cellulase activity of *Bacillus velezensis* strains 1, 2, and 3 from product samples. Not one of these comparative strains A to C could compete with the two high cellulase producers DSM 34978 and DSM 34674, as may be seen from table 1.

### 1.4.4 Summary

These data show that both strains *Bacillus velezensis* DSM 34978 and *Bacillus velezensis* DSM 34674 produce the highest concentrations of proteases and cellulases which are released in the environment to degrade several types of stains of organic origin and are better than already used strains of commercially available cleaning products. In addition, *Bacillus velezensis* strain DSM 34978 produces the highest concentration of amylases, further improving its efficacy as microbial cleaning agent.

### II. Determination of protease activity of Priestia megaterium DSM 34980

Two different *Priestia megaterium* strains and one *Bacillus velezensis* strain were tested for protease activity in a testing procedure according to **Assay A-II.**

*Priestia megaterium* DSM 34980 was isolated from soil rifled by wild boar.

One further *Priestia megaterium* strain (abbreviated as "*Priestia megaterium* strain 4") and one further *Bacillus velezensis* strain (abbreviated as *"Bacillus velezensis* strain 5") were isolated from commercially available cleaning products D and E, respectively, and investigated as comparisons.

Strains of interest were grown separately on **TSA**-agar medium, overnight at 37 °C. Then, each strain was collected, washed three times with phosphate-buffered saline ("**PBS**"; Sigma, P4417-100TAB; 8000 x g centrifugation, 3 min, room temperature), and adjusted to an optical density (OD at λ = 600 nm) equal to 0.1. The test consists of applying 10 µl of the single strain suspensions onto skim milk media surfaces which mimic the food-like dirt that might be deposited on surfaces.

For observing milk protease, skim milk agar medium (skim milk powder 28 g/L, casein enzymatic hydrolysate 5 g/L, yeast extract 2.5 g/L, glucose 1 g/L, agar 15 g/L) was used. The plates were incubated for seven days at room temperature. Following the incubation, the respective protease enzymatic activity was indicated by a halo of degradation [diameter ("**Ø**") in cm] surrounding active spots of growing bacteria.

The results are summarized in the following table 2:

**Table 2: Protease activities measured via a qualitative plate assay by determining the halo degradation size (Ø in cm) which is visible of a grown colony on plate. The experiments were performed in two biological replicates and the means and standard deviations ("σ") were given. Strain 4 and 5 were isolated from one commercially available cleaning product and are compared.**

| **Strain** | **Protease activity cells** |
|---|---|
| | **Ø** halo of degradation [cm] |
| **DSM 34980** | 2.33 (**σ** 0.1) |
| **Strain 4 (*Priestia megaterium*)** | 0.75 (**σ** 0.2) |
| **Strain 5 (*Bacillus velezensis*)** | 1.8 (**σ** 0.35) |

Surprisingly, *Priestia megaterium* DSM 34980 showed a halo of degradation of **Ø** size of 2.33 (± 0.1) cm. Another strain of the same species (strain 4), used in a commercially available cleaning product, showed a 1.58 cm smaller halo of degradation, i.e. of a **Ø** size of 0.75 (± 0.2) cm. A further comparative strain (strain 5) of the species *Bacillus velezensis* showed a better performance, however, still inferior to the performance of *Priestia megaterium* strain DSM 34980 (table 2).

*Priestia megaterium* strain DSM 34980 hence showed the most extended halo on skim milk protease plates. Thus, this strain sowed the highest degradation, indicating that during a long-term growth many milk proteases could be produced and were most likely cell-wall bound.

### III. Determination of lipase activity of Bacillus subtilis DSM 34981

Three different *Bacillus subtilis* strains were tested for lipase activity in a testing procedure according to **Assay G.**

*Bacillus subtilis* DSM 34981 was isolated from an environmental soil sample. Two further *Bacillus subtilis* strains (abbreviated as "*Bacillus subtilis* strains 6 and 7) were isolated from commercially available cleaning products D and B, respectively, and investigated as comparisons.

In these tests, the ability to directly measure the hydrolysis of free **p-NP** due to a photometric assay was used. The test was performed with 4 mmol/L 4-nitrophenyloctanonate ("**4-NP-C8**"; Sigma-Aldrich, CAS:1956-10-1). A stock solution **S_{4NPC8}** [confer step **(G-3)** supra] was prepared in *iso*-propanol and further diluted in 0.1 M Tris-buffer pH 8.5. 20 µl of this stock solution of **4-NP-C8** was used in each test.

For the preparation of the test solution, each bacterial strain of interest was pre-cultivated in 10 ml tryptic-soy-broth ["**TSB**"; Merck- 105459; peptone from casein 17.0 g/L; peptone from soymeal 3.0 g/L; D(+)glucose monohydrate 2.5 g/L; sodium chloride 5.0 g/L; di-potassium hydrogen phosphate 2.5 g/L)) at 37 °C and 200 rpm overnight (16 h).

A new main culture was inoculated with pre-culture to an OD at λ = 660 nm of 0.1. Beforehand the pre-culture was washed twice with main culture medium **MCM₀** (described in the context of **Assay J**). The main culture was incubated at 37 °C and 200 rpm agitation for 4 hours. Afterwards, the cultures were harvested by centrifugation (5000 x g, 3 min, room temperature) and adjusted to OD 0.25 in 180 µl 0.1 M Tris-buffer. 180 µl bacterial cell suspension was mixed with 20 µl **4-NP-C8** substrate solution **S_{4NPC8}** (the preparation of **S_{4NC8}** is described in step **(G-3)** of **Assay G**) in a 96-well microliter plate (Greiner bio one, Ref. 655101) and incubated at 25 °C for 60 min in a Tecan spark plate reader. The determination was carried out in the Tecan plate reader at λ = 410 nm. Every 2 min, a measurement was done and after 60 min the final absorbance at λ = 410 nm was used for the evaluation. For the determination of the baseline, 20 µl substrate solution **S_{4NPC8}** was mixed with 180 µl Tris buffer and as positive control different concentrations of a pure lipase from *Candida rugosa* (Sigma-Aldrich, L174) were used. Additionally, a p-nitrophenol standard series (0-10 mmol/L) was prepared and used to quantify the amount of released **p-NP** (yellow) to determine the lipase activity in the different *Bacillus* suspensions. The activity of expressed lipase is given in mU/ml as mean value from two independent biological experiments.

The results are summarized in table 3:

**Table 3: Lipase activities in mU/ml or in mU/OD1 are shown as mean values ("σ"=standard deviation) of two biological replicates. For the quantification of lipase activity, the para-nitrophenyl (p-NP) ester with a C8 chain was used. The quantification was made based on a p-NP standard curve. Strain 4 and 5 from product D and B belong to the Bacillus species subtilis as known for the DSM 34981.**

| **Strain** | **Lipase activity** | |
|---|---|---|
| | mU/ml | mU/OD1 |
| **DSM 34981** | 3.40 (**σ** 0.97) | 2.72 (**σ** 0.77) |
| **Strain 6, product D** | 0.753 (**σ** 0.06) | 0.60 (**σ** 0.04) |
| **Strain 7, product B** | 3.07 (**σ** 0.20) | 2.46 (**σ** 0.16) |

Surprisingly, *Bacillus subtilis* DSM 34981 showed the highest lipase production. The strain was isolated from an environmental soil sample and could hydrolyze 4-nitrophenyloctanonate into the C8 chain and p-nitrophenol. Already after 30 min, almost all of the 4 mM substrate was degraded and after 60 min the activity was measured with 3.40 mU/ml which is comparable to the activity of 5µg/ml lipase from *Candida rugosa.* Tested strains 6 and 7 from commercially available cleaning products show an even lower activity tested for the same substrate (table 3).

*Bacillus subtilis* DSM 34981 is hence an excellent candidate for degrading oil and grease indeed. The responsible lipases are cell-wall bound or could be produced during the 1-hour of incubation with the substrate.

### IV. Determination of valeric acid metabolism

### IV.1 Determination of tolerance to valeric acid and the ability to metabolize valeric acid

Different *Bacillus* and *Priestia* species were tested for their ability to grow on valeric acid medium and their ability to metabolize valeric acid. For these investigations, a testing procedure according to **Assay J** was used.

The tested *Bacillus velezensis* species were *Bacillus velezensis* DSM 34978 and
*Bacillus velezensis* DSM 34674.

The tested *Bacillus licheniformis* species was *Bacillus licheniformis* DSM 34977.

The tested *Priestia megaterium* species was *Priestia megaterium* DSM 34980.

The tested *Bacillus subtilis* species was *Bacillus subtilis* DSM 34981.

These five *Bacillus* / *Priestia* strains were tested in the presence of valeric acid ("**VA**") in a "malodor control test". In particular, in the malodor control test, it was tested whether these *Bacillus* / *Priestia* strains have the ability to tolerate and likewise to reduce 1000 ppm valeric acid. To this aim, the growth in a minimal medium with 1 wt.-% glucose as opposed to the growth in 1 wt.-% glucose with 1 wt.-% **VA** was compared.

Each *Bacillus* / *Priestia* strain of interest was pre-cultivated in 10 ml tryptic-soy-broth ["**TSB**"; Merck-105459; peptone from casein 17.0 g/L; peptone from soymeal 3.0 g/L; D (+) glucose monohydrate 2.5 g/L; sodium chloride 5.0 g/L; di-potassium hydrogen phosphate 2.5 g/L] at 30 °C and 200 rpm overnight (16 h). The cells were washed with NaCl solution and used for inoculating the main culture. For the main cultures, **MCM_{+Val}** medium as described in the context of **Assay J** and **MCM₀** as described in the context of **Assay J** were used as media. For the cultivation of different *Bacillus* / *Priestia* strains inoculated in different media compositions a small cultivation system, Woulter-Deutz, was used, which allowed to measure up to 24 batches in parallel.

Main cultures were inoculated with washed pre-culture to OD 0.1 and were incubated at 30 °C with 225 rpm for 48 hours. After 48 hours, the final optical density at λ = 660 nm was measured of each culture and subsequently the supernatants were harvested by centrifugation (13000 x g, 3 min, room temperature). The concentration of valeric acid in the samples was quantified using an analytic method (see point VII.5.1.5). The values were compared to the medium control without a *Bacillus* / *Priestia* inoculum. The percentage reduction of valeric acid based on the medium control was calculated for each batch with *Bacillus* / *Priestia* inoculum. Values were given as means of technical replicates.

The quantification of valeric acid was done by the following analytical method.

All samples and a series of calibration standards were mixed with an internal standard (2-methylhexanoic acid). An excess of NaCl was added and samples were diluted with 1 part 50 wt.-% sulfuric acid to 4 parts sample. The resulting mixture was extracted in a ratio of 1 part diethyl ether to 1.25 parts sample for 10 minutes and then centrifuged for 10 minutes at 3000 x g. The organic supernatant was removed and, if necessary, further diluted to a concentration range of 1 g/L. Samples and calibration standards were measured using an Agilent GC 8860 equipped with split/spitless injector and FID detector on a GC column (BP21 30m; ID 0.32 mm; film 0.25 µm) at a flow rate of 1 ml/min He. The separation was carried out by means of a temperature gradient from 130 °C to 180 °C within 8.5 min. The chromatograms were evaluated by means of a multi-point area evaluation linear without zero crossing with 2-methylhexanoic acid as the internal standard. Valeric acid was eluting at 5.4 min within the program.

### IV.2 Results

The optical density at λ = 660 nm (OD) of cultures without and with **VA** after 48 h was measured. In addition, the reduction of **VA** of cultures with **VA** was determined. The results are summarized in Table 4.

**Table 4: Malodor reduction. The final OD at λ = 660 nm was measured of approaches with and without VA after 48 h. Means were given. Delta OD was calculated based of final OD with VA to final OD without VA ("+" =increase, "- "= decrease). The VA in the different approaches was quantified in the supernatant and given as percentage reduction compared to the start concentration/medium without bacterial inoculation.**

| **Strain** | **OD after 48 h w/o VA** | **OD after 48 h with VA** | **Delta OD compared to w/o VA** | **VA reduction after 48 h in %** |
|---|---|---|---|---|
| **DSM 34978 (*B. velezensis*)** | 0.986 | 0.626 | -0.359 | 57 |
| **DSM 34977 (*B. licheniformis*)** | 0.377 | 1.662 | +1.285 | 23 |
| **DSM 34674 (*B. velezensis*)** | 0.926 | 0.472 | -0.454 | 41 |
| **DSM 34980** | 0.992 | 1.058 | +0.065 | 29 |
| **(*P. megaterium*)** | | | | |
| **DSM 34981 (*B. subtilis*)** | 0.719 | 1.329 | +0.610 | 8 |

From the results summarized in table 4, it follows that only three of the five strain samples, namely the samples containing strains of *Priestia megaterium* (DSM 34980), *Bacillus subtilis* (DSM 34981), *Bacillus licheniformis* (DSM 34977), showed not only tolerance to grow in presence of **VA**, but, in fact, improved growth in the presence of **VA.** In addition, they showed the ability to metabolize and therefore to reduce **VA** from the culture medium. In particular, the following observations were made:
- *Bacillus licheniformis* DSM 34977, DSM 34980, and DSM 34981 showed higher OD in presence of **VA** compared to the culture without **VA.**
   In particular, *Bacillus licheniformis* DSM 34977 showed the best performance when taking into account the **VA** reduction (23 % reduction of **VA** from the culture medium) and the generation of biomass (Δ OD = +1.285). Such accumulation of biomass in presence of **VA,** combined with the **VA** metabolization rate that was observed for DSM 34977 is beneficial for the reduction of **VA** over time and helps to reduce odor-causing compounds in a long-lasting manner. Such combination of advantageous properties (high degradation rate of valeric acid and high growth of biomass) makes *Bacillus licheniformis* DSM 34977 as efficient **VA** (and hence, malodor) removal agent with a long-lasting effect.
- In case of *Priestia megaterium* DSM 34980, similar observations as for *Bacillus licheniformis* DSM 34977 were made: *Priestia megaterium* DSM 34980 showed a production of biomass which was slightly increased in the presence of **VA** (Δ OD = +0.065), but showed a rate of metabolization and thus reduction of **VA** from the culture medium that was even higher (29 %) than the respective rate observed for *Bacillus licheniformis* DSM 34977. This points to the fact that this strain does not need **VA** for its metabolism, but the combination of these two observed features (slightly increased production of biomass with a high **VA** metabolization rate) also predestines this species as efficient **VA** (and hence, malodor) removal agent with a long-lasting effect. Both strains exhibit exceptional characteristics in reducing the odor-causing compound **VA,** which is the result of the combination of two advantageous properties, i.e. the production of biomass and **VA** reduction. That is why both strains can be used efficiently for **VA**-caused malodor reduction with long-lasting effect;
- Finally, *Bacillus subtilis* DSM 34981 also showed an accumulation of biomass during the incubation over 48 hours (Δ OD = +0.610) which is between the respective number observed for *Bacillus licheniformis* DSM 34977 and *Priestia megaterium* DSM 34980. The **VA** reduction over 48 hours was only 8 %. Nevertheless, the combination of these two properties also predestines *Bacillus subtilis* and in particular the respective strain DSM 34981 for **VA-caused** malodor reduction with long-lasting effect.

In summary, **E_{XJ; 1000 ppm}** for each strain with **Γ_{XJ; 1000 ppm}** > 1 may be determined as follows (given that the rate of metabolization of valeric acid medium **k_{XJ; 1000 ppm}** is proportional to the measured **VA** reduction and thus may be equated in the following for simplicity):
- *Bacillus licheniformis* DSM 34977, **k_{XJ; 1000 ppm}** ~ 0.23; **Γ_{XJ}; _{1000 ppm}** = 1.285 → **E_{XJ; 1000 ppm}** = 0.29555;
- *Priestia megaterium* DSM 34980, **k_{XJ; 1000 ppm}** ~ 0.29; **Γ_{XJ; 1000 ppm}** = 0.065 -> **E_{XJ; 1000 ppm}** = 0.01885;
- *Bacillus subtilis* DSM 34981, **k_{XJ; 1000 ppm}** ~ _{0.08}; **Γ_{XJ; 1000 ppm}** = 1.285 -> **E_{XJ; 1000 ppm}** = 0.1028.

From this, it follows that, of all strains with **Γ**_{**XJ;** 1000 ppm} > 1, *Bacillus licheniformis* DSM 34977 displayed the highest effectiveness **E**_{**XJ;** 1000 ppm} to reduce valeric acid (1000 ppm).
- On the other hand, *Bacillus velezensis* strains DSM 34978 (Δ OD = -0.359) and DSM 34674 (Δ OD = -0.454) showed a reduction in biomass over the course of the incubation period of 48 hours (**Γ_{XJ; 1000 ppm}** < 1). Nevertheless, the **VA** reduction rate observed for these *Bacillus velezensis* strains DSM 34978 (57 %) and DSM 34674 (41 %) was even higher than the respective rates observed for *Bacillus licheniformis* DSM 34977, *Priestia megaterium* DSM 34980, and *Bacillus subtilis* DSM 34981. Both *Bacillus velezensis* strains DSM 34978 and DSM 34674 strains were able to metabolize **VA** over the course of the incubation period, i.e. for 48 hours. However, as, they showed a decreased cell concentration after 48 hours in presence of **VA,** they demonstrated smaller tolerances for this organic acid.
   The high **VA** reduction rate, however, makes them predestined for use as a short-term agent for reduction of malodor that is caused by **VA.**

These observations show that the respective species *Priestia megaterium* (in particular DSM 34980), *Bacillus subtilis* (in particular DSM 34981), *Bacillus licheniformis* (in particular DSM 34977) showing a long-term effect for malodor removal may be combined with the respective *Bacillus velezensis* species (in particular strains DSM 34978 and DSM 34674) to give an effective agent for removal of malodor caused by valeric acid.

### V. Spreading of Bacillus strains in combination with biosurfactants

### V.1 Spreading assay

For the investigation of the spreading of the strains in combination with biosurfactants (Rewoferm RL200) and other additives (such as other surfactants), a new assay was established. 10⁹ spores of *Bacillus velezensis* DSM 34978 were produced by fermentation and freeze dried afterwards.

In example 1 (according to the invention), the spore biomass was incorporated into a 10 % water-dilution of rhamnolipids (Rewoferm RL200; "**RL200**")**.**

In comparative example 2, the spore biomass was incorporated into a 10 % water-dilution of a non-ionic surfactant (ethoxylated alcohol; "Tomadol 91-6" by Evonik Industries AG).

In comparative example 3, the spore biomass was incorporated into a 10 % water-dilution of the anionic surfactant **SLES** ("Texapon NSO IS"; BASF).

In blank example 4, the spore biomass was incorporated into water.

Each spore mixture was homogenized by shaking (1 hour and 200 rpm) and incubated at room temperature without shaking for a further 24 hours.

After the incubation, serial dilutions of the spore mixtures were made and plated onto motility agar. Motility agar plates containing 37 g/L Luria-Bertani (LB) agar (Merck-1 10283) with a reduced concentration of agar-agar (0.7 %) were used.

Plates were incubated for 10 h at 30 °C. Comparison of plates was made by photo documentation and **CFU**s [i.e. the spores that germinated into vegetative cells (standardized per gram of spore biomass)] were counted.

### V.2 Results

The following table gives the results of the counting of the **CFU**s in each case.

| Example | Additive | **CFU**/g |
|---|---|---|
| 1 (inventive) | rhamnolipid (**RL200**) | 1.26*10⁹ |
| 2 (comparative) | nonionic surfactant (ethoxylated alcohol) | 3.80*10⁸ |
| 3 (comparative) | anionic surfactant (**SLES**) | 6.00*10⁸ |
| 4 (blank) | None (water) | 6.07*10⁸ |

From the results, the following conclusions can be drawn:
The highest number of **CFU**s was counted in Example 1, i.e. with **RL200.** This number was higher than the number in the comparative examples 2 and 3 (nonionic surfactant, anionic surfactant) and also compared to the blank example 4.

This shows that spores that are incubated with **RL200** are dispersed over a broader area than when incubated with alternative surfactants or just in water. This experiment demonstrates the synergy of the combined used of bacterial spores and glycolipids, in particular rhamnolipids, for cleaning.

In conclusion, bacterial spores show an advantageous behavior in terms of spreading over and germinating on surfaces when combined with eco-friendly and sustainable biosurfactants such as rhamnolipids (here **RL200**). This advantage is not only observed in comparison with water, but also with other, typical surfactants that are commonly used in cleaning.

### Overview sequences

| **SEQ ID NO:** | **Strain** | **Description** |
|---|---|---|
| 1 | *Bacillus velezensis* strain DSM 34674 | DNA sequence coding for the 16S rRNA |
| 2 | *Bacillus velezensis* strain DSM 34674 | *rpoB* sequence |
| 3 | *Bacillus velezensis* strain DSM 34674 | *gyrB* sequence |
| 4 | *Bacillus velezensis* strain DSM 34674 | *groEL* sequence |
| 5 | *Bacillus velezensis* strain DSM 34674 | *yqfD* sequence |
| 6 | *Bacillus velezensis* strain DSM 34674 | *uvrC* sequence |
| 7 | *Bacillus velezensis* strain DSM 34978 | DNA sequence coding for the 16S rRNA |
| 8 | *Bacillus velezensis* strain DSM 34978 | *rpoB* sequence |
| 9 | *Bacillus velezensis* strain DSM 34978 | *gyrB* sequence |
| 10 | *Bacillus velezensis* strain DSM 34978 | *groEL*sequence |
| 11 | *Bacillus velezensis* strain DSM 34978 | DNA sequence coding for an ATP synthase C chain |
| 12 | *Bacillus velezensis* strain DSM 34978 | DNA sequence coding for a chemotaxis protein |
| 13 | *Bacillus subtilis* strain DSM 34981 | DNA sequence coding for the 16S rRNA |
| 14 | *Bacillus subtilis* strain DSM 34981 | *rpoB* sequence |
| 15 | *Bacillus subtilis* strain DSM 34981 | *gyrB* sequence |
| 16 | *Bacillus subtilis* strain DSM 34981 | *groEL* sequence |
| 17 | *Bacillus subtilis* strain DSM 34981 | *lipC* sequence |
| 18 | *Bacillus subtilis* strain DSM 34981 | *yqcA* sequence* |
| 19 | *Bacillus licheniformis* strain DSM 34977 | DNA sequence coding for the 16S rRNA |
| 20 | *Bacillus licheniformis* strain DSM 34977 | *rpoB* sequence |
| 21 | *Bacillus licheniformis* strain DSM 34977 | *gyrB* sequence |
| 22 | *Bacillus licheniformis* strain DSM 34977 | *groEL* sequence |
| 23 | *Bacillus licheniformis* strain DSM 34977 | *yqfD* sequence |
| 24 | *Bacillus licheniformis* strain DSM 34977 | *adaA* sequence |
| 25 | *Priestia megaterium* strain DSM 34980 | DNA sequence coding for the 16S rRNA |
| 26 | *Priestia megaterium* strain DSM 34980 | *rpoB* sequence |
| 27 | *Priestia megaterium* strain DSM 34980 | *gyrB* sequence |
| 28 | *Priestia megaterium* strain DSM 34980 | *groEL* sequence |
| 29 | *Priestia megaterium* strain DSM 34980 | *yqfD* sequence |

| | | |
|---|---|---|
| *** "*yqcA*" is not further characterized. "*adaA*" = methylphosphotriester-DNA-protein-cystein methyltransferase; "*groEL*" = chaperonin GroEL; "*gyrB*" = DNA gyrase subunit B; "*lipC*" = spore germination lipase; "*rpoB*" = DNA-directed RNA polymerase subunit β; "*uvrC*" = excinuclease ABC subunit; "*yqfD*" = sporulation protein. | | |

## Claims

1. A method for germinating bacterial spores **SP** on a surface **5** according to **variant 1, variant 2,** or **variant 3,**
wherein **variant 1** comprises the following steps:
a1) providing **SP** and, separately from **SP,** at least one biosurfactant **S_{Bio}**, wherein the biosurfactant **S_{Bio}** is a glycolipid,
b1) contacting **S** with **SP** and **S_{Bio}**, so that a mixture **M₁** comprising **SP** and at least one biosurfactant **S_{Bio}** is obtained, wherein mixture **M₁** is in contact with **S,**
c1) incubating **M₁** to germinate at least a part of the spores **SP** comprised by mixture **M₁;**
wherein **variant 2** comprises the following steps:
a2) providing a mixture **M*** comprising **SP** and at least one biosurfactant **S_{Bio},** wherein the biosurfactant **S_{Bio}** is a glycolipid,
b2) contacting **S** with the mixture **M*,** wherein **M*** is optionally incubated before contacting **S,** so that a mixture **M₂** comprising **SP** and at least one biosurfactant **S_{Bio}** is obtained, wherein mixture **M₂** is in contact with **S,**
c2) incubating **M₂** to germinate at least a part of the spores **SP** comprised by mixture **M₂;**
wherein **variant 3** comprises the following steps:
a3) providing a mixture **M*** comprising **SP** and at least one biosurfactant **S_{Bio},** wherein the biosurfactant **S_{Bio}** is a glycolipid,
b3)
o incubating mixture **M*,**
o then separating at least a part **π_{SP}** of the spores **SP** comprised by **M*** from at least a part of the biosurfactants **S_{Bio}** comprised by **M*,**
o contacting **5** with **π_{SP},**
so that a mixture **M₃** comprising at least a part **π_{SP}** of the spores **SP** and optionally at least one biosurfactant **S_{Bio}** is obtained, wherein mixture **M₃** is in contact with **S,**
c3) incubating **M₃** to germinate at least a part of the spores **SP** comprised by mixture **M₃.**

2. The method according to Claim 1, wherein the bacterial spores **SP** are selected from the group consisting of *Clostridium* spores, *Bacillus* spores, *Priestia* spores, *Desulfotomaculum* spores, *Heyndrickxia* spores, preferably selected from the group consisting of *Bacillus* spores, *Priestia* spores.

3. The method according to Claim 1 or 2, wherein the bacterial spores **SP** are selected from the group consisting of *Bacillus velezensis* spores, *Bacillus subtilis* spores, *Bacillus licheniformis* spores, *Priestia megaterium* spores.

4. The method according to one of Claims 1 to 3, wherein the bacterial spores **SP** are selected from the group consisting of spores of *Bacillus velezensis* DSM 34978, spores of mutants of *Bacillus velezensis* DSM 34978, spores of *Bacillus velezensis* DSM 34674, spores of mutants of *Bacillus velezensis* DSM 34674, spores of *Bacillus subtilis* DSM 34981, spores of mutants of *Bacillus subtilis* DSM 34981, spores of *Bacillus licheniformis* DSM 34977, spores of mutants of *Bacillus licheniformis* DSM 34977, spores of *Priestia megaterium* DSM 34980, spores of mutants of *Priestia megaterium* DSM 34980.

5. The method according to one of Claims 1 to 4, wherein the biosurfactant **S_{Bio}** is selected from the group consisting of rhamnolipids, glucolipids, sophorolipids, preferably rhamnolipids.

6. The method according to one of Claims 1 to 5, wherein,
when **variant 1** is carried out, the incubation in step c1) is carried out until at least 10 % of the spores **SP** that are comprised by mixture **M₁** have germinated, relative to the number of spores **SP** comprised by mixture **M₁** before mixture **M₁** is subjected to the incubation according to step c1),
when **variant 2** is carried out, the incubation in step c2) is carried out until at least 10 % of the spores **SP** that are comprised by mixture **M₂** have germinated, relative to the number of spores **SP** comprised by mixture **M₂** before mixture **M₂** is subjected to the incubation according to step c2),
when **variant 3** is carried out, the incubation in step c3) is carried out until at least 10 % of the spores **SP** that are comprised by mixture **M₃** have germinated, relative to the number of spores **SP** comprised by mixture **M₃** before mixture **M₃** is subjected to the incubation according to step c3).

7. The method according to one of Claims 1 to 6, wherein
mixture **M₁** in steps b1) and c1) of **variant 1** is an aqueous mixture,
mixture **M₂** in steps b2) and c2) of **variant 2** is an aqueous mixture, and
mixture **M₃** in steps b3) and c3) of **variant 3** is an aqueous mixture.

8. The method according to one of Claims 1 to 7, wherein surface **S** is a surface **So** of an object **O** or a surface **S_{B}** of the human or animal body **B.**

9. The method according to one of Claims 1 to 8,
wherein during step b1) and/or during step c1) of **variant 1,** at least one impurity **I** which is absorbed or adsorbed on the surface **5** is at least partially desorbed from the surface **5** and dispersed or dissolved in the mixture **M₁,**
wherein during step b2) and/or during step c2) of **variant 2,** at least one impurity **I** which is absorbed or adsorbed on the surface **5** is at least partially desorbed from the surface **5** and dispersed or dissolved in the mixture **M₂,** and
wherein during step b3) and/or during step c3) of **variant 3,** at least one impurity **I** which is absorbed or adsorbed on the surface **5** is at least partially desorbed from the surface **5** and dispersed or dissolved in the mixture **M₃.**

10. Use of at least one biosurfactant **S_{Bio}**, wherein biosurfactant **S_{Bio}** is a glycolipid, for dispersing bacterial spores **SP.**

11. The use according to Claim 10, wherein the bacterial spores **SP** are selected from the group consisting of *Clostridium* spores, *Bacillus* spores, *Priestia* spores, *Desulfotomaculum* spores, *Heyndrickxia* spores, preferably selected from the group consisting of *Bacillus* spores, *Priestia* spores.

12. The use according to Claim 11, wherein the bacterial spores **SP** are selected from the group consisting of *Bacillus velezensis* spores, *Bacillus subtilis* spores, *Bacillus licheniformis* spores, *Priestia megaterium* spores.

13. The use according to Claim 12, wherein the bacterial spores **SP** are selected from the group consisting of spores of *Bacillus velezensis* DSM 34978, spores of mutants of *Bacillus velezensis* DSM 34978, spores of *Bacillus velezensis* DSM 34674, spores of mutants of *Bacillus velezensis* DSM 34674, spores of *Bacillus subtilis* DSM 34981, spores of mutants of *Bacillus subtilis* DSM 34981, spores of *Bacillus licheniformis* DSM 34977, spores of mutants of *Bacillus licheniformis* DSM 34977, spores of *Priestia megaterium* DSM 34980, spores of mutants of *Priestia megaterium* DSM 34980.

14. The use according to one of Claims 10 to 13, wherein biosurfactant **S_{Bio}** is selected from the group consisting of rhamnolipids, glucolipids, sophorolipids, preferably rhamnolipids.

15. The use according to one of Claims 10 to 14, which is carried out in a method according to one of Claims 1 to 9.
